# EUROPEAN PATENT SPECIFICATION

(11) **EP 3 953 474 B1**
(45) Date of publication and mention of the grant of the patent: **01.07.2026**
(21) Application number: 20788075.8
(22) Date of filing: 09.04.2020
(51) Int. Cl.: C12N 15/113, C12N 15/11

(54) **CANCER-SPECIFIC MOLECULES AND METHODS OF USE THEREOF**
KREBSSPEZIFISCHE MOLEKÜLE UND VERFAHREN ZU IHRER VERWENDUNG
MOLÉCULES SPÉCIFIQUES AU CANCER ET LEURS MÉTHODES D'UTILISATION

(30) Priority: 09.04.2019 US 201962831604 P; 20.08.2019 US 201962889217 P; 06.12.2019 US 201962944913 P; 24.02.2020 US 202062980900 P
(43) Date of publication of application: 16.02.2022
(73) Proprietor: Envisagenics, Inc., New York, NY 10013 (US)
(72) Inventor: PINEDA, Maria Luisa, Long Island City, New York 11109 (US); AKERMAN, Martin, Huntington, New York 11743 (US); ARUN, Gayatri, Huntington Station, New York 11746 (US); DHINGRA, Priyanka, Jersey City, New Jersey 07302 (US); ANDERSON, Kendall, New York, New York 10007 (US); FREDERICK, Vanessa, Brooklyn, New York 11221 (US); YUDANIN, Naomi, New York, New York 10013 (US); MUNCH, Robin, New York, New York 10013 (US); ZHENG, Paulina, New York, New York 10280 (US); ABRAHAM, Anson, Roslyn Heights, New York 11577 (US)
(74) Representative: Mewburn Ellis LLP
(86) International application number: PCT/US2020/027534
(87) International publication number: WO 2020/210537

(56) References cited:
- WO-A1-2019/028440
- CN-A- 106 636 199
- US-A1- 2007 237 770
- US-A1- 2018 362 987
- US-B1- 6 284 535
- SCREEN MARK ET AL: "Abnormal Splicing of NEDD4 in Myotonic Dystrophy Type 2", vol. 184, no. 8, 1 August 2014 (2014-08-01), US, pages 2322 - 2332, XP093102860, ISSN: 0002-9440, Retrieved from the Internet <URL:http://dx.doi.org/10.1016/j.ajpath.2014.04.013> DOI: 10.1016/j.ajpath.2014.04.013
- DUNN D M ET AL: "Common variant of human NEDD4L activates a cryptic splice site to form a frameshifted transcript", JOURNAL OF HUMAN GENETICS, SPRINGER SINGAPORE, SINGAPORE, vol. 47, no. 12, 1 December 2002 (2002-12-01), pages 665 - 676, XP036953766, ISSN: 1434-5161, [retrieved on 20021201], DOI: 10.1007/S100380200102
- GUARNIERI A L ET AL: "The miR-106b-25 cluster mediates breast tumor initiation through activation of NOTCH1 via direct repression of NEDD4L", ONCOGENE, NATURE PUBLISHING GROUP UK, LONDON, vol. 37, no. 28, 17 April 2018 (2018-04-17), pages 3879 - 3893, XP036746096, ISSN: 0950-9232, [retrieved on 20180417], DOI: 10.1038/S41388-018-0239-7

## Description

### BACKGROUND

Cancer and genetic diseases affect more than millions of people in the U.S. Splicing deregulation can be a major hallmark of cancer and genetic diseases, affecting progression, metastasis, and therapy resistance. RNA splicing is the process by which introns, the non-protein coding regions of DNA, are removed from nascent precursor messenger RNA (pre-mRNA), and exons, the protein coding regions of DNA, are joined together to form mature messenger RNA (mRNA). RNA splicing errors may result in spliced RNA that fail to produce functional proteins, thereby causing genetic diseases including many types of cancers.

US2018/0362987A1 discloses methods for the targeted augmentation of nuclear gene output. US6284535B1 discloses splice variants of the heregulin gene, naria and uses thereof. US2007/0237770A1 discloses novel compositions and methods in cancer. WO2019/028440A1 discloses methods and compositions for modulating splicing. CN106636199A discloses methods and products for easily screening and obtaining target gene knockout cell lines using CRISPR/Cas9. Screen et al (2014) in Am. J. Pathol*.* disclose abnormal splicing of NEDD4 in Myotonic Dystrophy Type 2 and a possible link to statin adverse reactions. Dunn et al (2002) in J. Hum. Genet*.* disclose a common variant of NEDDL4 that activates a cryptic splice site to form a frameshifted transcript. Guarnieri et al (2018) in Oncogene disclose that the miR-106b-25 cluster mediates breast tumour initiation through activation of NOTCH1 via direct repression of NEDD4L.

### SUMMARY

RNA splicing is a form of RNA processing in which a newly made precursor messenger RNA (pre-mRNA) transcript is transformed into a mature messenger RNA (mRNA). During splicing, introns (intra-genic, non-coding regions) are removed and exons (coding regions) are joined together. RNA splicing not only provides functional mRNA, but may also be responsible for generating additional diversity (i.e., alternative splicing, alternative RNA splicing, or differential splicing). The alternative splicing may result in the production of different mRNAs from the same gene. The mRNAs that represent isoforms arising from a single gene can differ by the use of alternative exons or retention of an intron that disrupts two exons. This process often leads to different protein products that may have related or drastically different, even antagonistic, cellular functions. The alternative splicing may comprise one or more of exon skipping or cassette exon, mutually exclusive exons, alternative donor site, alternative acceptor site, intron retention, and any combination or variation thereof.

Multiple studies have shown the oncogenic activity of specific splicing events and splicing factors, such as SRSF1, SRSF2, ESRP1, and RBFOX1, in human and animal models. As such, cancer-associated splicing deregulation may be a novel source of clinically-applicable biomarkers and therapeutic targets.

The invention relates to an antisense compound comprising an oligonucleotide having a sequence selected from the group consisting of: 5'-GTGGGTTTCAGGGATTCTGA-3' (SEQ ID NO: 1), 5'-CCCTGATTCAGACAGCAGGG-3' (SEQ ID NO: 2), 5'-GCTGGCTTTGTCTGGATAGG-3' (SEQ ID NO: 3), 5'-TCTCACGTCACCTGCCTTAC-3' (SEQ ID NO: 4), and 5'-AGCGCTGCCACAGCAGTGGG-3' (SEQ ID NO: 5) or a complement thereof; compositions comprising such antisense compounds; and methods and uses thereof. The invention is defined by the claims.

Further disclosed herein are systems and methods for identifying therapeutic targets (e.g., disease-specific splicing events) in various diseases or illnesses such as cancers. Non-limiting examples of cancers may include prostate cancer, cancers of barrier tissues (i.e. colorectal cancer, lung cancer) and in other TGFb-rich sites like ovaries, AML/hematological disorders, respiratory system cancer, hepatocellular carcinoma (liver cancer) which may include both a TGFb-rich environment and chronic, potentially diet induced inflammation, thoracic cancer, stomach cancer, kidney cancer, pancreatic cancer, skin cancer, or combinations thereof. Examples of some other diseases may include, but are not limited to, autism (i.e., ST7 (ENV19)), lymphatic disease such as syndromic lymphedema-genetic disorder and milory disease, eye degenerative diseases, brain disease- peripheral neuropathy, neurometabolic disease, rare genetic neurological disorders-genetic motor neuron disease, psychiatric disorders, chronic inflammation / autoimmune disease, including IBD, crohn's disease, and similar gastrointestinal disorders, inflammation in pathogenic obesity, including hereditary, childhood, leptin and non-leptin dependent disease, hypertension and/or other comorbidities associated with western diets.

The methods of the present disclosure may also comprise detecting one or more biomarkers, for example, detecting a presence or an absence of specific DNA sequence, mRNA and/or protein isoforms. The presence or absence of the one or more biomarkers can be used to diagnose disease and/or track disease progression.

Also disclosed herein are methods for modulating therapeutic targets (e.g., disease-specific splicing events) using various types of modalities, such as oligonucleotides, small molecules, antibodies, or any combination thereof. In some examples, the modalities may switch pathogenic RNA isoforms to non-pathogenic RNA isoforms. In some examples, the modalities are oligonucleotides including splicing-switch oligonucleotides (SSO), antisense oligonucleotides (ASO), small interfering Ribonucleic Acid (siRNA), conjugated oligonucleotides, or a combination thereof that can knockdown specific isoforms. In some other example, the modalities are antibodies or cell-based (e.g., CAR-T) that may specifically recognize protein isoform of alternatively spliced RNA, and/or therapeutic compounds including ASO, small molecules or biologics that target the isoforms specifically to obtain therapeutic benefit.

Further disclosed are methods and systems for treating diseases or illnesses such as cancers. The systems and methods may comprises administering an effective amount to modalities to a subject having the diseases or illnesses. The modalities may be oligonucleotides, small molecules, antibodies, or any combination thereof, which are designed to achieve disease-specific targeting. For example, some of the disease-specific splicing events that have been identified can open up grooves for small molecule binding. In that case, small molecules can be designed to target the region that is created because of a splicing change. In another example, splicing changes of the membrane bound proteins can display altered surface epitopes that can be specifically targeted using antibodies.

An aspect of the present disclosure provides a method of modulating splicing in a pre-mRNA in a biological sample comprising: contacting the biological sample with an antisense compound comprising one or more oligonucleotides having at least 90% sequence identity to oligonucleotides selected from Tables 2-33.

In some aspects of the disclosure, the biological sample comprises a cell, a tissue, or a blood sample. In some aspects, the biological sample is *in vitro.* In some aspects, the biological sample is a cell. In some aspects, the method further comprises measuring viability of the cell. In some aspects, the measuring is over a predetermined time period. In some aspects, the method further comprises monitoring the viability of the cell over a predetermined time period. In some aspects, the method further comprises decreasing or increasing a concentration of the antisense compound based on the viability of the cell. In some aspects, the method further comprises decreasing or increasing a concentration of the antisense compound when the viability of the cell is below a cut-off value. In some aspects, the cut-off value is about 80%. In some aspects, the cut-off value is about 90%. In some aspects, the antisense compound comprises the one or more oligonucleotides at a concentration of greater than or equal to about 300 nM. In some aspects, the antisense compound comprises the one or more oligonucleotides at a concentration of less than or equal to about 450 nM. In some aspects, the one or more oligonucleotides comprise deoxyribonucleic acid (DNA), ribonucleic acid (RNA), or a combination thereof. In some aspects, the RNA comprises small interfering RNA (siRNA). In some aspects, the one or more oligonucleotides comprises single-stranded oligonucleotides, double-stranded oligonucleotides, or a combination thereof. In some aspects, the biological sample is from a subject having or suspected of having a disease or condition. In some aspects, the disease or condition comprises a genetic disease, a CNS disease, an inflammatory disease, a neurodegenerative disease, a cardiovascular disease, an autoimmune disease, or cancer. In some aspects, the disease is cancer. In some aspects, the cancer comprises lung cancer, kidney cancer, or breast cancer. In some aspects, the breast cancer is triple-negative breast cancer. In some aspects, the one or more nucleotides comprise oligonucleotides selected from Tables 2-33. In some aspects, the one or more oligonucleotides comprise a modified oligonucleotide. In some aspects, the modified oligonucleotide comprises at least one modified internucleotide linkage. In some aspects, the at least one modified internucleotide linkage is phosphorothioate linkage. In some aspects, the modified oligonucleotide comprises one or more modified nucleotides. In some aspects, the modified oligonucleotide comprises one or more modified nucleosides. In some aspects, a modified nucleoside of the one or more modified nucleosides comprises a modified sugar moiety. In some aspects, the modified sugar moiety is a 2'-substituted sugar moiety. In some aspects, the 2'-substituted sugar moiety comprises a modification selected from the group consisting of 2'-O-methoxyethyl, 2'-fluoro, 2'-dimethylaminooxyethoxy, 2'-dimethylaminoethoxyethoxy, 2'-guanidinium, 2'-O-guanidinium ethyl, 2'-carbamate, 2'aminooxy, 2'-acetamido, and locked nucleic acid. In some aspects, the modified oligonucleotide comprises a plurality of modified nucleosides each comprising a modified sugar moiety. In some aspects, at least a subset of the plurality of modified nucleosides are different from one another. In some aspects, the modulating comprises inducing or enhancing exon skipping. In some aspects, the modulating comprises inducing or enhancing exon inclusion. In some aspects, the modulating comprises promoting a splicing switch. In some aspects, the modulating comprises down-regulation or up-regulation of splicing. In some aspects, the antisense compound specifically binds to a segment of a pre-mRNA encoded by a gene comprising NEDD4L, MAP3K7, NFYA, ESYT2, MARK2, ST7, ARVCF, SYTL2, R3HDM1, COL4A3BP, TANGO2, SEPT9, ROBO1, FAM122B, CD47, LSR, PBX1, EPB41, ADAM15, EPB41L1, ABI1, FLNB, CTNND1, GPR160, ITGB3BP, INCENP, DENND1B, or CA12.

Another aspect of the present disclosure provides a pharmaceutical composition comprising (i) an antisense compound comprising one or more oligonucleotides having at least 90% sequence identity to oligonucleotides selected from Tables 2-33, and (ii) a pharmaceutically acceptable diluent or carrier.

In some aspects, the antisense compound comprises the one or more oligonucleotides at a concentration of greater than or equal to about 300 nM. In some aspects, the antisense compound comprises the one or more oligonucleotides at a concentration of less than or equal to about 450 nM. In some aspects, the one or more oligonucleotides comprise deoxyribonucleic acid (DNA), ribonucleic acid (RNA), or a combination thereof. In some aspects, the RNA comprises small interfering RNA (siRNA). In some aspects, the one or more oligonucleotides comprises single-stranded oligonucleotides, double-stranded oligonucleotides, or a combination thereof. In some aspects, the pharmaceutical composition is used for treating or alleviating a disease or condition. In some aspects, the disease comprises a genetic disease, a CNS disease, an inflammatory disease, a neurodegenerative disease, a cardiovascular disease, an autoimmune disease, or cancer. In some aspects, the disease or condition is cancer. In some aspects, the cancer comprises lung cancer, kidney cancer, or breast cancer. In some aspects, the breast cancer is triple-negative breast cancer. In some aspects, the one or more nucleotides comprise oligonucleotides selected from Tables 2-33. In some aspects, the one or more oligonucleotides comprise a modified oligonucleotide. In some aspects, the modified oligonucleotide comprises at least one modified internucleotide linkage. In some aspects, the at least one modified internucleotide linkage is phosphorothioate linkage. In some aspects, the modified oligonucleotide comprises one or more modified nucleotides. In some aspects, the modified oligonucleotide comprises one or more modified nucleosides. In some aspects, a modified nucleoside of the one or more modified nucleosides comprises a modified sugar moiety. In some aspects, the modified sugar moiety is a 2'-substituted sugar moiety. In some aspects, the 2'-substituted sugar moiety comprises a modification selected from the group consisting of 2'-O-methoxyethyl, 2'-fluoro, 2'-dimethylaminooxyethoxy, 2'-dimethylaminoethoxyethoxy, 2'-guanidinium, 2'-O-guanidinium ethyl, 2'-carbamate, 2'aminooxy, 2'-acetamido, and locked nucleic acid. In some aspects, the modified oligonucleotide comprises a plurality of modified nucleosides each comprising a modified sugar moiety. In some aspects, at least a subset of the plurality of modified nucleosides are different from one another. In some aspects, the pharmaceutical composition is used for modulating splicing of a pre-mRNA encoded by a gene comprising NEDD4L, MAP3K7, NFYA, ESYT2, MARK2, ST7, ARVCF, SYTL2, R3HDM1, COL4A3BP, TANGO2, SEPT9, ROBO1, FAM122B, CD47, LSR, PBX1, EPB41, ADAM15, EPB41L1, ABI1, FLNB, CTNND1, GPR160, ITGB3BP, INCENP, DENND1B, or CA12. In some aspects, the modulating comprises inducing or enhancing exon skipping. In some aspects, the modulating comprises inducing or enhancing exon inclusion. In some aspects, the modulating comprises promoting a splicing switch. In some aspects, the modulating comprises down-regulation or up-regulation of splicing.

Another aspect of the present disclosure provides an antisense compound for use in preparation of a medicament for the treatment of a disease or a condition, the antisense compound comprising one or more oligonucleotides having at least 90% sequence identity to oligonucleotides selected from Tables 2-33.

In some aspects, the antisense compounds comprise the one or more oligonucleotides at a concentration of greater than or equal to about 300 nM. In some aspects, the antisense compound comprises the one or more oligonucleotides at a concentration of the concentration is less than or equal to about 450 nM. In some aspects, the one or more oligonucleotides comprise deoxyribonucleic acid (DNA), ribonucleic acid (RNA), or a combination thereof. In some aspects, the RNA comprises small interfering RNA (siRNA). In some aspects, the one or more oligonucleotides comprises single-stranded oligonucleotides, double-stranded oligonucleotides, or a combination thereof. In some embodiments, the disease or condition comprises a genetic disease, a CNS disease, an inflammatory disease, a neurodegenerative disease, a cardiovascular disease, an autoimmune disease, or cancer. In some aspects, the disease is cancer. In some aspects, the cancer comprises lung cancer, kidney cancer, or breast cancer. In some aspects, the breast cancer is triple-negative breast cancer. In some aspects, the one or more nucleotides comprise oligonucleotides selected from Tables 2-33. In some aspects, the one or more oligonucleotides comprise a modified oligonucleotide. In some aspects, the modified oligonucleotide comprises at least one modified internucleotide linkage. In some aspects, the at least one modified internucleotide linkage is phosphorothioate linkage. In some aspects, the modified oligonucleotide comprises one or more modified nucleotides. In some aspects, the modified oligonucleotide comprises one or more modified nucleosides. In some aspects, a modified nucleoside of the one or more modified nucleosides comprises a modified sugar moiety. In some aspects, the modified sugar moiety is a 2'-substituted sugar moiety. In some aspects, the 2'-substituted sugar moiety comprises a modification selected from the group consisting of 2'-O-methoxyethyl, 2'-fluoro, 2'-dimethylaminooxyethoxy, 2'-dimethylaminoethoxyethoxy, 2'-guanidinium, 2'-O-guanidinium ethyl, 2'-carbamate, 2'aminooxy, 2'-acetamido and locked nucleic acid. In some aspects, the modified oligonucleotide comprises a plurality of modified nucleosides each comprising a modified sugar moiety. In some aspects, at least a subset of the plurality of modified nucleosides are different from one another. In some aspects, the treatment comprising modulating splicing of a pre-mRNA encoded by a gene comprising NEDD4L, MAP3K7, NFYA, ESYT2, MARK2, ST7, ARVCF, SYTL2, R3HDM1, COL4A3BP, TANGO2, SEPT9, ROBO1, FAM122B, CD47, LSR, PBX1, EPB41, ADAM15, EPB41L1, ABI1, FLNB, CTNND1, GPR160, ITGB3BP, INCENP, DENND1B, or CA12. In some aspects, the modulating comprises inducing or enhancing exon skipping. In some aspects, the modulating comprises inducing or enhancing exon inclusion. In some embodiments, the modulating comprises promoting a splicing switch. In some aspects, the modulating comprises down-regulation or up-regulation of splicing.

Another aspect of the present disclosure provides a method of modulating splicing in a pre-mRNA in a biological sample comprising: contacting the biological sample with a composition which specifically binds to a segment of the pre-mRNA which is encoded by a gene selected from the group consisting of NEDD4L, MAP3K7, NFYA, ESYT2, MARK2, ST7, ARVCF, SYTL2, R3HDM1, COL4A3BP, TANGO2, SEPT9, ROBO1, FAM122B, CD47, LSR, PBX1, EPB41, ADAM15, EPB41L1, ABI1, FLNB, CTNND1, GPR160, ITGB3BP, INCENP, DENND1B, and CA12.

In some aspects, the segment of the pre-mRNA is 9-150 nucleotides in length. In some aspects, the composition comprises oligonucleotides. In some aspects, the oligonucleotides are sufficiently complementary to the segment of the pre-mRNA. In some aspects, the oligonucleotides have at least 80% sequence identity to the segment of the pre-mRNA. In some aspects, the oligonucleotides have at least 90% sequence identity to the segment of the pre-mRNA. In some aspects, the oligonucleotides comprise 10-50 nucleotides. In some aspects, the oligonucleotides comprise 15-30 nucleotides. In some aspects, the composition comprises small molecules, nucleic acid molecules, engineered cells, proteins, or a combination or modification thereof. In some aspects, the composition comprises a chimeric molecule. In some aspects, the chimeric molecule comprises a nucleic acid molecule and a protein. In some aspects, the nucleic acid molecule comprises DNA, RNA, PNA, or a combination or hybrid thereof. In some aspects, the composition induces or enhances exon skipping in the pre-mRNA. In some aspects, the composition induces or enhances exon inclusion in the pre-mRNA. In some aspects, the composition promotes a splicing switch in the pre-mRNA. In some aspects, the composition down-regulates or up-regulates of splicing in the pre-mRNA. In some aspects, the composition prevents splicing in the pre-mRNA.

Another aspect of the present disclosure provides a method for treating a disease or condition in a subject in need thereof, comprising: administering an effective amount of a composition to the subject, which composition specifically binds to a segment of a pre-mRNA which is encoded by a gene selected from the group consisting of NEDD4L, MAP3K7, NFYA, ESYT2, MARK2, ST7, ARVCF, SYTL2, R3HDM1, COL4A3BP, TANGO2, SEPT9, ROBO1, FAM122B, CD47, LSR, PBX1, EPB41, ADAM15, EPB41L1, ABI1, FLNB, CTNND1, GPR160, ITGB3BP, INCENP, DENND1B, and CA12, thereby modulating splicing in the pre-mRNA.

In some aspects, the segment of the pre-mRNA is 9-150 nucleotides in length. In some aspects, the composition comprises oligonucleotides. In some aspects, the oligonucleotides are sufficiently complementary to the segment of the pre-mRNA. In some aspects, the oligonucleotides have at least 80% sequence identity to the segment of the pre-mRNA. In some aspects, the oligonucleotides have at least 90% sequence identity to the segment of the pre-mRNA. In some aspects, the oligonucleotides comprise 10-50 nucleotides. In some aspects, the oligonucleotides comprise 15-30 nucleotides. In some aspects, the composition comprises small molecules, nucleic acid molecules, engineered cells, proteins, or a combination or modification thereof. In some aspects, the composition comprises a chimeric molecule. In some aspects, the chimeric molecule comprises a nucleic acid molecule and a protein. In some aspects, the nucleic acid molecule comprises DNA, RNA, PNA, or a combination or hybrid thereof. In some aspects, the composition induces or enhances exon skipping in the pre-mRNA. In some aspects, the composition induces or enhances exon inclusion in the pre-mRNA. In some aspects, the composition promotes a splicing switch in the pre-mRNA. In some aspects, the composition down-regulates or up-regulates of splicing in the pre-mRNA. In some aspects, the composition prevents splicing in the pre-mRNA. In some aspects, the effective amount comprises at least 300 nM of the composition. In some aspects, the effective amount comprises at most 500 nM of the composition. In some aspects, the disease or condition comprises a genetic disease, a CNS disease, an inflammatory disease, a neurodegenerative disease, a cardiovascular disease, an autoimmune disease, or cancer. In some aspects, the disease or condition is cancer. In some aspects, the cancer comprises lung cancer, kidney cancer, or breast cancer. In some aspects, the breast cancer is triple negative breast cancer.

Another aspect of the present disclosure provides a method for screening, diagnosis or prognosis of a disease or condition in a subject, comprising: (a) analyzing a biological sample from the subject to detect a level of expression of a protein isoform, which protein isoform is encoded by a gene selected from the group consisting of NEDD4L, MAP3K7, NFYA, ESYT2, MARK2, ST7, ARVCF, SYTL2, R3HDM1, COL4A3BP, TANGO2, SEPT9, ROBO1, FAM122B, CD47, LSR, PBX1, EPB41, ADAM15, EPB41L1, ABI1, FLNB, CTNND1, GPR160, ITGB3BP, INCENP, DENND1B, and CA12; and (b) determining a difference of the level of expression of the protein isoform in the biological sample relative to a level of expression of the protein isoform in a biological sample of a control, wherein the difference is indicative or predicative of the disease or condition.

In some aspects, the biological sample is a cell, a tissue, or a blood sample. In some aspects, (a) comprises quantitatively detecting an amount of the protein isoform in the biological sample. In some aspects, the difference comprises an increase or a decrease of the level of expression of the protein isoform in the biological sample relative to the level of expression of the protein isoform in the biological sample of the control. In some aspects, the increase of the level of expression of the protein isoform in the biological sample relative to the level of expression of the protein isoform in the biological sample of the control is indicative or predicative of the disease or condition. In some aspects, the decrease of the level of expression of the protein isoform in the biological sample relative to the level of expression of the protein isoform in the biological sample of the control is indicative or predicative of the disease or condition. In some aspects, the protein isoform comprises alternatively spliced protein isoforms. In some aspects, the alternatively spliced protein isoforms are formed by alternative splicing of the gene. In some aspects, the alternative splicing comprises exon skipping, exon inclusion, intron retention, competing 5' splice sites, competing 3' splice sites, multiple promoters, multiple poly(A) sites or a combination thereof. In some aspects, the method further comprises detecting a level of expression of the gene in the biological sample. In some aspects, the method further comprises detecting a presence or an absence of a difference of the level of expression of the gene in the biological sample of the subject relative to a level of expression of the gene in the biological sample in the control. In some aspects, the presence or the absence of the difference of the level of expression of the gene is further indicative or predicative of the disease or condition. In some aspects, the presence of the difference comprises an increase or a decrease of the level of expression of the gene in the biological sample of the subject relative to the level of expression of the gene in the biological sample in the control. In some aspects, the method further comprises monitoring a progression of the disease or condition in the subject. In some aspects, the monitoring comprises repeating (a) multiple times over a predetermined time period. In some aspects, the method further comprises providing a treatment to the subject upon diagnosis of the disease or condition in the subject (not claimed). In some aspects, the treatment (not claimed) comprises administering to the subject an effective amount of a composition which modulates the level of the protein isoform expression. In some aspects, the treatment (not claimed) comprises administering to the subject an effective amount of a composition which modulates splicing of the gene encoding the protein isoform. In some aspects, the disease or condition comprises a genetic disease, a CNS disease, an inflammatory disease, a neurodegenerative disease, a cardiovascular disease, an autoimmune disease, or cancer. In some aspects, the disease or condition is cancer. In some aspects, the cancer comprises lung cancer, kidney cancer, or breast cancer. In some aspects, the breast cancer is triple negative breast cancer.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects.

### BRIEF DESCRIPTION OF THE DRAWINGS

The invention is defined by the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**Figure 1** shows an exemplary oncoprint summary of recurring genomic aberration and transcriptional changes for splicing associated RNA binding proteins in triple-negative breast cancer (TNBC) patient samples.
**Figure 2** schematically illustrates an example of luminal versus TNBC RNA-seq data analysis and target selection.
**Figure 3** shows an examplary diagram of splicing changes in the Cancer Genome Atlas (TCGA) and cell lines showing independent and overlapping events.
**Figure 4** shows examplary reverse transcription polymerase chain reaction (RT-PCR) images showing differential splicing of selected candidates in luminal versus TNBC cell lines.
**Figure 5** shows exemplary Western blot of protein lysates from luminal and basal cell lines showing the isoform expression at the protein level for different genes.
**Figure 6** shows a survival analysis of breast cancer patients expressing NEDD4L inclusion versus skipped isoforms showing poor overall survival for patents with skipped isoform.
**Figure 7** shows comparison of splicing differences and total gene expression differences across multiple breast cancer subtypes.
**Figure 8** illustrate SpliceLearn scores and corresponding eCLIP peaks around the NEDD4L exon trio. The scores can be used for designing oligo sequences and the bottom panel shows the splice switching experimental results in which the high scoring ASOs (GTGGGTTTCAGGGATTCTGA, CCCTGATTCAGACAGCAGGG significantly switched to the inclusion isoform in MDA-MB-231 cells.
**Figure 9** shows an exemplary experimental validation and quantitation of switching off NEDD4L in MCF7 and MDA-Mb-231 cells treated with 400nM specific oligos and controls treated with either lipofectamine or PBS. Radioactive RT-PCR is shown above and quantitation of the results is shown below.
**Figure 10A** shows an exemplary dose response curve for an SSO (GTGGGTTTCAGGGATTCTGA) targeting NEDD4L which promotes inclusion. The SSO treatment causes dose dependent viability loss in MDA-Mb-231 cells compared to MCF7 cells. The optimal LC50 value is about 370nM.
**Figure 10B** shows an exemplary dose response curve for an SSO (CCCTGATTCAGACAGCAGGG) targeting NEDD4L which promotes inclusion. SSO treatment causes dose dependent viability loss in MDA-Mb-231 cells compared to MCF7 cells. The optimal LC50 value is about 420nM.
**Figure 11** shows exemplary PCR validations for a candidate gene.
**Figure 12** shows exemplary PCR validations for a candidate gene.
**Figure 13** shows exemplary PCR validations for a candidate gene.
**Figure 14** shows exemplary PCR validations for a candidate gene.
**Figure 15** shows an exemplary survival analysis of breast cancer patients expressing long and short isoforms for a candidate gene.
**Figure 16** shows an exemplary survival analysis of breast cancer patients expressing long and short isoforms for a candidate gene.
**Figure 17** shows an example selection criteria table.
**Figure 18** shows how the selection criteria is used to identify more target genes than use of splicing alone.

### DETAILED DESCRIPTION

While various aspects of the disclosure have been shown and described herein, it will be obvious to those skilled in the art that such aspects are provided by way of example only. Numerous variations, changes, and substitutions may occur to those skilled in the art. It should be understood that various alternatives to the aspects of the disclosure described herein may be employed.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

### Identification of Candidate Targets for Therapeutic Development

In some aspects, methods and systems for detecting or identifying candidate drug targets are disclosed. The candidate drug targets may be associated with specific diseases, illnesses or conditions. The diseases, illnesses or conditions may comprise cancer. Non-limiting examples of diseases, illnesses or conditions may include but not limited to ductal carcinoma in duct tissue in a mammary gland, medullary carcinomas, colloid carcinomas, tubular carcinomas, breast cancer or subtypes thereof; ovarian cancer, including epithelial ovarian tumors such as adenocarcinoma in the ovary and an adenocarcinoma that has migrated from the ovary into the abdominal cavity, uterine cancer, cervical cancer such as adenocarcinoma in the cervix epithelial including squamous cell carcinoma and adenocarcinomas; prostate cancer, such as a prostate cancer selected from the following: an adenocarcinoma or an adenocarcinoma that has migrated to the bone; pancreatic cancer such as epithelioid carcinoma in the pancreatic duct tissue and an adenocarcinoma in a pancreatic duct; bladder cancer such as a transitional cell carcinoma in urinary bladder, urothelial carcinomas (transitional cell carcinomas), tumors in the urothelial cells that line the bladder, squamous cell carcinomas, adenocarcinomas, and small cell cancers; leukemia such as acute myeloid leukemia (AML), acute lymphocytic leukemia, chronic lymphocytic leukemia, chronic myeloid leukemia, hairy cell leukemia, myelodysplasia, myeloproliferative disorders, acute myelogenous leukemia (AML), chronic myelogenous leukemia (CML), mastocytosis, chronic lymphocytic leukemia (CLL), multiple myeloma (MM), and myelodysplastic syndrome (MDS); bone cancer; lung cancer such as non-small cell lung cancer (NSCLC), which is divided into squamous cell carcinomas, adenocarcinomas, and large cell undifferentiated carcinomas, and small cell lung cancer; skin cancer such as basal cell carcinoma, melanoma, squamous cell carcinoma and actinic keratosis, which is a skin condition that sometimes develops into squamous cell carcinoma; eye retinoblastoma; cutaneous or intraocular (eye) melanoma; primary liver cancer (cancer that begins in the liver); kidney cancer; thyroid cancer such as papillary, follicular, medullary and anaplastic; AIDS-related lymphoma such as diffuse large B-cell lymphoma, B-cell immunoblastic lymphoma and small non-cleaved cell lymphoma; Kaposi's Sarcoma; viral-induced cancers including hepatitis B virus (HBV), hepatitis C virus (HCV), and hepatocellular carcinoma; human lymphotropic virus-type 1 (HTLV-1) and adult T-cell leukemia/lymphoma; and human papilloma virus (HPV) and cervical cancer; central nervous system cancers (CNS) such as primary brain tumor, which includes gliomas (astrocytoma, anaplastic astrocytoma, or glioblastoma multiforme), Oligodendroglioma, Ependymoma, Meningioma, Lymphoma, Schwannoma, and Medulloblastoma; peripheral nervous system (PNS) cancers such as acoustic neuromas and malignant peripheral nerve sheath tumor (MPNST) including neurofibromas and schwannomas, malignant fibrous cytoma, malignant fibrous histiocytoma, malignant meningioma, malignant mesothelioma, and malignant mixed Mtillerian tumor; oral cavity and oropharyngeal cancer such as, hypopharyngeal cancer, laryngeal cancer, nasopharyngeal cancer, and oropharyngeal cancer; stomach cancer such as lymphomas, gastric stromal tumors, and carcinoid tumors; testicular cancer such as germ cell tumors (GCTs), which include seminomas and nonseminomas, and gonadal stromal tumors, which include Leydig cell tumors and Sertoli cell tumors; thymus cancer such as to thymomas, thymic carcinomas, Hodgkin disease, non-Hodgkin lymphomas carcinoids or carcinoid tumors; rectal cancer; and colon cancer. In some aspects, the pharmaceutical composition is for the treatment of a non-cancerous hyperproliferative disorder such as benign hyperplasia of the skin (e. g., psoriasis), restenosis, or prostate (e. g., benign prostatic hypertrophy (BPH)).

The candidate drug targets may comprise one or more genes that are differentially express, exons (e.g., exon duos or exon trios) that are differentially spliced, or a combination thereof. The methods and systems can be exon-centric and highly sensitive in detecting low-abundance aberrant mRNA isoforms.

Additionally, artificial intelligence (AI) may be utilized by the methods and systems are disclosed herein. The AI may comprise the use of machine learning algorithms, non-limiting examples of which may comprise supervised (or predictive) learning, semi-supervised learning, active learning, unsupervised machine learning, or reinforcement learning, support vector machines (SVM), linear, logistics, tress, random forest, xgboost, neural networks, deep neural networks, boosting techniques, bootstrapping techniques, ensemble techniques, or combinations thereof.

As disclosed herein, the systems or methods may comprise receiving data from a database. The database may be a public database (e.g., TCGA, GTEX, dbGAP), a private database, or a combination thereof. The database may comprise public data, proprietary data, or a combination thereof. The database may comprise clinical or biological data. The database may comprise RNA-seq data. The database may comprise data obtained from a variety of samples, e.g., greater than or equal to about 100, 200, 300, 400, 500, 600, 700, 800, 900, 1,000, 2,000, 3,000, 4,000, 5,000, 6,000, 7,000, 8,000, 9,000, 10,000, 15,000, 20,000, 25,000, 30,000, 40,000, 50,000, 60,000, 70,000, 80,000, 90,000, 100,000, 125,000, 150,000, 175,000, 200,000 samples, or more. At least a subset of the samples may be obtained from different subjects have the same or different diseases, illnesses or conditions.

The database may comprise data extracted or derived from samples from cell lines from certain diseases, illnesses or conditions, and/or from subjects having certain different diseases, illnesses or conditions. In some cases, the diseases, illnesses or conditions comprise breast cancer or subtypes thereof, for example, liminal A, luminal B, Her2+, TNBC. Non-limiting examples of cell lines may comprise BT483, CAMA1, EFM19, HCC1428, HCC712, IBEP2, KPL1, LY2, MCF7, MDAMB134, MDAMB134VI, MDAMB157, MDAMB175, MDAMB175VII, MDAMB231, MDAMB330, MDAMB361, MDAMB415, MDAMB435, MDAMB436, MDAMB453, MDAMB468, T47D, ZR751, ZR75B, BSMZ, BT474, EFM192A, IBEP1, IBEP3, UACC812, ZR7527, ZR7530, 21MT1, 21MT2, 21NT, 21PT, AU565, HCC1008, HCC1569, HCC1954, HCC202, HCC2218, HH315, HH375, KPL-4, OCUB-F, SKBR3, SKBR5, SUM190PT, SUM225CWN, UACC893, BT20, CAL148, DU4475, EMG3, HCC38, HCC1143, HCC1187, HCC1395, HCC1599, HCC1739, HCC1806, HCC1937, HCC2157, HCC3153, HCC70, HMT3522, KPL-3C, MA11, MFM223, SUM185PE, SUM229PE, BT549, CAL120, CAL851, HDQ-P1, Hs578T, SKBR7, SUM102PT, SUM1315M02, SUM149PT, SUM159PT, or any combination thereof.

The data may be subject to one or more analysis or processing steps. The data may be analyzed and/or quantified to identify information or event(s) such as a splicing event. The information or event(s) identified may be statistically significant or specific to one or more diseases, illnesses or conditions. The data analysis or processing may comprise mapping the data to genomes, transcriptomes, or a combination thereof. The data may be processed to remove any information that may not be related to genomes, transcriptomes, or a combination thereof. The data may be processed or analyzed based on one or more predetermined parameters or criteria including, such as types or subtypes of diseases, illnesses, or conditions.

In cases where a database comprises data associated with different types or subtypes of diseases, illnesses, or conditions, data related to each type or subtype may be analyzed or processed individually to identify information or an event(s) that may be statistically significant or specific to each type or subtype. The identified information or event(s) may be grouped together and compared to one or more controls to determine one or more candidate targets. Alternatively, the information or event(s) identified for each subtype or type may be compared with one another to generate a list of candidate targets. In some cases, the candidate targets comprise information or an event(s) that is identified or shared by at least two different types or subtypes.

The list of candidate targets may comprise any number of candidate targets, for example, greater than or equal to about 5, 10, 20, 30, 40, 50, 60, 70, 80, 90, 100, 120, 140, 160, 180, 200, 220, 240, 260, 280, 300, 350, 400, 450, 500, or more. In some cases, the list may comprise a number of candidate targets falling between any of the two values described above, for example, about 275.

At least a portion (e.g., at least about 10%, 20%, 30%, 40%, 50%, 60%, 70%, 80%, 90%, or more) of the candidate targets generated may be subjected to further data analysis or processing. The candidate targets may be arranged in certain order based upon one or more parameters or criteria. The candidate targets may be selected based upon one or more parameters or criteria, thereby generating a refined list of candidate targets. Non-limiting examples of the parameters which may be used to arrange the candidate targets comprise a splicing index, a disease index, a splice-switching oligonucleotides (SSO) druggability index, or any combination thereof.

The splicing index may be determined based at least in part on factors including e.g., splicing change in a sample (or data) analyzed as compared to a control, consistency and/or reproducibility of a given information or event(s) (e.g., in patient dataset(s)), recurrence of a given information or event(s) in multiple disease datasets, an absence of a given information or event in a normal or control dataset(s). Each factor may be given the same or a different weight in the determination and based on the determination, a score may be generated.

The disease index may be determined based at least in part on factors including e.g., an impact of a given information or event(s) such as a splice change on function of an expression product(s) such as a protein(s), the degree of association with a disease, illness, or condition, pathway analysis such as pathways listed in kyoto encyclopedia of genes and genomes (KEGG) database, literature evidence, or any combination thereof. Each factor may be given the same or a different weight in the determination and based on the determination, a score may be generated.

The SSO druggability index may be determined based at least in part on factors including e.g., an ability to identify unique and/or specific splice correcting molecules (e.g., oligo sequence(s)) using AI such as machine learning algorithms, a presence or absence of a strong enhanced crosslinking and immunoprecipitation (eCLIP) peak(s) mapped to the identified target (e.g., an exon(s)), a presence or absence of a disease specific expression of an isoform(s) as compared to the genotype-tissue expression (GTEx) normal data, or any combination thereof. Each factor may be given the same or a different weight in the determination and a score may be generated based on the determination.

**In** cases in which a refined list of candidate targets is generated, candidate targets comprised in the list may be subject to additional analysis or processing steps. For example, splicing events associated with at least a subset of the candidate targets may be subjected to an evaluation process. The evaluation process may evaluate for expression at various levels, for example, at the RNA level, at a protein level, or both. The evaluation process may confirm differential isoform expression in samples from different diseases (or subtypes thereof), illnesses, or conditions. Upon confirmation, the candidate targets may be selected and used for further development of therapeutics. In some cases, the selected targets comprise one or more genes. Non-limiting examples of the one or more genes may comprise NEDD4L (ENV2), MAP3K7 (ENV3), NFYA (ENV11), ESYT2 (ENV21), MARK2 (ENV18), ST7 (ENV19), ARVCF (ENV22), SYTL2 (ENV17), R3HDM1 (ENV23), COL4A3BP (ENV9), TANGO2 (ENV6), SEPT9 (ENV15), ROBO1 (ENV4), FAM122B (ENV5), CD47 (ENV13), LSR (ENV20), PBX1 (ENV16), EPB41 (ENV14), ADAM15 (ENV7), EPB41L1 (ENV8), ABI1 (ENV10), FLNB (ENV1), CTNND1 (ENV12), GPR160 (ENV24), ITGB3BP (ENV25), INCENP (ENV26), DENNDIB (ENV27), CA12 (ENV28), or any combination thereof.

### Modulation of Targets using various Modalities

In some aspects, methods and systems for modulating a splicing target(s) are disclosed. The modulation may comprise modulating a splicing event(s) associated with a target (e.g., a gene). The modulation may comprise promoting or facilitating a splice switching. For example, the modulation may comprise switching pathogenic isoforms to non-pathogenic isoforms.

The modulation may comprise the use of one or more compositions or molecules which may interact specifically with (e.g., hybridize) a target so as to control or alter splicing of the target or regulate expression of the target at the RNA level, protein level or both. The compositions or molecules may be targeted to any element or combination of elements (e.g., one or more genomic regions within a target) that regulate splicing, including such as the 3 'splice site, the 5' splice site, the branch point, the polypyrimidine tract, exonic splicing enhancers, exonic splicing silencers, intronic splicing enhancers, intronic splicing silencers, or any combination thereof.

The compositions or molecules may comprise e.g., small molecules, polymers (natural or synthetic), nucleotide sequences such as oligonucleotides or RNAs, a therapeutic agent(s), cells such as CAR-T cells, a protein such as an antibody, or any combination thereof.

The compositions or molecules may be admixed, encapsulated, conjugated, or otherwise associated with other molecules, molecule structures, or mixtures of compounds, for example liposomes, receptor targeted molecules, oral, rectal, topical or other formulation, for assisting in uptake, distribution, and/or absorption.

The compositions or molecules may be applied *in vivo* or *ex vivo.* To achieve target-specific, or disease-specific targeting, the compositions or molecules may be added at a certain concentration. For example, the compositions or molecules may have a concentration that is less than or equal to about 5 micromolar (µM), 4 µM, 3 µM, 2 µM, 1 µM, 900 nanomolar (nM), 800 nM, 700 nM, 650 nM, 600 nM, 550 nM, 500 nM, 450 nM, 400 nM, 350 nM, 300 nM, 250 nM, 200 nM, 150 nM, 100 nM, 50 nM, 10 nM, or less. The concentration may be greater than or equal to about 1 nM, 10 nM, 50 nM, 100 nM, 200 nM, 300 nM, 400 nM, 500 nM, 600 nM, 700 nM, 800 nM, 900 nM, or more. In some cases, the concentration may fall between any two of the values discussed above, for example, about 370 nM or 420 nM.

In some cases, the compositions or molecules comprise oligonucleotides. The oligonucleotides may comprise any number of nucleotides or nucleotide residues, for example, greater than or equal to about 5, 10, 12, 14, 16, 18, 20, 22, 24, 26, 28, 30, 32, 34, 36, 38, 40, 42, 44, 46, 48, 50, 55, 60 nucleotides or nucleotide residues, or more. In some cases, the oligonucleotides may comprise less than or equal to about 50, 45, 40, 35, 30, 29, 27, 25, 24, 23, 22, 21, 19, 18, 17, 16, 13, 10, 8, 6 nucleotides or nucleotide residues, or less. In some cases, the number of nucleotides or nucleotide residues comprised in the oligonucleotides may fall between any of the values described above, for example, about 16 (16-mer), 17 (17-mer), 18 (18-mer), 19 (19-mer), 20 (20-mer), 21 (16-mer), or 22 (22-mer). In some cases, the oligonucleotides comprise DNA molecules, RNA molecules, or a combination thereof.

In some cases, the oligonucleotides comprise antisense oligonucleotides. The antisense oligonucleotides may be DNA and/or RNA oligos which are complementary to a given sequence, which given sequence may be a region within a target gene.

The oligonucleotides may be prepared using various technologies such as solid phase synthesis, the phosphorothioates and/or alkylated derivatives. The nucleotides or nucleotide residues comprised in the oligonucleotides may comprise natural, unmodified nucleotides (e.g., cytosine, guanine, adenine, uracil or thymidine), modified nucleotides, or any combination thereof. In some cases, modified nucleotides or bases are used. The modification may be designed to enhance binding affinity. The modification may comprise chemical modifications. The modification may comprise backbone modifications, sugar ring modifications, or a combination thereof. The sugar ring modifications may comprise 2'-sugar modifications. As an example, an oligonucleotide of the present disclosure may comprise a phosphothioate-modified backbone and/or ribose sugar modified to contain methoxy ethane at 2'-position (2'MOE). The oligonucleotides comprising modified nucleotides or bases may not activate RNase H. In some cases, one or more (e.g., at least about 1, 2, 3, 4, 5, 6, 7, 8, 9, 10, 12, 14, 16, 18, 20, 22, 24, 26, 28, 30, 35, 40, 45, or more) of the internucleotide bridging phosphate residues are modified phosphates, such as methyl phosphonates, methyl phosphonothioates, phosphoromorpholidates, phosphoropiperazidates, phosphoroamidates, or any combination thereof. In some cases, one or more (e.g., at least about 1, 2, 3, 4, 5, 6, 7, 8, 9, 10, 12, 14, 16, 18, 20, 22, 24, 26, 28, 30, 35, 40, 45, or more) of the nucleotides or bases comprise a 2'-alkyl moiety (e.g., C1-C4 alkyl, linear or branched, saturated or unsaturated including e.g., methyl, ethyl, ethenyl, propyl, 1-propenyl, 2-propenyl, isopropyl, or combination or derivative thereof).

In some cases, the compositions or molecules comprise small molecules. The small molecules may comprise a broad range of chemical compounds that can switch the isoforms of the above-mentioned targets either at the pre-mRNA level or protein level. These compounds may be identified through high-throughput screening approaches using chemical libraries, wherein addition of a compound or a combination of compounds can induce an isoform switch or modulate (e.g., inhibit or enhance) the biological activity of a specific isoform of one or several of the genes (e.g., genes mentioned above or described elsewhere herein) either at the level of RNA or protein or both.

### Application

The systems and methods of the present disclosure can be used for determining or identifying a novel splicing event(s) or a target (e.g., a gene) to which the novel splicing event is associated with. The novel splicing event(s) may be statistically significant or specific to one or more given types or subtypes of diseases, illnesses or conditions. To identify the novel splicing events, the methods and systems of the present disclosure may receive data from one or more databases, public and/or private, which data may comprise biologically relevant data with respect to the types or subtypes of diseases, illnesses or conditions which are under investigation. The data may be analyzed, processed or annotated. The data analysis, processing, and/or annotation may be conducted using machine learning algorithms. The machine learning may be a supervised learning, an unsupervised learning, or a combination thereof. The algorithm may be a trained algorithm. The algorithm may be trained using a training set. The training set may comprise training samples. The training samples may be cell lines from certain diseases, illnesses, or conditions; samples obtained from subjects having certain diseases, illnesses, or conditions; controls including positive and/or negative controls; or any combination thereof.

The data analysis, processing, and/or annotation may generate a list of candidate targets which may potentially be used for therapeutic development. Candidate targets comprised in the list may be subjected to further screening process or analyses. Splicing of the candidate targets may be evaluated or validated. The evaluation or validation of the candidate targets may yield a refined list of targets which may be subjected to further therapeutic development.

Splicing of individual targets comprised in the refined list may be using compositions or molecules. The splicing may be modulated to promote switching of pathogenic isoforms to non-pathogenic isoforms. The compositions or molecules may be designed to target a select region or a combination of select regions within a target to achieve a disease-specific targeting. In some cases, the compositions or molecules are designed to modulate the splicing of two or more (e.g., at least 3 ,4, 5, 6, 7, 8, 9, 10, 11, 12, 13, 14, 15, or more) different targets. Compositions or molecules targeting different targets may be used sequentially, or simultaneously.

In some aspects, methods and systems for providing treatment to a subject having or suspected to have a disease, illness, or condition are disclosed (not claimed). The methods and systems may comprise obtaining a sample from the subject having or suspected to have a disease, illness, or condition. Biologically relevant data (e.g., DNA, RNA-seq data) may be derived or extracted from the sample. The biologically relevant data may be screened or processed to remove any data unrelated to genome(s) or transcriptome(s). The processed data may be subjected to one or more data analysis, processing or annotation processes which may identify one or more novel splicing events statistically significant or specific to the disease, illness, or condition the subject has or suspected to have. The splicing events identified may be further analyzed or filtered using one or more parameters or filtering criteria, which may generate a final list of splicing events and targets associated therewith for the treatment (not claimed).

Upon identification of the targets, the systems and methods may further comprise administering a therapeutically effective amount of compositions or molecules to the subject having or suspected to have the disease, illness, or condition. The administration may be conducted within a given time period. The subject may be monitored, and the amount of the compositions or molecules administered to the subject may be adjusted depending upon, the monitoring results. Additionally, the monitoring may comprise obtaining one or more samples from the subject while the subject is under treatment (not claimed). The one or more samples may be analyzed or tested to determine if the treatment is effective or not. If a treatment is determined to be ineffective, the treatment may be ceased and/or a different treatment (e.g., administering a different type of compositions or molecules) may be provided (not claimed).

### Examples

### Example 1- Identification of alternatively spliced transcripts in Triple Negative Breast Cancer (TNBC) and therapeutics to correct the splicing change

Breast cancer is the most commonly diagnosed cancer and the second leading cause of cancer mortality in women, with nearly 30% of primary disease diagnoses that result in metastatic breast cancer. One of the challenges in breast cancer treatment is to overcome its large heterogeneity and distinct cancer subtypes that may demand differential treatments including chemotherapy, hormonal therapy, and human epidermal growth factor receptor 2 (Her2-) targeted therapy (depending on the subtype). However, a significant number of patients may develop resistance to current standard of care therapies. This stresses the need for identification of novel targets and development of alternative therapies for complete disease remission.

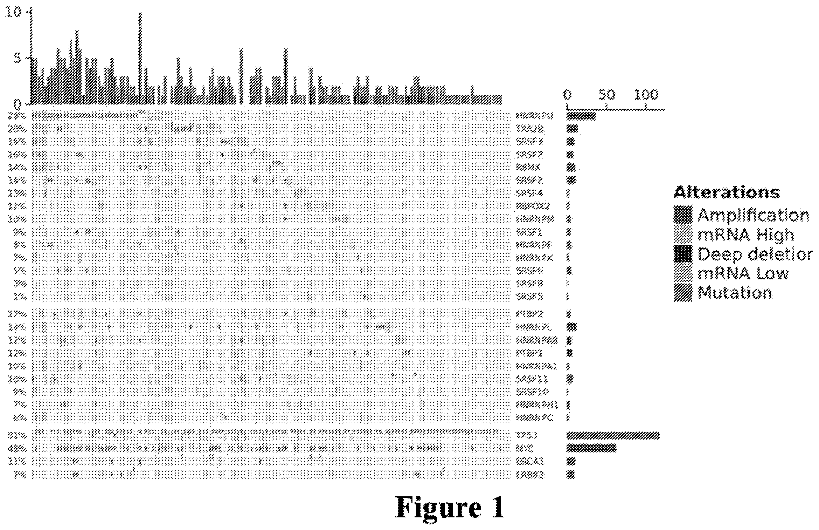

Splicing errors can be a source of coding variation in breast cancer. Aberrant splicing of several genes may occur even without DNA mutations or epigenetic changes due to mis-regulated expression of splicing factors in breast cancer. Multiple studies have indicated the oncogenic role of core splicing factors such as SRSF1, SRSF2, ESRP1, RBFOX1, etc. in breast cancer. For example, overexpression of SRSF1 may promote transformation of non-cancerous breast epithelial cells. Additionally, it has been suggested that spliceosomal components may be particularly essential factors in TNBC subtype, and the TNBC tumors sometimes show dependency on these factors. As an example, Figure 1 shows an oncoprint summary of recurring genomic aberration and transcriptional changes for splicing associated RNA binding proteins in the Cancer Genome Atlas (TCGA) breast cancer TNBC subtype patient samples.

Additionally, splice site mutations that result in expression of alternative isoforms have been reported in key breast cancer genes such as ESR1 encoding estrogen receptor alpha rendering resistance to first line drugs such as Tamoxifen in ER positive breast cancers. Mis-regulation of splicing factors have been shown to contribute to epithelial to mesenchymal switch by the production of mesenchymal isoforms of critical genes such as CD44 and FGFR2, thereby promoting tumor progression and metastasis. Given the suboptimal treatment options available for TNBC patients and the widespread splicing errors observed in TNBC patient RNA-seq data, disease specific alternative splicing can be a source of actionable candidates that can be therapeutically targeted.

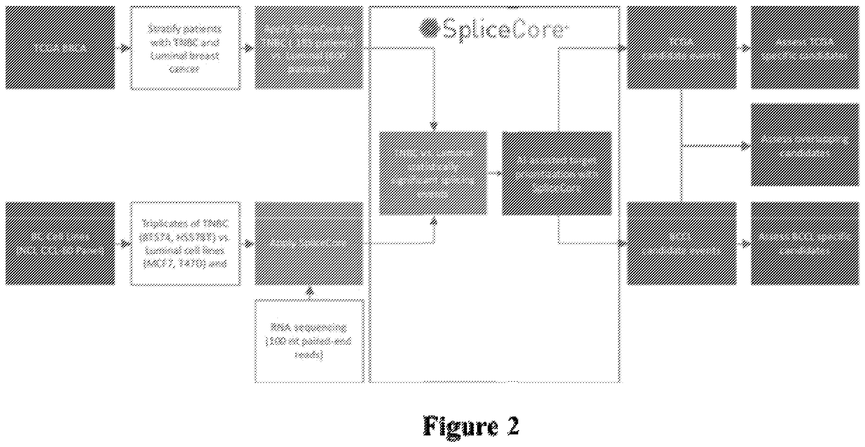

Systems of the present disclosure can be used to discover recurrent splicing changes in TNBC patient RNA-seq and design modalities such as antisense oligonucleotides that target the specific isoforms in order to promote splice-switching to achieve a therapeutic benefit. As discussed above or elsewhere herein, the systems may comprise the SpliceCore^{®} software platform described in International Patent Application No. WO2019/226804. In order to discover splicing changes that occur in TNBC subtypes, RNA-seq data from luminal subtype patients and TNBC subtype patients from TCGA breast cancer datasets are compared using the SpliceCore^{®} software platform. In addition, RNA-sequencing in triplicates on breast cancer cell lines is performed. Two representative luminal cell lines (i.e., MCF7, T47D) and two representative TNBC B subtype cell lines (i.e., HS578T, BT549) and one TNBC A subtype cell line (i.e., MDA-MB 468) are used. A flowchart of the SpliceCore^{®} analysis of the TCGA and the cell line RNA seq data is illustrated in Figure 2.

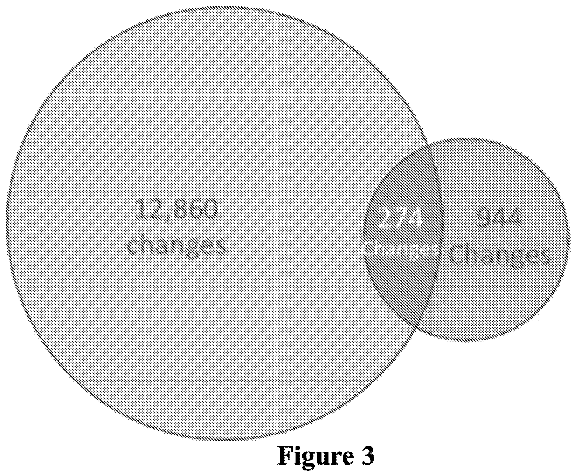

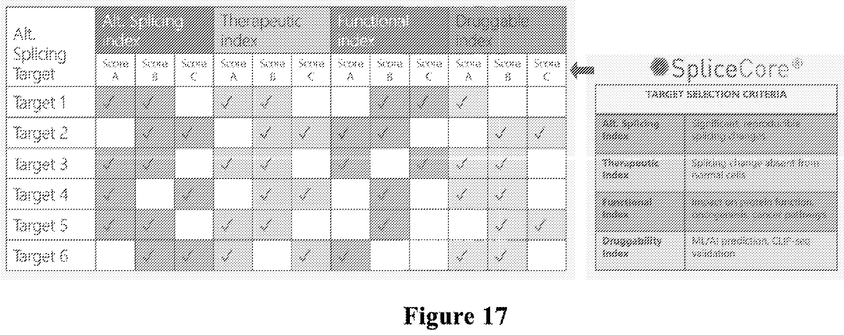

Comparison of the splicing changes between luminal breast cancer and basal breast cancer from TCGA and the cell lines may independently result in an identification of splicing changes that are distinct to TCGA (12,860 changes) and changes that are distinct to cell lines (944 changes) (Figure 3). Among those changes, there are about 274 splicing changes that are identified in both TCGA and cell lines (Figure 3). These 274 splicing changes are considered candidates for target selection and may be subject to further analysis. The analysis may be prioritized based on a number of parameters. The parameters (or buckets) for candidate selection may serve as diversified target selection criteria, which may include e.g., a splicing index, a disease index, a therapeutic index, a functional index, a splice-switching oligonucleotide (SSO) druggability index or any combination thereof. Example selection criteria and results are shown in Figure 17. The figure shows a representative illustration of candidate selection scoring matrix based on different parameters described above.

The splicing index can score for the splicing change (dPSI) in a given case and a control, consistency, reproducibility of a given event in patient datasets, and recurrence of a given event in multiple disease datasets and absent in normal datasets. The disease index can score for impact of the splicing change on protein function (i.e., "SpliceImpact^{™}"), disease association (integrated through Open Target scores), pathway analysis (KEGG), and literature evidence. The SSO druggability index can score for the ability to identify unique and specific splice correcting oligo sequence using machine learning (i.e., "SpliceLearn^{™} scores"), presence of strong eCLIP peaks mapped to the identified exons, and disease specific expression of the isoform (comparison with GTex normal data).

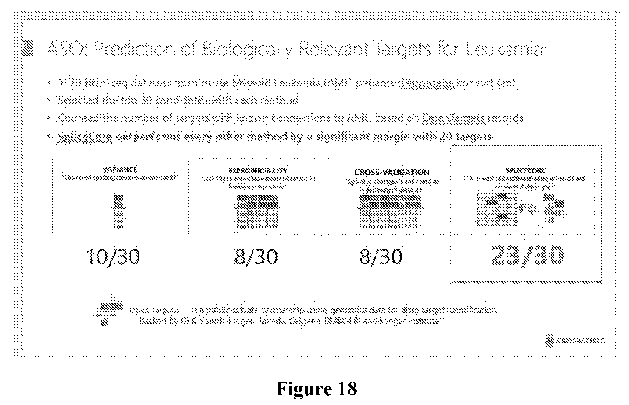

Figure 18 reveals the identification of biologically relevant targets for the treatment of leukemia as described herein. About 1178 RNA-seq datasets from Acute Myeloid Leukemia (AML) patients obtained from the Leucegene consortium were analyzed to identify potential therapeutic targets. Different approaches were used to analyze alternative splicing events including variance assessment (selection of strongest splicing changes above a cutoff), reproducibility (selection for splicing changes repeatedly observed in biological replicates), cross-validation (splicing changes confirmed in independent dataset(s)), and SpliceCore^{®} (software platform described in International Patent Application No. WO2019/226804. For each approach, the top 30 gene candidates were selected and the gene candidates that were also known to be connected to AML pathogenesis using records from OpenTargets (a public-private partnership using genomics data for drug target identification backed by GSK, Sanofi, Biogen, Takeda, Celgene, EMBL-EBI and Sanger Institute). As shown in Figure 18, 23 of the top 30 candidates identified by SpliceCore were known to be connected to AML. The other approaches identified few candidates known to be connected to AML: the variance approach identified 10 targets; the reproducibility approach identified 8 targets; and the cross-validation approach identified 8 targets.

In an exemplary application of the selection criteria in Figure 17, the alternative splicing index is determined by observing one or more alternative splicing event(s)/change(s) in in-house cell lines and public BRCA TCGA RNA-seq data; the therapeutic index is determined by confirming that the one or more alternative splicing event(s)/change(s) is/are disease-specific and is/are not found in normal breast tissues using public GTEx RNA-seq; the functional index is determined by noting that the score(s) for the one or more alternative splicing event(s)/change(s) generated by SpliceImpact (software platform described in International Patent Application No. WO2019/226804) are significantly disruptive; and the druggable index is determined by using SpliceLearn (software platform described in International Patent Application No. WO2019/226804) to predict that the one or more alternative splicing event(s)/change(s) is a drug target. In some cases, the drug target is an SSO modulatory target.

In an another exemplary application of the selection criteria in Figure 17, the alternative splicing index is determined by observing one or more alternative splicing event(s)/change(s) in in-house organoids and public BRCA TCGA RNA-seq from the Metabrick dataset; the therapeutic index is determined by confirming that the one or more alternative splicing event(s)/change(s) is/are disease-specific and is/are not found in various post-mortem tissues including liver, heart, muscle and/or kidney, using public GTEx RNA-seq; the functional index is determined by noting that the one or more alternative splicing event(s)/change(s) occur in one or more genes with high BRCA-association scores estimated using OpenTargets; and the druggable index is determined by confirming that binding of one or more oncogenic splicing factors to the one or more target genes with the one or more alternative splicing event(s)/change(s) using CLIP-seq data. In some cases, the one or more target genes may be blocked by ASO based in the selection criteria analyses.

In an another exemplary application of the selection criteria in Figure 17, the alternative splicing index is determined by observing one or more alternative splicing event(s)/change(s) in in-house cell lines and licensed RNA-seq from a partner; the therapeutic index is determined by confirming that the one or more alternative splicing event(s)/change(s) is/are disease-specific and is/are not found in normal tissues from partner RNA-seq; the functional index is determined by confirming that the one or more alternative splicing event(s)/change(s) are functionally related breast cancer using public literature; and the druggable index is determined by confirming that one or more alternative splicing event(s)/change(s) occurs in one or more genes that is/are known to be small molecule protein target(s).

Based on one or more of the above-mentioned filtering criteria or parameters, a total of 28 candidates whose splice changes may be significant in TNBC subtype (Table 1 - List of TNBC specific top scoring splicing events and their corresponding gene names) are selected. These candidates' splicing events are subsequently subjected to an evaluation for expression at the level of RNA through PCR in experimental models such as cell lines, primary cells, tissues, organoids, PDX tumors, patient tissue material, body fluids, etc. Wherever antibodies are available, splicing isoforms may also be evaluated for protein expression. Hybridization methods such as RNA-FISH can also be used to validate the specific isoform expression in tumor tissue sections.

**Table 1**

| **TXDBID** | **Gene (HUGO)** | **ENV Code** |
|---|---|---|
| CA-18-58335477-58335537.2267.0 | NEDD4L | ENV2 |
| CA-6-90544551-90544632.1 | MAP3K7 | ENV3 |
| CA-6-41080810-41080897.1 | NFYA | ENV11 |
| CA-7-158752780-158752843.1 | ESYT2 | ENV21 |
| CA-11-63903985-63904147.1 | MARK2 | ENV18 |
| CA-7-117134123-117134192.1 | ST7 | ENV19 |
| CA-22-19971215-19971335.1 | ARVCF | ENV22 |
| CA-11-85717482-85717530.1 | SYTL2 | ENV17 |
| CA-2-135641535-135641604.2 | R3HDM1 | ENV23 |
| CA-5-75399309-75399387.1 | COL4A3BP | ENV9 |
| CA-22-20053436-20053551.3 | TANGO2 | ENV6 |
| CA-17-77307140-77307197.5 | SEPT9 | ENV15 |
| CA-3-78647628-78647655.1 | ROBO1 | ENV4 |
| CA-X-134772142-134772283.1 | FAM122B | ENV5 |
| CA-3-58141857-58141929.1 | FLNB | ENV1 |
| CA-3-108050577-108050602.2 | CD47 | ENV13 |
| CA-1-29058588-29058645.2 | EPB41 | ENV14 |
| CA-1-164820071-164820184.1 | PBX1 | ENV16 |
| CA-19-35262545-35262692.1 | LSR | ENV20 |
| CA-1-155061417-155061489.4 | ADAM15 | ENV7 |
| CA-20-36209487-36209898.2 | EPB41L1 | ENV8 |
| CA-10-26755654-26755741.1 | ABI1 | ENV10 |
| CA-11-57791493-57791673.2 | CTNND 1 | ENV12 |
| CA-3-170082616-170082758.1 | GPR160 | ENV24 |
| CA-1-63447564-63447614.1 | ITGB3BP | ENV25 |
| CA-11-62141499-62141511.1 | INCENP | ENV26 |
| CA-1-197647054-197647114.1 | DENND1B | ENV27 |
| CA-15-63328097-63328130.1 | CA12 | ENV28 |

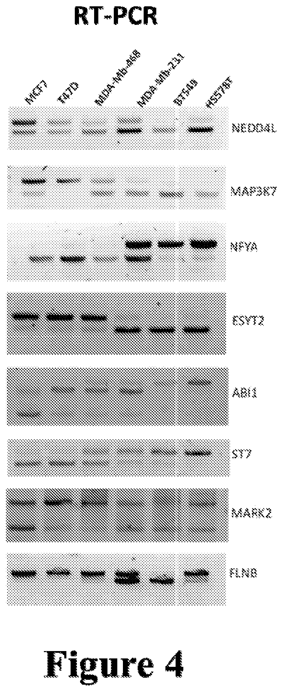

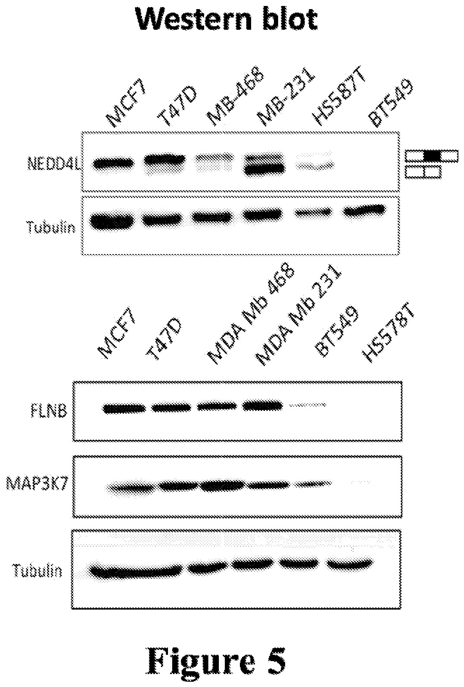

First, reverse transcription polymerase chain reaction (RT-PCR) is performed on selected candidates from the list of Table 1 to confirm the differential isoform expression in luminal versus the basal cell lines. Representative PCRs are shown in Figure 4 where specific expression of the long or the short isoform is enriched in TNBC cell lines versus luminal cell lines. Further, for a select group of candidates, western blot analysis is also performed to verify the protein expression, and the representative western blot images for those candidates are shown in Figure 5, which also shows differential isoform expression in protein lysates extracted from luminal and basal cell lines.

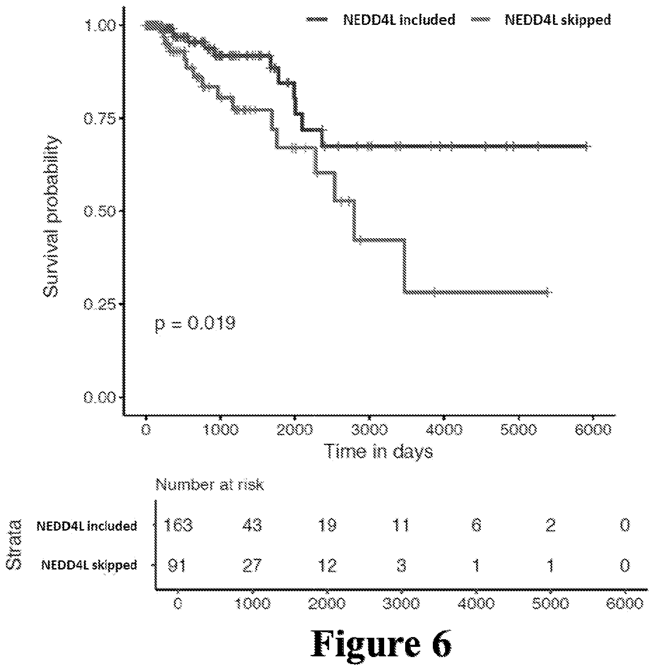

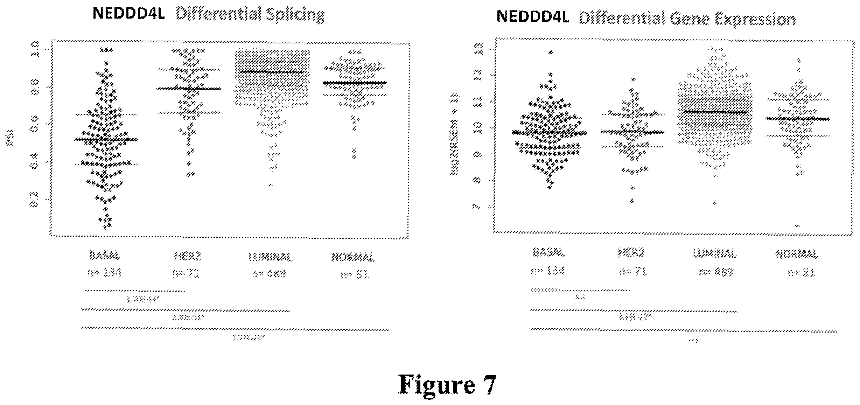

Next, specific candidates are focused on to test to see if they can be used as a good therapeutic target for the development therapeutic compounds. NEDD4L is suggested by the SpliceCore software platform as one of the top candidates and has shown the strongest dPSI change in TCGA data and very high reproducibility, along with known cancer association in a key signaling pathway (i.e., TGFbeta). By studying the impact of the observed splicing changes on protein function, it is determined that the skipping isoform (short isoform) enriched in TNBC subtype lacks a short loop region next to the WW domain which may be responsible for protein-protein interaction. The loop contains a Threonine residue that may undergo post-translational modification by a kinase, which can phosphorylate NEDD4L to maintain the homeostasis of TGFbeta signaling. The TNBC cancer-specific loss of the loop through alternative splicing may deregulate the signaling cascade leading to tumor progression. Additionally, it is observed that breast cancer patients expressing the inclusion isoform of NEDD4L have a better overall survival compared to the breast cancer patients that have the expression of the skipped isoform (Figure 6). Further analyses of the subtype stratified data from TCGA have shown that the NEDD4L-skipping isoform is significantly enriched in TNBC subtype compared to normal breast or luminal or HER2 subtypes of breast cancer. This difference has been observed only at the level of alternative splicing and there is only a modest difference in the total RNA expression of NEDD4L across these subtypes (Figure 7).

By using the SpliceCore^{®} software platform and a module called SpliceLearn, a machine-learning-based (ML-based) approach is used to predict nucleotide sequences with high likelihoods of promoting a splicing switch if blocked using a molecule (e.g., an oligonucleotide). These sequences are scored, and rank-ordered for every exon trio on the candidate list. Additional scoring criteria may include the RBP binding peaks which can be obtained from ENCODE eCLIP-seq data and/or in-house generated eCLIP-seq data for splicing regulatory proteins including but not limited to RBFOX2, TDP-43, HNRNPL.

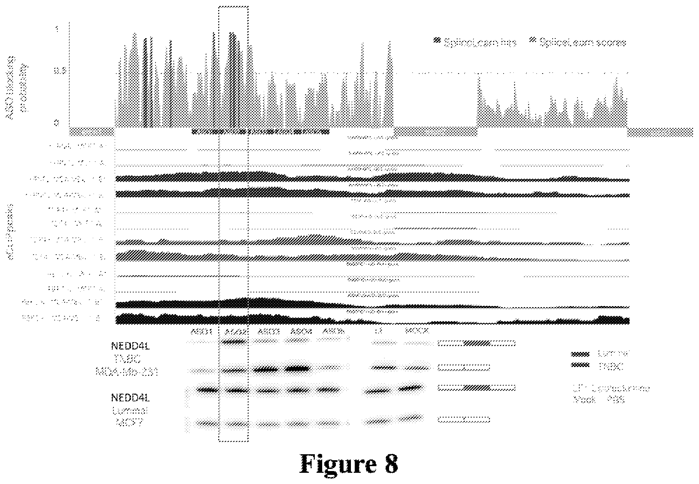

Next, a list of k-mer sequences can be generated that span across the high scoring regions within the exon trio and may further be filtered based on SSO specificity, repeat motifs, and off-target effects, and secondary structure (Figure 8). The top 5 oligos are chemically synthesized and may contain phosphothioate-modified backbone and/or ribose sugar uniformly modified to contain methoxy ethane in 2' position (2'MOE). Purified oligonucleotides can be subjected to functional assays in breast cancer cell lines.

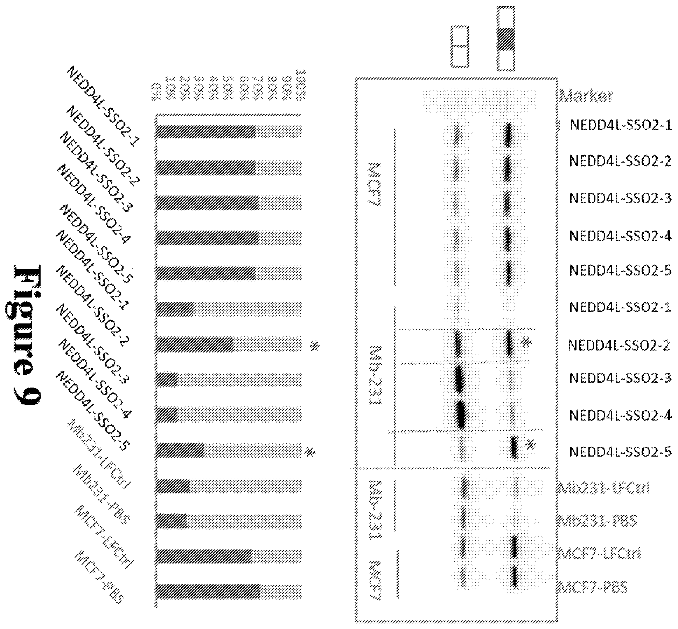

For NEDD4L, 5 different sequences (GCTGGCTTTGTCTGGATAGG, GTGGGTTTCAGGGATTCTGA, TCTCACGTCACCTGCCTTAC, AGCGCTGCCACAGCAGTGGG,CCCTGATTCAGACAGCAGGG) are tested in total, and 2 of those sequences are found to promote the inclusion of the middle exon significantly. GTGGGTTTCAGGGATTCTGA (SSO2-2) is shown to promote an average of 40% inclusion ratio in 3 out of 4 experiments, and (SSO2-5) CCCTGATTCAGACAGCAGGG is shown to promote an average of 30% inclusion ratio in 4 out of 4 experiments (Figure 9).

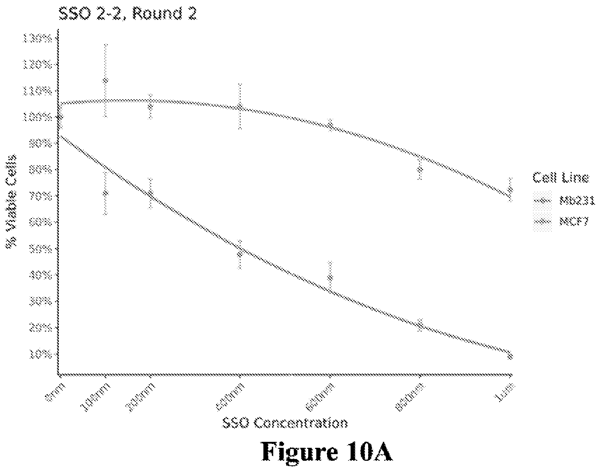

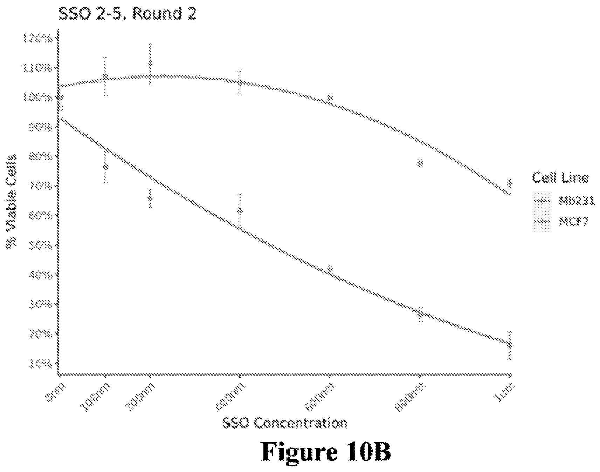

Dosage response experiments are performed to evaluate the LC50 value for the SSO compounds in 2 breast cancer cell lines - i.e., the MCF7 (luminal) and MDA-MB-231 (TNBC) cell lines. Transfection of the SSO compounds show a dose responsive loss of viability in the MDA-MB-231 cells and not in the MCF7 cells. The optimal dosing concentration in cell lines is found to be between 370nM- 420nM where >50% of the cell death has been observed. The cell viability can be evaluated by measuring the mitochondrial ATP flux using the Celltitre glow assay. The mean luminescence can be converted to percentage of viable cells after normalizing to control untransfected cells. The dose response curve for both the SSO on two different cell lines is shown in Figures 10A-10B.

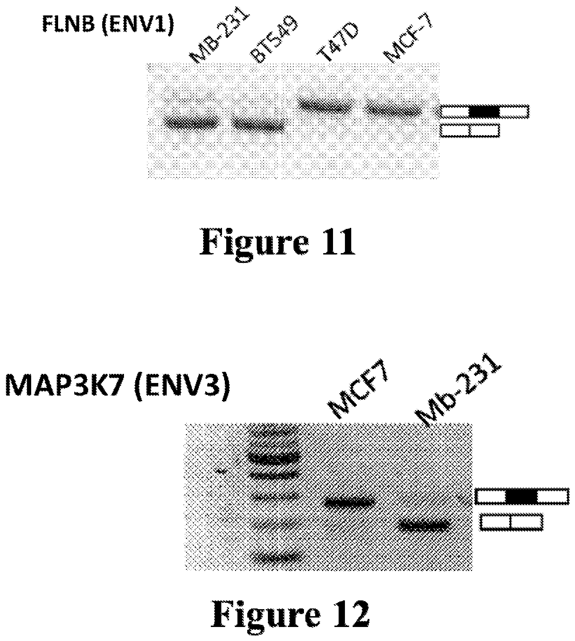

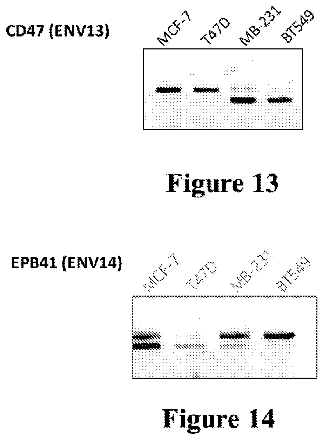

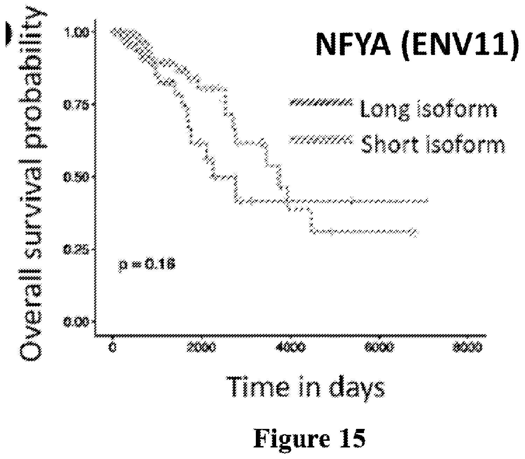

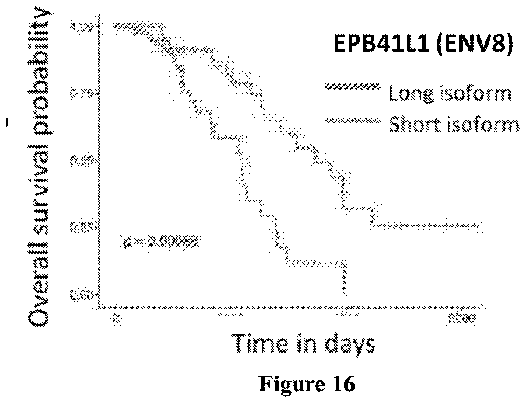

The criticality of alternative splicing events for TNMC tumors are also shown in Figure 11, Figure 12, Figure 13 and Figure 14. The alternative splicing events may be associated with one or more genes as described above or elsewhere herein, e.g., genes comprising NEDD4L (ENV2), MAP3K7 (ENV3), NFYA (ENV11), ESYT2 (ENV21), MARK2 (ENV18), ST7 (ENV19), ARVCF (ENV22), SYTL2 (ENV17), R3HDM1 (ENV23), COL4A3BP (ENV9), TANGO2 (ENV6), SEPT9 (ENV15), ROBO1 (ENV4), FAM122B (ENV5), CD47 (ENV13), LSR (ENV20), PBX1 (ENV16), EPB41 (ENV14), ADAM15 (ENV7), EPB41L1 (ENV8), ABI1 (ENV10), FLNB (ENV1), CTNND1 (ENV12), GPR160 (ENV24), ITGB3BP (ENV25), INCENP (ENV26), DENNDIB (ENV27), CA12 (ENV28), or a combination thereof. Survival analysis of TCGA BRCA patients containing long and short isoforms for candidates is conducted with results illustrated in Figure 15 and Figure 16, respectively. The candidates may be one or more genes as described above or elsewhere herein, e.g., genes comprising NEDD4L (ENV2), MAP3K7 (ENV3), NFYA (ENV11), ESYT2 (ENV21), MARK2 (ENV18), ST7 (ENV19), ARVCF (ENV22), SYTL2 (ENV17), R3HDM1 (ENV23), COL4A3BP (ENV9), TANGO2 (ENV6), SEPT9 (ENV15), ROBO1 (ENV4), FAM122B (ENV5), CD47 (ENV13), LSR (ENV20), PBX1 (ENV16), EPB41 (ENV14), ADAM15 (ENV7), EPB41L1 (ENV8), ABI1 (ENV10), FLNB (ENV1), CTNND1 (ENV12), GPR160 (ENV24), ITGB3BP (ENV25), INCENP (ENV26), DENNDIB (ENV27), CA12 (ENV28), or a combination thereof. The analysis shows significant different in overall survival in patients expressing either of the isoforms.

Thus, the above results show that TNBC subtype can be vulnerable to splicing changes. Using the software platform of the present disclosure, reproducible and high impact splicing changes can be identified and validated in RNA-seq datasets from patient samples. The candidates listed can potentially serve as a therapeutic target for TNBC breast cancer. The platform has also designed and nominated oligonucleotide sequences that can promote splice switching when targeted using antisense oligos. The platform-designed oligos are experimentally validated for NEDD4L using uniformly modified 2'MOE oligonucleotide chemistry. The NEDD4L alternative splicing is a splicing event specific to TNBC subtype of breast cancer. Targeting NEDD4L isoform switching using modalities such as antisense oligonucleotides exhibits selective viability loss in TNBC cells specifically in a dose responsive manner. As such, NEDD4L splicing can be an actionable event to develop therapeutic to treat TNBC patients.

It shall be understood that although the above example is related to TNBC RNA-seq datasets, the NEDD4L and other candidate splicing events as discussed above or elsewhere herein can also be important in other solid or hematological malignancies, as well as in neurological or metabolic diseases. One or more of the splicing changes can be responsible for pathogenesis or progression of such diseases, disorders, or conditions, and the therapeutic targeting of the present disclosure can be applied to such diseases, disorders, or conditions.

Alternatively or additionally, the splicing targets can be modulated using modalities including, but not limited to, small molecules, GAPMER oligonucleotides, siRNAs, CAR-T cells, or any combination thereof to achieve disease-specific targeting. For example, some of the disease-specific splicing events that have been identified can open up grooves for small molecule binding. In such case, small molecules can be designed to target the region that is created because of a splicing change. In another example, splicing changes of the membrane bound proteins can display altered surface epitopes which can be specifically targeted using antibodies. The SpliceCore software platform, and the methods as described above and elsewhere herein, can be used to analyze, design, and develop multimodal therapeutic targets for a wide range of disease indications.

### Example 2 - Target-specific anti-sense oligonucleotides (ASOs)

As described above and elsewhere herein, the ASOs can be identified based on exon position and SpliceLearn^{™} features such as RNA binding protein. The ASOs can be used for splice switching experiments to promote exon inclusion in the target candidates disclosed herein. In some cases, chemically-modified ASOs may be synthesized based on the ASOs identified. For example, one or more chemical modifications can be introduced in one or more ASOs. The chemical modification can comprise a phosphorothioate backbone modification and/or 2'-O-(2 Methoxyethyl) ribose modification (2'MOE) (modification of the ribose sugar). Sequences of the ASOs may be used for one or more *ex vivo* experiments on breast cancer cell lines to determine the effect of the ASOs on splice switching of the target gene candidates.

A select group of the ASOs may be used for further *in vitro* and *in vivo* experiments and preclinical studies. In some instances, the specific splice switching event can be identified to be strongly associated with triple negative breast cancer (TNBC). In some aspects, the ASOs may have potent splice switching effects with less toxicity. In some cases, the ASOs can be used in preclinical studies and/or in the therapeutic development of targeting of cancer-specific genes in patients. For example, an ASO can be used in the therapeutic development in the targeting of TNBC breast cancer patients by inducing a splice switch that can potentially have an anti-tumor effect.

Tables 2-8 list example target-specific oligonucleotide sequences of variable sequence lengths which may be used to induce an isoform switch or modulate (e.g., inhibit or enhance) the biological activity of a specific isoform of one or several of the genes described above or elsewhere herein (e.g., genes comprising one or more of NEDD4L (ENV2), MAP3K7 (ENV3), NFYA (ENV11), ESYT2 (ENV21), MARK2 (ENV18), ST7 (ENV19), ARVCF (ENV22), SYTL2 (ENV17), R3HDM1 (ENV23), COL4A3BP (ENV9), TANGO2 (ENV6), SEPT9 (ENV15), ROBO1 (ENV4), FAM122B (ENV5), CD47 (ENV13), LSR (ENV20), PBX1 (ENV16), EPB41 (ENV14), ADAM15 (ENV7), EPB41L1 (ENV8), ABI1 (ENV10), FLNB (ENV1), CTNND1 (ENV12), GPR160 (ENV24), ITGB3BP (ENV25), INCENP (ENV26), DENNDIB (ENV27), CA12 (ENV28).

In some cases, oligonucleotide sequences comprised in a table are specific for a single target. In some cases, oligonucleotide sequences comprised in a table are specific for more than one target. In some cases, oligonucleotide sequences comprised in more than one tables are specific for a single target. For example, oligonucleotide sequences comprised in Tables 2-8 may be specific for a single target. The target may be a gene selected from genes described above or elsewhere herein.

**Table 2: 16-mer target-specific ASOs**

| **CHR** | **START** | **END** | **STRAND** | **kmer** | **SEQUENCE** |
|---|---|---|---|---|---|
| chr3 | 58141828 | 58141843 | +/- | 16 | CCCAACTAATCTCCAT |
| chr3 | 58141829 | 58141844 | +/- | 16 | CCAACTAATCTCCATT |
| chr3 | 58141830 | 58141845 | +/- | 16 | CAACTAATCTCCATTT |
| chr3 | 58141831 | 58141846 | +/- | 16 | AACTAATCTCCATTTG |
| chr3 | 58141832 | 58141847 | +/- | 16 | ACTAATCTCCATTTGC |
| chr3 | 58141833 | 58141848 | +/- | 16 | CTAATCTCCATTTGCC |
| chr3 | 58141834 | 58141849 | +/- | 16 | TAATCTCCATTTGCCA |
| chr3 | 58141835 | 58141850 | +/- | 16 | AATCTCCATTTGCCAC |
| chr3 | 58141836 | 58141851 | +/- | 16 | ATCTCCATTTGCCACT |
| chr3 | 58141837 | 58141852 | +/- | 16 | TCTCCATTTGCCACTG |
| chr3 | 58141838 | 58141853 | +/- | 16 | CTCCATTTGCCACTGA |
| chr3 | 58141839 | 58141854 | +/- | 16 | TCCATTTGCCACTGAC |
| chr3 | 58141840 | 58141855 | +/- | 16 | CCATTTGCCACTGACC |
| chr3 | 58141841 | 58141856 | +/- | 16 | CATTTGCCACTGACCA |
| chr3 | 58141842 | 58141857 | +/- | 16 | ATTTGCCACTGACCAG |
| chr3 | 58141843 | 58141858 | +/- | 16 | TTTGCCACTGACCAGG |
| chr3 | 58141844 | 58141859 | +/- | 16 | TTGCCACTGACCAGGC |
| chr3 | 58141845 | 58141860 | +/- | 16 | TGCCACTGACCAGGCC |
| chr3 | 58141846 | 58141861 | +/- | 16 | GCCACTGACCAGGCCA |
| chr3 | 58141847 | 58141862 | +/- | 16 | CCACTGACCAGGCCAC |
| chr3 | 58141848 | 58141863 | +/- | 16 | CACTGACCAGGCCACA |
| chr3 | 58141849 | 58141864 | +/- | 16 | ACTGACCAGGCCACAG |
| chr3 | 58141850 | 58141865 | +/- | 16 | CTGACCAGGCCACAGA |
| chr3 | 58141851 | 58141866 | +/- | 16 | TGACCAGGCCACAGAT |
| chr3 | 58141852 | 58141867 | +/- | 16 | GACCAGGCCACAGATG |
| chr3 | 58141853 | 58141868 | +/- | 16 | ACCAGGCCACAGATGG |
| chr3 | 58141854 | 58141869 | +/- | 16 | CCAGGCCACAGATGGG |
| chr3 | 58141855 | 58141870 | +/- | 16 | CAGGCCACAGATGGGG |
| chr3 | 58141856 | 58141871 | +/- | 16 | AGGCCACAGATGGGGA |
| chr3 | 58141857 | 58141872 | +/- | 16 | GGCCACAGATGGGGAA |
| chr3 | 58141858 | 58141873 | +/- | 16 | GCCACAGATGGGGAAG |
| chr3 | 58141859 | 58141874 | +/- | 16 | CCACAGATGGGGAAGT |
| chr3 | 58141860 | 58141875 | +/- | 16 | CACAGATGGGGAAGTC |
| chr3 | 58141861 | 58141876 | +/- | 16 | ACAGATGGGGAAGTCA |
| chr3 | 58141862 | 58141877 | +/- | 16 | CAGATGGGGAAGTCAC |
| chr3 | 58141863 | 58141878 | +/- | 16 | AGATGGGGAAGTCACA |
| chr3 | 58141864 | 58141879 | +/- | 16 | GATGGGGAAGTCACAG |
| chr3 | 58141865 | 58141880 | +/- | 16 | ATGGGGAAGTCACAGC |
| chr3 | 58141866 | 58141881 | +/- | 16 | TGGGGAAGTCACAGCC |
| chr3 | 58141867 | 58141882 | +/- | 16 | GGGGAAGTCACAGCCG |
| chr3 | 58141868 | 58141883 | +/- | 16 | GGGAAGTCACAGCCGT |
| chr3 | 58141869 | 58141884 | +/- | 16 | GGAAGTCACAGCCGTG |
| chr3 | 58141870 | 58141885 | +/- | 16 | GAAGTCACAGCCGTGG |
| chr3 | 58141871 | 58141886 | +/- | 16 | AAGTCACAGCCGTGGA |
| chr3 | 58141872 | 58141887 | +/- | 16 | AGTCACAGCCGTGGAG |
| chr3 | 58141873 | 58141888 | +/- | 16 | GTCACAGCCGTGGAGG |
| chr3 | 58141874 | 58141889 | +/- | 16 | TCACAGCCGTGGAGGA |
| chr3 | 58141875 | 58141890 | +/- | 16 | CACAGCCGTGGAGGAG |
| chr3 | 58141876 | 58141891 | +/- | 16 | ACAGCCGTGGAGGAGG |
| chr3 | 58141877 | 58141892 | +/- | 16 | CAGCCGTGGAGGAGGC |
| chr3 | 58141878 | 58141893 | +/- | 16 | AGCCGTGGAGGAGGCA |
| chr3 | 58141879 | 58141894 | +/- | 16 | GCCGTGGAGGAGGCAC |
| chr3 | 58141880 | 58141895 | +/- | 16 | CCGTGGAGGAGGCACC |
| chr3 | 58141881 | 58141896 | +/- | 16 | CGTGGAGGAGGCACCG |
| chr3 | 58141882 | 58141897 | +/- | 16 | GTGGAGGAGGCACCGG |
| chr3 | 58141883 | 58141898 | +/- | 16 | TGGAGGAGGCACCGGT |
| chr3 | 58141884 | 58141899 | +/- | 16 | GGAGGAGGCACCGGTA |
| chr3 | 58141885 | 58141900 | +/- | 16 | GAGGAGGCACCGGTAA |
| chr3 | 58141886 | 58141901 | +/- | 16 | AGGAGGCACCGGTAAA |
| chr3 | 58141887 | 58141902 | +/- | 16 | GGAGGCACCGGTAAAT |
| chr3 | 58141888 | 58141903 | +/- | 16 | GAGGCACCGGTAAATG |
| chr3 | 58141889 | 58141904 | +/- | 16 | AGGCACCGGTAAATGC |
| chr3 | 58141890 | 58141905 | +/- | 16 | GGCACCGGTAAATGCA |
| chr3 | 58141891 | 58141906 | +/- | 16 | GCACCGGTAAATGCAT |
| chr3 | 58141892 | 58141907 | +/- | 16 | CACCGGTAAATGCATG |
| chr3 | 58141893 | 58141908 | +/- | 16 | ACCGGTAAATGCATGT |
| chr3 | 58141894 | 58141909 | +/- | 16 | CCGGTAAATGCATGTC |
| chr3 | 58141895 | 58141910 | +/- | 16 | CGGTAAATGCATGTCC |
| chr3 | 58141896 | 58141911 | +/- | 16 | GGTAAATGCATGTCCC |
| chr3 | 58141897 | 58141912 | +/- | 16 | GTAAATGCATGTCCCC |
| chr3 | 58141898 | 58141913 | +/- | 16 | TAAATGCATGTCCCCC |
| chr3 | 58141899 | 58141914 | +/- | 16 | AAATGCATGTCCCCCT |
| chr3 | 58141900 | 58141915 | +/- | 16 | AATGCATGTCCCCCTG |
| chr3 | 58141901 | 58141916 | +/- | 16 | ATGCATGTCCCCCTGG |
| chr3 | 58141902 | 58141917 | +/- | 16 | TGCATGTCCCCCTGGA |
| chr3 | 58141903 | 58141918 | +/- | 16 | GCATGTCCCCCTGGAT |
| chr3 | 58141904 | 58141919 | +/- | 16 | CATGTCCCCCTGGATT |
| chr3 | 58141905 | 58141920 | +/- | 16 | ATGTCCCCCTGGATTC |
| chr3 | 58141906 | 58141921 | +/- | 16 | TGTCCCCCTGGATTCA |
| chr3 | 58141907 | 58141922 | +/- | 16 | GTCCCCCTGGATTCAG |
| chr3 | 58141908 | 58141923 | +/- | 16 | TCCCCCTGGATTCAGG |
| chr3 | 58141909 | 58141924 | +/- | 16 | CCCCCTGGATTCAGGC |
| chr3 | 58141910 | 58141925 | +/- | 16 | CCCCTGGATTCAGGCC |
| chr3 | 58141911 | 58141926 | +/- | 16 | CCCTGGATTCAGGCCC |
| chr3 | 58141912 | 58141927 | +/- | 16 | CCTGGATTCAGGCCCT |
| chr3 | 58141913 | 58141928 | +/- | 16 | CTGGATTCAGGCCCTG |
| chr3 | 58141914 | 58141929 | +/- | 16 | TGGATTCAGGCCCTGG |
| chr3 | 58141915 | 58141930 | +/- | 16 | GGATTCAGGCCCTGGG |
| chr3 | 58141916 | 58141931 | +/- | 16 | GATTCAGGCCCTGGGT |
| chr3 | 58141917 | 58141932 | +/- | 16 | ATTCAGGCCCTGGGTA |
| chr3 | 58141918 | 58141933 | +/- | 16 | TTCAGGCCCTGGGTAC |
| chr3 | 58141919 | 58141934 | +/- | 16 | TCAGGCCCTGGGTACA |
| chr3 | 58141920 | 58141935 | +/- | 16 | CAGGCCCTGGGTACAA |
| chr3 | 58141921 | 58141936 | +/- | 16 | AGGCCCTGGGTACAAT |
| chr3 | 58141922 | 58141937 | +/- | 16 | GGCCCTGGGTACAATT |
| chr3 | 58141923 | 58141938 | +/- | 16 | GCCCTGGGTACAATTT |
| chr3 | 58141924 | 58141939 | +/- | 16 | CCCTGGGTACAATTTT |
| chr3 | 58141925 | 58141940 | +/- | 16 | CCTGGGTACAATTTTG |
| chr3 | 58141926 | 58141941 | +/- | 16 | CTGGGTACAATTTTGG |
| chr3 | 58141927 | 58141942 | +/- | 16 | TGGGTACAATTTTGGT |
| chr3 | 58141928 | 58141943 | +/- | 16 | GGGTACAATTTTGGTT |
| chr3 | 58141929 | 58141944 | +/- | 16 | GGTACAATTTTGGTTT |
| chr3 | 58141930 | 58141945 | +/- | 16 | GTACAATTTTGGTTTT |
| chr3 | 58141931 | 58141946 | +/- | 16 | TACAATTTTGGTTTTT |
| chr3 | 58141932 | 58141947 | +/- | 16 | ACAATTTTGGTTTTTT |
| chr3 | 58141933 | 58141948 | +/- | 16 | CAATTTTGGTTTTTTC |
| chr3 | 58141934 | 58141949 | +/- | 16 | AATTTTGGTTTTTTCC |
| chr3 | 58141935 | 58141950 | +/- | 16 | ATTTTGGTTTTTTCCT |
| chr3 | 58141936 | 58141951 | +/- | 16 | TTTTGGTTTTTTCCTT |
| chr3 | 58141937 | 58141952 | +/- | 16 | TTTGGTTTTTTCCTTT |
| chr3 | 58141938 | 58141953 | +/- | 16 | TTGGTTTTTTCCTTTT |
| chr3 | 58141939 | 58141954 | +/- | 16 | TGGTTTTTTCCTTTTT |
| chr3 | 58141940 | 58141955 | +/- | 16 | GGTTTTTTCCTTTTTG |
| chr3 | 58141941 | 58141956 | +/- | 16 | GTTTTTTCCTTTTTTGT |
| chr3 | 58141942 | 58141957 | +/- | 16 | TTTTTTCCTTTTTGTG |
| chr3 | 58141943 | 58141958 | +/- | 16 | TTTTTCCTTTTTGTGT |
| chr3 | 58141944 | 58141959 | +/- | 16 | TTTTCCTTTTTGTGTT |
| chr3 | 58141945 | 58141960 | +/- | 16 | TTTCCTTTTTGTGTTT |
| chr3 | 58141946 | 58141961 | +/- | 16 | TTCCTTTTTGTGTTTC |
| chr3 | 58141947 | 58141962 | +/- | 16 | TCCTTTTTGTGTTTCT |
| chr3 | 58141948 | 58141963 | +/- | 16 | CCTTTTTGTGTTTCTG |
| chr3 | 58141949 | 58141964 | +/- | 16 | CTTTTTGTGTTTCTGT |
| chr3 | 58141950 | 58141965 | +/- | 16 | TTTTTGTGTTTCTGTG |
| chr3 | 58141951 | 58141966 | +/- | 16 | TTTTGTGTTTCTGTGT |
| chr3 | 58141952 | 58141967 | +/- | 16 | TTTGTGTTTCTGTGTT |
| chr3 | 58141953 | 58141968 | +/- | 16 | TTGTGTTTCTGTGTTT |
| chr3 | 58141954 | 58141969 | +/- | 16 | TGTGTTTCTGTGTTTA |
| chr3 | 58141955 | 58141970 | +/- | 16 | GTGTTTCTGTGTTTAC |
| chr3 | 58141956 | 58141971 | +/- | 16 | TGTTTCTGTGTTTACT |
| chr3 | 58141957 | 58141972 | +/- | 16 | GTTTCTGTGTTTACTC |
| chr3 | 58141958 | 58141973 | +/- | 16 | TTTCTGTGTTTACTCA |
| chr3 | 58141959 | 58141974 | +/- | 16 | TTCTGTGTTTACTCAG |
| chr3 | 58141960 | 58141975 | +/- | 16 | TCTGTGTTTACTCAGC |
| chr3 | 58141961 | 58141976 | +/- | 16 | CTGTGTTTACTCAGCC |
| chr3 | 58141962 | 58141977 | +/- | 16 | TGTGTTTACTCAGCCT |
| chr3 | 58141963 | 58141978 | +/- | 16 | GTGTTTACTCAGCCTT |
| chr3 | 58141964 | 58141979 | +/- | 16 | TGTTTACTCAGCCTTC |
| chr3 | 58141965 | 58141980 | +/- | 16 | GTTTACTCAGCCTTCA |
| chr3 | 58141966 | 58141981 | +/- | 16 | TTTACTCAGCCTTCAT |
| chr3 | 58141967 | 58141982 | +/- | 16 | TTACTCAGCCTTCATT |
| chr3 | 58141968 | 58141983 | +/- | 16 | TACTCAGCCTTCATTT |
| chr3 | 58141969 | 58141984 | +/- | 16 | ACTCAGCCTTCATTTC |
| chr3 | 58141970 | 58141985 | +/- | 16 | CTCAGCCTTCATTTCA |
| chr3 | 58141971 | 58141986 | +/- | 16 | TCAGCCTTCATTTCAG |
| chr3 | 58141972 | 58141987 | +/- | 16 | CAGCCTTCATTTCAGA |
| chr3 | 58141973 | 58141988 | +/- | 16 | AGCCTTCATTTCAGAA |
| chr3 | 58141974 | 58141989 | +/- | 16 | GCCTTCATTTCAGAAA |
| chr3 | 58141975 | 58141990 | +/- | 16 | CCTTCATTTCAGAAAA |
| chr3 | 58141976 | 58141991 | +/- | 16 | CTTCATTTCAGAAAAT |
| chr3 | 58141977 | 58141992 | +/- | 16 | TTCATTTCAGAAAATC |
| chr3 | 58141978 | 58141993 | +/- | 16 | TCATTTCAGAAAATCT |
| chr3 | 58141979 | 58141994 | +/- | 16 | CATTTCAGAAAATCTG |
| chr3 | 58141980 | 58141995 | +/- | 16 | ATTTCAGAAAATCTGC |
| chr3 | 58141981 | 58141996 | +/- | 16 | TTTCAGAAAATCTGCC |
| chr3 | 58141982 | 58141997 | +/- | 16 | TTCAGAAAATCTGCCA |
| chr3 | 58141983 | 58141998 | +/- | 16 | TCAGAAAATCTGCCAT |
| chr3 | 58141984 | 58141999 | +/- | 16 | CAGAAAATCTGCCATC |
| chr3 | 58141985 | 58142000 | +/- | 16 | AGAAAATCTGCCATCT |
| chr3 | 58141986 | 58142001 | +/- | 16 | GAAAATCTGCCATCTG |
| chr3 | 58141987 | 58142002 | +/- | 16 | AAAATCTGCCATCTGC |
| chr3 | 58141988 | 58142003 | +/- | 16 | AAATCTGCCATCTGCT |
| chr3 | 58141989 | 58142004 | +/- | 16 | AATCTGCCATCTGCTT |
| chr3 | 58141990 | 58142005 | +/- | 16 | ATCTGCCATCTGCTTC |
| chr3 | 58141991 | 58142006 | +/- | 16 | TCTGCCATCTGCTTCT |
| chr3 | 58141992 | 58142007 | +/- | 16 | CTGCCATCTGCTTCTG |
| chr3 | 58141993 | 58142008 | +/- | 16 | TGCCATCTGCTTCTGG |
| chr3 | 58141994 | 58142009 | +/- | 16 | GCCATCTGCTTCTGGG |
| chr3 | 58141995 | 58142010 | +/- | 16 | CCATCTGCTTCTGGGA |
| chr3 | 58141996 | 58142011 | +/- | 16 | CATCTGCTTCTGGGAT |
| chr3 | 58141997 | 58142012 | +/- | 16 | ATCTGCTTCTGGGATT |
| chr3 | 58141998 | 58142013 | +/- | 16 | TCTGCTTCTGGGATTG |
| chr3 | 58141999 | 58142014 | +/- | 16 | CTGCTTCTGGGATTGC |
| chr3 | 58142000 | 58142015 | +/- | 16 | TGCTTCTGGGATTGCT |
| chr3 | 58142001 | 58142016 | +/- | 16 | GCTTCTGGGATTGCTT |
| chr3 | 58142002 | 58142017 | +/- | 16 | CTTCTGGGATTGCTTA |
| chr3 | 58142003 | 58142018 | +/- | 16 | TTCTGGGATTGCTTAA |
| chr3 | 58142004 | 58142019 | +/- | 16 | TCTGGGATTGCTTAAG |
| chr3 | 58142005 | 58142020 | +/- | 16 | CTGGGATTGCTTAAGC |
| chr3 | 58142006 | 58142021 | +/- | 16 | TGGGATTGCTTAAGCC |
| chr3 | 58142007 | 58142022 | +/- | 16 | GGGATTGCTTAAGCCC |
| chr3 | 58142008 | 58142023 | +/- | 16 | GGATTGCTTAAGCCCT |
| chr3 | 58142009 | 58142024 | +/- | 16 | GATTGCTTAAGCCCTG |
| chr3 | 58142010 | 58142025 | +/- | 16 | ATTGCTTAAGCCCTGT |
| chr3 | 58142011 | 58142026 | +/- | 16 | TTGCTTAAGCCCTGTG |
| chr3 | 58142012 | 58142027 | +/- | 16 | TGCTTAAGCCCTGTGG |
| chr3 | 58142013 | 58142028 | +/- | 16 | GCTTAAGCCCTGTGGG |
| chr3 | 58142014 | 58142029 | +/- | 16 | CTTAAGCCCTGTGGGT |
| chr3 | 58142015 | 58142030 | +/- | 16 | TTAAGCCCTGTGGGTG |
| chr3 | 58142016 | 58142031 | +/- | 16 | TAAGCCCTGTGGGTGT |
| chr3 | 58142017 | 58142032 | +/- | 16 | AAGCCCTGTGGGTGTC |
| chr3 | 58142018 | 58142033 | +/- | 16 | AGCCCTGTGGGTGTCC |
| chr3 | 58142019 | 58142034 | +/- | 16 | GCCCTGTGGGTGTCCT |
| chr3 | 58142020 | 58142035 | +/- | 16 | CCCTGTGGGTGTCCTG |
| chr3 | 58142021 | 58142036 | +/- | 16 | CCTGTGGGTGTCCTGG |
| chr3 | 58142022 | 58142037 | +/- | 16 | CTGTGGGTGTCCTGGT |
| chr3 | 58142023 | 58142038 | +/- | 16 | TGTGGGTGTCCTGGTC |
| chr3 | 58142024 | 58142039 | +/- | 16 | GTGGGTGTCCTGGTCA |
| chr3 | 58142025 | 58142040 | +/- | 16 | TGGGTGTCCTGGTCAT |
| chr3 | 58142026 | 58142041 | +/- | 16 | GGGTGTCCTGGTCATT |
| chr3 | 58142027 | 58142042 | +/- | 16 | GGTGTCCTGGTCATTG |
| chr3 | 58142028 | 58142043 | +/- | 16 | GTGTCCTGGTCATTGG |
| chr3 | 58142029 | 58142044 | +/- | 16 | TGTCCTGGTCATTGGT |

**Table 3: 17-mer target-specific ASOs**

| **CHR** | **START** | **END** | **STRAND** | **kmer** | **SEQUENCE** |
|---|---|---|---|---|---|
| chr3 | 58141828 | 58141844 | +/- | 17 | CCCAACTAATCTCCATT |
| chr3 | 58141829 | 58141845 | +/- | 17 | CCAACTAATCTCCATTT |
| chr3 | 58141830 | 58141846 | +/- | 17 | CAACTAATCTCCATTTG |
| chr3 | 58141831 | 58141847 | +/- | 17 | AACTAATCTCCATTTGC |
| chr3 | 58141832 | 58141848 | +/- | 17 | ACTAATCTCCATTTGCC |
| chr3 | 58141833 | 58141849 | +/- | 17 | CTAATCTCCATTTGCCA |
| chr3 | 58141834 | 58141850 | +/- | 17 | TAATCTCCATTTGCCAC |
| chr3 | 58141835 | 58141851 | +/- | 17 | AATCTCCATTTGCCACT |
| chr3 | 58141836 | 58141852 | +/- | 17 | ATCTCCATTTGCCACTG |
| chr3 | 58141837 | 58141853 | +/- | 17 | TCTCCATTTGCCACTGA |
| chr3 | 58141838 | 58141854 | +/- | 17 | CTCCATTTGCCACTGAC |
| chr3 | 58141839 | 58141855 | +/- | 17 | TCCATTTGCCACTGACC |
| chr3 | 58141840 | 58141856 | +/- | 17 | CCATTTGCCACTGACCA |
| chr3 | 58141841 | 58141857 | +/- | 17 | CATTTGCCACTGACCAG |
| chr3 | 58141842 | 58141858 | +/- | 17 | ATTTGCCACTGACCAGG |
| chr3 | 58141843 | 58141859 | +/- | 17 | TTTGCCACTGACCAGGC |
| chr3 | 58141844 | 58141860 | +/- | 17 | TTGCCACTGACCAGGCC |
| chr3 | 58141845 | 58141861 | +/- | 17 | TGCCACTGACCAGGCCA |
| chr3 | 58141846 | 58141862 | +/- | 17 | GCCACTGACCAGGCCAC |
| chr3 | 58141847 | 58141863 | +/- | 17 | CCACTGACCAGGCCACA |
| chr3 | 58141848 | 58141864 | +/- | 17 | CACTGACCAGGCCACAG |
| chr3 | 58141849 | 58141865 | +/- | 17 | ACTGACCAGGCCACAGA |
| chr3 | 58141850 | 58141866 | +/- | 17 | CTGACCAGGCCACAGAT |
| chr3 | 58141851 | 58141867 | +/- | 17 | TGACCAGGCCACAGATG |
| chr3 | 58141852 | 58141868 | +/- | 17 | GACCAGGCCACAGATGG |
| chr3 | 58141853 | 58141869 | +/- | 17 | ACCAGGCCACAGATGGG |
| chr3 | 58141854 | 58141870 | +/- | 17 | CCAGGCCACAGATGGGG |
| chr3 | 58141855 | 58141871 | +/- | 17 | CAGGCCACAGATGGGGA |
| chr3 | 58141856 | 58141872 | +/- | 17 | AGGCCACAGATGGGGAA |
| chr3 | 58141857 | 58141873 | +/- | 17 | GGCCACAGATGGGGAAG |
| chr3 | 58141858 | 58141874 | +/- | 17 | GCCACAGATGGGGAAGT |
| chr3 | 58141859 | 58141875 | +/- | 17 | CCACAGATGGGGAAGTC |
| chr3 | 58141860 | 58141876 | +/- | 17 | CACAGATGGGGAAGTCA |
| chr3 | 58141861 | 58141877 | +/- | 17 | ACAGATGGGGAAGTCAC |
| chr3 | 58141862 | 58141878 | +/- | 17 | CAGATGGGGAAGTCACA |
| chr3 | 58141863 | 58141879 | +/- | 17 | AGATGGGGAAGTCACAG |
| chr3 | 58141864 | 58141880 | +/- | 17 | GATGGGGAAGTCACAGC |
| chr3 | 58141865 | 58141881 | +/- | 17 | ATGGGGAAGTCACAGCC |
| chr3 | 58141866 | 58141882 | +/- | 17 | TGGGGAAGTCACAGCCG |
| chr3 | 58141867 | 58141883 | +/- | 17 | GGGGAAGTCACAGCCGT |
| chr3 | 58141868 | 58141884 | +/- | 17 | GGGAAGTCACAGCCGTG |
| chr3 | 58141869 | 58141885 | +/- | 17 | GGAAGTCACAGCCGTGG |
| chr3 | 58141870 | 58141886 | +/- | 17 | GAAGTCACAGCCGTGGA |
| chr3 | 58141871 | 58141887 | +/- | 17 | AAGTCACAGCCGTGGAG |
| chr3 | 58141872 | 58141888 | +/- | 17 | AGTCACAGCCGTGGAGG |
| chr3 | 58141873 | 58141889 | +/- | 17 | GTCACAGCCGTGGAGGA |
| chr3 | 58141874 | 58141890 | +/- | 17 | TCACAGCCGTGGAGGAG |
| chr3 | 58141875 | 58141891 | +/- | 17 | CACAGCCGTGGAGGAGG |
| chr3 | 58141876 | 58141892 | +/- | 17 | ACAGCCGTGGAGGAGGC |
| chr3 | 58141877 | 58141893 | +/- | 17 | CAGCCGTGGAGGAGGCA |
| chr3 | 58141878 | 58141894 | +/- | 17 | AGCCGTGGAGGAGGCAC |
| chr3 | 58141879 | 58141895 | +/- | 17 | GCCGTGGAGGAGGCACC |
| chr3 | 58141880 | 58141896 | +/- | 17 | CCGTGGAGGAGGCACCG |
| chr3 | 58141881 | 58141897 | +/- | 17 | CGTGGAGGAGGCACCGG |
| chr3 | 58141882 | 58141898 | +/- | 17 | GTGGAGGAGGCACCGGT |
| chr3 | 58141883 | 58141899 | +/- | 17 | TGGAGGAGGCACCGGTA |
| chr3 | 58141884 | 58141900 | +/- | 17 | GGAGGAGGCACCGGTAA |
| chr3 | 58141885 | 58141901 | +/- | 17 | GAGGAGGCACCGGTAAA |
| chr3 | 58141886 | 58141902 | +/- | 17 | AGGAGGCACCGGTAAAT |
| chr3 | 58141887 | 58141903 | +/- | 17 | GGAGGCACCGGTAAATG |
| chr3 | 58141888 | 58141904 | +/- | 17 | GAGGCACCGGTAAATGC |
| chr3 | 58141889 | 58141905 | +/- | 17 | AGGCACCGGTAAATGCA |
| chr3 | 58141890 | 58141906 | +/- | 17 | GGCACCGGTAAATGCAT |
| chr3 | 58141891 | 58141907 | +/- | 17 | GCACCGGTAAATGCATG |
| chr3 | 58141892 | 58141908 | +/- | 17 | CACCGGTAAATGCATGT |
| chr3 | 58141893 | 58141909 | +/- | 17 | ACCGGTAAATGCATGTC |
| chr3 | 58141894 | 58141910 | +/- | 17 | CCGGTAAATGCATGTCC |
| chr3 | 58141895 | 58141911 | +/- | 17 | CGGTAAATGCATGTCCC |
| chr3 | 58141896 | 58141912 | +/- | 17 | GGTAAATGCATGTCCCC |
| chr3 | 58141897 | 58141913 | +/- | 17 | GTAAATGCATGTCCCCC |
| chr3 | 58141898 | 58141914 | +/- | 17 | TAAATGCATGTCCCCCT |
| chr3 | 58141899 | 58141915 | +/- | 17 | AAATGCATGTCCCCCTG |
| chr3 | 58141900 | 58141916 | +/- | 17 | AATGCATGTCCCCCTGG |
| chr3 | 58141901 | 58141917 | +/- | 17 | ATGCATGTCCCCCTGGA |
| chr3 | 58141902 | 58141918 | +/- | 17 | TGCATGTCCCCCTGGAT |
| chr3 | 58141903 | 58141919 | +/- | 17 | GCATGTCCCCCTGGATT |
| chr3 | 58141904 | 58141920 | +/- | 17 | CATGTCCCCCTGGATTC |
| chr3 | 58141905 | 58141921 | +/- | 17 | ATGTCCCCCTGGATTCA |
| chr3 | 58141906 | 58141922 | +/- | 17 | TGTCCCCCTGGATTCAG |
| chr3 | 58141907 | 58141923 | +/- | 17 | GTCCCCCTGGATTCAGG |
| chr3 | 58141908 | 58141924 | +/- | 17 | TCCCCCTGGATTCAGGC |
| chr3 | 58141909 | 58141925 | +/- | 17 | CCCCCTGGATTCAGGCC |
| chr3 | 58141910 | 58141926 | +/- | 17 | CCCCTGGATTCAGGCCC |
| chr3 | 58141911 | 58141927 | +/- | 17 | CCCTGGATTCAGGCCCT |
| chr3 | 58141912 | 58141928 | +/- | 17 | CCTGGATTCAGGCCCTG |
| chr3 | 58141913 | 58141929 | +/- | 17 | CTGGATTCAGGCCCTGG |
| chr3 | 58141914 | 58141930 | +/- | 17 | TGGATTCAGGCCCTGGG |
| chr3 | 58141915 | 58141931 | +/- | 17 | GGATTCAGGCCCTGGGT |
| chr3 | 58141916 | 58141932 | +/- | 17 | GATTCAGGCCCTGGGTA |
| chr3 | 58141917 | 58141933 | +/- | 17 | ATTCAGGCCCTGGGTAC |
| chr3 | 58141918 | 58141934 | +/- | 17 | TTCAGGCCCTGGGTACA |
| chr3 | 58141919 | 58141935 | +/- | 17 | TCAGGCCCTGGGTACAA |
| chr3 | 58141920 | 58141936 | +/- | 17 | CAGGCCCTGGGTACAAT |
| chr3 | 58141921 | 58141937 | +/- | 17 | AGGCCCTGGGTACAATT |
| chr3 | 58141922 | 58141938 | +/- | 17 | GGCCCTGGGTACAATTT |
| chr3 | 58141923 | 58141939 | +/- | 17 | GCCCTGGGTACAATTTT |
| chr3 | 58141924 | 58141940 | +/- | 17 | CCCTGGGTACAATTTTG |
| chr3 | 58141925 | 58141941 | +/- | 17 | CCTGGGTACAATTTTGG |
| chr3 | 58141926 | 58141942 | +/- | 17 | CTGGGTACAATTTTGGT |
| chr3 | 58141927 | 58141943 | +/- | 17 | TGGGTACAATTTTGGTT |
| chr3 | 58141928 | 58141944 | +/- | 17 | GGGTACAATTTTGGTTT |
| chr3 | 58141929 | 58141945 | +/- | 17 | GGTACAATTTTGGTTTT |
| chr3 | 58141930 | 58141946 | +/- | 17 | GTACAATTTTGVTTTTT |
| chr3 | 58141931 | 58141947 | +/- | 17 | TACAATTTTGGTTTTTT |
| chr3 | 58141932 | 58141948 | +/- | 17 | ACAATTTTGGTTTTTTC |
| chr3 | 58141933 | 58141949 | +/- | 17 | CAATTTTGGTTTTTTCC |
| chr3 | 58141934 | 58141950 | +/- | 17 | AATTTTGGTTTTTTCCT |
| chr3 | 58141935 | 58141951 | +/- | 17 | ATTTTGGTTTTTTCCTT |
| chr3 | 58141936 | 58141952 | +/- | 17 | TTTTGGTTTTTTCCTTT |
| chr3 | 58141937 | 58141953 | +/- | 17 | TTTGGTTTTTTCCTTTT |
| chr3 | 58141938 | 58141954 | +/- | 17 | TTGGTTTTTTCCTTTTT |
| chr3 | 58141939 | 58141955 | +/- | 17 | TGGTTTTTTCCTTTTTG |
| chr3 | 58141940 | 58141956 | +/- | 17 | GGTTTTTTCCTTTTTGT |
| chr3 | 58141941 | 58141957 | +/- | 17 | GTTTTTTCCTTTTTGTG |
| chr3 | 58141942 | 58141958 | +/- | 17 | TTTTTTCCTTTTTGTGT |
| chr3 | 58141943 | 58141959 | +/- | 17 | TTTTTCCTTTTTGTGTT |
| chr3 | 58141944 | 58141960 | +/- | 17 | TTTTCCTTTTTGTGTTT |
| chr3 | 58141945 | 58141961 | +/- | 17 | TTTCCTTTTTGTGTTTC |
| chr3 | 58141946 | 58141962 | +/- | 17 | TTCCTTTTTGTGTTTCT |
| chr3 | 58141947 | 58141963 | +/- | 17 | TCCTTTTTGTGTTTCTG |
| chr3 | 58141948 | 58141964 | +/- | 17 | CCTTTTTGTGTTTCTGT |
| chr3 | 58141949 | 58141965 | +/- | 17 | CTTTTTGTGTTTCTGTG |
| chr3 | 58141950 | 58141966 | +/- | 17 | TTTTTGTGTTTCTGTGT |
| chr3 | 58141951 | 58141967 | +/- | 17 | TTTTGTGTTTCTGTGTT |
| chr3 | 58141952 | 58141968 | +/- | 17 | TTTGTGTTTCTGTGTTT |
| chr3 | 58141953 | 58141969 | +/- | 17 | TTGTGTTTCTGTGTTTA |
| chr3 | 58141954 | 58141970 | +/- | 17 | TGTGTTTCTGTGTTTAC |
| chr3 | 58141955 | 58141971 | +/- | 17 | GTGTTTCTGTGTTTACT |
| chr3 | 58141956 | 58141972 | +/- | 17 | TGTTTCTGTGTTTACTC |
| chr3 | 58141957 | 58141973 | +/- | 17 | GTTTCTGTGTTTACTCA |
| chr3 | 58141958 | 58141974 | +/- | 17 | TTTCTGTGTTTACTCAG |
| chr3 | 58141959 | 58141975 | +/- | 17 | TTCTGTGTTTACTCAGC |
| chr3 | 58141960 | 58141976 | +/- | 17 | TCTGTGTTTACTCAGCC |
| chr3 | 58141961 | 58141977 | +/- | 17 | CTGTGTTTACTCAGCCT |
| chr3 | 58141962 | 58141978 | +/- | 17 | TGTGTTTACTCAGCCTT |
| chr3 | 58141963 | 58141979 | +/- | 17 | GTGTTTACTCAGCCTTC |
| chr3 | 58141964 | 58141980 | +/- | 17 | TGTTTACTCAGCCTTCA |
| chr3 | 58141965 | 58141981 | +/- | 17 | GTTTACTCAGCCTTCAT |
| chr3 | 58141966 | 58141982 | +/- | 17 | TTTACTCAGCCTTCATT |
| chr3 | 58141967 | 58141983 | +/- | 17 | TTACTCAGCCTTCATTT |
| chr3 | 58141968 | 58141984 | +/- | 17 | TACTCAGCCTTCATTTC |
| chr3 | 58141969 | 58141985 | +/- | 17 | ACTCAGCCTTCATTTCA |
| chr3 | 58141970 | 58141986 | +/- | 17 | CTCAGCCTTCATTTCAG |
| chr3 | 58141971 | 58141987 | +/- | 17 | TCAGCCTTCATTTCAGA |
| chr3 | 58141972 | 58141988 | +/- | 17 | CAGCCTTCATTTCAGAA |
| chr3 | 58141973 | 58141989 | +/- | 17 | AGCCTTCATTTCAGAAA |
| chr3 | 58141974 | 58141990 | +/- | 17 | GCCTTCATTTCAGAAAA |
| chr3 | 58141975 | 58141991 | +/- | 17 | CCTTCATTTCAGAAAAT |
| chr3 | 58141976 | 58141992 | +/- | 17 | CTTCATTTCAGAAAATC |
| chr3 | 58141977 | 58141993 | +/- | 17 | TTCATTTCAGAAAATCT |
| chr3 | 58141978 | 58141994 | +/- | 17 | TCATTTCAGAAAATCTG |
| chr3 | 58141979 | 58141995 | +/- | 17 | CATTTCAGAAAATCTGC |
| chr3 | 58141980 | 58141996 | +/- | 17 | ATTTCAGAAAATCTGCC |
| chr3 | 58141981 | 58141997 | +/- | 17 | TTTCAGAAAATCTGCCA |
| chr3 | 58141982 | 58141998 | +/- | 17 | TTCAGAAAATCTGCCAT |
| chr3 | 58141983 | 58141999 | +/- | 17 | TCAGAAAATCTGCCATC |
| chr3 | 58141984 | 58142000 | +/- | 17 | CAGAAAATCTGCCATCT |
| chr3 | 58141985 | 58142001 | +/- | 17 | AGAAAATCTGCCATCTG |
| chr3 | 58141986 | 58142002 | +/- | 17 | GAAAATCTGCCATCTGC |
| chr3 | 58141987 | 58142003 | +/- | 17 | AAAATCTGCCATCTGCT |
| chr3 | 58141988 | 58142004 | +/- | 17 | AAATCTGCCATCTGCTT |
| chr3 | 58141989 | 58142005 | +/- | 17 | AATCTGCCATCTGCTTC |
| chr3 | 58141990 | 58142006 | +/- | 17 | ATCTGCCATCTGCTTCT |
| chr3 | 58141991 | 58142007 | +/- | 17 | TCTGCCATCTGCTTCTG |
| chr3 | 58141992 | 58142008 | +/- | 17 | CTGCCATCTGCTTCTGG |
| chr3 | 58141993 | 58142009 | +/- | 17 | TGCCATCTGCTTCTGGG |
| chr3 | 58141994 | 58142010 | +/- | 17 | GCCATCTGCTTCTGGGA |
| chr3 | 58141995 | 58142011 | +/- | 17 | CCATCTGCTTCTGGGAT |
| chr3 | 58141996 | 58142012 | +/- | 17 | CATCTGCTTCTGGGATT |
| chr3 | 58141997 | 58142013 | +/- | 17 | ATCTGCTTCTGGGATTG |
| chr3 | 58141998 | 58142014 | +/- | 17 | TCTGCTTCTGGGATTGC |
| chr3 | 58141999 | 58142015 | +/- | 17 | CTGCTTCTGGGATTGCT |
| chr3 | 58142000 | 58142016 | +/- | 17 | TGCTTCTGGGATTGCTT |
| chr3 | 58142001 | 58142017 | +/- | 17 | GCTTCTGGGATTGCTTA |
| chr3 | 58142002 | 58142018 | +/- | 17 | CTTCTGGGATTGCTTAA |
| chr3 | 58142003 | 58142019 | +/- | 17 | TTCTGGGATTGCTTAAG |
| chr3 | 58142004 | 58142020 | +/- | 17 | TCTGGGATTGCTTAAGC |
| chr3 | 58142005 | 58142021 | +/- | 17 | CTGGGATTGCTTAAGCC |
| chr3 | 58142006 | 58142022 | +/- | 17 | TGGGATTGCTTAAGCCC |
| chr3 | 58142007 | 58142023 | +/- | 17 | GGGATTGCTTAAGCCCT |
| chr3 | 58142008 | 58142024 | +/- | 17 | GGATTGCTTAAGCCCTG |
| chr3 | 58142009 | 58142025 | +/- | 17 | GATTGCTTAAGCCCTGT |
| chr3 | 58142010 | 58142026 | +/- | 17 | ATTGCTTAAGCCCTGTG |
| chr3 | 58142011 | 58142027 | +/- | 17 | TTGCTTAAGCCCTGTGG |
| chr3 | 58142012 | 58142028 | +/- | 17 | TGCTTAAGCCCTGTGGG |
| chr3 | 58142013 | 58142029 | +/- | 17 | GCTTAAGCCCTGTGGGT |
| chr3 | 58142014 | 58142030 | +/- | 17 | CTTAAGCCCTGTGGGTG |
| chr3 | 58142015 | 58142031 | +/- | 17 | TTAAGCCCTGTGGGTGT |
| chr3 | 58142016 | 58142032 | +/- | 17 | TAAGCCCTGTGGGTGTC |
| chr3 | 58142017 | 58142033 | +/- | 17 | AAGCCCTGTGGGTGTCC |
| chr3 | 58142018 | 58142034 | +/- | 17 | AGCCCTGTGGGTGTCCT |
| chr3 | 58142019 | 58142035 | +/- | 17 | GCCCTGTGGGTGTCCTG |
| chr3 | 58142020 | 58142036 | +/- | 17 | CCCTGTGGGTGTCCTGG |
| chr3 | 58142021 | 58142037 | +/- | 17 | CCTGTGGGTGTCCTGGT |
| chr3 | 58142022 | 58142038 | +/- | 17 | CTGTGGGTGTCCTGGTC |
| chr3 | 58142023 | 58142039 | +/- | 17 | TGTGGGTGTCCTGGTCA |
| chr3 | 58142024 | 58142040 | +/- | 17 | GTGGGTGTCCTGGTCAT |
| chr3 | 58142025 | 58142041 | +/- | 17 | TGGGTGTCCTGGTCATT |
| chr3 | 58142026 | 58142042 | +/- | 17 | GGGTGTCCTGGTCATTG |
| chr3 | 58142027 | 58142043 | +/- | 17 | GGTGTCCTGGTCATTGG |
| chr3 | 58142028 | 58142044 | +/- | 17 | GTGTCCTGGTCATTGGT |

**Table 4: 18-mer target-specific ASOs**

| **CHR** | **START** | **END** | **STRAND** | **kmer** | **SEQUENCE** |
|---|---|---|---|---|---|
| chr3 | 58141828 | 58141845 | +/- | 18 | CCCAACTAATCTCCATTT |
| chr3 | 58141829 | 58141846 | +/- | 18 | CCAACTAATCTCCATTTG |
| chr3 | 58141830 | 58141847 | +/- | 18 | CAACTAATCTCCATTTGC |
| chr3 | 58141831 | 58141848 | +/- | 18 | AACTAATCTCCATTTGCC |
| chr3 | 58141832 | 58141849 | +/- | 18 | ACTAATCTCCATTTGCCA |
| chr3 | 58141833 | 58141850 | +/- | 18 | CTAATCTCCATTTGCCAC |
| chr3 | 58141834 | 58141851 | +/- | 18 | TAATCTCCATTTGCCACT |
| chr3 | 58141835 | 58141852 | +/- | 18 | AATCTCCATTTGCCACTG |
| chr3 | 58141836 | 58141853 | +/- | 18 | ATCTCCATTTGCCACTGA |
| chr3 | 58141837 | 58141854 | +/- | 18 | TCTCCATTTGCCACTGAC |
| chr3 | 58141838 | 58141855 | +/- | 18 | CTCCATTTGCCACTGACC |
| chr3 | 58141839 | 58141856 | +/- | 18 | TCCATTTGCCACTGACCA |
| chr3 | 58141840 | 58141857 | +/- | 18 | CCATTTGCCACTGACCAG |
| chr3 | 58141841 | 58141858 | +/- | 18 | CATTTGCCACTGACCAGG |
| chr3 | 58141842 | 58141859 | +/- | 18 | ATTTGCCACTGACCAGGC |
| chr3 | 58141843 | 58141860 | +/- | 18 | TTTGCCACTGACCAGGCC |
| chr3 | 58141844 | 58141861 | +/- | 18 | TTGCCACTGACCAGGCCA |
| chr3 | 58141845 | 58141862 | +/- | 18 | TGCCACTGACCAGGCCAC |
| chr3 | 58141846 | 58141863 | +/- | 18 | GCCACTGACCAGGCCACA |
| chr3 | 58141847 | 58141864 | +/- | 18 | CCACTGACCAGGCCACAG |
| chr3 | 58141848 | 58141865 | +/- | 18 | CACTGACCAGGCCACAGA |
| chr3 | 58141849 | 58141866 | +/- | 18 | ACTGACCAGGCCACAGAT |
| chr3 | 58141850 | 58141867 | +/- | 18 | CTGACCAGGCCACAGATG |
| chr3 | 58141851 | 58141868 | +/- | 18 | TGACCAGGCCACAGATGG |
| chr3 | 58141852 | 58141869 | +/- | 18 | GACCAGGCCACAGATGGG |
| chr3 | 58141853 | 58141870 | +/- | 18 | ACCAGGCCACAGATGGGG |
| chr3 | 58141854 | 58141871 | +/- | 18 | CCAGGCCACAGATGGGGA |
| chr3 | 58141855 | 58141872 | +/- | 18 | CAGGCCACAGATGGGGAA |
| chr3 | 58141856 | 58141873 | +/- | 18 | AGGCCACAGATGGGGAAG |
| chr3 | 58141857 | 58141874 | +/- | 18 | GGCCACAGATGGGGAAGT |
| chr3 | 58141858 | 58141875 | +/- | 18 | GCCACAGATGGGGAAGTC |
| chr3 | 58141859 | 58141876 | +/- | 18 | CCACAGATGGGGAAGTCA |
| chr3 | 58141860 | 58141877 | +/- | 18 | CACAGATGGGGAAGTCAC |
| chr3 | 58141861 | 58141878 | +/- | 18 | ACAGATGGGGAAGTCACA |
| chr3 | 58141862 | 58141879 | +/- | 18 | CAGATGGGGAAGTCACAG |
| chr3 | 58141863 | 58141880 | +/- | 18 | AGATGGGGAAGTCACAGC |
| chr3 | 58141864 | 58141881 | +/- | 18 | GATGGGGAAGTCACAGCC |
| chr3 | 58141865 | 58141882 | +/- | 18 | ATGGGGAAGTCACAGCCG |
| chr3 | 58141866 | 58141883 | +/- | 18 | TGGGGAAGTCACAGCCGT |
| chr3 | 58141867 | 58141884 | +/- | 18 | GGGGAAGTCACAGCCGTG |
| chr3 | 58141868 | 58141885 | +/- | 18 | GGGAAGTCACAGCCGTGG |
| chr3 | 58141869 | 58141886 | +/- | 18 | GGAAGTCACAGCCGTGGA |
| chr3 | 58141870 | 58141887 | +/- | 18 | GAAGTCACAGCCGTGGAG |
| chr3 | 58141871 | 58141888 | +/- | 18 | AAGTCACAGCCGTGGAGG |
| chr3 | 58141872 | 58141889 | +/- | 18 | AGTCACAGCCGTGGAGGA |
| chr3 | 58141873 | 58141890 | +/- | 18 | GTCACAGCCGTGGAGGAG |
| chr3 | 58141874 | 58141891 | +/- | 18 | TCACAGCCGTGGAGGAGG |
| chr3 | 58141875 | 58141892 | +/- | 18 | CACAGCCGTGGAGGAGGC |
| chr3 | 58141876 | 58141893 | +/- | 18 | ACAGCCGTGGAGGAGGCA |
| chr3 | 58141877 | 58141894 | +/- | 18 | CAGCCGTGGAGGAGGCAC |
| chr3 | 58141878 | 58141895 | +/- | 18 | AGCCGTGGAGGAGGCACC |
| chr3 | 58141879 | 58141896 | +/- | 18 | GCCGTGGAGGAGGCACCG |
| chr3 | 58141880 | 58141897 | +/- | 18 | CCGTGGAGGAGGCACCGG |
| chr3 | 58141881 | 58141898 | +/- | 18 | CGTGGAGGAGGCACCGGT |
| chr3 | 58141882 | 58141899 | +/- | 18 | GTGGAGGAGGCACCGGTA |
| chr3 | 58141883 | 58141900 | +/- | 18 | TGGAGGAGGCACCGGTAA |
| chr3 | 58141884 | 58141901 | +/- | 18 | GGAGGAGGCACCGGTAAA |
| chr3 | 58141885 | 58141902 | +/- | 18 | GAGGAGGCACCGGTAAAT |
| chr3 | 58141886 | 58141903 | +/- | 18 | AGGAGGCACCGGTAAATG |
| chr3 | 58141887 | 58141904 | +/- | 18 | GGAGGCACCGGTAAATGC |
| chr3 | 58141888 | 58141905 | +/- | 18 | GAGGCACCGGTAAATGCA |
| chr3 | 58141889 | 58141906 | +/- | 18 | AGGCACCGGTAAATGCAT |
| chr3 | 58141890 | 58141907 | +/- | 18 | GGCACCGGTAAATGCATG |
| chr3 | 58141891 | 58141908 | +/- | 18 | GCACCGGTAAATGCATGT |
| chr3 | 58141892 | 58141909 | +/- | 18 | CACCGGTAAATGCATGTC |
| chr3 | 58141893 | 58141910 | +/- | 18 | ACCGGTAAATGCATGTCC |
| chr3 | 58141894 | 58141911 | +/- | 18 | CCGGTAAATGCATGTCCC |
| chr3 | 58141895 | 58141912 | +/- | 18 | CGGTAAATGCATGTCCCC |
| chr3 | 58141896 | 58141913 | +/- | 18 | GGTAAATGCATGTCCCCC |
| chr3 | 58141897 | 58141914 | +/- | 18 | GTAAATGCATGTCCCCCT |
| chr3 | 58141898 | 58141915 | +/- | 18 | TAAATGCATGTCCCCCTG |
| chr3 | 58141899 | 58141916 | +/- | 18 | AAATGCATGTCCCCCTGG |
| chr3 | 58141900 | 58141917 | +/- | 18 | AATGCATGTCCCCCTGGA |
| chr3 | 58141901 | 58141918 | +/- | 18 | ATGCATGTCCCCCTGGAT |
| chr3 | 58141902 | 58141919 | +/- | 18 | TGCATGTCCCCCTGGATT |
| chr3 | 58141903 | 58141920 | +/- | 18 | GCATGTCCCCCTGGATTC |
| chr3 | 58141904 | 58141921 | +/- | 18 | CATGTCCCCCTGGATTCA |
| chr3 | 58141905 | 58141922 | +/- | 18 | ATGTCCCCCTGGATTCAG |
| chr3 | 58141906 | 58141923 | +/- | 18 | TGTCCCCCTGGATTCAGG |
| chr3 | 58141907 | 58141924 | +/- | 18 | GTCCCCCTGGATTCAGGC |
| chr3 | 58141908 | 58141925 | +/- | 18 | TCCCCCTGGATTCAGGCC |
| chr3 | 58141909 | 58141926 | +/- | 18 | CCCCCTGGATTCAGGCCC |
| chr3 | 58141910 | 58141927 | +/- | 18 | CCCCTGGATTCAGGCCCT |
| chr3 | 58141911 | 58141928 | +/- | 18 | CCCTGGATTCAGGCCCTG |
| chr3 | 58141912 | 58141929 | +/- | 18 | CCTGGATTCAGGCCCTGG |
| chr3 | 58141913 | 58141930 | +/- | 18 | CTGGATTCAGGCCCTGGG |
| chr3 | 58141914 | 58141931 | +/- | 18 | TGGATTCAGGCCCTGGGT |
| chr3 | 58141915 | 58141932 | +/- | 18 | GGATTCAGGCCCTGGGTA |
| chr3 | 58141916 | 58141933 | +/- | 18 | GATTCAGGCCCTGGGTAC |
| chr3 | 58141917 | 58141934 | +/- | 18 | ATTCAGGCCCTGGGTACA |
| chr3 | 58141918 | 58141935 | +/- | 18 | TTCAGGCCCTGGGTACAA |
| chr3 | 58141919 | 58141936 | +/- | 18 | TCAGGCCCTGGGTACAAT |
| chr3 | 58141920 | 58141937 | +/- | 18 | CAGGCCCTGGGTACAATT |
| chr3 | 58141921 | 58141938 | +/- | 18 | AGGCCCTGGGTACAATTT |
| chr3 | 58141922 | 58141939 | +/- | 18 | GGCCCTGGGTACAATTTT |
| chr3 | 58141923 | 58141940 | +/- | 18 | GCCCTGGGTACAATTTTG |
| chr3 | 58141924 | 58141941 | +/- | 18 | CCCTGGGTACAATTTTGG |
| chr3 | 58141925 | 58141942 | +/- | 18 | CCTGGGTACAATTTTGGT |
| chr3 | 58141926 | 58141943 | +/- | 18 | CTGGGTACAATTTTGGTT |
| chr3 | 58141927 | 58141944 | +/- | 18 | TGGGTACAATTTTGGTTT |
| chr3 | 58141928 | 58141945 | +/- | 18 | GGGTACAATTTTGGTTTT |
| chr3 | 58141929 | 58141946 | +/- | 18 | GGTACAATTTTGGTTTTT |
| chr3 | 58141930 | 58141947 | +/- | 18 | GTACAATTTTGGTTTTTT |
| chr3 | 58141931 | 58141948 | +/- | 18 | TACAATTTTGGTTTTTTC |
| chr3 | 58141932 | 58141949 | +/- | 18 | ACAATTTTGGTTTTTTCC |
| chr3 | 58141933 | 58141950 | +/- | 18 | CAATTTTGGTTTTTTCCTT |
| chr3 | 58141934 | 58141951 | +/- | 18 | AATTTTGGTTTTTTCCTT |
| chr3 | 58141935 | 58141952 | +/- | 18 | ATTTTGGTTTTTTCCTTT |
| chr3 | 58141936 | 58141953 | +/- | 18 | TTTTGGTTTTTTCCTTTT |
| chr3 | 58141937 | 58141954 | +/- | 18 | TTTGGTTTTTTCCTTTTT |
| chr3 | 58141938 | 58141955 | +/- | 18 | TTGGTTTTTTCCTTTTTG |
| chr3 | 58141939 | 58141956 | +/- | 18 | TGGTTTTTTCCTTTTTGT |
| chr3 | 58141940 | 58141957 | +/- | 18 | GGTTTTTTCCTTTTTGTG |
| chr3 | 58141941 | 58141958 | +/- | 18 | GTTTTTTCCTTTTTGTGT |
| chr3 | 58141942 | 58141959 | +/- | 18 | TTTTTTCCTTTTTGTGTT |
| chr3 | 58141943 | 58141960 | +/- | 18 | TTTTTCCTTTTTGTGTTT |
| chr3 | 58141944 | 58141961 | +/- | 18 | TTTTCLI I I I IGTGTTTC |
| chr3 | 58141945 | 58141962 | +/- | 18 | TTTCCTTTTTGTGTTTCT |
| chr3 | 58141946 | 58141963 | +/- | 18 | TTCCTTTTTGTGTTTCTG |
| chr3 | 58141947 | 58141964 | +/- | 18 | TCCTTTTTGTGTTTCTGT |
| chr3 | 58141948 | 58141965 | +/- | 18 | CCTTTTTGTGTTTCTGTG |
| chr3 | 58141949 | 58141966 | +/- | 18 | CTTTTTGTGTTTCTGTGT |
| chr3 | 58141950 | 58141967 | +/- | 18 | TTTTTGTGTTTCTGTGTT |
| chr3 | 58141951 | 58141968 | +/- | 18 | TTTTGTGTTTCTGTGTTT |
| chr3 | 58141952 | 58141969 | +/- | 18 | TTTGTGTTTCTGTGTTTA |
| chr3 | 58141953 | 58141970 | +/- | 18 | TTGTGTTTCTGTGTTTAC |
| chr3 | 58141954 | 58141971 | +/- | 18 | TGTGTTTCTGTGTTTACT |
| chr3 | 58141955 | 58141972 | +/- | 18 | GTGTTTCTGTGTTTACTC |
| chr3 | 58141956 | 58141973 | +/- | 18 | TGTTTCTGTGTTTACTCA |
| chr3 | 58141957 | 58141974 | +/- | 18 | GTTTCTGTGTTTACTCAG |
| chr3 | 58141958 | 58141975 | +/- | 18 | TTTCTGTGTTTACTCAGC |
| chr3 | 58141959 | 58141976 | +/- | 18 | TTCTGTGTTTACTCAGCC |
| chr3 | 58141960 | 58141977 | +/- | 18 | TCTGTGTTTACTCAGCCT |
| chr3 | 58141961 | 58141978 | +/- | 18 | CTGTGTTTACTCAGCCTT |
| chr3 | 58141962 | 58141979 | +/- | 18 | TGTGTTTACTCAGCCTTC |
| chr3 | 58141963 | 58141980 | +/- | 18 | GTGTTTACTCAGCCTTCA |
| chr3 | 58141964 | 58141981 | +/- | 18 | TGTTTACTCAGCCTTCAT |
| chr3 | 58141965 | 58141982 | +/- | 18 | GTTTACTCAGCCTTCATT |
| chr3 | 58141966 | 58141983 | +/- | 18 | TTTACTCAGCCTTCATTT |
| chr3 | 58141967 | 58141984 | +/- | 18 | TTACTCAGCCTTCATTTC |
| chr3 | 58141968 | 58141985 | +/- | 18 | TACTCAGCCTTCATTTCA |
| chr3 | 58141969 | 58141986 | +/- | 18 | ACTCAGCCTTCATTTCAG |
| chr3 | 58141970 | 58141987 | +/- | 18 | CTCAGCCTTCATTTCAGA |
| chr3 | 58141971 | 58141988 | +/- | 18 | TCAGCCTTCATTTCAGAA |
| chr3 | 58141972 | 58141989 | +/- | 18 | CAGCCTTCATTTCAGAAA |
| chr3 | 58141973 | 58141990 | +/- | 18 | AGCCTTCATTTCAGAAAA |
| chr3 | 58141974 | 58141991 | +/- | 18 | GCCTTCATTTCAGAAAAT |
| chr3 | 58141975 | 58141992 | +/- | 18 | CCTTCATTTCAGAAAATC |
| chr3 | 58141976 | 58141993 | +/- | 18 | CTTCATTTCAGAAAATCT |
| chr3 | 58141977 | 58141994 | +/- | 18 | TTCATTTCAGAAAATCTG |
| chr3 | 58141978 | 58141995 | +/- | 18 | TCATTTCAGAAAATCTGC |
| chr3 | 58141979 | 58141996 | +/- | 18 | CATTTCAGAAAATCTGCC |
| chr3 | 58141980 | 58141997 | +/- | 18 | ATTTCAGAAAATCTGCCA |
| chr3 | 58141981 | 58141998 | +/- | 18 | TTTCAGAAAATCTGCCAT |
| chr3 | 58141982 | 58141999 | +/- | 18 | TTCAGAAAATCTGCCATC |
| chr3 | 58141983 | 58142000 | +/- | 18 | TCAGAAAATCTGCCATCT |
| chr3 | 58141984 | 58142001 | +/- | 18 | CAGAAAATCTGCCATCTG |
| chr3 | 58141985 | 58142002 | +/- | 18 | AGAAAATCTGCCATCTGC |
| chr3 | 58141986 | 58142003 | +/- | 18 | GAAAATCTGCCATCTGCT |
| chr3 | 58141987 | 58142004 | +/- | 18 | AAAATCTGCCATCTGCTT |
| chr3 | 58141988 | 58142005 | +/- | 18 | AAATCTGCCATCTGCTTC |
| chr3 | 58141989 | 58142006 | +/- | 18 | AATCTGCCATCTGCTTCT |
| chr3 | 58141990 | 58142007 | +/- | 18 | ATCTGCCATCTGCTTCTG |
| chr3 | 58141991 | 58142008 | +/- | 18 | TCTGCCATCTGCTTCTGG |
| chr3 | 58141992 | 58142009 | +/- | 18 | CTGCCATCTGCTTCTGGG |
| chr3 | 58141993 | 58142010 | +/- | 18 | TGCCATCTGCTTCTGGGA |
| chr3 | 58141994 | 58142011 | +/- | 18 | GCCATCTGCTTCTGGGAT |
| chr3 | 58141995 | 58142012 | +/- | 18 | CCATCTGCTTCTGGGATT |
| chr3 | 58141996 | 58142013 | +/- | 18 | CATCTGCTTCTGGGATTG |
| chr3 | 58141997 | 58142014 | +/- | 18 | ATCTGCTTCTGGGATTGC |
| chr3 | 58141998 | 58142015 | +/- | 18 | TCTGCTTCTGGGATTGCT |
| chr3 | 58141999 | 58142016 | +/- | 18 | CTGCTTCTGGGATTGCTT |
| chr3 | 58142000 | 58142017 | +/- | 18 | TGCTTCTGGGATTGCTTA |
| chr3 | 58142001 | 58142018 | +/- | 18 | GCTTCTGGGATTGCTTAA |
| chr3 | 58142002 | 58142019 | +/- | 18 | CTTCTGGGATTGCTTAAG |
| chr3 | 58142003 | 58142020 | +/- | 18 | TTCTGGGATTGCTTAAGC |
| chr3 | 58142004 | 58142021 | +/- | 18 | TCTGGGATTGCTTAAGCC |
| chr3 | 58142005 | 58142022 | +/- | 18 | CTGGGATTGCTTAAGCCC |
| chr3 | 58142006 | 58142023 | +/- | 18 | TGGGATTGCTTAAGCCCT |
| chr3 | 58142007 | 58142024 | +/- | 18 | GGGATTGCTTAAGCCCTG |
| chr3 | 58142008 | 58142025 | +/- | 18 | GGATTGCTTAAGCCCTGT |
| chr3 | 58142009 | 58142026 | +/- | 18 | GATTGCTTAAGCCCTGTG |
| chr3 | 58142010 | 58142027 | +/- | 18 | ATTGCTTAAGCCCTGTGG |
| chr3 | 58142011 | 58142028 | +/- | 18 | TTGCTTAAGCCCTGTGGG |
| chr3 | 58142012 | 58142029 | +/- | 18 | TGCTTAAGCCCTGTGGGT |
| chr3 | 58142013 | 58142030 | +/- | 18 | GCTTAAGCCCTGTGGGTG |
| chr3 | 58142014 | 58142031 | +/- | 18 | CTTAAGCCCTGTGGGTGT |
| chr3 | 58142015 | 58142032 | +/- | 18 | TTAAGCCCTGTGGGTGTC |
| chr3 | 58142016 | 58142033 | +/- | 18 | TAAGCCCTGTGGGTGTCC |
| chr3 | 58142017 | 58142034 | +/- | 18 | AAGCCCTGTGGGTGTCCT |
| chr3 | 58142018 | 58142035 | +/- | 18 | AGCCCTGTGGGTGTCCTG |
| chr3 | 58142019 | 58142036 | +/- | 18 | GCCCTGTGGGTGTCCTGG |
| chr3 | 58142020 | 58142037 | +/- | 18 | CCCTGTGGGTGTCCTGGT |
| chr3 | 58142021 | 58142038 | +/- | 18 | CCTGTGGGTGTCCTGGTC |
| chr3 | 58142022 | 58142039 | +/- | 18 | CTGTGGGTGTCCTGGTCA |
| chr3 | 58142023 | 58142040 | +/- | 18 | TGTGGGTGTCCTGGTCAT |
| chr3 | 58142024 | 58142041 | +/- | 18 | GTGGGTGTCCTGGTCATT |
| chr3 | 58142025 | 58142042 | +/- | 18 | TGGGTGTCCTGGTCATTG |
| chr3 | 58142026 | 58142043 | +/- | 18 | GGGTGTCCTGGTCATTGG |
| chr3 | 58142027 | 58142044 | +/- | 18 | GGTGTCCTGGTCATTGGT |

**Table 5: 19-mer target-specific ASOs**

| **CHR** | **START** | **END** | **STRAND** | **kmer** | **SEQUENCE** |
|---|---|---|---|---|---|
| chr3 | 58141828 | 58141846 | +/- | 19 | CCCAACTAATCTCCATTTG |
| chr3 | 58141829 | 58141847 | +/- | 19 | CCAACTAATCTCCATTTGC |
| chr3 | 58141830 | 58141848 | +/- | 19 | CAACTAATCTCCATTTGCC |
| chr3 | 58141831 | 58141849 | +/- | 19 | AACTAATCTCCATTTGCCA |
| chr3 | 58141832 | 58141850 | +/- | 19 | ACTAATCTCCATTTGCCAC |
| chr3 | 58141833 | 58141851 | +/- | 19 | CTAATCTCCATTTGCCACT |
| chr3 | 58141834 | 58141852 | +/- | 19 | TAATCTCCATTTGCCACTG |
| chr3 | 58141835 | 58141853 | +/- | 19 | AATCTCCATTTGCCACTGA |
| chr3 | 58141836 | 58141854 | +/- | 19 | ATCTCCATTTGCCACTGAC |
| chr3 | 58141837 | 58141855 | +/- | 19 | TCTCCATTTGCCACTGACC |
| chr3 | 58141838 | 58141856 | +/- | 19 | CTCCATTTGCCACTGACCA |
| chr3 | 58141839 | 58141857 | +/- | 19 | TCCATTTGCCACTGACCAG |
| chr3 | 58141840 | 58141858 | +/- | 19 | CCATTTGCCACTGACCAGG |
| chr3 | 58141841 | 58141859 | +/- | 19 | CATTTGCCACTGACCAGGC |
| chr3 | 58141842 | 58141860 | +/- | 19 | ATTTGCCACTGACCAGGCC |
| chr3 | 58141843 | 58141861 | +/- | 19 | TTTGCCACTGACCAGGCCA |
| chr3 | 58141844 | 58141862 | +/- | 19 | TTGCCACTGACCAGGCCAC |
| chr3 | 58141845 | 58141863 | +/- | 19 | TGCCACTGACCAGGCCACA |
| chr3 | 58141846 | 58141864 | +/- | 19 | GCCACTGACCAGGCCACAG |
| chr3 | 58141847 | 58141865 | +/- | 19 | CCACTGACCAGGCCACAGA |
| chr3 | 58141848 | 58141866 | +/- | 19 | CACTGACCAGGCCACAGAT |
| chr3 | 58141849 | 58141867 | +/- | 19 | ACTGACCAGGCCACAGATG |
| chr3 | 58141850 | 58141868 | +/- | 19 | CTGACCAGGCCACAGATGG |
| chr3 | 58141851 | 58141869 | +/- | 19 | TGACCAGGCCACAGATGGG |
| chr3 | 58141852 | 58141870 | +/- | 19 | GACCAGGCCACAGATGGGG |
| chr3 | 58141853 | 58141871 | +/- | 19 | ACCAGGCCACAGATGGGGA |
| chr3 | 58141854 | 58141872 | +/- | 19 | CCAGGCCACAGATGGGGAA |
| chr3 | 58141855 | 58141873 | +/- | 19 | CAGGCCACAGATGGGGAAG |
| chr3 | 58141856 | 58141874 | +/- | 19 | AGGCCACAGATGGGGAAGT |
| chr3 | 58141857 | 58141875 | +/- | 19 | GGCCACAGATGGGGAAGTC |
| chr3 | 58141858 | 58141876 | +/- | 19 | GCCACAGATGGGGAAGTCA |
| chr3 | 58141859 | 58141877 | +/- | 19 | CCACAGATGGGGAAGTCAC |
| chr3 | 58141860 | 58141878 | +/- | 19 | CACAGATGGGGAAGTCACA |
| chr3 | 58141861 | 58141879 | +/- | 19 | ACAGATGGGGAAGTCACAG |
| chr3 | 58141862 | 58141880 | +/- | 19 | CAGATGGGGAAGTCACAGC |
| chr3 | 58141863 | 58141881 | +/- | 19 | AGATGGGGAAGTCACAGCC |
| chr3 | 58141864 | 58141882 | +/- | 19 | GATGGGGAAGTCACAGCCG |
| chr3 | 58141865 | 58141883 | +/- | 19 | ATGGGGAAGTCACAGCCGT |
| chr3 | 58141866 | 58141884 | +/- | 19 | TGGGGAAGTCACAGCCGTG |
| chr3 | 58141867 | 58141885 | +/- | 19 | GGGGAAGTCACAGCCGTGG |
| chr3 | 58141868 | 58141886 | +/- | 19 | GGGAAGTCACAGCCGTGGA |
| chr3 | 58141869 | 58141887 | +/- | 19 | GGAAGTCACAGCCGTGGAG |
| chr3 | 58141870 | 58141888 | +/- | 19 | GAAGTCACAGCCGTGGAGG |
| chr3 | 58141871 | 58141889 | +/- | 19 | AAGTCACAGCCGTGGAGGA |
| chr3 | 58141872 | 58141890 | +/- | 19 | AGTCACAGCCGTGGAGGAG |
| chr3 | 58141873 | 58141891 | +/- | 19 | GTCACAGCCGTGGAGGAGG |
| chr3 | 58141874 | 58141892 | +/- | 19 | TCACAGCCGTGGAGGAGGC |
| chr3 | 58141875 | 58141893 | +/- | 19 | CACAGCCGTGGAGGAGGCA |
| chr3 | 58141876 | 58141894 | +/- | 19 | ACAGCCGTGGAGGAGGCAC |
| chr3 | 58141877 | 58141895 | +/- | 19 | CAGCCGTGGAGGAGGCACC |
| chr3 | 58141878 | 58141896 | +/- | 19 | AGCCGTGGAGGAGGCACCG |
| chr3 | 58141879 | 58141897 | +/- | 19 | GCCGTGGAGGAGGCACCGG |
| chr3 | 58141880 | 58141898 | +/- | 19 | CCGTGGAGGAGGCACCGGT |
| chr3 | 58141881 | 58141899 | +/- | 19 | CGTGGAGGAGGCACCGGTA |
| chr3 | 58141882 | 58141900 | +/- | 19 | GTGGAGGAGGCACCGGTAA |
| chr3 | 58141883 | 58141901 | +/- | 19 | TGGAGGAGGCACCGGTAAA |
| chr3 | 58141884 | 58141902 | +/- | 19 | GGAGGAGGCACCGGTAAAT |
| chr3 | 58141885 | 58141903 | +/- | 19 | GAGGAGGCACCGGTAAATG |
| chr3 | 58141886 | 58141904 | +/- | 19 | AGGAGGCACCGGTAAATGC |
| chr3 | 58141887 | 58141905 | +/- | 19 | GGAGGCACCGGTAAATGCA |
| chr3 | 58141888 | 58141906 | +/- | 19 | GAGGCACCGGTAAATGCAT |
| chr3 | 58141889 | 58141907 | +/- | 19 | AGGCACCGGTAAATGCATG |
| chr3 | 58141890 | 58141908 | +/- | 19 | GGCACCGGTAAATGCATGT |
| chr3 | 58141891 | 58141909 | +/- | 19 | GCACCGGTAAATGCATGTC |
| chr3 | 58141892 | 58141910 | +/- | 19 | CACCGGTAAATGCATGTCC |
| chr3 | 58141893 | 58141911 | +/- | 19 | ACCGGTAAATGCATGTCCC |
| chr3 | 58141894 | 58141912 | +/- | 19 | CCGGTAAATGCATGTCCCC |
| chr3 | 58141895 | 58141913 | +/- | 19 | CGGTAAATGCATGTCCCCC |
| chr3 | 58141896 | 58141914 | +/- | 19 | GGTAAATGCATGTCCCCCT |
| chr3 | 58141897 | 58141915 | +/- | 19 | GTAAATGCATGTCCCCCTG |
| chr3 | 58141898 | 58141916 | +/- | 19 | TAAATGCATGTCCCCCTGG |
| chr3 | 58141899 | 58141917 | +/- | 19 | AAATGCATGTCCCCCTGGA |
| chr3 | 58141900 | 58141918 | +/- | 19 | AATGCATGTCCCCCTGGAT |
| chr3 | 58141901 | 58141919 | +/- | 19 | ATGCATGTCCCCCTGGATT |
| chr3 | 58141902 | 58141920 | +/- | 19 | TGCATGTCCCCCTGGATTC |
| chr3 | 58141903 | 58141921 | +/- | 19 | GCATGTCCCCCTGGATTCA |
| chr3 | 58141904 | 58141922 | +/- | 19 | CATGTCCCCCTGGATTCAG |
| chr3 | 58141905 | 58141923 | +/- | 19 | ATGTCCCCCTGGATTCAGG |
| chr3 | 58141906 | 58141924 | +/- | 19 | TGTCCCCCTGGATTCAGGC |
| chr3 | 58141907 | 58141925 | +/- | 19 | GTCCCCCTGGATTCAGGCC |
| chr3 | 58141908 | 58141926 | +/- | 19 | TCCCCCTGGATTCAGGCCC |
| chr3 | 58141909 | 58141927 | +/- | 19 | CCCCCTGGATTCAGGCCCT |
| chr3 | 58141910 | 58141928 | +/- | 19 | CCCCTGGATTCAGGCCCTG |
| chr3 | 58141911 | 58141929 | +/- | 19 | CCCTGGATTCAGGCCCTGG |
| chr3 | 58141912 | 58141930 | +/- | 19 | CCTGGATTCAGGCCCTGGG |
| chr3 | 58141913 | 58141931 | +/- | 19 | CTGGATTCAGGCCCTGGGT |
| chr3 | 58141914 | 58141932 | +/- | 19 | TGGATTCAGGCCCTGGGTA |
| chr3 | 58141915 | 58141933 | +/- | 19 | GGATTCAGGCCCTGGGTAC |
| chr3 | 58141916 | 58141934 | +/- | 19 | GATTCAGGCCCTGGGTACA |
| chr3 | 58141917 | 58141935 | +/- | 19 | ATTCAGGCCCTGGGTACAA |
| chr3 | 58141918 | 58141936 | +/- | 19 | TTCAGGCCCTGGGTACAAT |
| chr3 | 58141919 | 58141937 | +/- | 19 | TCAGGCCCTGGGTACAATT |
| chr3 | 58141920 | 58141938 | +/- | 19 | CAGGCCCTGGGTACAATTT |
| chr3 | 58141921 | 58141939 | +/- | 19 | AGGCCCTGGGTACAATTTT |
| chr3 | 58141922 | 58141940 | +/- | 19 | GGCCCTGGGTACAATTTTG |
| chr3 | 58141923 | 58141941 | +/- | 19 | GCCCTGGGTACAATTTTGG |
| chr3 | 58141924 | 58141942 | +/- | 19 | CCCTGGGTACAATTTTGGT |
| chr3 | 58141925 | 58141943 | +/- | 19 | CCTGGGTACAATTTTGGTT |
| chr3 | 58141926 | 58141944 | +/- | 19 | CTGGGTACAATTTTGGTTT |
| chr3 | 58141927 | 58141945 | +/- | 19 | TGGGTACAATTTTGGTTTT |
| chr3 | 58141928 | 58141946 | +/- | 19 | GGGTACAATTTTGGTTTTT |
| chr3 | 58141929 | 58141947 | +/- | 19 | GGTACAATTTTGGTTTTT |
| chr3 | 58141930 | 58141948 | +/- | 19 | GTACAATTTTGGTTTTTTC |
| chr3 | 58141931 | 58141949 | +/- | 19 | TACAATTTTGGTTTTTCC |
| chr3 | 58141932 | 58141950 | +/- | 19 | ACAATTTTGGTTTTTCCT |
| chr3 | 58141933 | 58141951 | +/- | 19 | CAATTTTGGTTTTTTCCTT |
| chr3 | 58141934 | 58141952 | +/- | 19 | AATTTTGGTTTTTTCCTTT |
| chr3 | 58141935 | 58141953 | +/- | 19 | ATTTTGGTTTTTTCCTTTT |
| chr3 | 58141936 | 58141954 | +/- | 19 | TTTTGGTTTTTCCTTTTT |
| chr3 | 58141937 | 58141955 | +/- | 19 | TTTGGTTTTTTCCTTTTTG |
| chr3 | 58141938 | 58141956 | +/- | 19 | TTGGTTTTTTTCCTTTTTGT |
| chr3 | 58141939 | 58141957 | +/- | 19 | TGGTTTTTTCCTTTTTGTG |
| chr3 | 58141940 | 58141958 | +/- | 19 | GGTTTTTTCCTTTTTGTGT |
| chr3 | 58141941 | 58141959 | +/- | 19 | GTTTTTTCCTTTTTGTGTT |
| chr3 | 58141942 | 58141960 | +/- | 19 | TTTTTTCCTTTTTGTGTTT |
| chr3 | 58141943 | 58141961 | +/- | 19 | TTTTTCCTTTTTGTGTTTC |
| chr3 | 58141944 | 58141962 | +/- | 19 | TTTTCCTTTTTGTGTTTCT |
| chr3 | 58141945 | 58141963 | +/- | 19 | TTTCCTTTTTGTGTTTCTG |
| chr3 | 58141946 | 58141964 | +/- | 19 | TTCCTTTTTGTGTTTCTGT |
| chr3 | 58141947 | 58141965 | +/- | 19 | TCCTTTTTGTGTTTCTGTG |
| chr3 | 58141948 | 58141966 | +/- | 19 | CCTTTTTGTGTTTCTGTGT |
| chr3 | 58141949 | 58141967 | +/- | 19 | CTTTTTGTGTTTCTGTGTT |
| chr3 | 58141950 | 58141968 | +/- | 19 | TTTTTGTGTTTCTGTGTTT |
| chr3 | 58141951 | 58141969 | +/- | 19 | TTTTGTGTTTCTGTGTTTA |
| chr3 | 58141952 | 58141970 | +/- | 19 | TTTGTGTTTCTGTGTTTAC |
| chr3 | 58141953 | 58141971 | +/- | 19 | TTGTGTTTCTGTGTTTACT |
| chr3 | 58141954 | 58141972 | +/- | 19 | TGTGTTTCTGTGTTTACTC |
| chr3 | 58141955 | 58141973 | +/- | 19 | GTGTTTCTGTGTTTACTCA |
| chr3 | 58141956 | 58141974 | +/- | 19 | TGTTTCTGTGTTTACTCAG |
| chr3 | 58141957 | 58141975 | +/- | 19 | GTTTCTGTGTTTACTCAGC |
| chr3 | 58141958 | 58141976 | +/- | 19 | TTTCTGTGTTTACTCAGCC |
| chr3 | 58141959 | 58141977 | +/- | 19 | TTCTGTGTTTACTCAGCCT |
| chr3 | 58141960 | 58141978 | +/- | 19 | TCTGTGTTTACTCAGCCTT |
| chr3 | 58141961 | 58141979 | +/- | 19 | CTGTGTTTACTCAGCCTTC |
| chr3 | 58141962 | 58141980 | +/- | 19 | TGTGTTTACTCAGCCTTCA |
| chr3 | 58141963 | 58141981 | +/- | 19 | GTGTTTACTCAGCCTTCAT |
| chr3 | 58141964 | 58141982 | +/- | 19 | TGTTTACTCAGCCTTCATT |
| chr3 | 58141965 | 58141983 | +/- | 19 | GTTTACTCAGCCTTCATTT |
| chr3 | 58141966 | 58141984 | +/- | 19 | TTTACTCAGCCTTCATTTC |
| chr3 | 58141967 | 58141985 | +/- | 19 | TTACTCAGCCTTCATTTCA |
| chr3 | 58141968 | 58141986 | +/- | 19 | TACTCAGCCTTCATTTCAG |
| chr3 | 58141969 | 58141987 | +/- | 19 | ACTCAGCCTTCATTTCAGA |
| chr3 | 58141970 | 58141988 | +/- | 19 | CTCAGCCTTCATTTCAGAA |
| chr3 | 58141971 | 58141989 | +/- | 19 | TCAGCCTTCATTTCAGAAA |
| chr3 | 58141972 | 58141990 | +/- | 19 | CAGCCTTCATTTCAGAAAA |
| chr3 | 58141973 | 58141991 | +/- | 19 | AGCCTTCATTTCAGAAAAT |
| chr3 | 58141974 | 58141992 | +/- | 19 | GCCTTCATTTCAGAAAATC |
| chr3 | 58141975 | 58141993 | +/- | 19 | CCTTCATTTCAGAAAATCT |
| chr3 | 58141976 | 58141994 | +/- | 19 | CTTCATTTCAGAAAATCTG |
| chr3 | 58141977 | 58141995 | +/- | 19 | TTCATTTCAGAAAATCTGC |
| chr3 | 58141978 | 58141996 | +/- | 19 | TCATTTCAGAAAATCTGCC |
| chr3 | 58141979 | 58141997 | +/- | 19 | CATTTCAGAAAATCTGCCA |
| chr3 | 58141980 | 58141998 | +/- | 19 | ATTTCAGAAAATCTGCCAT |
| chr3 | 58141981 | 58141999 | +/- | 19 | TTTCAGAAAATCTGCCATC |
| chr3 | 58141982 | 58142000 | +/- | 19 | TTCAGAAAATCTGCCATCT |
| chr3 | 58141983 | 58142001 | +/- | 19 | TCAGAAAATCTGCCATCTG |
| chr3 | 58141984 | 58142002 | +/- | 19 | CAGAAAATCTGCCATCTGC |
| chr3 | 58141985 | 58142003 | +/- | 19 | AGAAAATCTGCCATCTGCT |
| chr3 | 58141986 | 58142004 | +/- | 19 | GAAAATCTGCCATCTGCTT |
| chr3 | 58141987 | 58142005 | +/- | 19 | AAAATCTGCCATCTGCTTC |
| chr3 | 58141988 | 58142006 | +/- | 19 | AAATCTGCCATCTGCTTCT |
| chr3 | 58141989 | 58142007 | +/- | 19 | AATCTGCCATCTGCTTCTG |
| chr3 | 58141990 | 58142008 | +/- | 19 | ATCTGCCATCTGCTTCTGG |
| chr3 | 58141991 | 58142009 | +/- | 19 | TCTGCCATCTGCTTCTGGG |
| chr3 | 58141992 | 58142010 | +/- | 19 | CTGCCATCTGCTTCTGGGA |
| chr3 | 58141993 | 58142011 | +/- | 19 | TGCCATCTGCTTCTGGGAT |
| chr3 | 58141994 | 58142012 | +/- | 19 | GCCATCTGCTTCTGGGATT |
| chr3 | 58141995 | 58142013 | +/- | 19 | CCATCTGCTTCTGGGATTG |
| chr3 | 58141996 | 58142014 | +/- | 19 | CATCTGCTTCTGGGATTGC |
| chr3 | 58141997 | 58142015 | +/- | 19 | ATCTGCTTCTGGGATTGCT |
| chr3 | 58141998 | 58142016 | +/- | 19 | TCTGCTTCTGGGATTGCTT |
| chr3 | 58141999 | 58142017 | +/- | 19 | CTGCTTCTGGGATTGCTTA |
| chr3 | 58142000 | 58142018 | +/- | 19 | TGCTTCTGGGATTGCTTAA |
| chr3 | 58142001 | 58142019 | +/- | 19 | GCTTCTGGGATTGCTTAAG |
| chr3 | 58142002 | 58142020 | +/- | 19 | CTTCTGGGATTGCTTAAGC |
| chr3 | 58142003 | 58142021 | +/- | 19 | TTCTGGGATTGCTTAAGCC |
| chr3 | 58142004 | 58142022 | +/- | 19 | TCTGGGATTGCTTAAGCCC |
| chr3 | 58142005 | 58142023 | +/- | 19 | CTGGGATTGCTTAAGCCCT |
| chr3 | 58142006 | 58142024 | +/- | 19 | TGGGATTGCTTAAGCCCTG |
| chr3 | 58142007 | 58142025 | +/- | 19 | GGGATTGCTTAAGCCCTGT |
| chr3 | 58142008 | 58142026 | +/- | 19 | GGATTGCTTAAGCCCTGTG |
| chr3 | 58142009 | 58142027 | +/- | 19 | GATTGCTTAAGCCCTGTGG |
| chr3 | 58142010 | 58142028 | +/- | 19 | ATTGCTTAAGCCCTGTGGG |
| chr3 | 58142011 | 58142029 | +/- | 19 | TTGCTTAAGCCCTGTGGGT |
| chr3 | 58142012 | 58142030 | +/- | 19 | TGCTTAAGCCCTGTGGGTG |
| chr3 | 58142013 | 58142031 | +/- | 19 | GCTTAAGCCCTGTGGGTGT |
| chr3 | 58142014 | 58142032 | +/- | 19 | CTTAAGCCCTGTGGGTGTC |
| chr3 | 58142015 | 58142033 | +/- | 19 | TTAAGCCCTGTGGGTGTCC |
| chr3 | 58142016 | 58142034 | +/- | 19 | TAAGCCCTGTGGGTGTCCT |
| chr3 | 58142017 | 58142035 | +/- | 19 | AAGCCCTGTGGGTGTCCTG |
| chr3 | 58142018 | 58142036 | +/- | 19 | AGCCCTGTGGGTGTCCTGG |
| chr3 | 58142019 | 58142037 | +/- | 19 | GCCCTGTGGGTGTCCTGGT |
| chr3 | 58142020 | 58142038 | +/- | 19 | CCCTGTGGGTGTCCTGGTC |
| chr3 | 58142021 | 58142039 | +/- | 19 | CCTGTGGGTGTCCTGGTCA |
| chr3 | 58142022 | 58142040 | +/- | 19 | CTGTGGGTGTCCTGGTCAT |
| chr3 | 58142023 | 58142041 | +/- | 19 | TGTGGGTGTCCTGGTCATT |
| chr3 | 58142024 | 58142042 | +/- | 19 | GTGGGTGTCCTGGTCATTG |
| chr3 | 58142025 | 58142043 | +/- | 19 | TGGGTGTCCTGGTCATTGG |
| chr3 | 58142026 | 58142044 | +/- | 19 | GGGTGTCCTGGTCATTGGT |
| | | | +/- | | |

**Table 6: 20-mer target-specific ASOs**

| **CHR** | **START** | **END** | **STRAND** | **kmer** | **SEQUENCE** |
|---|---|---|---|---|---|
| chr3 | 58141828 | 58141847 | +/- | 20 | CCCAACTAATCTCCATTTGC |
| chr3 | 58141829 | 58141848 | +/- | 20 | CCAACTAATCTCCATTTGCC |
| chr3 | 58141830 | 58141849 | +/- | 20 | CAACTAATCTCCATTTGCCA |
| chr3 | 58141831 | 58141850 | +/- | 20 | AACTAATCTCCATTTGCCAC |
| chr3 | 58141832 | 58141851 | +/- | 20 | ACTAATCTCCATTTGCCACT |
| chr3 | 58141833 | 58141852 | +/- | 20 | CTAATCTCCATTTGCCACTG |
| chr3 | 58141834 | 58141853 | +/- | 20 | TAATCTCCATTTGCCACTGA |
| chr3 | 58141835 | 58141854 | +/- | 20 | AATCTCCATTTGCCACTGAC |
| chr3 | 58141836 | 58141855 | +/- | 20 | ATCTCCATTTGCCACTGACC |
| chr3 | 58141837 | 58141856 | +/- | 20 | TCTCCATTTGCCACTGACCA |
| chr3 | 58141838 | 58141857 | +/- | 20 | CTCCATTTGCCACTGACCAG |
| chr3 | 58141839 | 58141858 | +/- | 20 | TCCATTTGCCACTGACCAGG |
| chr3 | 58141840 | 58141859 | +/- | 20 | CCATTTGCCACTGACCAGGC |
| chr3 | 58141841 | 58141860 | +/- | 20 | CATTTGCCACTGACCAGGCC |
| chr3 | 58141842 | 58141861 | +/- | 20 | ATTTGCCACTGACCAGGCCA |
| chr3 | 58141843 | 58141862 | +/- | 20 | TTTGCCACTGACCAGGCCAC |
| chr3 | 58141844 | 58141863 | +/- | 20 | TTGCCACTGACCAGGCCACA |
| chr3 | 58141845 | 58141864 | +/- | 20 | TGCCACTGACCAGGCCACAG |
| chr3 | 58141846 | 58141865 | +/- | 20 | GCCACTGACCAGGCCACAGA |
| chr3 | 58141847 | 58141866 | +/- | 20 | CCACTGACCAGGCCACAGAT |
| chr3 | 58141848 | 58141867 | +/- | 20 | CACTGACCAGGCCACAGATG |
| chr3 | 58141849 | 58141868 | +/- | 20 | ACTGACCAGGCCACAGATGG |
| chr3 | 58141850 | 58141869 | +/- | 20 | CTGACCAGGCCACAGATGGG |
| chr3 | 58141851 | 58141870 | +/- | 20 | TGACCAGGCCACAGATGGGG |
| chr3 | 58141852 | 58141871 | +/- | 20 | GACCAGGCCACAGATGGGGA |
| chr3 | 58141853 | 58141872 | +/- | 20 | ACCAGGCCACAGATGGGGAA |
| chr3 | 58141854 | 58141873 | +/- | 20 | CCAGGCCACAGATGGGGAAG |
| chr3 | 58141855 | 58141874 | +/- | 20 | CAGGCCACAGATGGGGAAGT |
| chr3 | 58141856 | 58141875 | +/- | 20 | AGGCCACAGATGGGGAAGTC |
| chr3 | 58141857 | 58141876 | +/- | 20 | GGCCACAGATGGGGAAGTCA |
| chr3 | 58141858 | 58141877 | +/- | 20 | GCCACAGATGGGGAAGTCAC |
| chr3 | 58141859 | 58141878 | +/- | 20 | CCACAGATGGGGAAGTCACA |
| chr3 | 58141860 | 58141879 | +/- | 20 | CACAGATGGGGAAGTCACAG |
| chr3 | 58141861 | 58141880 | +/- | 20 | ACAGATGGGGAAGTCACAGC |
| chr3 | 58141862 | 58141881 | +/- | 20 | CAGATGGGGAAGTCACAGCC |
| chr3 | 58141863 | 58141882 | +/- | 20 | AGATGGGGAAGTCACAGCCG |
| chr3 | 58141864 | 58141883 | +/- | 20 | GATGGGGAAGTCACAGCCGT |
| chr3 | 58141865 | 58141884 | +/- | 20 | ATGGGGAAGTCACAGCCGTG |
| chr3 | 58141866 | 58141885 | +/- | 20 | TGGGGAAGTCACAGCCGTGG |
| chr3 | 58141867 | 58141886 | +/- | 20 | GGGGAAGTCACAGCCGTGGA |
| chr3 | 58141868 | 58141887 | +/- | 20 | GGGAAGTCACAGCCGTGGAG |
| chr3 | 58141869 | 58141888 | +/- | 20 | GGAAGTCACAGCCGTGGAGG |
| chr3 | 58141870 | 58141889 | +/- | 20 | GAAGTCACAGCCGTGGAGGA |
| chr3 | 58141871 | 58141890 | +/- | 20 | AAGTCACAGCCGTGGAGGAG |
| chr3 | 58141872 | 58141891 | +/- | 20 | AGTCACAGCCGTGGAGGAGG |
| chr3 | 58141873 | 58141892 | +/- | 20 | GTCACAGCCGTGGAGGAGGC |
| chr3 | 58141874 | 58141893 | +/- | 20 | TCACAGCCGTGGAGGAGGCA |
| chr3 | 58141875 | 58141894 | +/- | 20 | CACAGCCGTGGAGGAGGCAC |
| chr3 | 58141876 | 58141895 | +/- | 20 | ACAGCCGTGGAGGAGGCACC |
| chr3 | 58141877 | 58141896 | +/- | 20 | CAGCCGTGGAGGAGGCACCG |
| chr3 | 58141878 | 58141897 | +/- | 20 | AGCCGTGGAGGAGGCACCGG |
| chr3 | 58141879 | 58141898 | +/- | 20 | GCCGTGGAGGAGGCACCGGT |
| chr3 | 58141880 | 58141899 | +/- | 20 | CCGTGGAGGAGGCACCGGTA |
| chr3 | 58141881 | 58141900 | +/- | 20 | CGTGGAGGAGGCACCGGTAA |
| chr3 | 58141882 | 58141901 | +/- | 20 | GTGGAGGAGGCACCGGTAAA |
| chr3 | 58141883 | 58141902 | +/- | 20 | TGGAGGAGGCACCGGTAAAT |
| chr3 | 58141884 | 58141903 | +/- | 20 | GGAGGAGGCACCGGTAAATG |
| chr3 | 58141885 | 58141904 | +/- | 20 | GAGGAGGCACCGGTAAATGC |
| chr3 | 58141886 | 58141905 | +/- | 20 | AGGAGGCACCGGTAAATGCA |
| chr3 | 58141887 | 58141906 | +/- | 20 | GGAGGCACCGGTAAATGCAT |
| chr3 | 58141888 | 58141907 | +/- | 20 | GAGGCACCGGTAAATGCATG |
| chr3 | 58141889 | 58141908 | +/- | 20 | AGGCACCGGTAAATGCATGT |
| chr3 | 58141890 | 58141909 | +/- | 20 | GGCACCGGTAAATGCATGTC |
| chr3 | 58141891 | 58141910 | +/- | 20 | GCACCGGTAAATGCATGTCC |
| chr3 | 58141892 | 58141911 | +/- | 20 | CACCGGTAAATGCATGTCCC |
| chr3 | 58141893 | 58141912 | +/- | 20 | ACCGGTAAATGCATGTCCCC |
| chr3 | 58141894 | 58141913 | +/- | 20 | CCGGTAAATGCATGTCCCCC |
| chr3 | 58141895 | 58141914 | +/- | 20 | CGGTAAATGCATGTCCCCCT |
| chr3 | 58141896 | 58141915 | +/- | 20 | GGTAAATGCATGTCCCCCTG |
| chr3 | 58141897 | 58141916 | +/- | 20 | GTAAATGCATGTCCCCCTGG |
| chr3 | 58141898 | 58141917 | +/- | 20 | TAAATGCATGTCCCCCTGGA |
| chr3 | 58141899 | 58141918 | +/- | 20 | AAATGCATGTCCCCCTGGAT |
| chr3 | 58141900 | 58141919 | +/- | 20 | AATGCATGTCCCCCTGGATT |
| chr3 | 58141901 | 58141920 | +/- | 20 | ATGCATGTCCCCCTGGATTC |
| chr3 | 58141902 | 58141921 | +/- | 20 | TGCATGTCCCCCTGGATTCA |
| chr3 | 58141903 | 58141922 | +/- | 20 | GCATGTCCCCCTGGATTCAG |
| chr3 | 58141904 | 58141923 | +/- | 20 | CATGTCCCCCTGGATTCAGG |
| chr3 | 58141905 | 58141924 | +/- | 20 | ATGTCCCCCTGGATTCAGGC |
| chr3 | 58141906 | 58141925 | +/- | 20 | TGTCCCCCTGGATTCAGGCC |
| chr3 | 58141907 | 58141926 | +/- | 20 | GTCCCCCTGGATTCAGGCCC |
| chr3 | 58141908 | 58141927 | +/- | 20 | TCCCCCTGGATTCAGGCCCT |
| chr3 | 58141909 | 58141928 | +/- | 20 | CCCCCTGGATTCAGGCCCTG |
| chr3 | 58141910 | 58141929 | +/- | 20 | CCCCTGGATTCAGGCCCTGG |
| chr3 | 58141911 | 58141930 | +/- | 20 | CCCTGGATTCAGGCCCTGGG |
| chr3 | 58141912 | 58141931 | +/- | 20 | CCTGGATTCAGGCCCTGGGT |
| chr3 | 58141913 | 58141932 | +/- | 20 | CTGGATTCAGGCCCTGGGTA |
| chr3 | 58141914 | 58141933 | +/- | 20 | TGGATTCAGGCCCTGGGTAC |
| chr3 | 58141915 | 58141934 | +/- | 20 | GGATTCAGGCCCTGGGTACA |
| chr3 | 58141916 | 58141935 | +/- | 20 | GATTCAGGCCCTGGGTACAA |
| chr3 | 58141917 | 58141936 | +/- | 20 | ATTCAGGCCCTGGGTACAAT |
| chr3 | 58141918 | 58141937 | +/- | 20 | TTCAGGCCCTGGGTACAATT |
| chr3 | 58141919 | 58141938 | +/- | 20 | TCAGGCCCTGGGTACAATTT |
| chr3 | 58141920 | 58141939 | +/- | 20 | CAGGCCCTGGGTACAATTTT |
| chr3 | 58141921 | 58141940 | +/- | 20 | AGGCCCTGGGTACAATTTTG |
| chr3 | 58141922 | 58141941 | +/- | 20 | GGCCCTGGGTACAATTTTGG |
| chr3 | 58141923 | 58141942 | +/- | 20 | GCCCTGGGTACAATTTTGGT |
| chr3 | 58141924 | 58141943 | +/- | 20 | CCCTGGGTACAATTTTGGTT |
| chr3 | 58141925 | 58141944 | +/- | 20 | CCTGGGTACAATTTTGGTTT |
| chr3 | 58141926 | 58141945 | +/- | 20 | CTGGGTACAATTTTGGTTTT |
| chr3 | 58141927 | 58141946 | +/- | 20 | TGGGTACAATTTTGGTTTTT |
| chr3 | 58141928 | 58141947 | +/- | 20 | GGGTACAATTTTGGTTTTTT |
| chr3 | 58141929 | 58141948 | +/- | 20 | GGTACAATTTTGGTTTTTTC |
| chr3 | 58141930 | 58141949 | +/- | 20 | GTACAATTTTGGTTTTTTCC |
| chr3 | 58141931 | 58141950 | +/- | 20 | TACAATTTTGGTTTTTTCCT |
| chr3 | 58141932 | 58141951 | +/- | 20 | ACAATTTTGGTTTTTTCCTT |
| chr3 | 58141933 | 58141952 | +/- | 20 | CAATTTTGGTTTTTTCCTTT |
| chr3 | 58141934 | 58141953 | +/- | 20 | AATTTTGGTTTTTTCCTTTT |
| chr3 | 58141935 | 58141954 | +/- | 20 | ATTTTGGTTTTTTCCTTTTT |
| chr3 | 58141936 | 58141955 | +/- | 20 | TTTTGGTTTTTTCCTTTTTG |
| chr3 | 58141937 | 58141956 | +/- | 20 | TTTGGTTTTTTCCTTTTTGT |
| chr3 | 58141938 | 58141957 | +/- | 20 | TTGGTTTTTTCCTTTTTGTG |
| chr3 | 58141939 | 58141958 | +/- | 20 | TGGTTTTTTCCTTTTTGTGT |
| chr3 | 58141940 | 58141959 | +/- | 20 | GGTTTTTTCCTTTTTGTGTT |
| chr3 | 58141941 | 58141960 | +/- | 20 | GTTTTTTCCTTTTTGTGTTT |
| chr3 | 58141942 | 58141961 | +/- | 20 | TTTTTTCCTTTTTGTGTTTC |
| chr3 | 58141943 | 58141962 | +/- | 20 | TTTTTCCTTTTTGTGTTTCT |
| chr3 | 58141944 | 58141963 | +/- | 20 | TTTTCCTTTTTGTGTTTCTG |
| chr3 | 58141945 | 58141964 | +/- | 20 | TTTCCTTTTTGTGTTTCTGT |
| chr3 | 58141946 | 58141965 | +/- | 20 | TTCCTTTTTGTGTTTCTGTG |
| chr3 | 58141947 | 58141966 | +/- | 20 | TCCTTTTTGTGTTTCTGTGT |
| chr3 | 58141948 | 58141967 | +/- | 20 | CCTTTTTGTGTTTCTGTGTT |
| chr3 | 58141949 | 58141968 | +/- | 20 | CTTTTTGTGTTTCTGTGTTT |
| chr3 | 58141950 | 58141969 | +/- | 20 | TTTTTGTGTTTCTGTGTTTA |
| chr3 | 58141951 | 58141970 | +/- | 20 | TTTTGTGTTTCTGTGTTTAC |
| chr3 | 58141952 | 58141971 | +/- | 20 | TTTGTGTTTCTGTGTTTACT |
| chr3 | 58141953 | 58141972 | +/- | 20 | TTGTGTTTCTGTGTTTACTC |
| chr3 | 58141954 | 58141973 | +/- | 20 | TGTGTTTCTGTGTTTACTCA |
| chr3 | 58141955 | 58141974 | +/- | 20 | GTGTTTCTGTGTTTACTCAG |
| chr3 | 58141956 | 58141975 | +/- | 20 | TGTTTCTGTGTTTACTCAGC |
| chr3 | 58141957 | 58141976 | +/- | 20 | GTTTCTGTGTTTACTCAGCC |
| chr3 | 58141958 | 58141977 | +/- | 20 | TTTCTGTGTTTACTCAGCCT |
| chr3 | 58141959 | 58141978 | +/- | 20 | TTCTGTGTTTACTCAGCCTT |
| chr3 | 58141960 | 58141979 | +/- | 20 | TCTGTGTTTACTCAGCCTTC |
| chr3 | 58141961 | 58141980 | +/- | 20 | CTGTGTTTACTCAGCCTTCA |
| chr3 | 58141962 | 58141981 | +/- | 20 | TGTGTTTACTCAGCCTTCAT |
| chr3 | 58141963 | 58141982 | +/- | 20 | GTGTTTACTCAGCCTTCATT |
| chr3 | 58141964 | 58141983 | +/- | 20 | TGTTTACTCAGCCTTCATTT |
| chr3 | 58141965 | 58141984 | +/- | 20 | GTTTACTCAGCCTTCATTTC |
| chr3 | 58141966 | 58141985 | +/- | 20 | TTTACTCAGCCTTCATTTCA |
| chr3 | 58141967 | 58141986 | +/- | 20 | TTACTCAGCCTTCATTTCAG |
| chr3 | 58141968 | 58141987 | +/- | 20 | TACTCAGCCTTCATTTCAGA |
| chr3 | 58141969 | 58141988 | +/- | 20 | ACTCAGCCTTCATTTCAGAA |
| chr3 | 58141970 | 58141989 | +/- | 20 | CTCAGCCTTCATTTCAGAAA |
| chr3 | 58141971 | 58141990 | +/- | 20 | TCAGCCTTCATTTCAGAAAA |
| chr3 | 58141972 | 58141991 | +/- | 20 | CAGCCTTCATTTCAGAAAAT |
| chr3 | 58141973 | 58141992 | +/- | 20 | AGCCTTCATTTCAGAAAATC |
| chr3 | 58141974 | 58141993 | +/- | 20 | GCCTTCATTTCAGAAAATCT |
| chr3 | 58141975 | 58141994 | +/- | 20 | CCTTCATTTCAGAAAATCTG |
| chr3 | 58141976 | 58141995 | +/- | 20 | CTTCATTTCAGAAAATCTGC |
| chr3 | 58141977 | 58141996 | +/- | 20 | TTCATTTCAGAAAATCTGCC |
| chr3 | 58141978 | 58141997 | +/- | 20 | TCATTTCAGAAAATCTGCCA |
| chr3 | 58141979 | 58141998 | +/- | 20 | CATTTCAGAAAATCTGCCAT |
| chr3 | 58141980 | 58141999 | +/- | 20 | ATTTCAGAAAATCTGCCATC |
| chr3 | 58141981 | 58142000 | +/- | 20 | TTTCAGAAAATCTGCCATCT |
| chr3 | 58141982 | 58142001 | +/- | 20 | TTCAGAAAATCTGCCATCTG |
| chr3 | 58141983 | 58142002 | +/- | 20 | TCAGAAAATCTGCCATCTGC |
| chr3 | 58141984 | 58142003 | +/- | 20 | CAGAAAATCTGCCATCTGCT |
| chr3 | 58141985 | 58142004 | +/- | 20 | AGAAAATCTGCCATCTGCTT |
| chr3 | 58141986 | 58142005 | +/- | 20 | GAAAATCTGCCATCTGCTTC |
| chr3 | 58141987 | 58142006 | +/- | 20 | AAAATCTGCCATCTGCTTCT |
| chr3 | 58141988 | 58142007 | +/- | 20 | AAATCTGCCATCTGCTTCTG |
| chr3 | 58141989 | 58142008 | +/- | 20 | AATCTGCCATCTGCTTCTGG |
| chr3 | 58141990 | 58142009 | +/- | 20 | ATCTGCCATCTGCTTCTGGG |
| chr3 | 58141991 | 58142010 | +/- | 20 | TCTGCCATCTGCTTCTGGGA |
| chr3 | 58141992 | 58142011 | +/- | 20 | CTGCCATCTGCTTCTGGGAT |
| chr3 | 58141993 | 58142012 | +/- | 20 | TGCCATCTGCTTCTGGGATT |
| chr3 | 58141994 | 58142013 | +/- | 20 | GCCATCTGCTTCTGGGATTG |
| chr3 | 58141995 | 58142014 | +/- | 20 | CCATCTGCTTCTGGGATTGC |
| chr3 | 58141996 | 58142015 | +/- | 20 | CATCTGCTTCTGGGATTGCT |
| chr3 | 58141997 | 58142016 | +/- | 20 | ATCTGCTTCTGGGATTGCTT |
| chr3 | 58141998 | 58142017 | +/- | 20 | TCTGCTTCTGGGATTGCTTA |
| chr3 | 58141999 | 58142018 | +/- | 20 | CTGCTTCTGGGATTGCTTAA |
| chr3 | 58142000 | 58142019 | +/- | 20 | TGCTTCTGGGATTGCTTAAG |
| chr3 | 58142001 | 58142020 | +/- | 20 | GCTTCTGGGATTGCTTAAGC |
| chr3 | 58142002 | 58142021 | +/- | 20 | CTTCTGGGATTGCTTAAGCC |
| chr3 | 58142003 | 58142022 | +/- | 20 | TTCTGGGATTGCTTAAGCCC |
| chr3 | 58142004 | 58142023 | +/- | 20 | TCTGGGATTGCTTAAGCCCT |
| chr3 | 58142005 | 58142024 | +/- | 20 | CTGGGATTGCTTAAGCCCTG |
| chr3 | 58142006 | 58142025 | +/- | 20 | TGGGATTGCTTAAGCCCTGT |
| chr3 | 58142007 | 58142026 | +/- | 20 | GGGATTGCTTAAGCCCTGTG |
| chr3 | 58142008 | 58142027 | +/- | 20 | GGATTGCTTAAGCCCTGTGG |
| chr3 | 58142009 | 58142028 | +/- | 20 | GATTGCTTAAGCCCTGTGGG |
| chr3 | 58142010 | 58142029 | +/- | 20 | ATTGCTTAAGCCCTGTGGGT |
| chr3 | 58142011 | 58142030 | +/- | 20 | TTGCTTAAGCCCTGTGGGTG |
| chr3 | 58142012 | 58142031 | +/- | 20 | TGCTTAAGCCCTGTGGGTGT |
| chr3 | 58142013 | 58142032 | +/- | 20 | GCTTAAGCCCTGTGGGTGTC |
| chr3 | 58142014 | 58142033 | +/- | 20 | CTTAAGCCCTGTGGGTGTCC |
| chr3 | 58142015 | 58142034 | +/- | 20 | TTAAGCCCTGTGGGTGTCCT |
| chr3 | 58142016 | 58142035 | +/- | 20 | TAAGCCCTGTGGGTGTCCTG |
| chr3 | 58142017 | 58142036 | +/- | 20 | AAGCCCTGTGGGTGTCCTGG |
| chr3 | 58142018 | 58142037 | +/- | 20 | AGCCCTGTGGGTGTCCTGGT |
| chr3 | 58142019 | 58142038 | +/- | 20 | GCCCTGTGGGTGTCCTGGTC |
| chr3 | 58142020 | 58142039 | +/- | 20 | CCCTGTGGGTGTCCTGGTCA |
| chr3 | 58142021 | 58142040 | +/- | 20 | CCTGTGGGTGTCCTGGTCAT |
| chr3 | 58142022 | 58142041 | +/- | 20 | CTGTGGGTGTCCTGGTCATT |
| chr3 | 58142023 | 58142042 | +/- | 20 | TGTGGGTGTCCTGGTCATTG |
| chr3 | 58142024 | 58142043 | +/- | 20 | GTGGGTGTCCTGGTCATTGG |
| chr3 | 58142025 | 58142044 | +/- | 20 | TGGGTGTCCTGGTCATTGGT |

**Table 7: 21-mer target-specific ASOs**

| **CHR** | **START** | **END** | **STRAND** | **kmer** | **SEQUENCE** |
|---|---|---|---|---|---|
| chr3 | 58141828 | 58141848 | +/- | 21 | CCCAACTAATCTCCATTTGCC |
| chr3 | 58141829 | 58141849 | +/- | 21 | CCAACTAATCTCCATTTGCCA |
| chr3 | 58141830 | 58141850 | +/- | 21 | CAACTAATCTCCATTTGCCAC |
| chr3 | 58141831 | 58141851 | +/- | 21 | AACTAATCTCCATTTGCCACT |
| chr3 | 58141832 | 58141852 | +/- | 21 | ACTAATCTCCATTTGCCACTG |
| chr3 | 58141833 | 58141853 | +/- | 21 | CTAATCTCCATTTGCCACTGA |
| chr3 | 58141834 | 58141854 | +/- | 21 | TAATCTCCATTTGCCACTGAC |
| chr3 | 58141835 | 58141855 | +/- | 21 | AATCTCCATTTGCCACTGACC |
| chr3 | 58141836 | 58141856 | +/- | 21 | ATCTCCATTTGCCACTGACCA |
| chr3 | 58141837 | 58141857 | +/- | 21 | TCTCCATTTGCCACTGACCAG |
| chr3 | 58141838 | 58141858 | +/- | 21 | CTCCATTTGCCACTGACCAGG |
| chr3 | 58141839 | 58141859 | +/- | 21 | TCCATTTGCCACTGACCAGGC |
| chr3 | 58141840 | 58141860 | +/- | 21 | CCATTTGCCACTGACCAGGCC |
| chr3 | 58141841 | 58141861 | +/- | 21 | CATTTGCCACTGACCAGGCCA |
| chr3 | 58141842 | 58141862 | +/- | 21 | ATTTGCCACTGACCAGGCCAC |
| chr3 | 58141843 | 58141863 | +/- | 21 | TTTGCCACTGACCAGGCCACA |
| chr3 | 58141844 | 58141864 | +/- | 21 | TTGCCACTGACCAGGCCACAG |
| chr3 | 58141845 | 58141865 | +/- | 21 | TGCCACTGACCAGGCCACAGA |
| chr3 | 58141846 | 58141866 | +/- | 21 | GCCACTGACCAGGCCACAGAT |
| chr3 | 58141847 | 58141867 | +/- | 21 | CCACTGACCAGGCCACAGATG |
| chr3 | 58141848 | 58141868 | +/- | 21 | CACTGACCAGGCCACAGATGG |
| chr3 | 58141849 | 58141869 | +/- | 21 | ACTGACCAGGCCACAGATGGG |
| chr3 | 58141850 | 58141870 | +/- | 21 | CTGACCAGGCCACAGATGGGG |
| chr3 | 58141851 | 58141871 | +/- | 21 | TGACCAGGCCACAGATGGGGA |
| chr3 | 58141852 | 58141872 | +/- | 21 | GACCAGGCCACAGATGGGGAA |
| chr3 | 58141853 | 58141873 | +/- | 21 | ACCAGGCCACAGATGGGGAAG |
| chr3 | 58141854 | 58141874 | +/- | 21 | CCAGGCCACAGATGGGGAAGT |
| chr3 | 58141855 | 58141875 | +/- | 21 | CAGGCCACAGATGGGGAAGTC |
| chr3 | 58141856 | 58141876 | +/- | 21 | AGGCCACAGATGGGGAAGTCA |
| chr3 | 58141857 | 58141877 | +/- | 21 | GGCCACAGATGGGGAAGTCAC |
| chr3 | 58141858 | 58141878 | +/- | 21 | GCCACAGATGGGGAAGTCACA |
| chr3 | 58141859 | 58141879 | +/- | 21 | CCACAGATGGGGAAGTCACAG |
| chr3 | 58141860 | 58141880 | +/- | 21 | CACAGATGGGGAAGTCACAGC |
| chr3 | 58141861 | 58141881 | +/- | 21 | ACAGATGGGGAAGTCACAGCC |
| chr3 | 58141862 | 58141882 | +/- | 21 | CAGATGGGGAAGTCACAGCCG |
| chr3 | 58141863 | 58141883 | +/- | 21 | AGATGGGGAAGTCACAGCCGT |
| chr3 | 58141864 | 58141884 | +/- | 21 | GATGGGGAAGTCACAGCCGTG |
| chr3 | 58141865 | 58141885 | +/- | 21 | ATGGGGAAGTCACAGCCGTGG |
| chr3 | 58141866 | 58141886 | +/- | 21 | TGGGGAAGTCACAGCCGTGGA |
| chr3 | 58141867 | 58141887 | +/- | 21 | GGGGAAGTCACAGCCGTGGAG |
| chr3 | 58141868 | 58141888 | +/- | 21 | GGGAAGTCACAGCCGTGGAGG |
| chr3 | 58141869 | 58141889 | +/- | 21 | GGAAGTCACAGCCGTGGAGGA |
| chr3 | 58141870 | 58141890 | +/- | 21 | GAAGTCACAGCCGTGGAGGAG |
| chr3 | 58141871 | 58141891 | +/- | 21 | AAGTCACAGCCGTGGAGGAGG |
| chr3 | 58141872 | 58141892 | +/- | 21 | AGTCACAGCCGTGGAGGAGGC |
| chr3 | 58141873 | 58141893 | +/- | 21 | GTCACAGCCGTGGAGGAGGCA |
| chr3 | 58141874 | 58141894 | +/- | 21 | TCACAGCCGTGGAGGAGGCAC |
| chr3 | 58141875 | 58141895 | +/- | 21 | CACAGCCGTGGAGGAGGCACC |
| chr3 | 58141876 | 58141896 | +/- | 21 | ACAGCCGTGGAGGAGGCACCG |
| chr3 | 58141877 | 58141897 | +/- | 21 | CAGCCGTGGAGGAGGCACCGG |
| chr3 | 58141878 | 58141898 | +/- | 21 | AGCCGTGGAGGAGGCACCGGT |
| chr3 | 58141879 | 58141899 | +/- | 21 | GCCGTGGAGGAGGCACCGGTA |
| chr3 | 58141880 | 58141900 | +/- | 21 | CCGTGGAGGAGGCACCGGTAA |
| chr3 | 58141881 | 58141901 | +/- | 21 | CGTGGAGGAGGCACCGGTAAA |
| chr3 | 58141882 | 58141902 | +/- | 21 | GTGGAGGAGGCACCGGTAAAT |
| chr3 | 58141883 | 58141903 | +/- | 21 | TGGAGGAGGCACCGGTAAATG |
| chr3 | 58141884 | 58141904 | +/- | 21 | GGAGGAGGCACCGGTAAATGC |
| chr3 | 58141885 | 58141905 | +/- | 21 | GAGGAGGCACCGGTAAATGCA |
| chr3 | 58141886 | 58141906 | +/- | 21 | AGGAGGCACCGGTAAATGCAT |
| chr3 | 58141887 | 58141907 | +/- | 21 | GGAGGCACCGGTAAATGCATG |
| chr3 | 58141888 | 58141908 | +/- | 21 | GAGGCACCGGTAAATGCATGT |
| chr3 | 58141889 | 58141909 | +/- | 21 | AGGCACCGGTAAATGCATGTC |
| chr3 | 58141890 | 58141910 | +/- | 21 | GGCACCGGTAAATGCATGTCC |
| chr3 | 58141891 | 58141911 | +/- | 21 | GCACCGGTAAATGCATGTCCC |
| chr3 | 58141892 | 58141912 | +/- | 21 | CACCGGTAAATGCATGTCCCC |
| chr3 | 58141893 | 58141913 | +/- | 21 | ACCGGTAAATGCATGTCCCCC |
| chr3 | 58141894 | 58141914 | +/- | 21 | CCGGTAAATGCATGTCCCCCT |
| chr3 | 58141895 | 58141915 | +/- | 21 | CGGTAAATGCATGTCCCCCTG |
| chr3 | 58141896 | 58141916 | +/- | 21 | GGTAAATGCATGTCCCCCTGG |
| chr3 | 58141897 | 58141917 | +/- | 21 | GTAAATGCATGTCCCCCTGGA |
| chr3 | 58141898 | 58141918 | +/- | 21 | TAAATGCATGTCCCCCTGGAT |
| chr3 | 58141899 | 58141919 | +/- | 21 | AAATGCATGTCCCCCTGGATT |
| chr3 | 58141900 | 58141920 | +/- | 21 | AATGCATGTCCCCCTGGATTC |
| chr3 | 58141901 | 58141921 | +/- | 21 | ATGCATGTCCCCCTGGATTCA |
| chr3 | 58141902 | 58141922 | +/- | 21 | TGCATGTCCCCCTGGATTCAG |
| chr3 | 58141903 | 58141923 | +/- | 21 | GCATGTCCCCCTGGATTCAGG |
| chr3 | 58141904 | 58141924 | +/- | 21 | CATGTCCCCCTGGATTCAGGC |
| chr3 | 58141905 | 58141925 | +/- | 21 | ATGTCCCCCTGGATTCAGGCC |
| chr3 | 58141906 | 58141926 | +/- | 21 | TGTCCCCCTGGATTCAGGCCC |
| chr3 | 58141907 | 58141927 | +/- | 21 | GTCCCCCTGGATTCAGGCCCT |
| chr3 | 58141908 | 58141928 | +/- | 21 | TCCCCCTGGATTCAGGCCCTG |
| chr3 | 58141909 | 58141929 | +/- | 21 | CCCCCTGGATTCAGGCCCTGG |
| chr3 | 58141910 | 58141930 | +/- | 21 | CCCCTGGATTCAGGCCCTGGG |
| chr3 | 58141911 | 58141931 | +/- | 21 | CCCTGGATTCAGGCCCTGGGT |
| chr3 | 58141912 | 58141932 | +/- | 21 | CCTGGATTCAGGCCCTGGGTA |
| chr3 | 58141913 | 58141933 | +/- | 21 | CTGGATTCAGGCCCTGGGTAC |
| chr3 | 58141914 | 58141934 | +/- | 21 | TGGATTCAGGCCCTGGGTACA |
| chr3 | 58141915 | 58141935 | +/- | 21 | GGATTCAGGCCCTGGGTACAA |
| chr3 | 58141916 | 58141936 | +/- | 21 | GATTCAGGCCCTGGGTACAAT |
| chr3 | 58141917 | 58141937 | +/- | 21 | ATTCAGGCCCTGGGTACAATT |
| chr3 | 58141918 | 58141938 | +/- | 21 | TTCAGGCCCTGGGTACAATTT |
| chr3 | 58141919 | 58141939 | +/- | 21 | TCAGGCCCTGGGTACAATTTT |
| chr3 | 58141920 | 58141940 | +/- | 21 | CAGGCCCTGGGTACAATTTTG |
| chr3 | 58141921 | 58141941 | +/- | 21 | AGGCCCTGGGTACAATTTTGG |
| chr3 | 58141922 | 58141942 | +/- | 21 | GGCCCTGGGTACAATTTTGGT |
| chr3 | 58141923 | 58141943 | +/- | 21 | GCCCTGGGTACAATTTTGGTT |
| chr3 | 58141924 | 58141944 | +/- | 21 | CCCTGGGTACAATTTTGGTTT |
| chr3 | 58141925 | 58141945 | +/- | 21 | CCTGGGTACAATTTTGGTTTT |
| chr3 | 58141926 | 58141946 | +/- | 21 | CTGGGTACAATTTTGGTTTTT |
| chr3 | 58141927 | 58141947 | +/- | 21 | TGGGTACAATTTTGGTTTTTT |
| chr3 | 58141928 | 58141948 | +/- | 21 | GGGTACAATTTTGGTTTTTTC |
| chr3 | 58141929 | 58141949 | +/- | 21 | GGTACAATTTTGGTTTTTTCC |
| chr3 | 58141930 | 58141950 | +/- | 21 | GTACAATTTTGGTTTTTTCCT |
| chr3 | 58141931 | 58141951 | +/- | 21 | TACAATTTTGGTTTTTTCCTT |
| chr3 | 58141932 | 58141952 | +/- | 21 | ACAATTTTGGTTTTTTCCTTT |
| chr3 | 58141933 | 58141953 | +/- | 21 | CAATTTTGGTTTTTTCCTTTT |
| chr3 | 58141934 | 58141954 | +/- | 21 | AATTTTGGTTTTTTCCTTTTT |
| chr3 | 58141935 | 58141955 | +/- | 21 | ATTTTGGTTTTTTCCTTTTTG |
| chr3 | 58141936 | 58141956 | +/- | 21 | TTTTGGTTTTTTCCTTTTTGT |
| chr3 | 58141937 | 58141957 | +/- | 21 | TTITGGTTTTTTCCTTTTTGTG |
| chr3 | 58141938 | 58141958 | +/- | 21 | TTGGTTTTTTCCTTTTTGTGT |
| chr3 | 58141939 | 58141959 | +/- | 21 | TGGTTTTTTCCTTTTTGTGTT |
| chr3 | 58141940 | 58141960 | +/- | 21 | GGTTTTTTCCTTTTTGTGTTT |
| chr3 | 58141941 | 58141961 | +/- | 21 | GTTTTTTCCTTTTTTGTGTTTC |
| chr3 | 58141942 | 58141962 | +/- | 21 | TTTTTTCCTTTTTGTGTTTCT |
| chr3 | 58141943 | 58141963 | +/- | 21 | TTTTTCCTTTTTGTGTTTCTG |
| chr3 | 58141944 | 58141964 | +/- | 21 | TTTTCCTTTTTGTGTTTCTGT |
| chr3 | 58141945 | 58141965 | +/- | 21 | TTTCCTTTTGTGTTTCTGTG |
| chr3 | 58141946 | 58141966 | +/- | 21 | TTCCTTTTTGTGTTTCTGTGT |
| chr3 | 58141947 | 58141967 | +/- | 21 | TCCTTTTTIGTGTTTCTGTGTT |
| chr3 | 58141948 | 58141968 | +/- | 21 | CCTTTTTGTGTTTCTGTGTTT |
| chr3 | 58141949 | 58141969 | +/- | 21 | CTTTTIGTGTTTCTGTGTTTA |
| chr3 | 58141950 | 58141970 | +/- | 21 | TTTTTGTGTTTCTGTGTTTAC |
| chr3 | 58141951 | 58141971 | +/- | 21 | TTTTGTGTTTCTGTGTTTACT |
| chr3 | 58141952 | 58141972 | +/- | 21 | TTTGTGTTTCTGTGTTTACTC |
| chr3 | 58141953 | 58141973 | +/- | 21 | TTGTGTTTCTGTGTTTACTCA |
| chr3 | 58141954 | 58141974 | +/- | 21 | TGTGTTTCTGTGTTTACTCAG |
| chr3 | 58141955 | 58141975 | +/- | 21 | GTGTTTCTGTGTTTACTCAGC |
| chr3 | 58141956 | 58141976 | +/- | 21 | TGTTTCTGTGTTTACTCAGCC |
| chr3 | 58141957 | 58141977 | +/- | 21 | GTTTCTGTGTTTACTCAGCCT |
| chr3 | 58141958 | 58141978 | +/- | 21 | TTTCTGTGTTTACTCAGCCTT |
| chr3 | 58141959 | 58141979 | +/- | 21 | TTCTGTGTTTACTCAGCCTTC |
| chr3 | 58141960 | 58141980 | +/- | 21 | TCTGTGTTTACTCAGCCTTCA |
| chr3 | 58141961 | 58141981 | +/- | 21 | CTGTGTTTACTCAGCCTTCAT |
| chr3 | 58141962 | 58141982 | +/- | 21 | TGTGTTTACTCAGCCTTCATT |
| chr3 | 58141963 | 58141983 | +/- | 21 | GTGTTTACTCAGCCTTCATTT |
| chr3 | 58141964 | 58141984 | +/- | 21 | TGTTTACTCAGCCTTCATTTC |
| chr3 | 58141965 | 58141985 | +/- | 21 | GTTTACTCAGCCTTCATTTCA |
| chr3 | 58141966 | 58141986 | +/- | 21 | TTTACTCAGCCTTCATTTCAG |
| chr3 | 58141967 | 58141987 | +/- | 21 | TTACTCAGCCTTCATTTCAGA |
| chr3 | 58141968 | 58141988 | +/- | 21 | TACTCAGCCTTCATTTCAGAA |
| chr3 | 58141969 | 58141989 | +/- | 21 | ACTCAGCCTTCATTTCAGAAA |
| chr3 | 58141970 | 58141990 | +/- | 21 | CTCAGCCTTCATTTCAGAAAA |
| chr3 | 58141971 | 58141991 | +/- | 21 | TCAGCCTTCATTTCAGAAAAT |
| chr3 | 58141972 | 58141992 | +/- | 21 | CAGCCTTCATTTCAGAAAATC |
| chr3 | 58141973 | 58141993 | +/- | 21 | AGCCTTCATTTCAGAAAATCT |
| chr3 | 58141974 | 58141994 | +/- | 21 | GCCTTCATTTCAGAAAATCTG |
| chr3 | 58141975 | 58141995 | +/- | 21 | CCTTCATTTCAGAAAATCTGC |
| chr3 | 58141976 | 58141996 | +/- | 21 | CTTCATTTCAGAAAATCTGCC |
| chr3 | 58141977 | 58141997 | +/- | 21 | TTCATTTCAGAAAATCTGCCA |
| chr3 | 58141978 | 58141998 | +/- | 21 | TCATTTCAGAAAATCTGCCAT |
| chr3 | 58141979 | 58141999 | +/- | 21 | CATTTCAGAAAATCTGCCATC |
| chr3 | 58141980 | 58142000 | +/- | 21 | ATTTCAGAAAATCTGCCATCT |
| chr3 | 58141981 | 58142001 | +/- | 21 | TTTCAGAAAATCTGCCATCTG |
| chr3 | 58141982 | 58142002 | +/- | 21 | TTCAGAAAATCTGCCATCTGC |
| chr3 | 58141983 | 58142003 | +/- | 21 | TCAGAAAATCTGCCATCTGCT |
| chr3 | 58141984 | 58142004 | +/- | 21 | CAGAAAATCTGCCATCTGCTT |
| chr3 | 58141985 | 58142005 | +/- | 21 | AGAAAATCTGCCATCTGCTTC |
| chr3 | 58141986 | 58142006 | +/- | 21 | GAAAATCTGCCATCTGCTTCT |
| chr3 | 58141987 | 58142007 | +/- | 21 | AAAATCTGCCATCTGCTTCTG |
| chr3 | 58141988 | 58142008 | +/- | 21 | AAATCTGCCATCTGCTTCTGG |
| chr3 | 58141989 | 58142009 | +/- | 21 | AATCTGCCATCTGCTTCTGGG |
| chr3 | 58141990 | 58142010 | +/- | 21 | ATCTGCCATCTGCTTCTGGGA |
| chr3 | 58141991 | 58142011 | +/- | 21 | TCTGCCATCTGCTTCTGGGAT |
| chr3 | 58141992 | 58142012 | +/- | 21 | CTG CCATCTG CTTCTGG GATT |
| chr3 | 58141993 | 58142013 | +/- | 21 | TGCCATCTGCTTCTGGGATTG |
| chr3 | 58141994 | 58142014 | +/- | 21 | GCCATCTGCTTCTGGGATTGC |
| chr3 | 58141995 | 58142015 | +/- | 21 | CCATCTGCTTCTGGGATTGCT |
| chr3 | 58141996 | 58142016 | +/- | 21 | CATCTGCTTCTGGGATTGCTT |
| chr3 | 58141997 | 58142017 | +/- | 21 | ATCTGCTTCTGGGATTGCTTA |
| chr3 | 58141998 | 58142018 | +/- | 21 | TCTGCTTCTGGGATTGCTTAA |
| chr3 | 58141999 | 58142019 | +/- | 21 | CTGCTTCTGGGATTGCTTAAG |
| chr3 | 58142000 | 58142020 | +/- | 21 | TGCTTCTGGGATTGCTTAAGC |
| chr3 | 58142001 | 58142021 | +/- | 21 | GCTTCTGGGATTGCTTAAGCC |
| chr3 | 58142002 | 58142022 | +/- | 21 | CTTCTGGGATTGCTTAAGCCC |
| chr3 | 58142003 | 58142023 | +/- | 21 | TTCTGGGATTGCTTAAGCCCT |
| chr3 | 58142004 | 58142024 | +/- | 21 | TCTGGGATTGCTTAAGCCCTG |
| chr3 | 58142005 | 58142025 | +/- | 21 | CTGGGATTGCTTAAGCCCTGT |
| chr3 | 58142006 | 58142026 | +/- | 21 | TGGGATTGCTTAAGCCCTGTG |
| chr3 | 58142007 | 58142027 | +/- | 21 | GGGATTGCTTAAGCCCTGTGG |
| chr3 | 58142008 | 58142028 | +/- | 21 | GGATTGCTTAAGCCCTGTGGG |
| chr3 | 58142009 | 58142029 | +/- | 21 | GATTGCTTAAGCCCTGTGGGT |
| chr3 | 58142010 | 58142030 | +/- | 21 | ATTGCTTAAGCCCTGTGGGTG |
| chr3 | 58142011 | 58142031 | +/- | 21 | TTGCTTAAGCCCTGTGGGTGT |
| chr3 | 58142012 | 58142032 | +/- | 21 | TGCTTAAGCCCTGTGGGTGTC |
| chr3 | 58142013 | 58142033 | +/- | 21 | GCTTAAGCCCTGTGGGTGTCC |
| chr3 | 58142014 | 58142034 | +/- | 21 | CTTAAGCCCTGTGGGTGTCCT |
| chr3 | 58142015 | 58142035 | +/- | 21 | TTAAGCCCTGTGGGTGTCCTG |
| chr3 | 58142016 | 58142036 | +/- | 21 | TAAGCCCTGTGGGTGTCCTGG |
| chr3 | 58142017 | 58142037 | +/- | 21 | AAGCCCTGTGGGTGTCCTGGT |
| chr3 | 58142018 | 58142038 | +/- | 21 | AGCCCTGTGGGTGTCCTGGTC |
| chr3 | 58142019 | 58142039 | +/- | 21 | GCCCTGTGGGTGTCCTGGTCA |
| chr3 | 58142020 | 58142040 | +/- | 21 | CCCTGTGGGTGTCCTGGTCAT |
| chr3 | 58142021 | 58142041 | +/- | 21 | CCTGTGGGTGTCCTGGTCATT |
| chr3 | 58142022 | 58142042 | +/- | 21 | CTGTGGGTGTCCTGGTCATTG |
| chr3 | 58142023 | 58142043 | +/- | 21 | TGTGGGTGTCCTGGTCATTGG |
| chr3 | 58142024 | 58142044 | +/- | 21 | GTGGGTGTCCTGGTCATTGGT |

**Table 8: 22-mer target-specific ASOs**

| **CHR** | **START** | **END** | **STRAND** | **kmer** | **SEQUENCE** |
|---|---|---|---|---|---|
| chr3 | 58141828 | 58141849 | +/- | 22 | CCCAACTAATCTCCATTTGCCA |
| chr3 | 58141829 | 58141850 | +/- | 22 | CCAACTAATCTCCATTTGCCAC |
| chr3 | 58141830 | 58141851 | +/- | 22 | CAACTAATCTCCATTTGCCACT |
| chr3 | 58141831 | 58141852 | +/- | 22 | AACTAATCTCCATTTGCCACTG |
| chr3 | 58141832 | 58141853 | +/- | 22 | ACTAATCTCCATTTGCCACTGA |
| chr3 | 58141833 | 58141854 | +/- | 22 | CTAATCTCCATTTGCCACTGAC |
| chr3 | 58141834 | 58141855 | +/- | 22 | TAATCTCCATTTGCCACTGACC |
| chr3 | 58141835 | 58141856 | +/- | 22 | AATCTCCATTTGCCACTGACCA |
| chr3 | 58141836 | 58141857 | +/- | 22 | ATCTCCATTTGCCACTGACCAG |
| chr3 | 58141837 | 58141858 | +/- | 22 | TCTCCATTTGCCACTGACCAGG |
| chr3 | 58141838 | 58141859 | +/- | 22 | CTCCATTTGCCACTGACCAGGC |
| chr3 | 58141839 | 58141860 | +/- | 22 | TCCATTTGCCACTGACCAGGCC |
| chr3 | 58141840 | 58141861 | +/- | 22 | CCATTTGCCACTGACCAGGCCA |
| chr3 | 58141841 | 58141862 | +/- | 22 | CATTTGCCACTGACCAGGCCAC |
| chr3 | 58141842 | 58141863 | +/- | 22 | ATTTGCCACTGACCAGGCCACA |
| chr3 | 58141843 | 58141864 | +/- | 22 | TTTGCCACTGACCAGGCCACAG |
| chr3 | 58141844 | 58141865 | +/- | 22 | TTGCCACTGACCAGGCCACAGA |
| chr3 | 58141845 | 58141866 | +/- | 22 | TGCCACTGACCAGGCCACAGAT |
| chr3 | 58141846 | 58141867 | +/- | 22 | GCCACTGACCAGGCCACAGATG |
| chr3 | 58141847 | 58141868 | +/- | 22 | CCACTGACCAGGCCACAGATGG |
| chr3 | 58141848 | 58141869 | +/- | 22 | CACTGACCAGGCCACAGATGGG |
| chr3 | 58141849 | 58141870 | +/- | 22 | ACTGACCAGGCCACAGATGGGG |
| chr3 | 58141850 | 58141871 | +/- | 22 | CTGACCAGGCCACAGATGGGGA |
| chr3 | 58141851 | 58141872 | +/- | 22 | TGACCAGGCCACAGATGGGGAA |
| chr3 | 58141852 | 58141873 | +/- | 22 | GACCAGGCCACAGATGGGGAAG |
| chr3 | 58141853 | 58141874 | +/- | 22 | ACCAGGCCACAGATGGGGAAGT |
| chr3 | 58141854 | 58141875 | +/- | 22 | CCAGGCCACAGATGGGGAAGTC |
| chr3 | 58141855 | 58141876 | +/- | 22 | CAGGCCACAGATGGGGAAGTCA |
| chr3 | 58141856 | 58141877 | +/- | 22 | AGGCCACAGATGGGGAAGTCAC |
| chr3 | 58141857 | 58141878 | +/- | 22 | GGCCACAGATGGGGAAGTCACA |
| chr3 | 58141858 | 58141879 | +/- | 22 | GCCACAGATGGGGAAGTCACAG |
| chr3 | 58141859 | 58141880 | +/- | 22 | CCACAGATGGGGAAGTCACAGC |
| chr3 | 58141860 | 58141881 | +/- | 22 | CACAGATGGGGAAGTCACAGCC |
| chr3 | 58141861 | 58141882 | +/- | 22 | ACAGATGGGGAAGTCACAGCCG |
| chr3 | 58141862 | 58141883 | +/- | 22 | CAGATGGGGAAGTCACAGCCGT |
| chr3 | 58141863 | 58141884 | +/- | 22 | AGATGGGGAAGTCACAGCCGTG |
| chr3 | 58141864 | 58141885 | +/- | 22 | GATGGGGAAGTCACAGCCGTGG |
| chr3 | 58141865 | 58141886 | +/- | 22 | ATGGGGAAGTCACAGCCGTGGA |
| chr3 | 58141866 | 58141887 | +/- | 22 | TGGGGAAGTCACAGCCGTGGAG |
| chr3 | 58141867 | 58141888 | +/- | 22 | GGGGAAGTCACAGCCGTGGAGG |
| chr3 | 58141868 | 58141889 | +/- | 22 | GGGAAGTCACAGCCGTGGAGGA |
| chr3 | 58141869 | 58141890 | +/- | 22 | GGAAGTCACAGCCGTGGAGGAG |
| chr3 | 58141870 | 58141891 | +/- | 22 | GAAGTCACAGCCGTGGAGGAGG |
| chr3 | 58141871 | 58141892 | +/- | 22 | AAGTCACAGCCGTGGAGGAGGC |
| chr3 | 58141872 | 58141893 | +/- | 22 | AGTCACAGCCGTGGAGGAGGCA |
| chr3 | 58141873 | 58141894 | +/- | 22 | GTCACAGCCGTGGAGGAGGCAC |
| chr3 | 58141874 | 58141895 | +/- | 22 | TCACAGCCGTGGAGGAGGCACC |
| chr3 | 58141875 | 58141896 | +/- | 22 | CACAGCCGTGGAGGAGGCACCG |
| chr3 | 58141876 | 58141897 | +/- | 22 | ACAGCCGTGGAGGAGGCACCGG |
| chr3 | 58141877 | 58141898 | +/- | 22 | CAGCCGTGGAGGAGGCACCGGT |
| chr3 | 58141878 | 58141899 | +/- | 22 | AGCCGTGGAGGAGGCACCGGTA |
| chr3 | 58141879 | 58141900 | +/- | 22 | GCCGTGGAGGAGGCACCGGTAA |
| chr3 | 58141880 | 58141901 | +/- | 22 | CCGTGGAGGAGGCACCGGTAAA |
| chr3 | 58141881 | 58141902 | +/- | 22 | CGTGGAGGAGGCACCGGTAAAT |
| chr3 | 58141882 | 58141903 | +/- | 22 | GTGGAGGAGGCACCGGTAAATG |
| chr3 | 58141883 | 58141904 | +/- | 22 | TGGAGGAGGCACCGGTAAATGC |
| chr3 | 58141884 | 58141905 | +/- | 22 | GGAGGAGGCACCGGTAAATGCA |
| chr3 | 58141885 | 58141906 | +/- | 22 | GAGGAGGCACCGGTAAATGCAT |
| chr3 | 58141886 | 58141907 | +/- | 22 | AGGAGGCACCGGTAAATGCATG |
| chr3 | 58141887 | 58141908 | +/- | 22 | GGAGGCACCGGTAAATGCATGT |
| chr3 | 58141888 | 58141909 | +/- | 22 | GAGGCACCGGTAAATGCATGTC |
| chr3 | 58141889 | 58141910 | +/- | 22 | AGGCACCGGTAAATGCATGTCC |
| chr3 | 58141890 | 58141911 | +/- | 22 | GGCACCGGTAAATGCATGTCCC |
| chr3 | 58141891 | 58141912 | +/- | 22 | GCACCGGTAAATGCATGTCCCC |
| chr3 | 58141892 | 58141913 | +/- | 22 | CACCGGTAAATGCATGTCCCCC |
| chr3 | 58141893 | 58141914 | +/- | 22 | ACCGGTAAATGCATGTCCCCCT |
| chr3 | 58141894 | 58141915 | +/- | 22 | CCGGTAAATGCATGTCCCCCTG |
| chr3 | 58141895 | 58141916 | +/- | 22 | CGGTAAATGCATGTCCCCCTGG |
| chr3 | 58141896 | 58141917 | +/- | 22 | GGTAAATGCATGTCCCCCTGGA |
| chr3 | 58141897 | 58141918 | +/- | 22 | GTAAATG CATGTCCCCCTG GAT |
| chr3 | 58141898 | 58141919 | +/- | 22 | TAAATGCATGTCCCCCTGGATT |
| chr3 | 58141899 | 58141920 | +/- | 22 | AAATGCATGTCCCCCTGGATTC |
| chr3 | 58141900 | 58141921 | +/- | 22 | AATGCATGTCCCCCTGGATTCA |
| chr3 | 58141901 | 58141922 | +/- | 22 | ATGCATGTCCCCCTGGATTCAG |
| chr3 | 58141902 | 58141923 | +/- | 22 | TGCATGTCCCCCTGGATTCAGG |
| chr3 | 58141903 | 58141924 | +/- | 22 | GCATGTCCCCCTGGATTCAGGC |
| chr3 | 58141904 | 58141925 | +/- | 22 | CATGTCCCCCTGGATTCAGGCC |
| chr3 | 58141905 | 58141926 | +/- | 22 | ATGTCCCCCTGGATTCAGGCCC |
| chr3 | 58141906 | 58141927 | +/- | 22 | TGTCCCCCTGGATTCAGGCCCT |
| chr3 | 58141907 | 58141928 | +/- | 22 | GTCCCCCTGGATTCAGGCCCTG |
| chr3 | 58141908 | 58141929 | +/- | 22 | TCCCCCTGGATTCAGGCCCTGG |
| chr3 | 58141909 | 58141930 | +/- | 22 | CCCCCTGGATTCAGGCCCTGGG |
| chr3 | 58141910 | 58141931 | +/- | 22 | CCCCTGGATTCAGGCCCTGGGT |
| chr3 | 58141911 | 58141932 | +/- | 22 | CCCTGGATTCAGGCCCTGGGTA |
| chr3 | 58141912 | 58141933 | +/- | 22 | CCTGGATTCAGGCCCTGGGTAC |
| chr3 | 58141913 | 58141934 | +/- | 22 | CTGGATTCAGGCCCTGGGTACA |
| chr3 | 58141914 | 58141935 | +/- | 22 | TGGATTCAGGCCCTGGGTACAA |
| chr3 | 58141915 | 58141936 | +/- | 22 | GGATTCAGGCCCTGGGTACAAT |
| chr3 | 58141916 | 58141937 | +/- | 22 | GATTCAGGCCCTGGGTACAATT |
| chr3 | 58141917 | 58141938 | +/- | 22 | ATTCAGGCCCTGGGTACAATTT |
| chr3 | 58141918 | 58141939 | +/- | 22 | TTCAGGCCCTGGGTACAATTTT |
| chr3 | 58141919 | 58141940 | +/- | 22 | TCAGGCCCTGGGTACAATTTTG |
| chr3 | 58141920 | 58141941 | +/- | 22 | CAGGCCCTGGGTACAATTTTGG |
| chr3 | 58141921 | 58141942 | +/- | 22 | AGGCCCTGGGTACAATTTTGGT |
| chr3 | 58141922 | 58141943 | +/- | 22 | GGCCCTGGGTACAATTTTGGTT |
| chr3 | 58141923 | 58141944 | +/- | 22 | GCCCTGGGTACAATTTTGGTTT |
| chr3 | 58141924 | 58141945 | +/- | 22 | CCCTGGGTACAATTTTGGTTTT |
| chr3 | 58141925 | 58141946 | +/- | 22 | CCTGGGTACAATTTTGGTTTTT |
| chr3 | 58141926 | 58141947 | +/- | 22 | CTGGGTACAATTTTGGTTTTTT |
| chr3 | 58141927 | 58141948 | +/- | 22 | TGGGTACAATTTTGGTTTTTTC |
| chr3 | 58141928 | 58141949 | +/- | 22 | GGGTACAATTTTGGTTTTTTCC |
| chr3 | 58141929 | 58141950 | +/- | 22 | G GTACAATTTTG VIIIIIICCT |
| chr3 | 58141930 | 58141951 | +/- | 22 | GTACAATTTTGVTTTTTTCCTT |
| chr3 | 58141931 | 58141952 | +/- | 22 | TACAATTTTGGTTTTTTCCTTT |
| chr3 | 58141932 | 58141953 | +/- | 22 | ACAATTTTGGTTTTTTCCTTTT |
| chr3 | 58141933 | 58141954 | +/- | 22 | CAATTTTGGTTTTTTCCTTTTT |
| chr3 | 58141934 | 58141955 | +/- | 22 | AATTTTGGTTTTTTCCTTTTTG |
| chr3 | 58141935 | 58141956 | +/- | 22 | ATTTTGGTTTTTTCCTTTTTTGT |
| chr3 | 58141936 | 58141957 | +/- | 22 | TTTTGGTTTTTTCCTTTTTGTG |
| chr3 | 58141937 | 58141958 | +/- | 22 | TTTGGTTTTTTCCTTTTTGTGT |
| chr3 | 58141938 | 58141959 | +/- | 22 | TTGGTTTTTTCCTTTTIGTGTT |
| chr3 | 58141939 | 58141960 | +/- | 22 | TGGTTTTTTCCTTTTTGTGTTT |
| chr3 | 58141940 | 58141961 | +/- | 22 | GGI TTTTICCI TTTIGTGTTTC |
| chr3 | 58141941 | 58141962 | +/- | 22 | GTTTTTTCCTTTTTGTGTTTCT |
| chr3 | 58141942 | 58141963 | +/- | 22 | TTTTTTCCTTTTTGTGTTTCTG |
| chr3 | 58141943 | 58141964 | +/- | 22 | TTTTTCCTTTTTGTGTTTCTGT |
| chr3 | 58141944 | 58141965 | +/- | 22 | TTTTCCTTTTIGTGTTTCTGTG |
| chr3 | 58141945 | 58141966 | +/- | 22 | TTTCCTTTTTGTGTTTCTGTGT |
| chr3 | 58141946 | 58141967 | +/- | 22 | TTCCTTTTTGTGTTTCTGTGTT |
| chr3 | 58141947 | 58141968 | +/- | 22 | TCCTTTTTGTGTTTCTGTGTTT |
| chr3 | 58141948 | 58141969 | +/- | 22 | CCTTTTTGTGTTTCTGTGTTTA |
| chr3 | 58141949 | 58141970 | +/- | 22 | CTTTTTGTGTTTCTGTGTTTAC |
| chr3 | 58141950 | 58141971 | +/- | 22 | TTTTTGTGTTTCTGTGTTTACT |
| chr3 | 58141951 | 58141972 | +/- | 22 | TTTTGTGTTTCTGTGTTTACTC |
| chr3 | 58141952 | 58141973 | +/- | 22 | TTTGTGTTTCTGTGTTTACTCA |
| chr3 | 58141953 | 58141974 | +/- | 22 | TTGTGTTTCTGTGTTTACTCAG |
| chr3 | 58141954 | 58141975 | +/- | 22 | TGTGTTTCTGTGTTTACTCAGC |
| chr3 | 58141955 | 58141976 | +/- | 22 | GTGTTTCTGTGTTTACTCAGCC |
| chr3 | 58141956 | 58141977 | +/- | 22 | TGTTTCTGTGTTTACTCAGCCT |
| chr3 | 58141957 | 58141978 | +/- | 22 | GTTTCTGTGTTTACTCAGCCTT |
| chr3 | 58141958 | 58141979 | +/- | 22 | TTTCTGTGTTTACTCAGCCTTC |
| chr3 | 58141959 | 58141980 | +/- | 22 | TTCTGTGTTTACTCAGCCTTCA |
| chr3 | 58141960 | 58141981 | +/- | 22 | TCTGTGTTTACTCAGCCTTCAT |
| chr3 | 58141961 | 58141982 | +/- | 22 | CTGTGTTTACTCAGCCTTCATT |
| chr3 | 58141962 | 58141983 | +/- | 22 | TGTGTTTACTCAGCCTTCATTT |
| chr3 | 58141963 | 58141984 | +/- | 22 | GTGTTTACTCAGCCTTCATTTC |
| chr3 | 58141964 | 58141985 | +/- | 22 | TGTTTACTCAGCCTTCATTTCA |
| chr3 | 58141965 | 58141986 | +/- | 22 | GTTTACTCAGCCTTCATTTCAG |
| chr3 | 58141966 | 58141987 | +/- | 22 | TTTACTCAGCCTTCATTTCAGA |
| chr3 | 58141967 | 58141988 | +/- | 22 | TTACTCAGCCTTCATTTCAGAA |
| chr3 | 58141968 | 58141989 | +/- | 22 | TACTCAGCCTTCATTTCAGAAA |
| chr3 | 58141969 | 58141990 | +/- | 22 | ACTCAGCCTTCATTTCAGAAAA |
| chr3 | 58141970 | 58141991 | +/- | 22 | CTCAGCCTTCATTTCAGAAAAT |
| chr3 | 58141971 | 58141992 | +/- | 22 | TCAGCCTTCATTTCAGAAAATC |
| chr3 | 58141972 | 58141993 | +/- | 22 | CAGCCTTCATTTCAGAAAATCT |
| chr3 | 58141973 | 58141994 | +/- | 22 | AGCCTTCATTTCAGAAAATCTG |
| chr3 | 58141974 | 58141995 | +/- | 22 | GCCTTCATTTCAGAAAATCTGC |
| chr3 | 58141975 | 58141996 | +/- | 22 | CCTTCATTTCAGAAAATCTGCC |
| chr3 | 58141976 | 58141997 | +/- | 22 | CTTCATTTCAGAAAATCTGCCA |
| chr3 | 58141977 | 58141998 | +/- | 22 | TTCATTTCAGAAAATCTGCCAT |
| chr3 | 58141978 | 58141999 | +/- | 22 | TCATTTCAGAAAATCTGCCATC |
| chr3 | 58141979 | 58142000 | +/- | 22 | CATTTCAGAAAATCTGCCATCT |
| chr3 | 58141980 | 58142001 | +/- | 22 | ATTTCAGAAAATCTGCCATCTG |
| chr3 | 58141981 | 58142002 | +/- | 22 | TTTCAGAAAATCTGCCATCTGC |
| chr3 | 58141982 | 58142003 | +/- | 22 | TTCAGAAAATCTGCCATCTGCT |
| chr3 | 58141983 | 58142004 | +/- | 22 | TCAGAAAATCTGCCATCTGCTT |
| chr3 | 58141984 | 58142005 | +/- | 22 | CAGAAAATCTGCCATCTGCTTC |
| chr3 | 58141985 | 58142006 | +/- | 22 | AGAAAATCTGCCATCTGCTTCT |
| chr3 | 58141986 | 58142007 | +/- | 22 | GAAAATCTGCCATCTGCTTCTG |
| chr3 | 58141987 | 58142008 | +/- | 22 | AAAATCTGCCATCTGCTTCTGG |
| chr3 | 58141988 | 58142009 | +/- | 22 | AAATCTGCCATCTGCTTCTGGG |
| chr3 | 58141989 | 58142010 | +/- | 22 | AATCTGCCATCTGCTTCTGGGA |
| chr3 | 58141990 | 58142011 | +/- | 22 | ATCTGCCATCTGCTTCTGGGAT |
| chr3 | 58141991 | 58142012 | +/- | 22 | TCTGCCATCTGCTTCTGGGATT |
| chr3 | 58141992 | 58142013 | +/- | 22 | CTGCCATCTGCTTCTGGGATTG |
| chr3 | 58141993 | 58142014 | +/- | 22 | TGCCATCTGCTTCTGGGATTGC |
| chr3 | 58141994 | 58142015 | +/- | 22 | GCCATCTGCTTCTGGGATTGCT |
| chr3 | 58141995 | 58142016 | +/- | 22 | CCATCTGCTTCTGGGATTGCTT |
| chr3 | 58141996 | 58142017 | +/- | 22 | CATCTGCTTCTGGGATTGCTTA |
| chr3 | 58141997 | 58142018 | +/- | 22 | ATCTGCTTCTGGGATTGCTTAA |
| chr3 | 58141998 | 58142019 | +/- | 22 | TCTGCTTCTGGGATTGCTTAAG |
| chr3 | 58141999 | 58142020 | +/- | 22 | CTGCTTCTGGGATTGCTTAAGC |
| chr3 | 58142000 | 58142021 | +/- | 22 | TGCTTCTGGGATTGCTTAAGCC |
| chr3 | 58142001 | 58142022 | +/- | 22 | GCTTCTGGGATTGCTTAAGCCC |
| chr3 | 58142002 | 58142023 | +/- | 22 | CTTCTGGGATTGCTTAAGCCCT |
| chr3 | 58142003 | 58142024 | +/- | 22 | TTCTGGGATTGCTTAAGCCCTG |
| chr3 | 58142004 | 58142025 | +/- | 22 | TCTGGGATTGCTTAAGCCCTGT |
| chr3 | 58142005 | 58142026 | +/- | 22 | CTGGGATTGCTTAAGCCCTGTG |
| chr3 | 58142006 | 58142027 | +/- | 22 | TGGGATTGCTTAAGCCCTGTGG |
| chr3 | 58142007 | 58142028 | +/- | 22 | GGGATTGCTTAAGCCCTGTGGG |
| chr3 | 58142008 | 58142029 | +/- | 22 | GGATTGCTTAAGCCCTGTGGGT |
| chr3 | 58142009 | 58142030 | +/- | 22 | GATTGCTTAAGCCCTGTGGGTG |
| chr3 | 58142010 | 58142031 | +/- | 22 | ATTGCTTAAGCCCTGTGGGTGT |
| chr3 | 58142011 | 58142032 | +/- | 22 | TTGCTTAAGCCCTGTGGGTGTC |
| chr3 | 58142012 | 58142033 | +/- | 22 | TGCTTAAGCCCTGTGGGTGTCC |
| chr3 | 58142013 | 58142034 | +/- | 22 | GCTTAAGCCCTGTGGGTGTCCT |
| chr3 | 58142014 | 58142035 | +/- | 22 | CTTAAGCCCTGTGGGTGTCCTG |
| chr3 | 58142015 | 58142036 | +/- | 22 | TTAAGCCCTGTGGGTGTCCTGG |
| chr3 | 58142016 | 58142037 | +/- | 22 | TAAGCCCTGTGGGTGTCCTGGT |
| chr3 | 58142017 | 58142038 | +/- | 22 | AAGCCCTGTGGGTGTCCTGGTC |
| chr3 | 58142018 | 58142039 | +/- | 22 | AGCCCTGTGGGTGTCCTGGTCA |
| chr3 | 58142019 | 58142040 | +/- | 22 | GCCCTGTGGGTGTCCTGGTCAT |
| chr3 | 58142020 | 58142041 | +/- | 22 | CCCTGTGGGTGTCCTGGTCATT |
| chr3 | 58142021 | 58142042 | +/- | 22 | CCTGTGGGTGTCCTGGTCATTG |
| chr3 | 58142022 | 58142043 | +/- | 22 | CTGTGGGTGTCCTGGTCATTGG |
| chr3 | 58142023 | 58142044 | +/- | 22 | TGTGGGTGTCCTGGTCATTGGT |

Tables 9-15 comprise some additional example oligonucleotide sequences of variable sequence lengths that may be used to induce an isoform switch or modulate (e.g., inhibit or enhance) the biological activity of a specific isoform of one or several of the genes described above or elsewhere herein (e.g., genes comprising one or more of NEDD4L (ENV2), MAP3K7 (ENV3), NFYA (ENV11), ESYT2 (ENV21), MARK2 (ENV18), ST7 (ENV19), ARVCF (ENV22), SYTL2 (ENV17), R3HDM1 (ENV23), COL4A3BP (ENV9), TANGO2 (ENV6), SEPT9 (ENV15), ROBO1 (ENV4), FAM122B (ENV5), CD47 (ENV13), LSR (ENV20), PBX1 (ENV16), EPB41 (ENV14), ADAM15 (ENV7), EPB41L1 (ENV8), ABIl (ENV10), FLNB (ENV1), CTNND1 (ENV12), GPR160 (ENV24), ITGB3BP (ENV25), INCENP (ENV26), DENND1B (ENV27), CA12 (ENV28).

As discussed above, oligonucleotide sequences comprised in a table may be specific for a single target, or for more than one target. In some cases, oligonucleotide sequences comprised in more than one tables are specific for a single target. For example, oligonucleotide sequences comprised in Tables 9-15 may be specific for a single target. The target may be the same as or differ from the target which the oligonucleotide sequences comprised in Tables 2-8 are specific for. The target may be a gene selected from genes described above or elsewhere herein.

**Table 9: 16-mer target-specific**

| **CHR** | **START** | **END** | **STRAND** | **Kmer** | **SEQUENCE** |
|---|---|---|---|---|---|
| chr18 | 58335318 | 58335333 | +/- | 16 | GGTCATCAGCGACTGC |
| chr18 | 58335319 | 58335334 | +/- | 16 | GTCATCAGCGACTGCT |
| chr18 | 58335320 | 58335335 | +/- | 16 | TCATCAGCGACTGCTG |
| chr18 | 58335321 | 58335336 | +/- | 16 | CATCAGCGACTGCTGG |
| chr18 | 58335322 | 58335337 | +/- | 16 | ATCAGCGACTGCTGGC |
| chr18 | 58335323 | 58335338 | +/- | 16 | TCAGCGACTGCTGGCT |
| chr18 | 58335324 | 58335339 | +/- | 16 | CAGCGACTGCTGGCTT |
| chr18 | 58335325 | 58335340 | +/- | 16 | AGCGACTGCTGGCTTT |
| chr18 | 58335326 | 58335341 | +/- | 16 | GCGACTGCTGGCTTTG |
| chr18 | 58335327 | 58335342 | +/- | 16 | CGACTGCTGGCTTTGT |
| chr18 | 58335328 | 58335343 | +/- | 16 | GACTGCTGGCTTTGTC |
| chr18 | 58335329 | 58335344 | +/- | 16 | ACTGCTGGCTTTGTCT |
| chr18 | 58335330 | 58335345 | +/- | 16 | CTGCTGGCTTTGTCTG |
| chr18 | 58335331 | 58335346 | +/- | 16 | TGCTGGCTTTGTCTGG |
| chr18 | 58335332 | 58335347 | +/- | 16 | GCTGGCTTTGTCTGGA |
| chr18 | 58335333 | 58335348 | +/- | 16 | CTGGCTTTGTCTGGAT |
| chr18 | 58335334 | 58335349 | +/- | 16 | TGGCTTTGTCTGGATA |
| chr18 | 58335335 | 58335350 | +/- | 16 | GGCTTTGTCTGGATAG |
| chr18 | 58335336 | 58335351 | +/- | 16 | GCTTTGTCTGGATAGG |
| chr18 | 58335337 | 58335352 | +/- | 16 | CTTTGTCTGGATAGGG |
| chr18 | 58335338 | 58335353 | +/- | 16 | TTTGTCTGGATAGGGT |
| chr18 | 58335339 | 58335354 | +/- | 16 | TTGTCTGGATAGGGTG |
| chr18 | 58335340 | 58335355 | +/- | 16 | TGTCTGGATAGGGTGG |
| chr18 | 58335341 | 58335356 | +/- | 16 | GTCTGGATAGGGTGGG |
| chr18 | 58335342 | 58335357 | +/- | 16 | TCTGGATAGGGTGGGT |
| chr18 | 58335343 | 58335358 | +/- | 16 | CTGGATAGGGTGGGTT |
| chr18 | 58335344 | 58335359 | +/- | 16 | TGGATAGGGTGGGTTT |
| chr18 | 58335345 | 58335360 | +/- | 16 | GGATAGGGTGGGTTTC |
| chr18 | 58335346 | 58335361 | +/- | 16 | GATAGGGTGGGTTTCA |
| chr18 | 58335347 | 58335362 | +/- | 16 | ATAGGGTGGGTTTCAG |
| chr18 | 58335348 | 58335363 | +/- | 16 | TAGGGTGGGTTTCAGG |
| chr18 | 58335349 | 58335364 | +/- | 16 | AGGGTGGGTTTCAGGG |
| chr18 | 58335350 | 58335365 | +/- | 16 | GGGTGGGTTTCAGGGA |
| chr18 | 58335351 | 58335366 | +/- | 16 | GGTGGGTTTCAGGGAT |
| chr18 | 58335352 | 58335367 | +/- | 16 | GTGGGTTTCAGGGATT |
| chr18 | 58335353 | 58335368 | +/- | 16 | TGGGTTTCAGGGATTC |
| chr18 | 58335354 | 58335369 | +/- | 16 | GGGTTTCAGGGATTCT |
| chr18 | 58335355 | 58335370 | +/- | 16 | GGTTTCAGGGATTCTG |
| chr18 | 58335356 | 58335371 | +/- | 16 | GTTTCAGGGATTCTGA |
| chr18 | 58335357 | 58335372 | +/- | 16 | TTTCAGGGATTCTGAT |
| chr18 | 58335358 | 58335373 | +/- | 16 | TTCAGGGATTCTGATC |
| chr18 | 58335359 | 58335374 | +/- | 16 | TCAGGGATTCTGATCT |
| chr18 | 58335360 | 58335375 | +/- | 16 | CAGGGATTCTGATCTC |
| chr18 | 58335361 | 58335376 | +/- | 16 | AGGGATTCTGATCTCA |
| chr18 | 58335362 | 58335377 | +/- | 16 | GGGATTCTGATCTCAC |
| chr18 | 58335363 | 58335378 | +/- | 16 | GGATTCTGATCTCACG |
| chr18 | 58335364 | 58335379 | +/- | 16 | GATTCTGATCTCACGT |
| chr18 | 58335365 | 58335380 | +/- | 16 | ATTCTGATCTCACGTC |
| chr18 | 58335366 | 58335381 | +/- | 16 | TTCTGATCTCACGTCA |
| chr18 | 58335367 | 58335382 | +/- | 16 | TCTGATCTCACGTCAC |
| chr18 | 58335368 | 58335383 | +/- | 16 | CTGATCTCACGTCACC |
| chr18 | 58335369 | 58335384 | +/- | 16 | TGATCTCACGTCACCT |
| chr18 | 58335370 | 58335385 | +/- | 16 | GATCTCACGTCACCTG |
| chr18 | 58335371 | 58335386 | +/- | 16 | ATCTCACGTCACCTGC |
| chr18 | 58335372 | 58335387 | +/- | 16 | TCTCACGTCACCTGCC |
| chr18 | 58335373 | 58335388 | +/- | 16 | CTCACGTCACCTGCCT |
| chr18 | 58335374 | 58335389 | +/- | 16 | TCACGTCACCTGCCTT |
| chr18 | 58335375 | 58335390 | +/- | 16 | CACGTCACCTGCCTTA |
| chr18 | 58335376 | 58335391 | +/- | 16 | ACGTCACCTGCCTTAC |
| chr18 | 58335377 | 58335392 | +/- | 16 | CGTCACCTGCCTTACA |
| chr18 | 58335378 | 58335393 | +/- | 16 | GTCACCTGCCTTACAG |
| chr18 | 58335379 | 58335394 | +/- | 16 | TCACCTGCCTTACAGC |
| chr18 | 58335380 | 58335395 | +/- | 16 | CACCTGCCTTACAGCG |
| chr18 | 58335381 | 58335396 | +/- | 16 | ACCTGCCTTACAGCGC |
| chr18 | 58335382 | 58335397 | +/- | 16 | CCTGCCTTACAGCGCT |
| chr18 | 58335383 | 58335398 | +/- | 16 | CTGCCTTACAGCGCTG |
| chr18 | 58335384 | 58335399 | +/- | 16 | TGCCTTACAGCGCTGC |
| chr18 | 58335385 | 58335400 | +/- | 16 | GCCTTACAGCGCTGCC |
| chr18 | 58335386 | 58335401 | +/- | 16 | CCTTACAGCGCTGCCA |
| chr18 | 58335387 | 58335402 | +/- | 16 | CTTACAGCGCTGCCAC |
| chr18 | 58335388 | 58335403 | +/- | 16 | TTACAGCGCTGCCACA |
| chr18 | 58335389 | 58335404 | +/- | 16 | TACAGCGCTGCCACAG |
| chr18 | 58335390 | 58335405 | +/- | 16 | ACAGCGCTGCCACAGC |
| chr18 | 58335391 | 58335406 | +/- | 16 | CAGCGCTGCCACAGCA |
| chr18 | 58335392 | 58335407 | +/- | 16 | AGCGCTGCCACAGCAG |
| chr18 | 58335393 | 58335408 | +/- | 16 | GCGCTGCCACAGCAGT |
| chr18 | 58335394 | 58335409 | +/- | 16 | CGCTGCCACAGCAGTG |
| chr18 | 58335395 | 58335410 | +/- | 16 | GCTGCCACAGCAGTGG |
| chr18 | 58335396 | 58335411 | +/- | 16 | CTGCCACAGCAGTGGG |
| chr18 | 58335397 | 58335412 | +/- | 16 | TGCCACAGCAGTGGGC |
| chr18 | 58335398 | 58335413 | +/- | 16 | GCCACAGCAGTGGGCC |
| chr18 | 58335399 | 58335414 | +/- | 16 | CCACAGCAGTGGGCCC |
| chr18 | 58335400 | 58335415 | +/- | 16 | CACAGCAGTGGGCCCT |
| chr18 | 58335401 | 58335416 | +/- | 16 | ACAGCAGTGGGCCCTG |
| chr18 | 58335402 | 58335417 | +/- | 16 | CAGCAGTGGGCCCTGA |
| chr18 | 58335403 | 58335418 | +/- | 16 | AGCAGTGGGCCCTGAT |
| chr18 | 58335404 | 58335419 | +/- | 16 | GCAGTGGGCCCTGATT |
| chr18 | 58335405 | 58335420 | +/- | 16 | CAGTGGGCCCTGATTC |
| chr18 | 58335406 | 58335421 | +/- | 16 | AGTGGGCCCTGATTCA |
| chr18 | 58335407 | 58335422 | +/- | 16 | GTGGGCCCTGATTCAG |
| chr18 | 58335408 | 58335423 | +/- | 16 | TGGGCCCTGATTCAGA |
| chr18 | 58335409 | 58335424 | +/- | 16 | GGGCCCTGATTCAGAC |
| chr18 | 58335410 | 58335425 | +/- | 16 | GGCCCTGATTCAGACA |
| chr18 | 58335411 | 58335426 | +/- | 16 | GCCCTGATTCAGACAG |
| chr18 | 58335412 | 58335427 | +/- | 16 | CCCTGATTCAGACAGC |
| chr18 | 58335413 | 58335428 | +/- | 16 | CCTGATTCAGACAGCA |
| chr18 | 58335414 | 58335429 | +/- | 16 | CTGATTCAGACAGCAG |
| chr18 | 58335415 | 58335430 | +/- | 16 | TGATTCAGACAGCAGG |
| chr18 | 58335317 | 58335332 | +/- | 16 | TGGTCATCAGCGACTG |
| chr18 | 58335416 | 58335431 | +/- | 16 | GATTCAGACAGCAGGG |
| chr18 | 58335417 | 58335432 | +/- | 16 | ATTCAGACAGCAGGGG |
| chr18 | 58335418 | 58335433 | +/- | 16 | TTCAGACAGCAGGGGG |
| chr18 | 58335419 | 58335434 | +/- | 16 | TCAGACAGCAGGGGGT |
| chr18 | 58335420 | 58335435 | +/- | 16 | CAGACAGCAGGGGGTC |
| chr18 | 58335421 | 58335436 | +/- | 16 | AGACAGCAGGGGGTCA |
| chr18 | 58335422 | 58335437 | +/- | 16 | GACAGCAGGGGGTCAT |
| chr18 | 58335423 | 58335438 | +/- | 16 | ACAGCAGGGGGTCATC |
| chr18 | 58335424 | 58335439 | +/- | 16 | CAGCAGGGGGTCATCC |
| chr18 | 58335425 | 58335440 | +/- | 16 | AGCAGGGGGTCATCCC |
| chr18 | 58335426 | 58335441 | +/- | 16 | GCAGGGGGTCATCCCC |
| chr18 | 58335427 | 58335442 | +/- | 16 | CAGGGGGTCATCCCCT |
| chr18 | 58335428 | 58335443 | +/- | 16 | AGGGGGTCATCCCCTA |
| chr18 | 58335429 | 58335444 | +/- | 16 | GGGGGTCATCCCCTAA |
| chr18 | 58335430 | 58335445 | +/- | 16 | GGGGTCATCCCCTAAG |
| chr18 | 58335431 | 58335446 | +/- | 16 | GGGTCATCCCCTAAGT |
| chr18 | 58335432 | 58335447 | +/- | 16 | GGTCATCCCCTAAGTG |

**Table 10: 17-mer target-specific ASOs**

| **CHR** | **START** | **END** | **STRAND** | **Kmer** | **SEQUENCE** |
|---|---|---|---|---|---|
| chr18 | 58335318 | 58335334 | +/- | 17 | GGTCATCAGCGACTGCT |
| chr18 | 58335319 | 58335335 | +/- | 17 | GTCATCAGCGACTGCTG |
| chr18 | 58335320 | 58335336 | +/- | 17 | TCATCAGCGACTGCTGG |
| chr18 | 58335321 | 58335337 | +/- | 17 | CATCAGCGACTGCTGGC |
| chr18 | 58335322 | 58335338 | +/- | 17 | ATCAGCGACTGCTGGCT |
| chr18 | 58335323 | 58335339 | +/- | 17 | TCAGCGACTGCTGGCTT |
| chr18 | 58335324 | 58335340 | +/- | 17 | CAGCGACTGCTGGCTTT |
| chr18 | 58335325 | 58335341 | +/- | 17 | AGCGACTGCTGGCTTTG |
| chr18 | 58335326 | 58335342 | +/- | 17 | GCGACTGCTGGCTTTGT |
| chr18 | 58335327 | 58335343 | +/- | 17 | CGACTGCTGGCTTTGTC |
| chr18 | 58335328 | 58335344 | +/- | 17 | GACTGCTGGCTTTGTCT |
| chr18 | 58335329 | 58335345 | +/- | 17 | ACTGCTGGCTTTGTCTG |
| chr18 | 58335330 | 58335346 | +/- | 17 | CTGCTGGCTTTGTCTGG |
| chr18 | 58335331 | 58335347 | +/- | 17 | TGCTGGCTTTGTCTGGA |
| chr18 | 58335332 | 58335348 | +/- | 17 | GCTGGCTTTGTCTGGAT |
| chr18 | 58335333 | 58335349 | +/- | 17 | CTGGCTTTGTCTGGATA |
| chr18 | 58335334 | 58335350 | +/- | 17 | TGGCTTTGTCTGGATAG |
| chr18 | 58335335 | 58335351 | +/- | 17 | GGCTTTGTCTGGATAGG |
| chr18 | 58335336 | 58335352 | +/- | 17 | GCTTTGTCTGGATAGGG |
| chr18 | 58335337 | 58335353 | +/- | 17 | CTTTGTCTGGATAGGGT |
| chr18 | 58335338 | 58335354 | +/- | 17 | TTTGTCTGGATAGGGTG |
| chr18 | 58335339 | 58335355 | +/- | 17 | TTGTCTGGATAGGGTGG |
| chr18 | 58335340 | 58335356 | +/- | 17 | TGTCTGGATAGGGTGGG |
| chr18 | 58335341 | 58335357 | +/- | 17 | GTCTGGATAGGGTGGGT |
| chr18 | 58335342 | 58335358 | +/- | 17 | TCTGGATAGGGTGGGTT |
| chr18 | 58335343 | 58335359 | +/- | 17 | CTGGATAGGGTGGGTTT |
| chr18 | 58335344 | 58335360 | +/- | 17 | TGGATAGGGTGGGTTTC |
| chr18 | 58335345 | 58335361 | +/- | 17 | GGATAGGGTGGGTTTCA |
| chr18 | 58335346 | 58335362 | +/- | 17 | GATAGGGTGGGTTTCAG |
| chr18 | 58335347 | 58335363 | +/- | 17 | ATAGGGTGGGTTTCAGG |
| chr18 | 58335348 | 58335364 | +/- | 17 | TAGGGTGGGTTTCAGGG |
| chr18 | 58335349 | 58335365 | +/- | 17 | AGGGTGGGTTTCAGGGA |
| chr18 | 58335350 | 58335366 | +/- | 17 | GGGTGGGTTTCAGGGAT |
| chr18 | 58335351 | 58335367 | +/- | 17 | GGTGGGTTTCAGGGATT |
| chr18 | 58335352 | 58335368 | +/- | 17 | GTGGGTTTCAGGGATTC |
| chr18 | 58335353 | 58335369 | +/- | 17 | TGGGTTTCAGGGATTCT |
| chr18 | 58335354 | 58335370 | +/- | 17 | GGGTTTCAGGGATTCTG |
| chr18 | 58335355 | 58335371 | +/- | 17 | GGTTTCAGGGATTCTGA |
| chr18 | 58335356 | 58335372 | +/- | 17 | GTTTCAGGGATTCTGAT |
| chr18 | 58335357 | 58335373 | +/- | 17 | TTTCAGGGATTCTGATC |
| chr18 | 58335358 | 58335374 | +/- | 17 | TTCAGGGATTCTGATCT |
| chr18 | 58335359 | 58335375 | +/- | 17 | TCAGGGATTCTGATCTC |
| chr18 | 58335360 | 58335376 | +/- | 17 | CAGGGATTCTGATCTCA |
| chr18 | 58335361 | 58335377 | +/- | 17 | AGGGATTCTGATCTCAC |
| chr18 | 58335362 | 58335378 | +/- | 17 | GGGATTCTGATCTCACG |
| chr18 | 58335363 | 58335379 | +/- | 17 | GGATTCTGATCTCACGT |
| chr18 | 58335364 | 58335380 | +/- | 17 | GATTCTGATCTCACGTC |
| chr18 | 58335365 | 58335381 | +/- | 17 | ATTCTGATCTCACGTCA |
| chr18 | 58335366 | 58335382 | +/- | 17 | TTCTGATCTCACGTCAC |
| chr18 | 58335367 | 58335383 | +/- | 17 | TCTGATCTCACGTCACC |
| chr18 | 58335368 | 58335384 | +/- | 17 | CTGATCTCACGTCACCT |
| chr18 | 58335369 | 58335385 | +/- | 17 | TGATCTCACGTCACCTG |
| chr18 | 58335370 | 58335386 | +/- | 17 | GATCTCACGTCACCTGC |
| chr18 | 58335371 | 58335387 | +/- | 17 | ATCTCACGTCACCTGCC |
| chr18 | 58335372 | 58335388 | +/- | 17 | TCTCACGTCACCTGCCT |
| chr18 | 58335373 | 58335389 | +/- | 17 | CTCACGTCACCTGCCTT |
| chr18 | 58335374 | 58335390 | +/- | 17 | TCACGTCACCTGCCTTA |
| chr18 | 58335375 | 58335391 | +/- | 17 | CACGTCACCTGCCTTAC |
| chr18 | 58335376 | 58335392 | +/- | 17 | ACGTCACCTGCCTTACA |
| chr18 | 58335377 | 58335393 | +/- | 17 | CGTCACCTGCCTTACAG |
| chr18 | 58335378 | 58335394 | +/- | 17 | GTCACCTGCCTTACAGC |
| chr18 | 58335379 | 58335395 | +/- | 17 | TCACCTGCCTTACAGCG |
| chr18 | 58335380 | 58335396 | +/- | 17 | CACCTGCCTTACAGCGC |
| chr18 | 58335381 | 58335397 | +/- | 17 | ACCTGCCTTACAGCGCT |
| chr18 | 58335382 | 58335398 | +/- | 17 | CCTGCCTTACAGCGCTG |
| chr18 | 58335383 | 58335399 | +/- | 17 | CTGCCTTACAGCGCTGC |
| chr18 | 58335384 | 58335400 | +/- | 17 | TGCCTTACAGCGCTGCC |
| chr18 | 58335385 | 58335401 | +/- | 17 | GCCTTACAGCGCTGCCA |
| chr18 | 58335386 | 58335402 | +/- | 17 | CCTTACAGCGCTGCCAC |
| chr18 | 58335387 | 58335403 | +/- | 17 | CTTACAGCGCTGCCACA |
| chr18 | 58335388 | 58335404 | +/- | 17 | TTACAGCGCTGCCACAG |
| chr18 | 58335389 | 58335405 | +/- | 17 | TACAGCGCTGCCACAGC |
| chr18 | 58335390 | 58335406 | +/- | 17 | ACAGCGCTGCCACAGCA |
| chr18 | 58335391 | 58335407 | +/- | 17 | CAGCGCTGCCACAGCAG |
| chr18 | 58335392 | 58335408 | +/- | 17 | AGCGCTGCCACAGCAGT |
| chr18 | 58335393 | 58335409 | +/- | 17 | GCGCTGCCACAGCAGTG |
| chr18 | 58335394 | 58335410 | +/- | 17 | CGCTGCCACAGCAGTGG |
| chr18 | 58335395 | 58335411 | +/- | 17 | GCTGCCACAGCAGTGGG |
| chr18 | 58335396 | 58335412 | +/- | 17 | CTGCCACAGCAGTGGGC |
| chr18 | 58335397 | 58335413 | +/- | 17 | TGCCACAGCAGTGGGCC |
| chr18 | 58335398 | 58335414 | +/- | 17 | GCCACAGCAGTGGGCCC |
| chr18 | 58335399 | 58335415 | +/- | 17 | CCACAGCAGTGGGCCCT |
| chr18 | 58335400 | 58335416 | +/- | 17 | CACAGCAGTGGGCCCTG |
| chr18 | 58335401 | 58335417 | +/- | 17 | ACAGCAGTGGGCCCTGA |
| chr18 | 58335402 | 58335418 | +/- | 17 | CAGCAGTGGGCCCTGAT |
| chr18 | 58335403 | 58335419 | +/- | 17 | AGCAGTGGGCCCTGATT |
| chr18 | 58335404 | 58335420 | +/- | 17 | GCAGTGGGCCCTGATTC |
| chr18 | 58335405 | 58335421 | +/- | 17 | CAGTGGGCCCTGATTCA |
| chr18 | 58335406 | 58335422 | +/- | 17 | AGTGGGCCCTGATTCAG |
| chr18 | 58335407 | 58335423 | +/- | 17 | GTGGGCCCTGATTCAGA |
| chr18 | 58335408 | 58335424 | +/- | 17 | TGGGCCCTGATTCAGAC |
| chr18 | 58335409 | 58335425 | +/- | 17 | GGGCCCTGATTCAGACA |
| chr18 | 58335410 | 58335426 | +/- | 17 | GGCCCTGATTCAGACAG |
| chr18 | 58335411 | 58335427 | +/- | 17 | GCCCTGATTCAGACAGC |
| chr18 | 58335412 | 58335428 | +/- | 17 | CCCTGATTCAGACAGCA |
| chr18 | 58335413 | 58335429 | +/- | 17 | CCTGATTCAGACAGCAG |
| chr18 | 58335414 | 58335430 | +/- | 17 | CTGATTCAGACAGCAGG |
| chr18 | 58335317 | 58335333 | +/- | 17 | TGGTCATCAGCGACTGC |
| chr18 | 58335415 | 58335431 | +/- | 17 | TGATTCAGACAGCAGGG |
| chr18 | 58335416 | 58335432 | +/- | 17 | GATTCAGACAGCAGGGG |
| chr18 | 58335417 | 58335433 | +/- | 17 | ATTCAGACAGCAGGGGG |
| chr18 | 58335418 | 58335434 | +/- | 17 | TTCAGACAGCAGGGGGT |
| chr18 | 58335419 | 58335435 | +/- | 17 | TCAGACAGCAGGGGGTC |
| chr18 | 58335420 | 58335436 | +/- | 17 | CAGACAGCAGGGGGTCA |
| chr18 | 58335421 | 58335437 | +/- | 17 | AGACAGCAGGGGGTCAT |
| chr18 | 58335422 | 58335438 | +/- | 17 | GACAGCAGGGGGTCATC |
| chr18 | 58335423 | 58335439 | +/- | 17 | ACAGCAGGGGGTCATCC |
| chr18 | 58335424 | 58335440 | +/- | 17 | CAGCAGGGGGTCATCCC |
| chr18 | 58335425 | 58335441 | +/- | 17 | AGCAGGGGGTCATCCCC |
| chr18 | 58335426 | 58335442 | +/- | 17 | GCAGGGGGTCATCCCCT |
| chr18 | 58335427 | 58335443 | +/- | 17 | CAGGGGGTCATCCCCTA |
| chr18 | 58335428 | 58335444 | +/- | 17 | AGGGGGTCATCCCCTAA |
| chr18 | 58335429 | 58335445 | +/- | 17 | GGGGGTCATCCCCTAAG |
| chr18 | 58335430 | 58335446 | +/- | 17 | GGGGTCATCCCCTAAGT |
| chr18 | 58335431 | 58335447 | +/- | 17 | GGGTCATCCCCTAAGTG |

**Table 11: 18-mer target-specific ASOs**

| **CHR** | **START** | **END** | **STRAND** | **Kmer** | **SEQUENCE** |
|---|---|---|---|---|---|
| chr18 | 58335318 | 58335335 | +/- | 18 | GGTCATCAGCGACTGCTG |
| chr18 | 58335319 | 58335336 | +/- | 18 | GTCATCAGCGACTGCTGG |
| chr18 | 58335320 | 58335337 | +/- | 18 | TCATCAGCGACTGCTGGC |
| chr18 | 58335321 | 58335338 | +/- | 18 | CATCAGCGACTGCTGGCT |
| chr18 | 58335322 | 58335339 | +/- | 18 | ATCAGCGACTGCTGGCTT |
| chr18 | 58335323 | 58335340 | +/- | 18 | TCAGCGACTGCTGGCTTT |
| chr18 | 58335324 | 58335341 | +/- | 18 | CAGCGACTGCTGGCTTTG |
| chr18 | 58335325 | 58335342 | +/- | 18 | AGCGACTGCTGGCTTTGT |
| chr18 | 58335326 | 58335343 | +/- | 18 | GCGACTGCTGGCTTTGTC |
| chr18 | 58335327 | 58335344 | +/- | 18 | CGACTGCTGGCTTTGTCT |
| chr18 | 58335328 | 58335345 | +/- | 18 | GACTGCTGGCTTTGTCTG |
| chr18 | 58335329 | 58335346 | +/- | 18 | ACTGCTGGCTTTGTCTGG |
| chr18 | 58335330 | 58335347 | +/- | 18 | CTGCTGGCTTTGTCTGGA |
| chr18 | 58335331 | 58335348 | +/- | 18 | TGCTGGCTTTGTCTGGAT |
| chr18 | 58335332 | 58335349 | +/- | 18 | GCTGGCTTTGTCTGGATA |
| chr18 | 58335333 | 58335350 | +/- | 18 | CTG GCTTTGTCTG GATAG |
| chr18 | 58335334 | 58335351 | +/- | 18 | TGGCTTTGTCTGGATAGG |
| chr18 | 58335335 | 58335352 | +/- | 18 | GGCTTTGTCTGGATAGGG |
| chr18 | 58335336 | 58335353 | +/- | 18 | GCTTTGTCTGGATAGGGT |
| chr18 | 58335337 | 58335354 | +/- | 18 | CTTTGTCTGGATAGGGTG |
| chr18 | 58335338 | 58335355 | +/- | 18 | TTTGTCTGGATAGGGTGG |
| chr18 | 58335339 | 58335356 | +/- | 18 | TTGTCTGGATAGGGTGGG |
| chr18 | 58335340 | 58335357 | +/- | 18 | TGTCTGGATAGGGTGGGT |
| chr18 | 58335341 | 58335358 | +/- | 18 | GTCTGGATAGGGTGGGTT |
| chr18 | 58335342 | 58335359 | +/- | 18 | TCTGGATAGGGTGGGTTT |
| chr18 | 58335343 | 58335360 | +/- | 18 | CTGGATAGGGTGGGTTTC |
| chr18 | 58335344 | 58335361 | +/- | 18 | TGGATAGGGTGGGTTTCA |
| chr18 | 58335345 | 58335362 | +/- | 18 | GGATAGGGTGGGTTTCAG |
| chr18 | 58335346 | 58335363 | +/- | 18 | GATAGGGTGGGTTTCAGG |
| chr18 | 58335347 | 58335364 | +/- | 18 | ATAGGGTGGGTTTCAGGG |
| chr18 | 58335348 | 58335365 | +/- | 18 | TAGGGTGGGTTTCAGGGA |
| chr18 | 58335349 | 58335366 | +/- | 18 | AGGGTGGGTTTCAGGGAT |
| chr18 | 58335350 | 58335367 | +/- | 18 | GGGTGGGTTTCAGGGATT |
| chr18 | 58335351 | 58335368 | +/- | 18 | GGTGGGTTTCAGGGATTC |
| chr18 | 58335352 | 58335369 | +/- | 18 | GTGGGTTTCAGGGATTCT |
| chr18 | 58335353 | 58335370 | +/- | 18 | TGGGTTTCAGGGATTCTG |
| chr18 | 58335354 | 58335371 | +/- | 18 | GGGTTTCAGGGATTCTGA |
| chr18 | 58335355 | 58335372 | +/- | 18 | GGTTTCAGGGATTCTGAT |
| chr18 | 58335356 | 58335373 | +/- | 18 | GTTTCAGGGATTCTGATC |
| chr18 | 58335357 | 58335374 | +/- | 18 | TTTCAGGGATTCTGATCT |
| chr18 | 58335358 | 58335375 | +/- | 18 | TTCAGGGATTCTGATCTC |
| chr18 | 58335359 | 58335376 | +/- | 18 | TCAGGGATTCTGATCTCA |
| chr18 | 58335360 | 58335377 | +/- | 18 | CAGGGATTCTGATCTCAC |
| chr18 | 58335361 | 58335378 | +/- | 18 | AGGGATTCTGATCTCACG |
| chr18 | 58335362 | 58335379 | +/- | 18 | GGGATTCTGATCTCACGT |
| chr18 | 58335363 | 58335380 | +/- | 18 | GGATTCTGATCTCACGTC |
| chr18 | 58335364 | 58335381 | +/- | 18 | GATTCTGATCTCACGTCA |
| chr18 | 58335365 | 58335382 | +/- | 18 | ATTCTGATCTCACGTCAC |
| chr18 | 58335366 | 58335383 | +/- | 18 | TTCTGATCTCACGTCACC |
| chr18 | 58335367 | 58335384 | +/- | 18 | TCTGATCTCACGTCACCT |
| chr18 | 58335368 | 58335385 | +/- | 18 | CTGATCTCACGTCACCTG |
| chr18 | 58335369 | 58335386 | +/- | 18 | TGATCTCACGTCACCTGC |
| chr18 | 58335370 | 58335387 | +/- | 18 | GATCTCACGTCACCTGCC |
| chr18 | 58335371 | 58335388 | +/- | 18 | ATCTCACGTCACCTGCCT |
| chr18 | 58335372 | 58335389 | +/- | 18 | TCTCACGTCACCTGCCTT |
| chr18 | 58335373 | 58335390 | +/- | 18 | CTCACGTCACCTGCCTTA |
| chr18 | 58335374 | 58335391 | +/- | 18 | TCACGTCACCTGCCTTAC |
| chr18 | 58335375 | 58335392 | +/- | 18 | CACGTCACCTGCCTTACA |
| chr18 | 58335376 | 58335393 | +/- | 18 | ACGTCACCTGCCTTACAG |
| chr18 | 58335377 | 58335394 | +/- | 18 | CGTCACCTGCCTTACAGC |
| chr18 | 58335378 | 58335395 | +/- | 18 | GTCACCTGCCTTACAGCG |
| chr18 | 58335379 | 58335396 | +/- | 18 | TCACCTGCCTTACAGCGC |
| chr18 | 58335380 | 58335397 | +/- | 18 | CACCTGCCTTACAGCGCT |
| chr18 | 58335381 | 58335398 | +/- | 18 | ACCTGCCTTACAGCGCTG |
| chr18 | 58335382 | 58335399 | +/- | 18 | CCTGCCTTACAGCGCTGC |
| chr18 | 58335383 | 58335400 | +/- | 18 | CTGCCTTACAGCGCTGCC |
| chr18 | 58335384 | 58335401 | +/- | 18 | TGCCTTACAGCGCTGCCA |
| chr18 | 58335385 | 58335402 | +/- | 18 | GCCTTACAGCGCTGCCAC |
| chr18 | 58335386 | 58335403 | +/- | 18 | CCTTACAGCGCTGCCACA |
| chr18 | 58335387 | 58335404 | +/- | 18 | CTTACAGCGCTGCCACAG |
| chr18 | 58335388 | 58335405 | +/- | 18 | TTACAGCGCTGCCACAGC |
| chr18 | 58335389 | 58335406 | +/- | 18 | TACAGCGCTGCCACAGCA |
| chr18 | 58335390 | 58335407 | +/- | 18 | ACAGCGCTGCCACAGCAG |
| chr18 | 58335391 | 58335408 | +/- | 18 | CAGCGCTGCCACAGCAGT |
| chr18 | 58335392 | 58335409 | +/- | 18 | AGCGCTGCCACAGCAGTG |
| chr18 | 58335393 | 58335410 | +/- | 18 | GCGCTGCCACAGCAGTGG |
| chr18 | 58335394 | 58335411 | +/- | 18 | CGCTGCCACAGCAGTGGG |
| chr18 | 58335395 | 58335412 | +/- | 18 | GCTGCCACAGCAGTGGGC |
| chr18 | 58335396 | 58335413 | +/- | 18 | CTGCCACAGCAGTGGGCC |
| chr18 | 58335397 | 58335414 | +/- | 18 | TGCCACAGCAGTGGGCCC |
| chr18 | 58335398 | 58335415 | +/- | 18 | GCCACAGCAGTGGGCCCT |
| chr18 | 58335399 | 58335416 | +/- | 18 | CCACAGCAGTGGGCCCTG |
| chr18 | 58335400 | 58335417 | +/- | 18 | CACAGCAGTGGGCCCTGA |
| chr18 | 58335401 | 58335418 | +/- | 18 | ACAGCAGTGGGCCCTGAT |
| chr18 | 58335402 | 58335419 | +/- | 18 | CAGCAGTGGGCCCTGATT |
| chr18 | 58335403 | 58335420 | +/- | 18 | AGCAGTGGGCCCTGATTC |
| chr18 | 58335404 | 58335421 | +/- | 18 | GCAGTGGGCCCTGATTCA |
| chr18 | 58335405 | 58335422 | +/- | 18 | CAGTGGGCCCTGATTCAG |
| chr18 | 58335406 | 58335423 | +/- | 18 | AGTGGGCCCTGATTCAGA |
| chr18 | 58335407 | 58335424 | +/- | 18 | GTGGGCCCTGATTCAGAC |
| chr18 | 58335408 | 58335425 | +/- | 18 | TGGGCCCTGATTCAGACA |
| chr18 | 58335409 | 58335426 | +/- | 18 | GGGCCCTGATTCAGACAG |
| chr18 | 58335410 | 58335427 | +/- | 18 | GGCCCTGATTCAGACAGC |
| chr18 | 58335411 | 58335428 | +/- | 18 | GCCCTGATTCAGACAGCA |
| chr18 | 58335412 | 58335429 | +/- | 18 | CCCTGATTCAGACAGCAG |
| chr18 | 58335413 | 58335430 | +/- | 18 | CCTGATTCAGACAGCAGG |
| chr18 | 58335317 | 58335334 | +/- | 18 | TGGTCATCAGCGACTGCT |
| chr18 | 58335414 | 58335431 | +/- | 18 | CTGATTCAGACAGCAGGG |
| chr18 | 58335415 | 58335432 | +/- | 18 | TGATTCAGACAGCAGGGG |
| chr18 | 58335416 | 58335433 | +/- | 18 | GATTCAGACAGCAGGGGG |
| chr18 | 58335417 | 58335434 | +/- | 18 | ATTCAGACAGCAGGGGGT |
| chr18 | 58335418 | 58335435 | +/- | 18 | TTCAGACAGCAGGGGGTC |
| chr18 | 58335419 | 58335436 | +/- | 18 | TCAGACAGCAGGGGGTCA |
| chr18 | 58335420 | 58335437 | +/- | 18 | CAGACAGCAGGGGGTCAT |
| chr18 | 58335421 | 58335438 | +/- | 18 | AGACAGCAGGGGGTCATC |
| chr18 | 58335422 | 58335439 | +/- | 18 | GACAGCAGGGGGTCATCC |
| chr18 | 58335423 | 58335440 | +/- | 18 | ACAGCAGGGGGTCATCCC |
| chr18 | 58335424 | 58335441 | +/- | 18 | CAGCAGGGGGTCATCCCC |
| chr18 | 58335425 | 58335442 | +/- | 18 | AGCAGGGGGTCATCCCCT |
| chr18 | 58335426 | 58335443 | +/- | 18 | GCAGGGGGTCATCCCCTA |
| chr18 | 58335427 | 58335444 | +/- | 18 | CAGGGGGTCATCCCCTAA |
| chr18 | 58335428 | 58335445 | +/- | 18 | AGGGGGTCATCCCCTAAG |
| chr18 | 58335429 | 58335446 | +/- | 18 | GGGGGTCATCCCCTAAGT |
| chr18 | 58335430 | 58335447 | +/- | 18 | GGGGTCATCCCCTAAGTG |

**Table 12: 19-mer target-specific ASOs**

| **CHR** | **START** | **END** | **STRAND** | **Kmer** | **SEQUENCE** |
|---|---|---|---|---|---|
| chr18 | 58335318 | 58335336 | +/- | 19 | GGTCATCAGCGACTGCTGG |
| chr18 | 58335319 | 58335337 | +/- | 19 | GTCATCAGCGACTGCTGGC |
| chr18 | 58335320 | 58335338 | +/- | 19 | TCATCAGCGACTGCTGGCT |
| chr18 | 58335321 | 58335339 | +/- | 19 | CATCAGCGACTGCTGGCTT |
| chr18 | 58335322 | 58335340 | +/- | 19 | ATCAGCGACTGCTGGCTTT |
| chr18 | 58335323 | 58335341 | +/- | 19 | TCAGCGACTGCTGGCTTTG |
| chr18 | 58335324 | 58335342 | +/- | 19 | CAGCGACTGCTGGCTTTGT |
| chr18 | 58335325 | 58335343 | +/- | 19 | AGCGACTGCTGGCTTTGTC |
| chr18 | 58335326 | 58335344 | +/- | 19 | GCGACTGCTGGCTTTGTCT |
| chr18 | 58335327 | 58335345 | +/- | 19 | CGACTGCTGGCTTTGTCTG |
| chr18 | 58335328 | 58335346 | +/- | 19 | GACTGCTGGCTTTGTCTGG |
| chr18 | 58335329 | 58335347 | +/- | 19 | ACTGCTGGCTTTGTCTGGA |
| chr18 | 58335330 | 58335348 | +/- | 19 | CTGCTGGCTTTGTCTGGAT |
| chr18 | 58335331 | 58335349 | +/- | 19 | TGCTGGCTTTGTCTGGATA |
| chr18 | 58335332 | 58335350 | +/- | 19 | GCTGGCTTTGTCTGGATAG |
| chr18 | 58335333 | 58335351 | +/- | 19 | CTGGCTTTGTCTGGATAGG |
| chr18 | 58335334 | 58335352 | +/- | 19 | TGGCTTTGTCTGGATAGGG |
| chr18 | 58335335 | 58335353 | +/- | 19 | GGCTTTGTCTGGATAGGGT |
| chr18 | 58335336 | 58335354 | +/- | 19 | GCTTTGTCTGGATAGGGTG |
| chr18 | 58335337 | 58335355 | +/- | 19 | CTTTGTCTGGATAGGGTGG |
| chr18 | 58335338 | 58335356 | +/- | 19 | TTTGTCTGGATAGGGTGGG |
| chr18 | 58335339 | 58335357 | +/- | 19 | TTGTCTGGATAGGGTGGGT |
| chr18 | 58335340 | 58335358 | +/- | 19 | TGTCTGGATAGGGTGGGTT |
| chr18 | 58335341 | 58335359 | +/- | 19 | GTCTGGATAGGGTGGGTTT |
| chr18 | 58335342 | 58335360 | +/- | 19 | TCTGGATAGGGTGGGTTTC |
| chr18 | 58335343 | 58335361 | +/- | 19 | CTGGATAGGGTGGGTTTCA |
| chr18 | 58335344 | 58335362 | +/- | 19 | TGGATAGGGTGGGTTTCAG |
| chr18 | 58335345 | 58335363 | +/- | 19 | GGATAGGGTGGGTTTCAGG |
| chr18 | 58335346 | 58335364 | +/- | 19 | GATAGGGTGGGTTTCAGGG |
| chr18 | 58335347 | 58335365 | +/- | 19 | ATAGGGTGGGTTTCAGGGA |
| chr18 | 58335348 | 58335366 | +/- | 19 | TAGGGTGGGTTTCAGGGAT |
| chr18 | 58335349 | 58335367 | +/- | 19 | AGGGTGGGTTTCAGGGATT |
| chr18 | 58335350 | 58335368 | +/- | 19 | GGGTGGGTTTCAGGGATTC |
| chr18 | 58335351 | 58335369 | +/- | 19 | GGTGGGTTTCAGGGATTCT |
| chr18 | 58335352 | 58335370 | +/- | 19 | GTGGGTTTCAGGGATTCTG |
| chr18 | 58335353 | 58335371 | +/- | 19 | TGGGTTTCAGGGATTCTGA |
| chr18 | 58335354 | 58335372 | +/- | 19 | GGGTTTCAGGGATTCTGAT |
| chr18 | 58335355 | 58335373 | +/- | 19 | GGTTTCAGGGATTCTGATC |
| chr18 | 58335356 | 58335374 | +/- | 19 | GTTTCAGGGATTCTGATCT |
| chr18 | 58335357 | 58335375 | +/- | 19 | TTTCAGGGATTCTGATCTC |
| chr18 | 58335358 | 58335376 | +/- | 19 | TTCAGGGATTCTGATCTCA |
| chr18 | 58335359 | 58335377 | +/- | 19 | TCAGGGATTCTGATCTCAC |
| chr18 | 58335360 | 58335378 | +/- | 19 | CAGGGATTCTGATCTCACG |
| chr18 | 58335361 | 58335379 | +/- | 19 | AGGGATTCTGATCTCACGT |
| chr18 | 58335362 | 58335380 | +/- | 19 | GGGATTCTGATCTCACGTC |
| chr18 | 58335363 | 58335381 | +/- | 19 | GGATTCTGATCTCACGTCA |
| chr18 | 58335364 | 58335382 | +/- | 19 | GATTCTGATCTCACGTCAC |
| chr18 | 58335365 | 58335383 | +/- | 19 | ATTCTGATCTCACGTCACC |
| chr18 | 58335366 | 58335384 | +/- | 19 | TTCTGATCTCACGTCACCT |
| chr18 | 58335367 | 58335385 | +/- | 19 | TCTGATCTCACGTCACCTG |
| chr18 | 58335368 | 58335386 | +/- | 19 | CTGATCTCACGTCACCTGC |
| chr18 | 58335369 | 58335387 | +/- | 19 | TGATCTCACGTCACCTGCC |
| chr18 | 58335370 | 58335388 | +/- | 19 | GATCTCACGTCACCTGCCT |
| chr18 | 58335371 | 58335389 | +/- | 19 | ATCTCACGTCACCTGCCTT |
| chr18 | 58335372 | 58335390 | +/- | 19 | TCTCACGTCACCTGCCTTA |
| chr18 | 58335373 | 58335391 | +/- | 19 | CTCACGTCACCTGCCTTAC |
| chr18 | 58335374 | 58335392 | +/- | 19 | TCACGTCACCTGCCTTACA |
| chr18 | 58335375 | 58335393 | +/- | 19 | CACGTCACCTGCCTTACAG |
| chr18 | 58335376 | 58335394 | +/- | 19 | ACGTCACCTGCCTTACAGC |
| chr18 | 58335377 | 58335395 | +/- | 19 | CGTCACCTGCCTTACAGCG |
| chr18 | 58335378 | 58335396 | +/- | 19 | GTCACCTGCCTTACAGCGC |
| chr18 | 58335379 | 58335397 | +/- | 19 | TCACCTGCCTTACAGCGCT |
| chr18 | 58335380 | 58335398 | +/- | 19 | CACCTGCCTTACAGCGCTG |
| chr18 | 58335381 | 58335399 | +/- | 19 | ACCTGCCTTACAGCGCTGC |
| chr18 | 58335382 | 58335400 | +/- | 19 | CCTGCCTTACAGCGCTGCC |
| chr18 | 58335383 | 58335401 | +/- | 19 | CTGCCTTACAGCGCTGCCA |
| chr18 | 58335384 | 58335402 | +/- | 19 | TGCCTTACAGCGCTGCCAC |
| chr18 | 58335385 | 58335403 | +/- | 19 | GCCTTACAGCGCTGCCACA |
| chr18 | 58335386 | 58335404 | +/- | 19 | CCTTACAGCGCTGCCACAG |
| chr18 | 58335387 | 58335405 | +/- | 19 | CTTACAGCGCTGCCACAGC |
| chr18 | 58335388 | 58335406 | +/- | 19 | TTACAGCGCTGCCACAGCA |
| chr18 | 58335389 | 58335407 | +/- | 19 | TACAGCGCTGCCACAGCAG |
| chr18 | 58335390 | 58335408 | +/- | 19 | ACAGCGCTGCCACAGCAGT |
| chr18 | 58335391 | 58335409 | +/- | 19 | CAGCGCTGCCACAGCAGTG |
| chr18 | 58335392 | 58335410 | +/- | 19 | AGCGCTGCCACAGCAGTGG |
| chr18 | 58335393 | 58335411 | +/- | 19 | GCGCTGCCACAGCAGTGGG |
| chr18 | 58335394 | 58335412 | +/- | 19 | CGCTGCCACAGCAGTGGGC |
| chr18 | 58335395 | 58335413 | +/- | 19 | GCTGCCACAGCAGTGGGCC |
| chr18 | 58335396 | 58335414 | +/- | 19 | CTGCCACAGCAGTGGGCCC |
| chr18 | 58335397 | 58335415 | +/- | 19 | TGCCACAGCAGTGGGCCCT |
| chr18 | 58335398 | 58335416 | +/- | 19 | GCCACAGCAGTGGGCCCTG |
| chr18 | 58335399 | 58335417 | +/- | 19 | CCACAGCAGTGGGCCCTGA |
| chr18 | 58335400 | 58335418 | +/- | 19 | CACAGCAGTGGGCCCTGAT |
| chr18 | 58335401 | 58335419 | +/- | 19 | ACAGCAGTGGGCCCTGATT |
| chr18 | 58335402 | 58335420 | +/- | 19 | CAGCAGTGGGCCCTGATTC |
| chr18 | 58335403 | 58335421 | +/- | 19 | AGCAGTGGGCCCTGATTCA |
| chr18 | 58335404 | 58335422 | +/- | 19 | GCAGTGGGCCCTGATTCAG |
| chr18 | 58335405 | 58335423 | +/- | 19 | CAGTGGGCCCTGATTCAGA |
| chr18 | 58335406 | 58335424 | +/- | 19 | AGTGGGCCCTGATTCAGAC |
| chr18 | 58335407 | 58335425 | +/- | 19 | GTGGGCCCTGATTCAGACA |
| chr18 | 58335408 | 58335426 | +/- | 19 | TGGGCCCTGATTCAGACAG |
| chr18 | 58335409 | 58335427 | +/- | 19 | GGGCCCTGATTCAGACAGC |
| chr18 | 58335410 | 58335428 | +/- | 19 | GGCCCTGATTCAGACAGCA |
| chr18 | 58335411 | 58335429 | +/- | 19 | GCCCTGATTCAGACAGCAG |
| chr18 | 58335412 | 58335430 | +/- | 19 | CCCTGATTCAGACAGCAGG |
| chr18 | 58335317 | 58335335 | +/- | 19 | TGGTCATCAGCGACTGCTG |
| chr18 | 58335413 | 58335431 | +/- | 19 | CCTGATTCAGACAGCAGGG |
| chr18 | 58335414 | 58335432 | +/- | 19 | CTGATTCAGACAGCAGGGG |
| chr18 | 58335415 | 58335433 | +/- | 19 | TGATTCAGACAGCAGGGGG |
| chr18 | 58335416 | 58335434 | +/- | 19 | GATTCAGACAGCAGGGGGT |
| chr18 | 58335417 | 58335435 | +/- | 19 | ATTCAGACAGCAGGGGGTC |
| chr18 | 58335418 | 58335436 | +/- | 19 | TTCAGACAGCAGGGGGTCA |
| chr18 | 58335419 | 58335437 | +/- | 19 | TCAGACAGCAGGGGGTCAT |
| chr18 | 58335420 | 58335438 | +/- | 19 | CAGACAGCAGGGGGTCATC |
| chr18 | 58335421 | 58335439 | +/- | 19 | AGACAGCAGGGGGTCATCC |
| chr18 | 58335422 | 58335440 | +/- | 19 | GACAGCAGGGGGTCATCCC |
| chr18 | 58335423 | 58335441 | +/- | 19 | ACAGCAGGGGGTCATCCCC |
| chr18 | 58335424 | 58335442 | +/- | 19 | CAGCAGGGGGTCATCCCCT |
| chr18 | 58335425 | 58335443 | +/- | 19 | AGCAGGGGGTCATCCCCTA |
| chr18 | 58335426 | 58335444 | +/- | 19 | GCAGGGGGTCATCCCCTAA |
| chr18 | 58335427 | 58335445 | +/- | 19 | CAGGGGGTCATCCCCTAAG |
| chr18 | 58335428 | 58335446 | +/- | 19 | AGGGGGTCATCCCCTAAGT |
| chr18 | 58335429 | 58335447 | +/- | 19 | GGGGGTCATCCCCTAAGTG |

**Table 13: 20-mer target-specific ASOs**

| **CHR** | **START** | **END** | **STRAND** | **Kmer** | **SEQUENCE** |
|---|---|---|---|---|---|
| chr18 | 58335318 | 58335337 | +/- | 20 | GGTCATCAGCGACTGCTGGC |
| chr18 | 58335319 | 58335338 | +/- | 20 | GTCATCAGCGACTGCTGGCT |
| chr18 | 58335320 | 58335339 | +/- | 20 | TCATCAGCGACTGCTGGCTT |
| chr18 | 58335321 | 58335340 | +/- | 20 | CATCAGCGACTGCTGGCTTT |
| chr18 | 58335322 | 58335341 | +/- | 20 | ATCAGCGACTGCTGGCTTTG |
| chr18 | 58335323 | 58335342 | +/- | 20 | TCAGCGACTGCTGGCTTTGT |
| chr18 | 58335324 | 58335343 | +/- | 20 | CAGCGACTGCTGGCTTTGTC |
| chr18 | 58335325 | 58335344 | +/- | 20 | AGCGACTGCTGGCTTTGTCT |
| chr18 | 58335326 | 58335345 | +/- | 20 | GCGACTGCTGGCTTTGTCTG |
| chr18 | 58335327 | 58335346 | +/- | 20 | CGACTGCTGGCTTTGTCTGG |
| chr18 | 58335328 | 58335347 | +/- | 20 | GACTGCTGGCTTTGTCTGGA |
| chr18 | 58335329 | 58335348 | +/- | 20 | ACTGCTGGCTTTGTCTGGAT |
| chr18 | 58335330 | 58335349 | +/- | 20 | CTGCTG GCTTTGTCTG GATA |
| chr18 | 58335331 | 58335350 | +/- | 20 | TGCTGGCTTTGTCTGGATAG |
| chr18 | 58335332 | 58335351 | +/- | 20 | GCTGGCTTTGTCTGGATAGG |
| chr18 | 58335333 | 58335352 | +/- | 20 | CTGGCTTTGTCTGGATAGGG |
| chr18 | 58335334 | 58335353 | +/- | 20 | TGGCTTTGTCTGGATAGGGT |
| chr18 | 58335335 | 58335354 | +/- | 20 | GGCTTTGTCTGGATAGGGTG |
| chr18 | 58335336 | 58335355 | +/- | 20 | GCTTTGTCTGGATAGGGTGG |
| chr18 | 58335337 | 58335356 | +/- | 20 | CTTTGTCTGGATAGGGTGGG |
| chr18 | 58335338 | 58335357 | +/- | 20 | TTTGTCTGGATAGGGTGGGT |
| chr18 | 58335339 | 58335358 | +/- | 20 | TTGTCTGGATAGGGTGGGTT |
| chr18 | 58335340 | 58335359 | +/- | 20 | TGTCTGGATAGGGTGGGTTT |
| chr18 | 58335341 | 58335360 | +/- | 20 | GTCTGGATAGGGTGGGTTTC |
| chr18 | 58335342 | 58335361 | +/- | 20 | TCTGGATAGGGTGGGTTTCA |
| chr18 | 58335343 | 58335362 | +/- | 20 | CTGGATAGGGTGGGTTTCAG |
| chr18 | 58335344 | 58335363 | +/- | 20 | TGGATAGGGTGGGTTTCAGG |
| chr18 | 58335345 | 58335364 | +/- | 20 | GGATAGGGTGGGTTTCAGGG |
| chr18 | 58335346 | 58335365 | +/- | 20 | GATAGGGTGGGTTTCAGGGA |
| chr18 | 58335347 | 58335366 | +/- | 20 | ATAGGGTGGGTTTCAGGGAT |
| chr18 | 58335348 | 58335367 | +/- | 20 | TAGGGTGGGTTTCAGGGATT |
| chr18 | 58335349 | 58335368 | +/- | 20 | AGGGTGGGTTTCAGGGATTC |
| chr18 | 58335350 | 58335369 | +/- | 20 | GGGTGGGTTTCAGGGATTCT |
| chr18 | 58335351 | 58335370 | +/- | 20 | GGTGGGTTTCAGGGATTCTG |
| chr18 | 58335352 | 58335371 | +/- | 20 | GTGGGTTTCAGGGATTCTGA |
| chr18 | 58335353 | 58335372 | +/- | 20 | TGGGTTTCAGGGATTCTGAT |
| chr18 | 58335354 | 58335373 | +/- | 20 | GGGTTTCAGGGATTCTGATC |
| chr18 | 58335355 | 58335374 | +/- | 20 | GGTTTCAGGGATTCTGATCT |
| chr18 | 58335356 | 58335375 | +/- | 20 | GTTTCAGGGATTCTGATCTC |
| chr18 | 58335357 | 58335376 | +/- | 20 | TTTCAGGGATTCTGATCTCA |
| chr18 | 58335358 | 58335377 | +/- | 20 | TTCAGGGATTCTGATCTCAC |
| chr18 | 58335359 | 58335378 | +/- | 20 | TCAGGGATTCTGATCTCACG |
| chr18 | 58335360 | 58335379 | +/- | 20 | CAGGGATTCTGATCTCACGT |
| chr18 | 58335361 | 58335380 | +/- | 20 | AGGGATTCTGATCTCACGTC |
| chr18 | 58335362 | 58335381 | +/- | 20 | GGGATTCTGATCTCACGTCA |
| chr18 | 58335363 | 58335382 | +/- | 20 | GGATTCTGATCTCACGTCAC |
| chr18 | 58335364 | 58335383 | +/- | 20 | GATTCTGATCTCACGTCACC |
| chr18 | 58335365 | 58335384 | +/- | 20 | ATTCTGATCTCACGTCACCT |
| chr18 | 58335366 | 58335385 | +/- | 20 | TTCTGATCTCACGTCACCTG |
| chr18 | 58335367 | 58335386 | +/- | 20 | TCTGATCTCACGTCACCTGC |
| chr18 | 58335368 | 58335387 | +/- | 20 | CTGATCTCACGTCACCTGCC |
| chr18 | 58335369 | 58335388 | +/- | 20 | TGATCTCACGTCACCTGCCT |
| chr18 | 58335370 | 58335389 | +/- | 20 | GATCTCACGTCACCTGCCTT |
| chr18 | 58335371 | 58335390 | +/- | 20 | ATCTCACGTCACCTGCCTTA |
| chr18 | 58335372 | 58335391 | +/- | 20 | TCTCACGTCACCTGCCTTAC |
| chr18 | 58335373 | 58335392 | +/- | 20 | CTCACGTCACCTGCCTTACA |
| chr18 | 58335374 | 58335393 | +/- | 20 | TCACGTCACCTGCCTTACAG |
| chr18 | 58335375 | 58335394 | +/- | 20 | CACGTCACCTGCCTTACAGC |
| chr18 | 58335376 | 58335395 | +/- | 20 | ACGTCACCTGCCTTACAGCG |
| chr18 | 58335377 | 58335396 | +/- | 20 | CGTCACCTGCCTTACAGCGC |
| chr18 | 58335378 | 58335397 | +/- | 20 | GTCACCTGCCTTACAGCGCT |
| chr18 | 58335379 | 58335398 | +/- | 20 | TCACCTGCCTTACAGCGCTG |
| chr18 | 58335380 | 58335399 | +/- | 20 | CACCTGCCTTACAGCGCTGC |
| chr18 | 58335381 | 58335400 | +/- | 20 | ACCTGCCTTACAGCGCTGCC |
| chr18 | 58335382 | 58335401 | +/- | 20 | CCTGCCTTACAGCGCTGCCA |
| chr18 | 58335383 | 58335402 | +/- | 20 | CTGCCTTACAGCGCTGCCAC |
| chr18 | 58335384 | 58335403 | +/- | 20 | TGCCTTACAGCGCTGCCACA |
| chr18 | 58335385 | 58335404 | +/- | 20 | GCCTTACAGCGCTGCCACAG |
| chr18 | 58335386 | 58335405 | +/- | 20 | CCTTACAGCGCTGCCACAGC |
| chr18 | 58335387 | 58335406 | +/- | 20 | CTTACAGCGCTGCCACAGCA |
| chr18 | 58335388 | 58335407 | +/- | 20 | TTACAGCGCTGCCACAGCAG |
| chr18 | 58335389 | 58335408 | +/- | 20 | TACAGCGCTGCCACAGCAGT |
| chr18 | 58335390 | 58335409 | +/- | 20 | ACAGCGCTGCCACAGCAGTG |
| chr18 | 58335391 | 58335410 | +/- | 20 | CAGCGCTGCCACAGCAGTGG |
| chr18 | 58335392 | 58335411 | +/- | 20 | AGCGCTGCCACAGCAGTGGG |
| chr18 | 58335393 | 58335412 | +/- | 20 | GCGCTGCCACAGCAGTGGGC |
| chr18 | 58335394 | 58335413 | +/- | 20 | CGCTGCCACAGCAGTGGGCC |
| chr18 | 58335395 | 58335414 | +/- | 20 | GCTGCCACAGCAGTGGGCCC |
| chr18 | 58335396 | 58335415 | +/- | 20 | CTGCCACAGCAGTGGGCCCT |
| chr18 | 58335397 | 58335416 | +/- | 20 | TGCCACAGCAGTGGGCCCTG |
| chr18 | 58335398 | 58335417 | +/- | 20 | GCCACAGCAGTGGGCCCTGA |
| chr18 | 58335399 | 58335418 | +/- | 20 | CCACAGCAGTGGGCCCTGAT |
| chr18 | 58335400 | 58335419 | +/- | 20 | CACAGCAGTGGGCCCTGATT |
| chr18 | 58335401 | 58335420 | +/- | 20 | ACAGCAGTGGGCCCTGATTC |
| chr18 | 58335402 | 58335421 | +/- | 20 | CAGCAGTGGGCCCTGATTCA |
| chr18 | 58335403 | 58335422 | +/- | 20 | AGCAGTGGGCCCTGATTCAG |
| chr18 | 58335404 | 58335423 | +/- | 20 | GCAGTGGGCCCTGATTCAGA |
| chr18 | 58335405 | 58335424 | +/- | 20 | CAGTGGGCCCTGATTCAGAC |
| chr18 | 58335406 | 58335425 | +/- | 20 | AGTGGGCCCTGATTCAGACA |
| chr18 | 58335407 | 58335426 | +/- | 20 | GTGGGCCCTGATTCAGACAG |
| chr18 | 58335408 | 58335427 | +/- | 20 | TGGGCCCTGATTCAGACAGC |
| chr18 | 58335409 | 58335428 | +/- | 20 | GGGCCCTGATTCAGACAGCA |
| chr18 | 58335410 | 58335429 | +/- | 20 | GGCCCTGATTCAGACAGCAG |
| chr18 | 58335411 | 58335430 | +/- | 20 | GCCCTGATTCAGACAGCAGG |
| chr18 | 58335317 | 58335336 | +/- | 20 | TGGTCATCAGCGACTGCTGG |
| chr18 | 58335412 | 58335431 | +/- | 20 | CCCTGATTCAGACAGCAGGG |
| chr18 | 58335413 | 58335432 | +/- | 20 | CCTGATTCAGACAGCAGGGG |
| chr18 | 58335414 | 58335433 | +/- | 20 | CTGATTCAGACAGCAGGGGG |
| chr18 | 58335415 | 58335434 | +/- | 20 | TGATTCAGACAGCAGGGGGT |
| chr18 | 58335416 | 58335435 | +/- | 20 | GATTCAGACAGCAGGGGGTC |
| chr18 | 58335417 | 58335436 | +/- | 20 | ATTCAGACAGCAGGGGGTCA |
| chr18 | 58335418 | 58335437 | +/- | 20 | TTCAGACAGCAGGGGGTCAT |
| chr18 | 58335419 | 58335438 | +/- | 20 | TCAGACAGCAGGGGGTCATC |
| chr18 | 58335420 | 58335439 | +/- | 20 | CAGACAGCAGGGGGTCATCC |
| chr18 | 58335421 | 58335440 | +/- | 20 | AGACAGCAGGGGGTCATCCC |
| chr18 | 58335422 | 58335441 | +/- | 20 | GACAGCAGGGGGTCATCCCC |
| chr18 | 58335423 | 58335442 | +/- | 20 | ACAGCAGGGGGTCATCCCCT |
| chr18 | 58335424 | 58335443 | +/- | 20 | CAGCAGGGGGTCATCCCCTA |
| chr18 | 58335425 | 58335444 | +/- | 20 | AGCAGGGGGTCATCCCCTAA |
| chr18 | 58335426 | 58335445 | +/- | 20 | GCAGGGGGTCATCCCCTAAG |
| chr18 | 58335427 | 58335446 | +/- | 20 | CAGGGGGTCATCCCCTAAGT |
| chr18 | 58335428 | 58335447 | +/- | 20 | AGGGGGTCATCCCCTAAGTG |

**Table 14: 21-mer target-specific ASOs**

| **CHR** | **START** | **END** | **STRAND** | **Kmer** | **SEQUENCE** |
|---|---|---|---|---|---|
| chr18 | 58335318 | 58335338 | +/- | 21 | GGTCATCAGCGACTGCTGGCT |
| chr18 | 58335319 | 58335339 | +/- | 21 | GTCATCAGCGACTGCTGGCTT |
| chr18 | 58335320 | 58335340 | +/- | 21 | TCATCAGCGACTGCTGGCTTT |
| chr18 | 58335321 | 58335341 | +/- | 21 | CATCAGCGACTGCTGGCTTTG |
| chr18 | 58335322 | 58335342 | +/- | 21 | ATCAGCGACTGCTGGCTTTGT |
| chr18 | 58335323 | 58335343 | +/- | 21 | TCAGCGACTGCTGGCTTTGTC |
| chr18 | 58335324 | 58335344 | +/- | 21 | CAGCGACTGCTGGCTTTGTCT |
| chr18 | 58335325 | 58335345 | +/- | 21 | AGCGACTGCTGGCTTTGTCTG |
| chr18 | 58335326 | 58335346 | +/- | 21 | GCGACTGCTGGCTTTGTCTGG |
| chr18 | 58335327 | 58335347 | +/- | 21 | CGACTGCTGGCTTTGTCTGGA |
| chr18 | 58335328 | 58335348 | +/- | 21 | GACTG CTG GCTTTGTCTG GAT |
| chr18 | 58335329 | 58335349 | +/- | 21 | ACTGCTGGCTTTGTCTGGATA |
| chr18 | 58335330 | 58335350 | +/- | 21 | CTGCTGGCTTTGTCTGGATAG |
| chr18 | 58335331 | 58335351 | +/- | 21 | TGCTGGCTTTGTCTGGATAGG |
| chr18 | 58335332 | 58335352 | +/- | 21 | GCTGGCTTTGTCTGGATAGGG |
| chr18 | 58335333 | 58335353 | +/- | 21 | CTGGCTTTGTCTGGATAGGGT |
| chr18 | 58335334 | 58335354 | +/- | 21 | TGGCTTTGTCTGGATAGGGTG |
| chr18 | 58335335 | 58335355 | +/- | 21 | GGCTTTGTCTGGATAGGGTGG |
| chr18 | 58335336 | 58335356 | +/- | 21 | GCTTTGTCTGGATAGGGTGGG |
| chr18 | 58335337 | 58335357 | +/- | 21 | CTTTGTCTGGATAGGGTGGGT |
| chr18 | 58335338 | 58335358 | +/- | 21 | TTTGTCTGGATAGGGTGGGTT |
| chr18 | 58335339 | 58335359 | +/- | 21 | TTGTCTGGATAGGGTGGGTTT |
| chr18 | 58335340 | 58335360 | +/- | 21 | TGTCTGGATAGGGTGGGTTTC |
| chr18 | 58335341 | 58335361 | +/- | 21 | GTCTGGATAGGGTGGGTTTCA |
| chr18 | 58335342 | 58335362 | +/- | 21 | TCTGGATAGGGTGGGTTTCAG |
| chr18 | 58335343 | 58335363 | +/- | 21 | CTGGATAGGGTGGGTTTCAGG |
| chr18 | 58335344 | 58335364 | +/- | 21 | TGGATAGGGTGGGTTTCAGGG |
| chr18 | 58335345 | 58335365 | +/- | 21 | GGATAGGGTGGGTTTCAGGGA |
| chr18 | 58335346 | 58335366 | +/- | 21 | GATAGGGTGGGTTTCAGGGAT |
| chr18 | 58335347 | 58335367 | +/- | 21 | ATAGGGTGGGTTTCAGGGATT |
| chr18 | 58335348 | 58335368 | +/- | 21 | TAGGGTGGGTTTCAGGGATTC |
| chr18 | 58335349 | 58335369 | +/- | 21 | AGGGTGGGTTTCAGGGATTCT |
| chr18 | 58335350 | 58335370 | +/- | 21 | GGGTGGGTTTCAGGGATTCTG |
| chr18 | 58335351 | 58335371 | +/- | 21 | GGTGGGTTTCAGGGATTCTGA |
| chr18 | 58335352 | 58335372 | +/- | 21 | GTGGGTTTCAGGGATTCTGAT |
| chr18 | 58335353 | 58335373 | +/- | 21 | TGGGTTTCAGGGATTCTGATC |
| chr18 | 58335354 | 58335374 | +/- | 21 | GGGTTTCAGGGATTCTGATCT |
| chr18 | 58335355 | 58335375 | +/- | 21 | GGTTTCAGGGATTCTGATCTC |
| chr18 | 58335356 | 58335376 | +/- | 21 | GTTTCAGGGATTCTGATCTCA |
| chr18 | 58335357 | 58335377 | +/- | 21 | TTTCAGGGATTCTGATCTCAC |
| chr18 | 58335358 | 58335378 | +/- | 21 | TTCAGGGATTCTGATCTCACG |
| chr18 | 58335359 | 58335379 | +/- | 21 | TCAGGGATTCTGATCTCACGT |
| chr18 | 58335360 | 58335380 | +/- | 21 | CAGGGATTCTGATCTCACGTC |
| chr18 | 58335361 | 58335381 | +/- | 21 | AGGGATTCTGATCTCACGTCA |
| chr18 | 58335362 | 58335382 | +/- | 21 | GGGATTCTGATCTCACGTCAC |
| chr18 | 58335363 | 58335383 | +/- | 21 | GGATTCTGATCTCACGTCACC |
| chr18 | 58335364 | 58335384 | +/- | 21 | GATTCTGATCTCACGTCACCT |
| chr18 | 58335365 | 58335385 | +/- | 21 | ATTCTGATCTCACGTCACCTG |
| chr18 | 58335366 | 58335386 | +/- | 21 | TTCTGATCTCACGTCACCTGC |
| chr18 | 58335367 | 58335387 | +/- | 21 | TCTGATCTCACGTCACCTGCC |
| chr18 | 58335368 | 58335388 | +/- | 21 | CTGATCTCACGTCACCTGCCT |
| chr18 | 58335369 | 58335389 | +/- | 21 | TGATCTCACGTCACCTGCCTT |
| chr18 | 58335370 | 58335390 | +/- | 21 | GATCTCACGTCACCTGCCTTA |
| chr18 | 58335371 | 58335391 | +/- | 21 | ATCTCACGTCACCTGCCTTAC |
| chr18 | 58335372 | 58335392 | +/- | 21 | TCTCACGTCACCTGCCTTACA |
| chr18 | 58335373 | 58335393 | +/- | 21 | CTCACGTCACCTGCCTTACAG |
| chr18 | 58335374 | 58335394 | +/- | 21 | TCACGTCACCTGCCTTACAGC |
| chr18 | 58335375 | 58335395 | +/- | 21 | CACGTCACCTGCCTTACAGCG |
| chr18 | 58335376 | 58335396 | +/- | 21 | ACGTCACCTGCCTTACAGCGC |
| chr18 | 58335377 | 58335397 | +/- | 21 | CGTCACCTGCCTTACAGCGCT |
| chr18 | 58335378 | 58335398 | +/- | 21 | GTCACCTGCCTTACAGCGCTG |
| chr18 | 58335379 | 58335399 | +/- | 21 | TCACCTGCCTTACAGCGCTGC |
| chr18 | 58335380 | 58335400 | +/- | 21 | CACCTGCCTTACAGCGCTGCC |
| chr18 | 58335381 | 58335401 | +/- | 21 | ACCTGCCTTACAGCGCTGCCA |
| chr18 | 58335382 | 58335402 | +/- | 21 | CCTGCCTTACAGCGCTGCCAC |
| chr18 | 58335383 | 58335403 | +/- | 21 | CTGCCTTACAGCGCTGCCACA |
| chr18 | 58335384 | 58335404 | +/- | 21 | TGCCTTACAGCGCTGCCACAG |
| chr18 | 58335385 | 58335405 | +/- | 21 | GCCTTACAGCGCTGCCACAGC |
| chr18 | 58335386 | 58335406 | +/- | 21 | CCTTACAGCGCTGCCACAGCA |
| chr18 | 58335387 | 58335407 | +/- | 21 | CTTACAGCGCTGCCACAGCAG |
| chr18 | 58335388 | 58335408 | +/- | 21 | TTACAGCGCTGCCACAGCAGT |
| chr18 | 58335389 | 58335409 | +/- | 21 | TACAGCGCTGCCACAGCAGTG |
| chr18 | 58335390 | 58335410 | +/- | 21 | ACAGCGCTGCCACAGCAGTGG |
| chr18 | 58335391 | 58335411 | +/- | 21 | CAGCGCTGCCACAGCAGTGGG |
| chr18 | 58335392 | 58335412 | +/- | 21 | AGCGCTGCCACAGCAGTGGGC |
| chr18 | 58335393 | 58335413 | +/- | 21 | GCGCTGCCACAGCAGTGGGCC |
| chr18 | 58335394 | 58335414 | +/- | 21 | CGCTGCCACAGCAGTGGGCCC |
| chr18 | 58335395 | 58335415 | +/- | 21 | GCTGCCACAGCAGTGGGCCCT |
| chr18 | 58335396 | 58335416 | +/- | 21 | CTGCCACAGCAGTGGGCCCTG |
| chr18 | 58335397 | 58335417 | +/- | 21 | TGCCACAGCAGTGGGCCCTGA |
| chr18 | 58335398 | 58335418 | +/- | 21 | GCCACAGCAGTGGGCCCTGAT |
| chr18 | 58335399 | 58335419 | +/- | 21 | CCACAGCAGTGGGCCCTGATT |
| chr18 | 58335400 | 58335420 | +/- | 21 | CACAGCAGTGGGCCCTGATTC |
| chr18 | 58335401 | 58335421 | +/- | 21 | ACAGCAGTGGGCCCTGATTCA |
| chr18 | 58335402 | 58335422 | +/- | 21 | CAGCAGTGGGCCCTGATTCAG |
| chr18 | 58335403 | 58335423 | +/- | 21 | AGCAGTGGGCCCTGATTCAGA |
| chr18 | 58335404 | 58335424 | +/- | 21 | GCAGTGGGCCCTGATTCAGAC |
| chr18 | 58335405 | 58335425 | +/- | 21 | CAGTGGGCCCTGATTCAGACA |
| chr18 | 58335406 | 58335426 | +/- | 21 | AGTGGGCCCTGATTCAGACAG |
| chr18 | 58335407 | 58335427 | +/- | 21 | GTGGGCCCTGATTCAGACAGC |
| chr18 | 58335408 | 58335428 | +/- | 21 | TGGGCCCTGATTCAGACAGCA |
| chr18 | 58335409 | 58335429 | +/- | 21 | GGGCCCTGATTCAGACAGCAG |
| chr18 | 58335410 | 58335430 | +/- | 21 | GGCCCTGATTCAGACAGCAGG |
| chr18 | 58335317 | 58335337 | +/- | 21 | TGGTCATCAGCGACTGCTGGC |
| chr18 | 58335411 | 58335431 | +/- | 21 | GCCCTGATTCAGACAGCAGGG |
| chr18 | 58335412 | 58335432 | +/- | 21 | CCCTGATTCAGACAGCAGGGG |
| chr18 | 58335413 | 58335433 | +/- | 21 | CCTGATTCAGACAGCAGGGGG |
| chr18 | 58335414 | 58335434 | +/- | 21 | CTGATTCAGACAGCAGGGGGT |
| chr18 | 58335415 | 58335435 | +/- | 21 | TGATTCAGACAGCAGGGGGTC |
| chr18 | 58335416 | 58335436 | +/- | 21 | GATTCAGACAGCAGGGGGTCA |
| chr18 | 58335417 | 58335437 | +/- | 21 | ATTCAGACAGCAGGGGGTCAT |
| chr18 | 58335418 | 58335438 | +/- | 21 | TTCAGACAGCAGGGGGTCATC |
| chr18 | 58335419 | 58335439 | +/- | 21 | TCAGACAGCAGGGGGTCATCC |
| chr18 | 58335420 | 58335440 | +/- | 21 | CAGACAGCAGGGGGTCATCCC |
| chr18 | 58335421 | 58335441 | +/- | 21 | AGACAGCAGGGGGTCATCCCC |
| chr18 | 58335422 | 58335442 | +/- | 21 | GACAGCAGGGGGTCATCCCCT |
| chr18 | 58335423 | 58335443 | +/- | 21 | ACAGCAGGGGGTCATCCCCTA |
| chr18 | 58335424 | 58335444 | +/- | 21 | CAGCAGGGGGTCATCCCCTAA |
| chr18 | 58335425 | 58335445 | +/- | 21 | AGCAGGGGGTCATCCCCTAAG |
| chr18 | 58335426 | 58335446 | +/- | 21 | GCAGGGGGTCATCCCCTAAGT |
| chr18 | 58335427 | 58335447 | +/- | 21 | CAGGGGGTCATCCCCTAAGTG |

**Table 15: 22-mer target-specific ASOs**

| **CHR** | **START** | **END** | **STRAND** | **Kmer** | **SEQUENCE** |
|---|---|---|---|---|---|
| chr18 | 58335318 | 58335339 | +/- | 22 | GGTCATCAGCGACTGCTGGCTT |
| chr18 | 58335319 | 58335340 | +/- | 22 | GTCATCAGCGACTGCTGGCTTT |
| chr18 | 58335320 | 58335341 | +/- | 22 | TCATCAGCGACTGCTGGCTTTG |
| chr18 | 58335321 | 58335342 | +/- | 22 | CATCAGCGACTGCTGGCTTTGT |
| chr18 | 58335322 | 58335343 | +/- | 22 | ATCAGCGACTGCTGGCTTTGTC |
| chr18 | 58335323 | 58335344 | +/- | 22 | TCAGCGACTGCTGGCTTTGTCT |
| chr18 | 58335324 | 58335345 | +/- | 22 | CAGCGACTGCTGGCTTTGTCTG |
| chr18 | 58335325 | 58335346 | +/- | 22 | AGCGACTGCTGGCTTTGTCTGG |
| chr18 | 58335326 | 58335347 | +/- | 22 | GCGACTGCTGGCTTTGTCTGGA |
| chr18 | 58335327 | 58335348 | +/- | 22 | CGACTGCTGGCTTTGTCTGGAT |
| chr18 | 58335328 | 58335349 | +/- | 22 | GACTGCTGGCTTTGTCTGGATA |
| chr18 | 58335329 | 58335350 | +/- | 22 | ACTGCTGGCTTTGTCTGGATAG |
| chr18 | 58335330 | 58335351 | +/- | 22 | CTGCTGGCTTTGTCTGGATAGG |
| chr18 | 58335331 | 58335352 | +/- | 22 | TGCTGGCTTTGTCTGGATAGGG |
| chr18 | 58335332 | 58335353 | +/- | 22 | GCTGGCTTTGTCTGGATAGGGT |
| chr18 | 58335333 | 58335354 | +/- | 22 | CTGGCTTTGTCTGGATAGGGTG |
| chr18 | 58335334 | 58335355 | +/- | 22 | TGGCTTTGTCTGGATAGGGTGG |
| chr18 | 58335335 | 58335356 | +/- | 22 | GGCTTTGTCTGGATAGGGTGGG |
| chr18 | 58335336 | 58335357 | +/- | 22 | GCTTTGTCTGGATAGGGTGGGT |
| chr18 | 58335337 | 58335358 | +/- | 22 | CTTTGTCTGGATAGGGTGGGTT |
| chr18 | 58335338 | 58335359 | +/- | 22 | TTTGTCTGGATAGGGTGGGTTT |
| chr18 | 58335339 | 58335360 | +/- | 22 | TTGTCTGGATAGGGTGGGTTTC |
| chr18 | 58335340 | 58335361 | +/- | 22 | TGTCTGGATAGGGTGGGTTTCA |
| chr18 | 58335341 | 58335362 | +/- | 22 | GTCTGGATAGGGTGGGTTTCAG |
| chr18 | 58335342 | 58335363 | +/- | 22 | TCTGGATAGGGTGGGTTTCAGG |
| chr18 | 58335343 | 58335364 | +/- | 22 | CTGGATAGGGTGGGTTTCAGGG |
| chr18 | 58335344 | 58335365 | +/- | 22 | TGGATAGGGTGGGTTTCAGGGA |
| chr18 | 58335345 | 58335366 | +/- | 22 | GGATAGGGTGGGTTTCAGGGAT |
| chr18 | 58335346 | 58335367 | +/- | 22 | GATAGGGTGGGTTTCAGGGATT |
| chr18 | 58335347 | 58335368 | +/- | 22 | ATAGGGTGGGTTTCAGGGATTC |
| chr18 | 58335348 | 58335369 | +/- | 22 | TAGGGTGGGTTTCAGGGATTCT |
| chr18 | 58335349 | 58335370 | +/- | 22 | AGGGTGGGTTTCAGGGATTCTG |
| chr18 | 58335350 | 58335371 | +/- | 22 | GGGTGGGTTTCAGGGATTCTGA |
| chr18 | 58335351 | 58335372 | +/- | 22 | GGTGGGTTTCAGGGATTCTGAT |
| chr18 | 58335352 | 58335373 | +/- | 22 | GTGGGTTTCAGGGATTCTGATC |
| chr18 | 58335353 | 58335374 | +/- | 22 | TGGGTTTCAGGGATTCTGATCT |
| chr18 | 58335354 | 58335375 | +/- | 22 | GGGTTTCAGGGATTCTGATCTC |
| chr18 | 58335355 | 58335376 | +/- | 22 | GGTTTCAGGGATTCTGATCTCA |
| chr18 | 58335356 | 58335377 | +/- | 22 | GTTTCAGGGATTCTGATCTCAC |
| chr18 | 58335357 | 58335378 | +/- | 22 | TTTCAGGGATTCTGATCTCACG |
| chr18 | 58335358 | 58335379 | +/- | 22 | TTCAGGGATTCTGATCTCACGT |
| chr18 | 58335359 | 58335380 | +/- | 22 | TCAGGGATTCTGATCTCACGTC |
| chr18 | 58335360 | 58335381 | +/- | 22 | CAGGGATTCTGATCTCACGTCA |
| chr18 | 58335361 | 58335382 | +/- | 22 | AGGGATTCTGATCTCACGTCAC |
| chr18 | 58335362 | 58335383 | +/- | 22 | GGGATTCTGATCTCACGTCACC |
| chr18 | 58335363 | 58335384 | +/- | 22 | GGATTCTGATCTCACGTCACCT |
| chr18 | 58335364 | 58335385 | +/- | 22 | GATTCTGATCTCACGTCACCTG |
| chr18 | 58335365 | 58335386 | +/- | 22 | ATTCTGATCTCACGTCACCTGC |
| chr18 | 58335366 | 58335387 | +/- | 22 | TTCTGATCTCACGTCACCTGCC |
| chr18 | 58335367 | 58335388 | +/- | 22 | TCTGATCTCACGTCACCTGCCT |
| chr18 | 58335368 | 58335389 | +/- | 22 | CTGATCTCACGTCACCTGCCTT |
| chr18 | 58335369 | 58335390 | +/- | 22 | TGATCTCACGTCACCTGCCTTA |
| chr18 | 58335370 | 58335391 | +/- | 22 | GATCTCACGTCACCTGCCTTAC |
| chr18 | 58335371 | 58335392 | +/- | 22 | ATCTCACGTCACCTGCCTTACA |
| chr18 | 58335372 | 58335393 | +/- | 22 | TCTCACGTCACCTGCCTTACAG |
| chr18 | 58335373 | 58335394 | +/- | 22 | CTCACGTCACCTGCCTTACAGC |
| chr18 | 58335374 | 58335395 | +/- | 22 | TCACGTCACCTGCCTTACAGCG |
| chr18 | 58335375 | 58335396 | +/- | 22 | CACGTCACCTGCCTTACAGCGC |
| chr18 | 58335376 | 58335397 | +/- | 22 | ACGTCACCTGCCTTACAGCGCT |
| chr18 | 58335377 | 58335398 | +/- | 22 | CGTCACCTGCCTTACAGCGCTG |
| chr18 | 58335378 | 58335399 | +/- | 22 | GTCACCTGCCTTACAGCGCTGC |
| chr18 | 58335379 | 58335400 | +/- | 22 | TCACCTGCCTTACAGCGCTGCC |
| chr18 | 58335380 | 58335401 | +/- | 22 | CACCTGCCTTACAGCGCTGCCA |
| chr18 | 58335381 | 58335402 | +/- | 22 | ACCTGCCTTACAGCGCTGCCAC |
| chr18 | 58335382 | 58335403 | +/- | 22 | CCTGCCTTACAGCGCTGCCACA |
| chr18 | 58335383 | 58335404 | +/- | 22 | CTGCCTTACAGCGCTGCCACAG |
| chr18 | 58335384 | 58335405 | +/- | 22 | TGCCTTACAGCGCTGCCACAGC |
| chr18 | 58335385 | 58335406 | +/- | 22 | GCCTTACAGCGCTGCCACAGCA |
| chr18 | 58335386 | 58335407 | +/- | 22 | CCTTACAGCGCTGCCACAGCAG |
| chr18 | 58335387 | 58335408 | +/- | 22 | CTTACAGCGCTGCCACAGCAGT |
| chr18 | 58335388 | 58335409 | +/- | 22 | TTACAGCGCTGCCACAGCAGTG |
| chr18 | 58335389 | 58335410 | +/- | 22 | TACAGCGCTGCCACAGCAGTGG |
| chr18 | 58335390 | 58335411 | +/- | 22 | ACAGCGCTGCCACAGCAGTGGG |
| chr18 | 58335391 | 58335412 | +/- | 22 | CAGCGCTGCCACAGCAGTGGGC |
| chr18 | 58335392 | 58335413 | +/- | 22 | AGCGCTGCCACAGCAGTGGGCC |
| chr18 | 58335393 | 58335414 | +/- | 22 | GCGCTGCCACAGCAGTGGGCCC |
| chr18 | 58335394 | 58335415 | +/- | 22 | CGCTGCCACAGCAGTGGGCCCT |
| chr18 | 58335395 | 58335416 | +/- | 22 | GCTGCCACAGCAGTGGGCCCTG |
| chr18 | 58335396 | 58335417 | +/- | 22 | CTGCCACAGCAGTGGGCCCTGA |
| chr18 | 58335397 | 58335418 | +/- | 22 | TGCCACAGCAGTGGGCCCTGAT |
| chr18 | 58335398 | 58335419 | +/- | 22 | GCCACAGCAGTGGGCCCTGATT |
| chr18 | 58335399 | 58335420 | +/- | 22 | CCACAGCAGTGGGCCCTGATTC |
| chr18 | 58335400 | 58335421 | +/- | 22 | CACAGCAGTGGGCCCTGATTCA |
| chr18 | 58335401 | 58335422 | +/- | 22 | ACAGCAGTGGGCCCTGATTCAG |
| chr18 | 58335402 | 58335423 | +/- | 22 | CAGCAGTGGGCCCTGATTCAGA |
| chr18 | 58335403 | 58335424 | +/- | 22 | AGCAGTGGGCCCTGATTCAGAC |
| chr18 | 58335404 | 58335425 | +/- | 22 | GCAGTGGGCCCTGATTCAGACA |
| chr18 | 58335405 | 58335426 | +/- | 22 | CAGTGGGCCCTGATTCAGACAG |
| chr18 | 58335406 | 58335427 | +/- | 22 | AGTGGGCCCTGATTCAGACAGC |
| chr18 | 58335407 | 58335428 | +/- | 22 | GTGGGCCCTGATTCAGACAGCA |
| chr18 | 58335408 | 58335429 | +/- | 22 | TGGGCCCTGATTCAGACAGCAG |
| chr18 | 58335409 | 58335430 | +/- | 22 | GGGCCCTGATTCAGACAGCAGG |
| chr18 | 58335317 | 58335338 | +/- | 22 | TGGTCATCAGCGACTGCTGGCT |
| chr18 | 58335410 | 58335431 | +/- | 22 | GGCCCTGATTCAGACAGCAGGG |
| chr18 | 58335411 | 58335432 | +/- | 22 | GCCCTGATTCAGACAGCAGGGG |
| chr18 | 58335412 | 58335433 | +/- | 22 | CCCTGATTCAGACAGCAGGGGG |
| chr18 | 58335413 | 58335434 | +/- | 22 | CCTGATTCAGACAGCAGGGGGT |
| chr18 | 58335414 | 58335435 | +/- | 22 | CTGATTCAGACAGCAGGGGGTC |
| chr18 | 58335415 | 58335436 | +/- | 22 | TGATTCAGACAGCAGGGGGTCA |
| chr18 | 58335416 | 58335437 | +/- | 22 | GATTCAGACAGCAGGGGGTCAT |
| chr18 | 58335417 | 58335438 | +/- | 22 | ATTCAGACAGCAGGGGGTCATC |
| chr18 | 58335418 | 58335439 | +/- | 22 | TTCAGACAGCAGGGGGTCATCC |
| chr18 | 58335419 | 58335440 | +/- | 22 | TCAGACAGCAGGGGGTCATCCC |
| chr18 | 58335420 | 58335441 | +/- | 22 | CAGACAGCAGGGGGTCATCCCC |
| chr18 | 58335421 | 58335442 | +/- | 22 | AGACAGCAGGGGGTCATCCCCT |
| chr18 | 58335422 | 58335443 | +/- | 22 | GACAGCAGGGGGTCATCCCCTA |
| chr18 | 58335423 | 58335444 | +/- | 22 | ACAGCAGGGGGTCATCCCCTAA |
| chr18 | 58335424 | 58335445 | +/- | 22 | CAGCAGGGGGTCATCCCCTAAG |
| chr18 | 58335425 | 58335446 | +/- | 22 | AGCAGGGGGTCATCCCCTAAGT |
| chr18 | 58335426 | 58335447 | +/- | 22 | GCAGGGGGTCATCCCCTAAGTG |

Also disclosed herein are some additional target-specific ASOs (Tables 16-33) which may be used methods and/or systems of the present disclosure. The ASOs may be used to induce an isoform switch or modulate (e.g., inhibit or enhance) the biological activity of a specific isoform of one or several of the genes described above or elsewhere herein (e.g., genes comprising one or more of NEDD4L (ENV2), MAP3K7 (ENV3), NFYA (ENV11), ESYT2 (ENV21), MARK2 (ENV18), ST7 (ENV19), ARVCF (ENV22), SYTL2 (ENV17), R3HDM1 (ENV23), COL4A3BP (ENV9), TANGO2 (ENV6), SEPT9 (ENV15), ROBO1 (ENV4), FAM122B (ENV5), CD47 (ENV13), LSR (ENV20), PBX1 (ENV16), EPB41 (ENV14), ADAM15 (ENV7), EPB41L1 (ENV8), ABI1 (ENV10), FLNB (ENV1), CTNND1 (ENV12), GPR160 (ENV24), ITGB3BP (ENV25), INCENP (ENV26), DENND1B (ENV27), CA12 (ENV28)).

As discussed above, oligonucleotide sequences comprised in a table may be specific for a single target, or for more than one target. In some cases, oligonucleotide sequences comprised in more than one tables are specific for a single target. For example, oligonucleotide sequences comprised in Tables 16-33 may each be specific for a single target. The targets may be the same as or differ from the target(s) which the oligonucleotide sequences comprised in Tables 2-15 are specific for. The target(s) may be one or more genes described above or elsewhere herein. In some cases, ASOs included in Table 16 are specific for NEDD4L (ENV2). In some cases, ASOs included in Table 17 are specific for MAP3K7 (ENV3). In some cases, ASOs included in Table 18 are specific for ROBO1 (ENV4). In some cases, ASOs included in Table 19 are specific for FAM122B (ENV5). In some cases, ASOs included in Table 20 are specific for TANGO2 (ENV6). In some cases, ASOs included in Table 21 are specific for ADAM15 (ENV7). In some cases, ASOs included in Table 22 are specific for EPB41L1 (ENV8). In some cases, ASOs included in Table 23 are specific for COL4A3BP (ENV9). In some cases, ASOs included in Table 23 are specific for ABIl (ENV10). In some cases, ASOs included in Table 24 are specific for NFYA (ENV11). In some cases, ASOs included in Table 26 are specific for CTNND1 (ENV12). In some cases, ASOs included in Table 27 are specific for SEPT9 (ENV15). In some cases, ASOs included in Table 28 are specific for SYTL2 (ENV17). In some cases, ASOs included in Table 29 are specific for MARK2 (ENV18). In some cases, ASOs included in Table 30 are specific for ST7 (ENV19). In some cases, ASOs included in Table 31 are specific for ESYT2 (ENV21). In some cases, ASOs included in Table 32 are specific for ARVCF (ENV22). In some cases, ASOs included in Table 33 are specific for R3HDM1 (ENV23).

**Table 16: 25-mer target-specific ASOs**

| **CHR** | **START** | **END** | **STRAND** | **kmer** | **SEQUENCE** |
|---|---|---|---|---|---|
| chr18 | 58335427 | 58335451 | + | 25 | CAGGGGGTCATCCCCTAAGTGCATT |
| chr18 | 58335428 | 58335452 | + | 25 | AGGGGGTCATCCCCTAAGTGCATTT |
| chr18 | 58335429 | 58335453 | + | 25 | GGGGGTCATCCCCTAAGTGCATTTC |
| chr18 | 58335430 | 58335454 | + | 25 | GGGGTCATCCCCTAAGTGCATTTCA |
| chr18 | 58335431 | 58335455 | + | 25 | GGGTCATCCCCTAAGTGCATTTCAC |
| chr18 | 58335432 | 58335456 | + | 25 | GGTCATCCCCTAAGTGCATTTCACT |
| chr18 | 58335433 | 58335457 | + | 25 | GTCATCCCCTAAGTGCATTTCACTG |
| chr18 | 58335434 | 58335458 | + | 25 | TCATCCCCTAAGTGCATTTCACTGA |
| chr18 | 58335435 | 58335459 | + | 25 | CATCCCCTAAGTGCATTTCACTGAT |
| chr18 | 58335436 | 58335460 | + | 25 | ATCCCCTAAGTGCATTTCACTGATG |
| chr18 | 58335437 | 58335461 | + | 25 | TCCCCTAAGTGCATTTCACTGATGT |
| chr18 | 58335438 | 58335462 | + | 25 | CCCCTAAGTGCATTTCACTGATGTA |
| chr18 | 58335439 | 58335463 | + | 25 | CCCTAAGTGCATTTCACTGATGTAA |
| chr18 | 58335440 | 58335464 | + | 25 | CCTAAGTGCATTTCACTGATGTAAA |
| chr18 | 58335441 | 58335465 | + | 25 | CTAAGTGCATTTCACTGATGTAAAT |
| chr18 | 58335442 | 58335466 | + | 25 | TAAGTGCATTTCACTGATGTAAATT |
| chr18 | 58335443 | 58335467 | + | 25 | AAGTGCATTTCACTGATGTAAATTA |
| chr18 | 58335444 | 58335468 | + | 25 | AGTGCATTTCACTGATGTAAATTAT |
| chr18 | 58335445 | 58335469 | + | 25 | GTGCATTTCACTGATGTAAATTATC |
| chr18 | 58335446 | 58335470 | + | 25 | TGCATTTCACTGATGTAAATTATCA |
| chr18 | 58335447 | 58335471 | + | 25 | GCATTTCACTGATGTAAATTATCAT |
| chr18 | 58335448 | 58335472 | + | 25 | CATTTCACTGATGTAAATTATCATT |
| chr18 | 58335449 | 58335473 | + | 25 | ATTTCACTGATGTAAATTATCATTC |
| chr18 | 58335450 | 58335474 | + | 25 | TTTCACTGATGTAAATTATCATTCA |
| chr18 | 58335451 | 58335475 | + | 25 | TTCACTGATGTAAATTATCATTCAC |
| chr18 | 58335452 | 58335476 | + | 25 | TCACTGATGTAAATTATCATTCACA |
| chr18 | 58335453 | 58335477 | + | 25 | CACTGATGTAAATTATCATTCACAG |
| chr18 | 58335454 | 58335478 | + | 25 | ACTGATGTAAATTATCATTCACAGC |
| chr18 | 58335455 | 58335479 | + | 25 | CTGATGTAAATTATCATTCACAGCC |
| chr18 | 58335456 | 58335480 | + | 25 | TGATGTAAATTATCATTCACAGCCC |
| chr18 | 58335457 | 58335481 | + | 25 | GATGTAAATTATCATTCACAGCCCA |
| chr18 | 58335458 | 58335482 | + | 25 | ATGTAAATTATCATTCACAGCCCAG |
| chr18 | 58335459 | 58335483 | + | 25 | TGTAAATTATCATTCACAGCCCAGT |
| chr18 | 58335460 | 58335484 | + | 25 | GTAAATTATCATTCACAGCCCAGTG |
| chr18 | 58335461 | 58335485 | + | 25 | TAAATTATCATTCACAGCCCAGTGC |
| chr18 | 58335462 | 58335486 | + | 25 | AAATTATCATTCACAGCCCAGTGCC |
| chr18 | 58335463 | 58335487 | + | 25 | AATTATCATTCACAGCCCAGTGCCC |
| chr18 | 58335464 | 58335488 | + | 25 | ATTATCATTCACAGCCCAGTGCCCC |
| chr18 | 58335465 | 58335489 | + | 25 | TTATCATTCACAGCCCAGTGCCCCA |
| chr18 | 58335466 | 58335490 | + | 25 | TATCATTCACAGCCCAGTGCCCCAG |
| chr18 | 58335467 | 58335491 | + | 25 | ATCATTCACAGCCCAGTGCCCCAGC |
| chr18 | 58335468 | 58335492 | + | 25 | TCATTCACAGCCCAGTGCCCCAGCT |
| chr18 | 58335469 | 58335493 | + | 25 | CATTCACAGCCCAGTGCCCCAGCTG |
| chr18 | 58335470 | 58335494 | + | 25 | ATTCACAGCCCAGTGCCCCAGCTGG |
| chr18 | 58335471 | 58335495 | + | 25 | TTCACAGCCCAGTGCCCCAGCTGGG |
| chr18 | 58335472 | 58335496 | + | 25 | TCACAGCCCAGTGCCCCAGCTGGGA |
| chr18 | 58335473 | 58335497 | + | 25 | CACAGCCCAGTGCCCCAGCTGGGAG |
| chr18 | 58335474 | 58335498 | + | 25 | ACAGCCCAGTGCCCCAGCTGGGAGA |
| chr18 | 58335475 | 58335499 | + | 25 | CAGCCCAGTGCCCCAGCTGGGAGAG |
| chr18 | 58335476 | 58335500 | + | 25 | AGCCCAGTGCCCCAGCTGGGAGAGC |
| chr18 | 58335477 | 58335501 | + | 25 | GCCCAGTGCCCCAGCTGGGAGAGCG |
| chr18 | 58335478 | 58335502 | + | 25 | CCCAGTGCCCCAGCTGGGAGAGCGC |
| chr18 | 58335479 | 58335503 | + | 25 | CCAGTGCCCCAGCTGGGAGAGCGCG |
| chr18 | 58335480 | 58335504 | + | 25 | CAGTGCCCCAGCTGGGAGAGCGCGT |
| chr18 | 58335481 | 58335505 | + | 25 | AGTGCCCCAGCTGGGAGAGCGCGTT |
| chr18 | 58335482 | 58335506 | + | 25 | GTGCCCCAGCTGGGAGAGCGCGTTC |
| chr18 | 58335483 | 58335507 | + | 25 | TGCCCCAGCTGGGAGAGCGCGTTCA |
| chr18 | 58335484 | 58335508 | + | 25 | GCCCCAGCTGGGAGAGCGCGTTCAT |
| chr18 | 58335485 | 58335509 | + | 25 | CCCCAGCTGGGAGAGCGCGTTCATC |
| chr18 | 58335486 | 58335510 | + | 25 | CCCAGCTGGGAGAGCGCGTTCATCA |
| chr18 | 58335487 | 58335511 | + | 25 | CCAGCTGGGAGAGCGCGTTCATCAA |
| chr18 | 58335488 | 58335512 | + | 25 | CAGCTGGGAGAGCGCGTTCATCAAC |
| chr18 | 58335489 | 58335513 | + | 25 | AGCTGGGAGAGCGCGTTCATCAACT |
| chr18 | 58335490 | 58335514 | + | 25 | GCTGGGAGAGCGCGTTCATCAACTG |
| chr18 | 58335491 | 58335515 | + | 25 | CTGGGAGAGCGCGTTCATCAACTGT |
| chr18 | 58335492 | 58335516 | + | 25 | TGGGAGAGCGCGTTCATCAACTGTC |
| chr18 | 58335493 | 58335517 | + | 25 | GGGAGAGCGCGTTCATCAACTGTCA |
| chr18 | 58335494 | 58335518 | + | 25 | GGAGAGCGCGTTCATCAACTGTCAC |
| chr18 | 58335495 | 58335519 | + | 25 | GAGAGCGCGTTCATCAACTGTCACG |
| chr18 | 58335496 | 58335520 | + | 25 | AGAGCGCGTTCATCAACTGTCACGG |
| chr18 | 58335497 | 58335521 | + | 25 | GAGCGCGTTCATCAACTGTCACGGG |
| chr18 | 58335498 | 58335522 | + | 25 | AGCGCGTTCATCAACTGTCACGGGT |
| chr18 | 58335499 | 58335523 | + | 25 | GCGCGTTCATCAACTGTCACGGGTG |
| chr18 | 58335500 | 58335524 | + | 25 | CGCGTTCATCAACTGTCACGGGTGG |
| chr18 | 58335501 | 58335525 | + | 25 | GCGTTCATCAACTGTCACGGGTGGT |
| chr18 | 58335502 | 58335526 | + | 25 | CGTTCATCAACTGTCACGGGTGGTG |
| chr18 | 58335503 | 58335527 | + | 25 | GTTCATCAACTGTCACGGGTGGTGA |
| chr18 | 58335504 | 58335528 | + | 25 | TTCATCAACTGTCACGGGTGGTGAG |
| chr18 | 58335505 | 58335529 | + | 25 | TCATCAACTGTCACGGGTGGTGAGG |
| chr18 | 58335506 | 58335530 | + | 25 | CATCAACTGTCACGGGTGGTGAGGA |
| chr18 | 58335507 | 58335531 | + | 25 | ATCAACTGTCACGGGTGGTGAGGAA |
| chr18 | 58335508 | 58335532 | + | 25 | TCAACTGTCACGGGTGGTGAGGAAC |
| chr18 | 58335509 | 58335533 | + | 25 | CAACTGTCACGGGTGGTGAGGAACC |
| chr18 | 58335510 | 58335534 | + | 25 | AACTGTCACGGGTGGTGAGGAACCA |
| chr18 | 58335511 | 58335535 | + | 25 | ACTGTCACGGGTGGTGAGGAACCAA |
| chr18 | 58335512 | 58335536 | + | 25 | CTGTCACGGGTGGTGAGGAACCAAC |
| chr18 | 58335513 | 58335537 | + | 25 | TGTCACGGGTGGTGAGGAACCAACG |
| chr18 | 58335514 | 58335538 | + | 25 | GTCACGGGTGGTGAGGAACCAACGG |
| chr18 | 58335515 | 58335539 | + | 25 | TCACGGGTGGTGAGGAACCAACGGT |
| chr18 | 58335516 | 58335540 | + | 25 | CACGGGTGGTGAGGAACCAACGGTA |
| chr18 | 58335517 | 58335541 | + | 25 | ACGGGTGGTGAGGAACCAACGGTAA |
| chr18 | 58335518 | 58335542 | + | 25 | CGGGTGGTGAGGAACCAACGGTAAT |
| chr18 | 58335519 | 58335543 | + | 25 | GGGTGGTGAGGAACCAACGGTAATG |
| chr18 | 58335520 | 58335544 | + | 25 | GGTGGTGAGGAACCAACGGTAATGA |
| chr18 | 58335521 | 58335545 | + | 25 | GTGGTGAGGAACCAACGGTAATGAT |
| chr18 | 58335522 | 58335546 | + | 25 | TGGTGAGGAACCAACGGTAATGATC |
| chr18 | 58335523 | 58335547 | + | 25 | GGTGAGGAACCAACGGTAATGATCC |
| chr18 | 58335524 | 58335548 | + | 25 | GTGAGGAACCAACGGTAATGATCCA |
| chr18 | 58335525 | 58335549 | + | 25 | TGAGGAACCAACGGTAATGATCCAC |
| chr18 | 58335526 | 58335550 | + | 25 | GAGGAACCAACGGTAATGATCCACT |
| chr18 | 58335527 | 58335551 | + | 25 | AGGAACCAACGGTAATGATCCACTT |
| chr18 | 58335528 | 58335552 | + | 25 | GGAACCAACGGTAATGATCCACTTT |
| chr18 | 58335529 | 58335553 | + | 25 | GAACCAACGGTAATGATCCACTTTA |
| chr18 | 58335530 | 58335554 | + | 25 | AACCAACGGTAATGATCCACTTTAT |
| chr18 | 58335531 | 58335555 | + | 25 | ACCAACGGTAATGATCCACTTTATC |
| chr18 | 58335532 | 58335556 | + | 25 | CCAACGGTAATGATCCACTTTATCA |
| chr18 | 58335533 | 58335557 | + | 25 | CAACGGTAATGATCCACTTTATCAG |
| chr18 | 58335534 | 58335558 | + | 25 | AACGGTAATGATCCACTTTATCAGA |
| chr18 | 58335535 | 58335559 | + | 25 | ACGGTAATGATCCACTTTATCAGAC |
| chr18 | 58335536 | 58335560 | + | 25 | CGGTAATGATCCACTTTATCAGACA |
| chr18 | 58335537 | 58335561 | + | 25 | GGTAATGATCCACTTTATCAGACAT |
| chr18 | 58335538 | 58335562 | + | 25 | GTAATGATCCACTTTATCAGACATC |
| chr18 | 58335539 | 58335563 | + | 25 | TAATGATCCACTTTATCAGACATCA |
| chr18 | 58335540 | 58335564 | + | 25 | AATGATCCACTTTATCAGACATCAA |
| chr18 | 58335541 | 58335565 | + | 25 | ATGATCCACTTTATCAGACATCAAT |
| chr18 | 58335542 | 58335566 | + | 25 | TGATCCACTTTATCAGACATCAATA |
| chr18 | 58335543 | 58335567 | + | 25 | GATCCACTTTATCAGACATCAATAG |
| chr18 | 58335544 | 58335568 | + | 25 | ATCCACTTTATCAGACATCAATAGC |
| chr18 | 58335545 | 58335569 | + | 25 | TCCACTTTATCAGACATCAATAGCA |
| chr18 | 58335546 | 58335570 | + | 25 | CCACTTTATCAGACATCAATAGCAA |
| chr18 | 58335547 | 58335571 | + | 25 | CACTTTATCAGACATCAATAGCAAG |
| chr18 | 58335548 | 58335572 | + | 25 | ACTTTATCAGACATCAATAGCAAGA |
| chr18 | 58335549 | 58335573 | + | 25 | CTTTATCAGACATCAATAGCAAGAG |
| chr18 | 58335550 | 58335574 | + | 25 | TTTATCAGACATCAATAGCAAGAGG |
| chr18 | 58335551 | 58335575 | + | 25 | TTATCAGACATCAATAGCAAGAGGC |
| chr18 | 58335552 | 58335576 | + | 25 | TATCAGACATCAATAGCAAGAGGCC |
| chr18 | 58335553 | 58335577 | + | 25 | ATCAGACATCAATAGCAAGAGGCCG |
| chr18 | 58335554 | 58335578 | + | 25 | TCAGACATCAATAGCAAGAGGCCGT |
| chr18 | 58335555 | 58335579 | + | 25 | CAGACATCAATAGCAAGAGGCCGTG |
| chr18 | 58335556 | 58335580 | + | 25 | AGACATCAATAGCAAGAGGCCGTGA |
| chr18 | 58335557 | 58335581 | + | 25 | GACATCAATAGCAAGAGGCCGTGAG |
| chr18 | 58335558 | 58335582 | + | 25 | ACATCAATAGCAAGAGGCCGTGAGG |
| chr18 | 58335559 | 58335583 | + | 25 | CATCAATAGCAAGAGGCCGTGAGGC |
| chr18 | 58335560 | 58335584 | + | 25 | ATCAATAGCAAGAGGCCGTGAGGCA |
| chr18 | 58335561 | 58335585 | + | 25 | TCAATAGCAAGAGGCCGTGAGGCAG |
| chr18 | 58335562 | 58335586 | + | 25 | CAATAGCAAGAGGCCGTGAGGCAGT |
| chr18 | 58335563 | 58335587 | + | 25 | AATAGCAAGAGGCCGTGAGGCAGTT |

**Table 17: 25-mer target-specific ASOs**

| **CHR** | **START** | **END** | **STRAND** | **kmer** | **SEQUENCE** |
|---|---|---|---|---|---|
| chr6 | 90544501 | 90544525 | + | 25 | TCATTATTCCGGTTCCACAGCTATT |
| chr6 | 90544502 | 90544526 | + | 25 | CATTATTCCGGTTCCACAGCTATTA |
| chr6 | 90544503 | 90544527 | + | 25 | ATTATTCCGGTTCCACAGCTATTAG |
| chr6 | 90544504 | 90544528 | + | 25 | TTATTCCGGTTCCACAGCTATTAGA |
| chr6 | 90544505 | 90544529 | + | 25 | TATTCCGGTTCCACAGCTATTAGAC |
| chr6 | 90544506 | 90544530 | + | 25 | ATTCCGGTTCCACAGCTATTAGACC |
| chr6 | 90544507 | 90544531 | + | 25 | TTCCGGTTCCACAGCTATTAGACCA |
| chr6 | 90544508 | 90544532 | + | 25 | TCCGGTTCCACAGCTATTAGACCAA |
| chr6 | 90544509 | 90544533 | + | 25 | CCGGTTCCACAGCTATTAGACCAAC |
| chr6 | 90544510 | 90544534 | + | 25 | CGGTTCCACAGCTATTAGACCAACT |
| chr6 | 90544511 | 90544535 | + | 25 | GGTTCCACAGCTATTAGACCAACTC |
| chr6 | 90544512 | 90544536 | + | 25 | GTTCCACAGCTATTAGACCAACTCA |
| chr6 | 90544513 | 90544537 | + | 25 | TTCCACAGCTATTAGACCAACTCAG |
| chr6 | 90544514 | 90544538 | + | 25 | TCCACAGCTATTAGACCAACTCAGG |
| chr6 | 90544515 | 90544539 | + | 25 | CCACAGCTATTAGACCAACTCAGGT |
| chr6 | 90544516 | 90544540 | + | 25 | CACAGCTATTAGACCAACTCAGGTG |
| chr6 | 90544517 | 90544541 | + | 25 | ACAGCTATTAGACCAACTCAGGTGG |
| chr6 | 90544518 | 90544542 | + | 25 | CAGCTATTAGACCAACTCAGGTGGT |
| chr6 | 90544519 | 90544543 | + | 25 | AGCTATTAGACCAACTCAGGTGGTC |
| chr6 | 90544520 | 90544544 | + | 25 | GCTATTAGACCAACTCAGGTGGTCT |
| chr6 | 90544521 | 90544545 | + | 25 | CTATTAGACCAACTCAGGTGGTCTA |
| chr6 | 90544522 | 90544546 | + | 25 | TATTAGACCAACTCAGGTGGTCTAT |
| chr6 | 90544523 | 90544547 | + | 25 | ATTAGACCAACTCAGGTGGTCTATG |
| chr6 | 90544524 | 90544548 | + | 25 | TTAGACCAACTCAGGTGGTCTATGA |
| chr6 | 90544525 | 90544549 | + | 25 | TAGACCAACTCAGGTGGTCTATGAT |
| chr6 | 90544526 | 90544550 | + | 25 | AGACCAACTCAGGTGGTCTATGATA |
| chr6 | 90544527 | 90544551 | + | 25 | GACCAACTCAGGTGGTCTATGATAC |
| chr6 | 90544528 | 90544552 | + | 25 | ACCAACTCAGGTGGTCTATGATACC |
| chr6 | 90544529 | 90544553 | + | 25 | CCAACTCAGGTGGTCTATGATACCT |
| chr6 | 90544530 | 90544554 | + | 25 | CAACTCAGGTGGTCTATGATACCTG |
| chr6 | 90544531 | 90544555 | + | 25 | AACTCAGGTGGTCTATGATACCTGA |
| chr6 | 90544532 | 90544556 | + | 25 | ACTCAGGTGGTCTATGATACCTGAT |
| chr6 | 90544533 | 90544557 | + | 25 | CTCAGGTGGTCTATGATACCTGATA |
| chr6 | 90544534 | 90544558 | + | 25 | TCAGGTGGTCTATGATACCTGATAT |
| chr6 | 90544535 | 90544559 | + | 25 | CAGGTGGTCTATGATACCTGATATG |
| chr6 | 90544536 | 90544560 | + | 25 | AGGTGGTCTATGATACCTGATATGA |
| chr6 | 90544537 | 90544561 | + | 25 | GGTGGTCTATGATACCTGATATGAC |
| chr6 | 90544538 | 90544562 | + | 25 | GTGGTCTATGATACCTGATATGACG |
| chr6 | 90544539 | 90544563 | + | 25 | TGGTCTATGATACCTGATATGACGA |
| chr6 | 90544540 | 90544564 | + | 25 | GGTCTATGATACCTGATATGACGAT |
| chr6 | 90544541 | 90544565 | + | 25 | GTCTATGATACCTGATATGACGATC |
| chr6 | 90544542 | 90544566 | + | 25 | TCTATGATACCTGATATGACGATCT |
| chr6 | 90544543 | 90544567 | + | 25 | CTATGATACCTGATATGACGATCTC |
| chr6 | 90544544 | 90544568 | + | 25 | TATGATACCTGATATGACGATCTCA |
| chr6 | 90544545 | 90544569 | + | 25 | ATGATACCTGATATGACGATCTCAG |
| chr6 | 90544546 | 90544570 | + | 25 | TGATACCTGATATGACGATCTCAGG |
| chr6 | 90544547 | 90544571 | + | 25 | GATACCTGATATGACGATCTCAGGG |
| chr6 | 90544548 | 90544572 | + | 25 | ATACCTGATATGACGATCTCAGGGA |
| chr6 | 90544549 | 90544573 | + | 25 | TACCTGATATGACGATCTCAGGGAC |
| chr6 | 90544550 | 90544574 | + | 25 | ACCTGATATGACGATCTCAGGGACA |
| chr6 | 90544551 | 90544575 | + | 25 | CCTGATATGACGATCTCAGGGACAT |
| chr6 | 90544552 | 90544576 | + | 25 | CTGATATGACGATCTCAGGGACATC |
| chr6 | 90544553 | 90544577 | + | 25 | TGATATGACGATCTCAGGGACATCC |
| chr6 | 90544554 | 90544578 | + | 25 | GATATGACGATCTCAGGGACATCCA |
| chr6 | 90544555 | 90544579 | + | 25 | ATATGACGATCTCAGGGACATCCAG |
| chr6 | 90544556 | 90544580 | + | 25 | TATGACGATCTCAGGGACATCCAGA |
| chr6 | 90544557 | 90544581 | + | 25 | ATGACGATCTCAGGGACATCCAGAA |
| chr6 | 90544558 | 90544582 | + | 25 | TGACGATCTCAGGGACATCCAGAAT |
| chr6 | 90544559 | 90544583 | + | 25 | GACGATCTCAGGGACATCCAGAATG |
| chr6 | 90544560 | 90544584 | + | 25 | ACGATCTCAGGGACATCCAGAATGT |
| chr6 | 90544561 | 90544585 | + | 25 | CGATCTCAGGGACATCCAGAATGTT |
| chr6 | 90544562 | 90544586 | + | 25 | GATCTCAGGGACATCCAGAATGTTG |
| chr6 | 90544563 | 90544587 | + | 25 | ATCTCAGGGACATCCAGAATGTTGC |
| chr6 | 90544564 | 90544588 | + | 25 | TCTCAGGGACATCCAGAATGTTGCC |
| chr6 | 90544565 | 90544589 | + | 25 | CTCAGGGACATCCAGAATGTTGCCA |
| chr6 | 90544566 | 90544590 | + | 25 | TCAGGGACATCCAGAATGTTGCCAA |
| chr6 | 90544567 | 90544591 | + | 25 | CAGGGACATCCAGAATGTTGCCAAA |
| chr6 | 90544568 | 90544592 | + | 25 | AGGGACATCCAGAATGTTGCCAAAT |
| chr6 | 90544569 | 90544593 | + | 25 | GGGACATCCAGAATGTTGCCAAATG |
| chr6 | 90544570 | 90544594 | + | 25 | GGACATCCAGAATGTTGCCAAATGA |
| chr6 | 90544571 | 90544595 | + | 25 | GACATCCAGAATGTTGCCAAATGAA |
| chr6 | 90544572 | 90544596 | + | 25 | ACATCCAGAATGTTGCCAAATGAAG |
| chr6 | 90544573 | 90544597 | + | 25 | CATCCAGAATGTTGCCAAATGAAGC |
| chr6 | 90544574 | 90544598 | + | 25 | ATCCAGAATGTTGCCAAATGAAGCA |
| chr6 | 90544575 | 90544599 | + | 25 | TCCAGAATGTTGCCAAATGAAGCAG |
| chr6 | 90544576 | 90544600 | + | 25 | CCAGAATGTTGCCAAATGAAGCAGT |
| chr6 | 90544577 | 90544601 | + | 25 | CAGAATGTTGCCAAATGAAGCAGTT |
| chr6 | 90544578 | 90544602 | + | 25 | AGAATGTTGCCAAATGAAGCAGTTT |
| chr6 | 90544579 | 90544603 | + | 25 | GAATGTTGCCAAATGAAGCAGTTTT |
| chr6 | 90544580 | 90544604 | + | 25 | AATGTTGCCAAATGAAGCAGTTTTA |
| chr6 | 90544581 | 90544605 | + | 25 | ATGTTGCCAAATGAAGCAGTTTTAC |
| chr6 | 90544582 | 90544606 | + | 25 | TGTTGCCAAATGAAGCAGTTTTACG |
| chr6 | 90544583 | 90544607 | + | 25 | GTTGCCAAATGAAGCAGTTTTACGG |
| chr6 | 90544584 | 90544608 | + | 25 | TTGCCAAATGAAGCAGTTTTACGGT |
| chr6 | 90544585 | 90544609 | + | 25 | TGCCAAATGAAGCAGTTTTACGGTG |
| chr6 | 90544586 | 90544610 | + | 25 | GCCAAATGAAGCAGTTTTACGGTGG |
| chr6 | 90544587 | 90544611 | + | 25 | CCAAATGAAGCAGTTTTACGGTGGC |
| chr6 | 90544588 | 90544612 | + | 25 | CAAATGAAGCAGTTTTACGGTGGCC |
| chr6 | 90544589 | 90544613 | + | 25 | AAATGAAGCAGTTTTACGGTGGCCC |
| chr6 | 90544590 | 90544614 | + | 25 | AATGAAGCAGTTTTACGGTGGCCCC |
| chr6 | 90544591 | 90544615 | + | 25 | ATGAAGCAGTTTTACGGTGGCCCCG |
| chr6 | 90544592 | 90544616 | + | 25 | TGAAGCAGTTTTACGGTGGCCCCGT |
| chr6 | 90544593 | 90544617 | + | 25 | GAAGCAGTTTTACGGTGGCCCCGTT |
| chr6 | 90544594 | 90544618 | + | 25 | AAGCAGTTTTACGGTGGCCCCGTTT |
| chr6 | 90544595 | 90544619 | + | 25 | AGCAGTTTTACGGTGGCCCCGTTTA |
| chr6 | 90544596 | 90544620 | + | 25 | GCAGTTTTACGGTGGCCCCGTTTAG |
| chr6 | 90544597 | 90544621 | + | 25 | CAGTTTTACGGTGGCCCCGTTTAGG |
| chr6 | 90544598 | 90544622 | + | 25 | AGTTTTACGGTGGCCCCGTTTAGGC |
| chr6 | 90544599 | 90544623 | + | 25 | GTTTTACGGTGGCCCCGTTTAGGCT |
| chr6 | 90544600 | 90544624 | + | 25 | TTTTACGGTGGCCCCGTTTAGGCTT |
| chr6 | 90544601 | 90544625 | + | 25 | TTTACGGTGGCCCCGTTTAGGCTTG |
| chr6 | 90544602 | 90544626 | + | 25 | TTACGGTGGCCCCGTTTAGGCTTGG |
| chr6 | 90544603 | 90544627 | + | 25 | TACGGTGGCCCCGTTTAGGCTTGGA |
| chr6 | 90544604 | 90544628 | + | 25 | ACGGTGGCCCCGTTTAGGCTTGGAA |
| chr6 | 90544605 | 90544629 | + | 25 | CGGTGGCCCCGTTTAGGCTTGGAAT |
| chr6 | 90544606 | 90544630 | + | 25 | GGTGGCCCCGTTTAGGCTTGGAATA |
| chr6 | 90544607 | 90544631 | + | 25 | GTGGCCCCGTTTAGGCTTGGAATAG |
| chr6 | 90544608 | 90544632 | + | 25 | TGGCCCCGTTTAGGCTTGGAATAGG |
| chr6 | 90544609 | 90544633 | + | 25 | GGCCCCGTTTAGGCTTGGAATAGGC |
| chr6 | 90544610 | 90544634 | + | 25 | GCCCCGTTTAGGCTTGGAATAGGCT |
| chr6 | 90544611 | 90544635 | + | 25 | CCCCGTTTAGGCTTGGAATAGGCTG |
| chr6 | 90544612 | 90544636 | + | 25 | CCCGTTTAGGCTTGGAATAGGCTGC |
| chr6 | 90544613 | 90544637 | + | 25 | CCGTTTAGGCTTGGAATAGGCTGCA |
| chr6 | 90544614 | 90544638 | + | 25 | CGTTTAGGCTTGGAATAGGCTGCAA |
| chr6 | 90544615 | 90544639 | + | 25 | GTTTAGGCTTGGAATAGGCTGCAAA |
| chr6 | 90544616 | 90544640 | + | 25 | TTTAGGCTTGGAATAGGCTGCAAAA |
| chr6 | 90544617 | 90544641 | + | 25 | TTAGGCTTGGAATAGGCTGCAAAAA |
| chr6 | 90544618 | 90544642 | + | 25 | TAGGCTTGGAATAGGCTGCAAAAAC |
| chr6 | 90544619 | 90544643 | + | 25 | AGGCTTGGAATAGGCTGCAAAAACA |
| chr6 | 90544620 | 90544644 | + | 25 | GGCTTGGAATAGGCTGCAAAAACAC |
| chr6 | 90544621 | 90544645 | + | 25 | GCTTGGAATAGGCTGCAAAAACACA |
| chr6 | 90544622 | 90544646 | + | 25 | CTTGGAATAGGCTGCAAAAACACAT |
| chr6 | 90544623 | 90544647 | + | 25 | TTGGAATAGGCTGCAAAAACACATA |
| chr6 | 90544624 | 90544648 | + | 25 | TGGAATAGGCTGCAAAAACACATAT |
| chr6 | 90544625 | 90544649 | + | 25 | GGAATAGGCTGCAAAAACACATATA |
| chr6 | 90544626 | 90544650 | + | 25 | GAATAGGCTGCAAAAACACATATAT |
| chr6 | 90544627 | 90544651 | + | 25 | AATAGGCTGCAAAAACACATATATA |
| chr6 | 90544628 | 90544652 | + | 25 | ATAGGCTGCAAAAACACATATATAC |
| chr6 | 90544629 | 90544653 | + | 25 | TAGGCTGCAAAAACACATATATACA |
| chr6 | 90544630 | 90544654 | + | 25 | AGGCTGCAAAAACACATATATACAG |
| chr6 | 90544631 | 90544655 | + | 25 | GGCTGCAAAAACACATATATACAGT |
| chr6 | 90544632 | 90544656 | + | 25 | GCTGCAAAAACACATATATACAGTA |
| chr6 | 90544633 | 90544657 | + | 25 | CTGCAAAAACACATATATACAGTAT |
| chr6 | 90544634 | 90544658 | + | 25 | TGCAAAAACACATATATACAGTATA |
| chr6 | 90544635 | 90544659 | + | 25 | GCAAAAACACATATATACAGTATAC |
| chr6 | 90544636 | 90544660 | + | 25 | CAAAAACACATATATACAGTATACA |
| chr6 | 90544637 | 90544661 | + | 25 | AAAAACACATATATACAGTATACAT |
| chr6 | 90544638 | 90544662 | + | 25 | AAAACACATATATACAGTATACATG |
| chr6 | 90544639 | 90544663 | + | 25 | AAACACATATATACAGTATACATGC |
| chr6 | 90544640 | 90544664 | + | 25 | AACACATATATACAGTATACATGCA |
| chr6 | 90544641 | 90544665 | + | 25 | ACACATATATACAGTATACATGCAA |
| chr6 | 90544642 | 90544666 | + | 25 | CACATATATACAGTATACATGCAAA |
| chr6 | 90544643 | 90544667 | + | 25 | ACATATATACAGTATACATGCAAAC |
| chr6 | 90544644 | 90544668 | + | 25 | CATATATACAGTATACATGCAAACA |
| chr6 | 90544645 | 90544669 | + | 25 | ATATATACAGTATACATGCAAACAA |
| chr6 | 90544646 | 90544670 | + | 25 | TATATACAGTATACATGCAAACAAC |
| chr6 | 90544647 | 90544671 | + | 25 | ATATACAGTATACATGCAAACAACC |
| chr6 | 90544648 | 90544672 | + | 25 | TATACAGTATACATGCAAACAACCA |
| chr6 | 90544649 | 90544673 | + | 25 | ATACAGTATACATGCAAACAACCAC |
| chr6 | 90544650 | 90544674 | + | 25 | TACAGTATACATGCAAACAACCACA |
| chr6 | 90544651 | 90544675 | + | 25 | ACAGTATACATGCAAACAACCACAA |
| chr6 | 90544652 | 90544676 | + | 25 | CAGTATACATGCAAACAACCACAAA |
| chr6 | 90544653 | 90544677 | + | 25 | AGTATACATGCAAACAACCACAAAC |
| chr6 | 90544654 | 90544678 | + | 25 | GTATACATGCAAACAACCACAAACA |
| chr6 | 90544655 | 90544679 | + | 25 | TATACATGCAAACAACCACAAACAG |
| chr6 | 90544656 | 90544680 | + | 25 | ATACATGCAAACAACCACAAACAGT |
| chr6 | 90544657 | 90544681 | + | 25 | TACATGCAAACAACCACAAACAGTA |
| chr6 | 90544658 | 90544682 | + | 25 | ACATGCAAACAACCACAAACAGTAA |

**Table 18: 25-mer target-specific ASOs**

| **CHR** | **START** | **END** | **STRAND** | **kmer** | **SEQUENCE** |
|---|---|---|---|---|---|
| chr3 | 78647578 | 78647602 | + | 25 | ACACATGCCAAACTAACAATGGAAA |
| chr3 | 78647579 | 78647603 | + | 25 | CACATGCCAAACTAACAATGGAAAG |
| chr3 | 78647580 | 78647604 | + | 25 | ACATGCCAAACTAACAATGGAAAGG |
| chr3 | 78647581 | 78647605 | + | 25 | CATGCCAAACTAACAATGGAAAGGA |
| chr3 | 78647582 | 78647606 | + | 25 | ATGCCAAACTAACAATGGAAAGGAG |
| chr3 | 78647583 | 78647607 | + | 25 | TGCCAAACTAACAATGGAAAGGAGG |
| chr3 | 78647584 | 78647608 | + | 25 | GCCAAACTAACAATGGAAAGGAGGA |
| chr3 | 78647585 | 78647609 | + | 25 | CCAAACTAACAATGGAAAGGAGGAG |
| chr3 | 78647586 | 78647610 | + | 25 | CAAACTAACAATGGAAAGGAGGAGG |
| chr3 | 78647587 | 78647611 | + | 25 | AAACTAACAATGGAAAGGAGGAGGG |
| chr3 | 78647588 | 78647612 | + | 25 | AACTAACAATGGAAAGGAGGAGGGT |
| chr3 | 78647589 | 78647613 | + | 25 | ACTAACAATGGAAAGGAGGAGGGTA |
| chr3 | 78647590 | 78647614 | + | 25 | CTAACAATGGAAAGGAGGAGGGTAA |
| chr3 | 78647591 | 78647615 | + | 25 | TAACAATGGAAAGGAGGAGGGTAAG |
| chr3 | 78647592 | 78647616 | + | 25 | AACAATGGAAAGGAGGAGGGTAAGT |
| chr3 | 78647593 | 78647617 | + | 25 | ACAATGGAAAGGAGGAGGGTAAGTG |
| chr3 | 78647594 | 78647618 | + | 25 | CAATGGAAAGGAGGAGGGTAAGTGC |
| chr3 | 78647595 | 78647619 | + | 25 | AATGGAAAGGAGGAGGGTAAGTGCA |
| chr3 | 78647596 | 78647620 | + | 25 | ATGGAAAGGAGGAGGGTAAGTGCAA |
| chr3 | 78647597 | 78647621 | + | 25 | TGGAAAGGAGGAGGGTAAGTGCAAA |
| chr3 | 78647598 | 78647622 | + | 25 | GGAAAGGAGGAGGGTAAGTGCAAAT |
| chr3 | 78647599 | 78647623 | + | 25 | GAAAGGAGGAGGGTAAGTGCAAATA |
| chr3 | 78647600 | 78647624 | + | 25 | AAAGGAGGAGGGTAAGTGCAAATAT |
| chr3 | 78647601 | 78647625 | + | 25 | AAGGAGGAGGGTAAGTGCAAATATA |
| chr3 | 78647602 | 78647626 | + | 25 | AGGAGGAGGGTAAGTGCAAATATAT |
| chr3 | 78647603 | 78647627 | + | 25 | GGAGGAGGGTAAGTGCAAATATATA |
| chr3 | 78647604 | 78647628 | + | 25 | GAGGAGGGTAAGTGCAAATATATAC |
| chr3 | 78647605 | 78647629 | + | 25 | AGGAGGGTAAGTGCAAATATATACC |
| chr3 | 78647606 | 78647630 | + | 25 | GGAGGGTAAGTGCAAATATATACCT |
| chr3 | 78647607 | 78647631 | + | 25 | GAGGGTAAGTGCAAATATATACCTG |
| chr3 | 78647608 | 78647632 | + | 25 | AGGGTAAGTGCAAATATATACCTGT |
| chr3 | 78647609 | 78647633 | + | 25 | GGGTAAGTGCAAATATATACCTGTT |
| chr3 | 78647610 | 78647634 | + | 25 | GGTAAGTGCAAATATATACCTGTTG |
| chr3 | 78647611 | 78647635 | + | 25 | GTAAGTGCAAATATATACCTGTTGG |
| chr3 | 78647612 | 78647636 | + | 25 | TAAGTGCAAATATATACCTGTTGGT |
| chr3 | 78647613 | 78647637 | + | 25 | AAGTGCAAATATATACCTGTTGGTG |
| chr3 | 78647614 | 78647638 | + | 25 | AGTGCAAATATATACCTGTTGGTGT |
| chr3 | 78647615 | 78647639 | + | 25 | GTGCAAATATATACCTGTTGGTGTG |
| chr3 | 78647616 | 78647640 | + | 25 | TGCAAATATATACCTGTTGGTGTGA |
| chr3 | 78647617 | 78647641 | + | 25 | GCAAATATATACCTGTTGGTGTGAA |
| chr3 | 78647618 | 78647642 | + | 25 | CAAATATATACCTGTTGGTGTGAAG |
| chr3 | 78647619 | 78647643 | + | 25 | AAATATATACCTGTTGGTGTGAAGG |
| chr3 | 78647620 | 78647644 | + | 25 | AATATATACCTGTTGGTGTGAAGGT |
| chr3 | 78647621 | 78647645 | + | 25 | ATATATACCTGTTGGTGTGAAGGTA |
| chr3 | 78647622 | 78647646 | + | 25 | TATATACCTGTTGGTGTGAAGGTAA |
| chr3 | 78647623 | 78647647 | + | 25 | ATATACCTGTTGGTGTGAAGGTAAA |
| chr3 | 78647624 | 78647648 | + | 25 | TATACCTGTTGGTGTGAAGGTAAAA |
| chr3 | 78647625 | 78647649 | + | 25 | ATACCTGTTGGTGTGAAGGTAAAAG |
| chr3 | 78647626 | 78647650 | + | 25 | TACCTGTTGGTGTGAAGGTAAAAGA |
| chr3 | 78647627 | 78647651 | + | 25 | ACCTGTTGGTGTGAAGGTAAAAGAC |
| chr3 | 78647628 | 78647652 | + | 25 | CCTGTTGGTGTGAAGGTAAAAGACG |
| chr3 | 78647629 | 78647653 | + | 25 | CTGTTGGTGTGAAGGTAAAAGACGG |
| chr3 | 78647630 | 78647654 | + | 25 | TGTTGGTGTGAAGGTAAAAGACGGG |
| chr3 | 78647631 | 78647655 | + | 25 | GTTGGTGTGAAGGTAAAAGACGGGA |
| chr3 | 78647632 | 78647656 | + | 25 | TTGGTGTGAAGGTAAAAGACGGGAC |
| chr3 | 78647633 | 78647657 | + | 25 | TGGTGTGAAGGTAAAAGACGGGACT |
| chr3 | 78647634 | 78647658 | + | 25 | GGTGTGAAGGTAAAAGACGGGACTG |
| chr3 | 78647635 | 78647659 | + | 25 | GTGTGAAGGTAAAAGACGGGACTGA |
| chr3 | 78647636 | 78647660 | + | 25 | TGTGAAGGTAAAAGACGGGACTGAA |
| chr3 | 78647637 | 78647661 | + | 25 | GTGAAGGTAAAAGACGGGACTGAAA |
| chr3 | 78647638 | 78647662 | + | 25 | TGAAGGTAAAAGACGGGACTGAAAA |
| chr3 | 78647639 | 78647663 | + | 25 | GAAGGTAAAAGACGGGACTGAAAAA |
| chr3 | 78647640 | 78647664 | + | 25 | AAGGTAAAAGACGGGACTGAAAAAT |
| chr3 | 78647641 | 78647665 | + | 25 | AGGTAAAAGACGGGACTGAAAAATC |
| chr3 | 78647642 | 78647666 | + | 25 | GGTAAAAGACGGGACTGAAAAATCA |
| chr3 | 78647643 | 78647667 | + | 25 | GTAAAAGACGGGACTGAAAAATCAA |
| chr3 | 78647644 | 78647668 | + | 25 | TAAAAGACGGGACTGAAAAATCAAA |
| chr3 | 78647645 | 78647669 | + | 25 | AAAAGACGGGACTGAAAAATCAAAA |
| chr3 | 78647646 | 78647670 | + | 25 | AAAGACGGGACTGAAAAATCAAAAC |
| chr3 | 78647647 | 78647671 | + | 25 | AAGACGGGACTGAAAAATCAAAACA |
| chr3 | 78647648 | 78647672 | + | 25 | AGACGGGACTGAAAAATCAAAACAA |
| chr3 | 78647649 | 78647673 | + | 25 | GACGGGACTGAAAAATCAAAACAAA |
| chr3 | 78647650 | 78647674 | + | 25 | ACGGGACTGAAAAATCAAAACAAAA |
| chr3 | 78647651 | 78647675 | + | 25 | CGGGACTGAAAAATCAAAACAAAAT |
| chr3 | 78647652 | 78647676 | + | 25 | GGGACTGAAAAATCAAAACAAAATA |
| chr3 | 78647653 | 78647677 | + | 25 | GGACTGAAAAATCAAAACAAAATAT |
| chr3 | 78647654 | 78647678 | + | 25 | GACTGAAAAATCAAAACAAAATATA |
| chr3 | 78647655 | 78647679 | + | 25 | ACTGAAAAATCAAAACAAAATATAA |
| chr3 | 78647656 | 78647680 | + | 25 | CTGAAAAATCAAAACAAAATATAAA |
| chr3 | 78647657 | 78647681 | + | 25 | TGAAAAATCAAAACAAAATATAAAC |
| chr3 | 78647658 | 78647682 | + | 25 | GAAAAATCAAAACAAAATATAAACC |
| chr3 | 78647659 | 78647683 | + | 25 | AAAAATCAAAACAAAATATAAACCA |
| chr3 | 78647660 | 78647684 | + | 25 | AAAATCAAAACAAAATATAAACCAG |
| chr3 | 78647661 | 78647685 | + | 25 | AAATCAAAACAAAATATAAACCAGT |
| chr3 | 78647662 | 78647686 | + | 25 | AATCAAAACAAAATATAAACCAGTT |
| chr3 | 78647663 | 78647687 | + | 25 | ATCAAAACAAAATATAAACCAGTTA |
| chr3 | 78647664 | 78647688 | + | 25 | TCAAAACAAAATATAAACCAGTTAT |
| chr3 | 78647665 | 78647689 | + | 25 | CAAAACAAAATATAAACCAGTTATT |
| chr3 | 78647666 | 78647690 | + | 25 | AAAACAAAATATAAACCAGTTATTA |
| chr3 | 78647667 | 78647691 | + | 25 | AAACAAAATATAAACCAGTTATTAA |
| chr3 | 78647668 | 78647692 | + | 25 | AACAAAATATAAACCAGTTATTAAG |
| chr3 | 78647669 | 78647693 | + | 25 | ACAAAATATAAACCAGTTATTAAGC |
| chr3 | 78647670 | 78647694 | + | 25 | CAAAATATAAACCAGTTATTAAGCT |
| chr3 | 78647671 | 78647695 | + | 25 | AAAATATAAACCAGTTATTAAGCTG |
| chr3 | 78647672 | 78647696 | + | 25 | AAATATAAACCAGTTATTAAGCTGA |
| chr3 | 78647673 | 78647697 | + | 25 | AATATAAACCAGTTATTAAGCTGAA |
| chr3 | 78647674 | 78647698 | + | 25 | ATATAAACCAGTTATTAAGCTGAAG |
| chr3 | 78647675 | 78647699 | + | 25 | TATAAACCAGTTATTAAGCTGAAGA |
| chr3 | 78647676 | 78647700 | + | 25 | ATAAACCAGTTATTAAGCTGAAGAG |
| chr3 | 78647677 | 78647701 | + | 25 | TAAACCAGTTATTAAGCTGAAGAGA |
| chr3 | 78647678 | 78647702 | + | 25 | AAACCAGTTATTAAGCTGAAGAGAG |
| chr3 | 78647679 | 78647703 | + | 25 | AACCAGTTATTAAGCTGAAGAGAGA |
| chr3 | 78647680 | 78647704 | + | 25 | ACCAGTTATTAAGCTGAAGAGAGAA |
| chr3 | 78647681 | 78647705 | + | 25 | CCAGTTATTAAGCTGAAGAGAGAAA |

**Table 19: 25-mer target-specific ASOs**

| **CHR** | **START** | **END** | **STRAND** | **kmer** | **SEQUENCE** |
|---|---|---|---|---|---|
| chrX | 134772092 | 134772116 | + | 25 | TTTATGTAGGAAACAGCAGTTAAAA |
| chrX | 134772093 | 134772117 | + | 25 | TTATGTAGGAAACAGCAGTTAAAAC |
| chrX | 134772094 | 134772118 | + | 25 | TATGTAGGAAACAGCAGTTAAAACG |
| chrX | 134772095 | 134772119 | + | 25 | ATGTAGGAAACAGCAGTTAAAACGT |
| chrX | 134772096 | 134772120 | + | 25 | TGTAGGAAACAGCAGTTAAAACGTT |
| chrX | 134772097 | 134772121 | + | 25 | GTAGGAAACAGCAGTTAAAACGTTG |
| chrX | 134772098 | 134772122 | + | 25 | TAGGAAACAGCAGTTAAAACGTTGA |
| chrX | 134772099 | 134772123 | + | 25 | AGGAAACAGCAGTTAAAACGTTGAA |
| chrX | 134772100 | 134772124 | + | 25 | GGAAACAGCAGTTAAAACGTTGAAT |
| chrX | 134772101 | 134772125 | + | 25 | GAAACAGCAGTTAAAACGTTGAATC |
| chrX | 134772102 | 134772126 | + | 25 | AAACAGCAGTTAAAACGTTGAATCA |
| chrX | 134772103 | 134772127 | + | 25 | AACAGCAGTTAAAACGTTGAATCAG |
| chrX | 134772104 | 134772128 | + | 25 | ACAGCAGTTAAAACGTTGAATCAGA |
| chrX | 134772105 | 134772129 | + | 25 | CAGCAGTTAAAACGTTGAATCAGAA |
| chrX | 134772106 | 134772130 | + | 25 | AGCAGTTAAAACGTTGAATCAGAAA |
| chrX | 134772107 | 134772131 | + | 25 | GCAGTTAAAACGTTGAATCAGAAAT |
| chrX | 134772108 | 134772132 | + | 25 | CAGTTAAAACGTTGAATCAGAAATG |
| chrX | 134772109 | 134772133 | + | 25 | AGTTAAAACGTTGAATCAGAAATGG |
| chrX | 134772110 | 134772134 | + | 25 | GTTAAAACGTTGAATCAGAAATGGT |
| chrX | 134772111 | 134772135 | + | 25 | TTAAAACGTTGAATCAGAAATGGTT |
| chrX | 134772112 | 134772136 | + | 25 | TAAAACGTTGAATCAGAAATGGTTA |
| chrX | 134772113 | 134772137 | + | 25 | AAAACGTTGAATCAGAAATGGTTAA |
| chrX | 134772114 | 134772138 | + | 25 | AAACGTTGAATCAGAAATGGTTAAG |
| chrX | 134772115 | 134772139 | + | 25 | AACGTTGAATCAGAAATGGTTAAGT |
| chrX | 134772116 | 134772140 | + | 25 | ACGTTGAATCAGAAATGGTTAAGTC |
| chrX | 134772117 | 134772141 | + | 25 | CGTTGAATCAGAAATGGTTAAGTCA |
| chrX | 134772118 | 134772142 | + | 25 | GTTGAATCAGAAATGGTTAAGTCAC |
| chrX | 134772119 | 134772143 | + | 25 | TTGAATCAGAAATGGTTAAGTCACT |
| chrX | 134772120 | 134772144 | + | 25 | TGAATCAGAAATGGTTAAGTCACTT |
| chrX | 134772121 | 134772145 | + | 25 | GAATCAGAAATGGTTAAGTCACTTG |
| chrX | 134772122 | 134772146 | + | 25 | AATCAGAAATGGTTAAGTCACTTGG |
| chrX | 134772123 | 134772147 | + | 25 | ATCAGAAATGGTTAAGTCACTTGGG |
| chrX | 134772124 | 134772148 | + | 25 | TCAGAAATGGTTAAGTCACTTGGGT |
| chrX | 134772125 | 134772149 | + | 25 | CAGAAATGGTTAAGTCACTTGGGTG |
| chrX | 134772126 | 134772150 | + | 25 | AGAAATGGTTAAGTCACTTGGGTGA |
| chrX | 134772127 | 134772151 | + | 25 | GAAATGGTTAAGTCACTTGGGTGAG |
| chrX | 134772128 | 134772152 | + | 25 | AAATGGTTAAGTCACTTGGGTGAGA |
| chrX | 134772129 | 134772153 | + | 25 | AATGGTTAAGTCACTTGGGTGAGAG |
| chrX | 134772130 | 134772154 | + | 25 | ATGGTTAAGTCACTTGGGTGAGAGA |
| chrX | 134772131 | 134772155 | + | 25 | TGGTTAAGTCACTTGGGTGAGAGAT |
| chrX | 134772132 | 134772156 | + | 25 | GGTTAAGTCACTTGGGTGAGAGATC |
| chrX | 134772133 | 134772157 | + | 25 | GTTAAGTCACTTGGGTGAGAGATCA |
| chrX | 134772134 | 134772158 | + | 25 | TTAAGTCACTTGGGTGAGAGATCAT |
| chrX | 134772135 | 134772159 | + | 25 | TAAGTCACTTGGGTGAGAGATCATC |
| chrX | 134772136 | 134772160 | + | 25 | AAGTCACTTGGGTGAGAGATCATCC |
| chrX | 134772137 | 134772161 | + | 25 | AGTCACTTGGGTGAGAGATCATCCA |
| chrX | 134772138 | 134772162 | + | 25 | GTCACTTGGGTGAGAGATCATCCAT |
| chrX | 134772139 | 134772163 | + | 25 | TCACTTGGGTGAGAGATCATCCATC |
| chrX | 134772140 | 134772164 | + | 25 | CACTTGGGTGAGAGATCATCCATCA |
| chrX | 134772141 | 134772165 | + | 25 | ACTTGGGTGAGAGATCATCCATCAA |
| chrX | 134772142 | 134772166 | + | 25 | CTTGGGTGAGAGATCATCCATCAAG |
| chrX | 134772143 | 134772167 | + | 25 | TTGGGTGAGAGATCATCCATCAAGA |
| chrX | 134772144 | 134772168 | + | 25 | TGGGTGAGAGATCATCCATCAAGAT |
| chrX | 134772145 | 134772169 | + | 25 | GGGTGAGAGATCATCCATCAAGATG |
| chrX | 134772146 | 134772170 | + | 25 | GGTGAGAGATCATCCATCAAGATGA |
| chrX | 134772147 | 134772171 | + | 25 | GTGAGAGATCATCCATCAAGATGAA |
| chrX | 134772148 | 134772172 | + | 25 | TGAGAGATCATCCATCAAGATGAAC |
| chrX | 134772149 | 134772173 | + | 25 | GAGAGATCATCCATCAAGATGAACG |
| chrX | 134772150 | 134772174 | + | 25 | AGAGATCATCCATCAAGATGAACGA |
| chrX | 134772151 | 134772175 | + | 25 | GAGATCATCCATCAAGATGAACGAA |
| chrX | 134772152 | 134772176 | + | 25 | AGATCATCCATCAAGATGAACGAAG |
| chrX | 134772153 | 134772177 | + | 25 | GATCATCCATCAAGATGAACGAAGA |
| chrX | 134772154 | 134772178 | + | 25 | ATCATCCATCAAGATGAACGAAGAG |
| chrX | 134772155 | 134772179 | + | 25 | TCATCCATCAAGATGAACGAAGAGC |
| chrX | 134772156 | 134772180 | + | 25 | CATCCATCAAGATGAACGAAGAGCA |
| chrX | 134772157 | 134772181 | + | 25 | ATCCATCAAGATGAACGAAGAGCAT |
| chrX | 134772158 | 134772182 | + | 25 | TCCATCAAGATGAACGAAGAGCATG |
| chrX | 134772159 | 134772183 | + | 25 | CCATCAAGATGAACGAAGAGCATGA |
| chrX | 134772160 | 134772184 | + | 25 | CATCAAGATGAACGAAGAGCATGAA |
| chrX | 134772161 | 134772185 | + | 25 | ATCAAGATGAACGAAGAGCATGAAT |
| chrX | 134772162 | 134772186 | + | 25 | TCAAGATGAACGAAGAGCATGAATT |
| chrX | 134772163 | 134772187 | + | 25 | CAAGATGAACGAAGAGCATGAATTG |
| chrX | 134772164 | 134772188 | + | 25 | AAGATGAACGAAGAGCATGAATTGG |
| chrX | 134772165 | 134772189 | + | 25 | AGATGAACGAAGAGCATGAATTGGA |
| chrX | 134772166 | 134772190 | + | 25 | GATGAACGAAGAGCATGAATTGGAG |
| chrX | 134772167 | 134772191 | + | 25 | ATGAACGAAGAGCATGAATTGGAGC |
| chrX | 134772168 | 134772192 | + | 25 | TGAACGAAGAGCATGAATTGGAGCA |
| chrX | 134772169 | 134772193 | + | 25 | GAACGAAGAGCATGAATTGGAGCAT |
| chrX | 134772170 | 134772194 | + | 25 | AACGAAGAGCATGAATTGGAGCATG |
| chrX | 134772171 | 134772195 | + | 25 | ACGAAGAGCATGAATTGGAGCATGC |
| chrX | 134772172 | 134772196 | + | 25 | CGAAGAGCATGAATTGGAGCATGCT |
| chrX | 134772173 | 134772197 | + | 25 | GAAGAGCATGAATTGGAGCATGCTA |
| chrX | 134772174 | 134772198 | + | 25 | AAGAGCATGAATTGGAGCATGCTAC |
| chrX | 134772175 | 134772199 | + | 25 | AGAGCATGAATTGGAGCATGCTACT |
| chrX | 134772176 | 134772200 | + | 25 | GAGCATGAATTGGAGCATGCTACTG |
| chrX | 134772177 | 134772201 | + | 25 | AGCATGAATTGGAGCATGCTACTGG |
| chrX | 134772178 | 134772202 | + | 25 | GCATGAATTGGAGCATGCTACTGGA |
| chrX | 134772179 | 134772203 | + | 25 | CATGAATTGGAGCATGCTACTGGAG |
| chrX | 134772180 | 134772204 | + | 25 | ATGAATTGGAGCATGCTACTGGAGA |
| chrX | 134772181 | 134772205 | + | 25 | TGAATTGGAGCATGCTACTGGAGAC |
| chrX | 134772182 | 134772206 | + | 25 | GAATTGGAGCATGCTACTGGAGACT |
| chrX | 134772183 | 134772207 | + | 25 | AATTGGAGCATGCTACTGGAGACTC |
| chrX | 134772184 | 134772208 | + | 25 | ATTGGAGCATGCTACTGGAGACTCT |
| chrX | 134772185 | 134772209 | + | 25 | TTGGAGCATGCTACTGGAGACTCTG |
| chrX | 134772186 | 134772210 | + | 25 | TGGAGCATGCTACTGGAGACTCTGC |
| chrX | 134772187 | 134772211 | + | 25 | GGAGCATGCTACTGGAGACTCTGCG |
| chrX | 134772188 | 134772212 | + | 25 | GAGCATGCTACTGGAGACTCTGCGG |
| chrX | 134772189 | 134772213 | + | 25 | AGCATGCTACTGGAGACTCTGCGGT |
| chrX | 134772190 | 134772214 | + | 25 | GCATGCTACTGGAGACTCTGCGGTA |
| chrX | 134772191 | 134772215 | + | 25 | CATGCTACTGGAGACTCTGCGGTAG |
| chrX | 134772192 | 134772216 | + | 25 | ATGCTACTGGAGACTCTGCGGTAGC |
| chrX | 134772193 | 134772217 | + | 25 | TGCTACTGGAGACTCTGCGGTAGCG |
| chrX | 134772194 | 134772218 | + | 25 | GCTACTGGAGACTCTGCGGTAGCGC |
| chrX | 134772195 | 134772219 | + | 25 | CTACTGGAGACTCTGCGGTAGCGCT |
| chrX | 134772196 | 134772220 | + | 25 | TACTGGAGACTCTGCGGTAGCGCTG |
| chrX | 134772197 | 134772221 | + | 25 | ACTGGAGACTCTGCGGTAGCGCTGC |
| chrX | 134772198 | 134772222 | + | 25 | CTGGAGACTCTGCGGTAGCGCTGCC |
| chrX | 134772199 | 134772223 | + | 25 | TGGAGACTCTGCGGTAGCGCTGCCT |
| chrX | 134772200 | 134772224 | + | 25 | GGAGACTCTGCGGTAGCGCTGCCTT |
| chrX | 134772201 | 134772225 | + | 25 | GAGACTCTGCGGTAGCGCTGCCTTT |
| chrX | 134772202 | 134772226 | + | 25 | AGACTCTGCGGTAGCGCTGCCTTTA |
| chrX | 134772203 | 134772227 | + | 25 | GACTCTGCGGTAGCGCTGCCTTTAG |
| chrX | 134772204 | 134772228 | + | 25 | ACTCTGCGGTAGCGCTGCCTTTAGC |
| chrX | 134772205 | 134772229 | + | 25 | CTCTGCGGTAGCGCTGCCTTTAGCC |
| chrX | 134772206 | 134772230 | + | 25 | TCTGCGGTAGCGCTGCCTTTAGCCA |
| chrX | 134772207 | 134772231 | + | 25 | CTGCGGTAGCGCTGCCTTTAGCCAG |
| chrX | 134772208 | 134772232 | + | 25 | TGCGGTAGCGCTGCCTTTAGCCAGC |
| chrX | 134772209 | 134772233 | + | 25 | GCGGTAGCGCTGCCTTTAGCCAGCG |
| chrX | 134772210 | 134772234 | + | 25 | CGGTAGCGCTGCCTTTAGCCAGCGG |
| chrX | 134772211 | 134772235 | + | 25 | GGTAGCGCTGCCTTTAGCCAGCGGG |
| chrX | 134772212 | 134772236 | + | 25 | GTAGCGCTGCCTTTAGCCAGCGGGT |
| chrX | 134772213 | 134772237 | + | 25 | TAGCGCTGCCTTTAGCCAGCGGGTC |
| chrX | 134772214 | 134772238 | + | 25 | AGCGCTGCCTTTAGCCAGCGGGTCT |
| chrX | 134772215 | 134772239 | + | 25 | GCGCTGCCTTTAGCCAGCGGGTCTG |
| chrX | 134772216 | 134772240 | + | 25 | CGCTGCCTTTAGCCAGCGGGTCTGA |
| chrX | 134772217 | 134772241 | + | 25 | GCTGCCTTTAGCCAGCGGGTCTGAG |
| chrX | 134772218 | 134772242 | + | 25 | CTGCCTTTAGCCAGCGGGTCTGAGG |
| chrX | 134772219 | 134772243 | + | 25 | TGCCTTTAGCCAGCGGGTCTGAGGA |
| chrX | 134772220 | 134772244 | + | 25 | GCCTTTAGCCAGCGGGTCTGAGGAT |
| chrX | 134772221 | 134772245 | + | 25 | CCTTTAGCCAGCGGGTCTGAGGATA |
| chrX | 134772222 | 134772246 | + | 25 | CTTTAGCCAGCGGGTCTGAGGATAA |
| chrX | 134772223 | 134772247 | + | 25 | TTTAGCCAGCGGGTCTGAGGATAAG |
| chrX | 134772224 | 134772248 | + | 25 | TTAGCCAGCGGGTCTGAGGATAAGC |
| chrX | 134772225 | 134772249 | + | 25 | TAGCCAGCGGGTCTGAGGATAAGCC |
| chrX | 134772226 | 134772250 | + | 25 | AGCCAGCGGGTCTGAGGATAAGCCA |
| chrX | 134772227 | 134772251 | + | 25 | GCCAGCGGGTCTGAGGATAAGCCAC |
| chrX | 134772228 | 134772252 | + | 25 | CCAGCGGGTCTGAGGATAAGCCACT |
| chrX | 134772229 | 134772253 | + | 25 | CAGCGGGTCTGAGGATAAGCCACTG |
| chrX | 134772230 | 134772254 | + | 25 | AGCGGGTCTGAGGATAAGCCACTGC |
| chrX | 134772231 | 134772255 | + | 25 | GCGGGTCTGAGGATAAGCCACTGCT |
| chrX | 134772232 | 134772256 | + | 25 | CGGGTCTGAGGATAAGCCACTGCTG |
| chrX | 134772233 | 134772257 | + | 25 | GGGTCTGAGGATAAGCCACTGCTGC |
| chrX | 134772234 | 134772258 | + | 25 | GGTCTGAGGATAAGCCACTGCTGCT |
| chrX | 134772235 | 134772259 | + | 25 | GTCTGAGGATAAGCCACTGCTGCTA |
| chrX | 134772236 | 134772260 | + | 25 | TCTGAGGATAAGCCACTGCTGCTAC |
| chrX | 134772237 | 134772261 | + | 25 | CTGAGGATAAGCCACTGCTGCTACT |
| chrX | 134772238 | 134772262 | + | 25 | TGAGGATAAGCCACTGCTGCTACTA |
| chrX | 134772239 | 134772263 | + | 25 | GAGGATAAGCCACTGCTGCTACTAC |
| chrX | 134772240 | 134772264 | + | 25 | AGGATAAGCCACTGCTGCTACTACT |
| chrX | 134772241 | 134772265 | + | 25 | GGATAAGCCACTGCTGCTACTACTG |
| chrX | 134772242 | 134772266 | + | 25 | GATAAGCCACTGCTGCTACTACTGC |
| chrX | 134772243 | 134772267 | + | 25 | ATAAGCCACTGCTGCTACTACTGCC |
| chrX | 134772244 | 134772268 | + | 25 | TAAGCCACTGCTGCTACTACTGCCA |
| chrX | 134772245 | 134772269 | + | 25 | AAGCCACTGCTGCTACTACTGCCAT |
| chrX | 134772246 | 134772270 | + | 25 | AGCCACTGCTGCTACTACTGCCATC |
| chrX | 134772247 | 134772271 | + | 25 | GCCACTGCTGCTACTACTGCCATCC |
| chrX | 134772248 | 134772272 | + | 25 | CCACTGCTGCTACTACTGCCATCCA |
| chrX | 134772249 | 134772273 | + | 25 | CACTGCTGCTACTACTGCCATCCAA |
| chrX | 134772250 | 134772274 | + | 25 | ACTGCTGCTACTACTGCCATCCAAA |
| chrX | 134772251 | 134772275 | + | 25 | CTGCTGCTACTACTGCCATCCAAAA |
| chrX | 134772252 | 134772276 | + | 25 | TGCTGCTACTACTGCCATCCAAAAT |
| chrX | 134772253 | 134772277 | + | 25 | GCTGCTACTACTGCCATCCAAAATA |
| chrX | 134772254 | 134772278 | + | 25 | CTGCTACTACTGCCATCCAAAATAT |
| chrX | 134772255 | 134772279 | + | 25 | TGCTACTACTGCCATCCAAAATATC |
| chrX | 134772256 | 134772280 | + | 25 | GCTACTACTGCCATCCAAAATATCT |
| chrX | 134772257 | 134772281 | + | 25 | CTACTACTGCCATCCAAAATATCTG |
| chrX | 134772258 | 134772282 | + | 25 | TACTACTGCCATCCAAAATATCTGA |
| chrX | 134772259 | 134772283 | + | 25 | ACTACTGCCATCCAAAATATCTGAA |
| chrX | 134772260 | 134772284 | + | 25 | CTACTGCCATCCAAAATATCTGAAC |
| chrX | 134772261 | 134772285 | + | 25 | TACTGCCATCCAAAATATCTGAACT |
| chrX | 134772262 | 134772286 | + | 25 | ACTGCCATCCAAAATATCTGAACTG |
| chrX | 134772263 | 134772287 | + | 25 | CTGCCATCCAAAATATCTGAACTGA |
| chrX | 134772264 | 134772288 | + | 25 | TGCCATCCAAAATATCTGAACTGAA |
| chrX | 134772265 | 134772289 | + | 25 | GCCATCCAAAATATCTGAACTGAAG |
| chrX | 134772266 | 134772290 | + | 25 | CCATCCAAAATATCTGAACTGAAGA |
| chrX | 134772267 | 134772291 | + | 25 | CATCCAAAATATCTGAACTGAAGAC |
| chrX | 134772268 | 134772292 | + | 25 | ATCCAAAATATCTGAACTGAAGACA |
| chrX | 134772269 | 134772293 | + | 25 | TCCAAAATATCTGAACTGAAGACAA |
| chrX | 134772270 | 134772294 | + | 25 | CCAAAATATCTGAACTGAAGACAAA |
| chrX | 134772271 | 134772295 | + | 25 | CAAAATATCTGAACTGAAGACAAAG |
| chrX | 134772272 | 134772296 | + | 25 | AAAATATCTGAACTGAAGACAAAGC |
| chrX | 134772273 | 134772297 | + | 25 | AAATATCTGAACTGAAGACAAAGCA |
| chrX | 134772274 | 134772298 | + | 25 | AATATCTGAACTGAAGACAAAGCAA |
| chrX | 134772275 | 134772299 | + | 25 | ATATCTGAACTGAAGACAAAGCAAA |
| chrX | 134772276 | 134772300 | + | 25 | TATCTGAACTGAAGACAAAGCAAAA |
| chrX | 134772277 | 134772301 | + | 25 | ATCTGAACTGAAGACAAAGCAAAAA |
| chrX | 134772278 | 134772302 | + | 25 | TCTGAACTGAAGACAAAGCAAAAAG |
| chrX | 134772279 | 134772303 | + | 25 | CTGAACTGAAGACAAAGCAAAAAGA |
| chrX | 134772280 | 134772304 | + | 25 | TGAACTGAAGACAAAGCAAAAAGAA |
| chrX | 134772281 | 134772305 | + | 25 | GAACTGAAGACAAAGCAAAAAGAAT |
| chrX | 134772282 | 134772306 | + | 25 | AACTGAAGACAAAGCAAAAAGAATT |
| chrX | 134772283 | 134772307 | + | 25 | ACTGAAGACAAAGCAAAAAGAATTA |
| chrX | 134772284 | 134772308 | + | 25 | CTGAAGACAAAGCAAAAAGAATTAT |
| chrX | 134772285 | 134772309 | + | 25 | TGAAGACAAAGCAAAAAGAATTATA |
| chrX | 134772286 | 134772310 | + | 25 | GAAGACAAAGCAAAAAGAATTATAA |
| chrX | 134772287 | 134772311 | + | 25 | AAGACAAAGCAAAAAGAATTATAAG |
| chrX | 134772288 | 134772312 | + | 25 | AGACAAAGCAAAAAGAATTATAAGC |
| chrX | 134772289 | 134772313 | + | 25 | GACAAAGCAAAAAGAATTATAAGCA |
| chrX | 134772290 | 134772314 | + | 25 | ACAAAGCAAAAAGAATTATAAGCAA |
| chrX | 134772291 | 134772315 | + | 25 | CAAAGCAAAAAGAATTATAAGCAAA |
| chrX | 134772292 | 134772316 | + | 25 | AAAGCAAAAAGAATTATAAGCAAAT |
| chrX | 134772293 | 134772317 | + | 25 | AAGCAAAAAGAATTATAAGCAAATG |
| chrX | 134772294 | 134772318 | + | 25 | AGCAAAAAGAATTATAAGCAAATGC |
| chrX | 134772295 | 134772319 | + | 25 | GCAAAAAGAATTATAAGCAAATGCA |
| chrX | 134772296 | 134772320 | + | 25 | CAAAAAGAATTATAAGCAAATGCAA |
| chrX | 134772297 | 134772321 | + | 25 | AAAAAGAATTATAAGCAAATGCAAC |
| chrX | 134772298 | 134772322 | + | 25 | AAAAGAATTATAAGCAAATGCAACT |
| chrX | 134772299 | 134772323 | + | 25 | AAAGAATTATAAGCAAATGCAACTA |
| chrX | 134772300 | 134772324 | + | 25 | AAGAATTATAAGCAAATGCAACTAT |
| chrX | 134772301 | 134772325 | + | 25 | AGAATTATAAGCAAATGCAACTATC |
| chrX | 134772302 | 134772326 | + | 25 | GAATTATAAGCAAATGCAACTATCA |
| chrX | 134772303 | 134772327 | + | 25 | AATTATAAGCAAATGCAACTATCAG |
| chrX | 134772304 | 134772328 | + | 25 | ATTATAAGCAAATGCAACTATCAGG |
| chrX | 134772305 | 134772329 | + | 25 | TTATAAGCAAATGCAACTATCAGGG |
| chrX | 134772306 | 134772330 | + | 25 | TATAAGCAAATGCAACTATCAGGGT |
| chrX | 134772307 | 134772331 | + | 25 | ATAAGCAAATGCAACTATCAGGGTA |
| chrX | 134772308 | 134772332 | + | 25 | TAAGCAAATGCAACTATCAGGGTAA |
| chrX | 134772309 | 134772333 | + | 25 | AAGCAAATGCAACTATCAGGGTAAA |

**Table 20: 25-mer target-specific ASOs**

| **CHR** | **START** | **END** | **STRAND** | **kmer** | **SEQUENCE** |
|---|---|---|---|---|---|
| chr22 | 20053386 | 20053410 | + | 25 | GAAGAGCACATGCCTGCAGCTGTGG |
| chr22 | 20053387 | 20053411 | + | 25 | AAGAGCACATGCCTGCAGCTGTGGA |
| chr22 | 20053388 | 20053412 | + | 25 | AGAGCACATGCCTGCAGCTGTGGAC |
| chr22 | 20053389 | 20053413 | + | 25 | GAGCACATGCCTGCAGCTGTGGACA |
| chr22 | 20053390 | 20053414 | + | 25 | AGCACATGCCTGCAGCTGTGGACAC |
| chr22 | 20053391 | 20053415 | + | 25 | GCACATGCCTGCAGCTGTGGACACA |
| chr22 | 20053392 | 20053416 | + | 25 | CACATGCCTGCAGCTGTGGACACAG |
| chr22 | 20053393 | 20053417 | + | 25 | ACATGCCTGCAGCTGTGGACACAGC |
| chr22 | 20053394 | 20053418 | + | 25 | CATGCCTGCAGCTGTGGACACAGCA |
| chr22 | 20053395 | 20053419 | + | 25 | ATGCCTGCAGCTGTGGACACAGCAT |
| chr22 | 20053396 | 20053420 | + | 25 | TGCCTGCAGCTGTGGACACAGCATC |
| chr22 | 20053397 | 20053421 | + | 25 | GCCTGCAGCTGTGGACACAGCATCT |
| chr22 | 20053398 | 20053422 | + | 25 | CCTGCAGCTGTGGACACAGCATCTG |
| chr22 | 20053399 | 20053423 | + | 25 | CTGCAGCTGTGGACACAGCATCTGT |
| chr22 | 20053400 | 20053424 | + | 25 | TGCAGCTGTGGACACAGCATCTGTC |
| chr22 | 20053401 | 20053425 | + | 25 | GCAGCTGTGGACACAGCATCTGTCC |
| chr22 | 20053402 | 20053426 | + | 25 | CAGCTGTGGACACAGCATCTGTCCC |
| chr22 | 20053403 | 20053427 | + | 25 | AGCTGTGGACACAGCATCTGTCCCC |
| chr22 | 20053404 | 20053428 | + | 25 | GCTGTGGACACAGCATCTGTCCCCT |
| chr22 | 20053405 | 20053429 | + | 25 | CTGTGGACACAGCATCTGTCCCCTG |
| chr22 | 20053406 | 20053430 | + | 25 | TGTGGACACAGCATCTGTCCCCTGC |
| chr22 | 20053407 | 20053431 | + | 25 | GTGGACACAGCATCTGTCCCCTGCC |
| chr22 | 20053408 | 20053432 | + | 25 | TGGACACAGCATCTGTCCCCTGCCT |
| chr22 | 20053409 | 20053433 | + | 25 | GGACACAGCATCTGTCCCCTGCCTA |
| chr22 | 20053410 | 20053434 | + | 25 | GACACAGCATCTGTCCCCTGCCTAC |
| chr22 | 20053411 | 20053435 | + | 25 | ACACAGCATCTGTCCCCTGCCTACA |
| chr22 | 20053412 | 20053436 | + | 25 | CACAGCATCTGTCCCCTGCCTACAG |
| chr22 | 20053413 | 20053437 | + | 25 | ACAGCATCTGTCCCCTGCCTACAGG |
| chr22 | 20053414 | 20053438 | + | 25 | CAGCATCTGTCCCCTGCCTACAGGT |
| chr22 | 20053415 | 20053439 | + | 25 | AGCATCTGTCCCCTGCCTACAGGTG |
| chr22 | 20053416 | 20053440 | + | 25 | GCATCTGTCCCCTGCCTACAGGTGA |
| chr22 | 20053417 | 20053441 | + | 25 | CATCTGTCCCCTGCCTACAGGTGAA |
| chr22 | 20053418 | 20053442 | + | 25 | ATCTGTCCCCTGCCTACAGGTGAAC |
| chr22 | 20053419 | 20053443 | + | 25 | TCTGTCCCCTGCCTACAGGTGAACT |
| chr22 | 20053420 | 20053444 | + | 25 | CTGTCCCCTGCCTACAGGTGAACTT |
| chr22 | 20053421 | 20053445 | + | 25 | TGTCCCCTGCCTACAGGTGAACTTG |
| chr22 | 20053422 | 20053446 | + | 25 | GTCCCCTGCCTACAGGTGAACTTGT |
| chr22 | 20053423 | 20053447 | + | 25 | TCCCCTGCCTACAGGTGAACTTGTC |
| chr22 | 20053424 | 20053448 | + | 25 | CCCCTGCCTACAGGTGAACTTGTCA |
| chr22 | 20053425 | 20053449 | + | 25 | CCCTGCCTACAGGTGAACTTGTCAC |
| chr22 | 20053426 | 20053450 | + | 25 | CCTGCCTACAGGTGAACTTGTCACC |
| chr22 | 20053427 | 20053451 | + | 25 | CTGCCTACAGGTGAACTTGTCACCC |
| chr22 | 20053428 | 20053452 | + | 25 | TGCCTACAGGTGAACTTGTCACCCA |
| chr22 | 20053429 | 20053453 | + | 25 | GCCTACAGGTGAACTTGTCACCCAC |
| chr22 | 20053430 | 20053454 | + | 25 | CCTACAGGTGAACTTGTCACCCACT |
| chr22 | 20053431 | 20053455 | + | 25 | CTACAGGTGAACTTGTCACCCACTT |
| chr22 | 20053432 | 20053456 | + | 25 | TACAGGTGAACTTGTCACCCACTTT |
| chr22 | 20053433 | 20053457 | + | 25 | ACAGGTGAACTTGTCACCCACTTTC |
| chr22 | 20053434 | 20053458 | + | 25 | CAGGTGAACTTGTCACCCACTTTCT |
| chr22 | 20053435 | 20053459 | + | 25 | AGGTGAACTTGTCACCCACTTTCTG |
| chr22 | 20053436 | 20053460 | + | 25 | GGTGAACTTGTCACCCACTTTCTGA |
| chr22 | 20053437 | 20053461 | + | 25 | GTGAACTTGTCACCCACTTTCTGAC |
| chr22 | 20053438 | 20053462 | + | 25 | TGAACTTGTCACCCACTTTCTGACC |
| chr22 | 20053439 | 20053463 | + | 25 | GAACTTGTCACCCACTTTCTGACCA |
| chr22 | 20053440 | 20053464 | + | 25 | AACTTGTCACCCACTTTCTGACCAC |
| chr22 | 20053441 | 20053465 | + | 25 | ACTTGTCACCCACTTTCTGACCACT |
| chr22 | 20053442 | 20053466 | + | 25 | CTTGTCACCCACTTTCTGACCACTG |
| chr22 | 20053443 | 20053467 | + | 25 | TTGTCACCCACTTTCTGACCACTGA |
| chr22 | 20053444 | 20053468 | + | 25 | TGTCACCCACTTTCTGACCACTGAC |
| chr22 | 20053445 | 20053469 | + | 25 | GTCACCCACTTTCTGACCACTGACG |
| chr22 | 20053446 | 20053470 | + | 25 | TCACCCACTTTCTGACCACTGACGT |
| chr22 | 20053447 | 20053471 | + | 25 | CACCCACTTTCTGACCACTGACGTG |
| chr22 | 20053448 | 20053472 | + | 25 | ACCCACTTTCTGACCACTGACGTGG |
| chr22 | 20053449 | 20053473 | + | 25 | CCCACTTTCTGACCACTGACGTGGA |
| chr22 | 20053450 | 20053474 | + | 25 | CCACTTTCTGACCACTGACGTGGAC |
| chr22 | 20053451 | 20053475 | + | 25 | CACTTTCTGACCACTGACGTGGACA |
| chr22 | 20053452 | 20053476 | + | 25 | ACTTTCTGACCACTGACGTGGACAG |
| chr22 | 20053453 | 20053477 | + | 25 | CTTTCTGACCACTGACGTGGACAGC |
| chr22 | 20053454 | 20053478 | + | 25 | TTTCTGACCACTGACGTGGACAGCT |
| chr22 | 20053455 | 20053479 | + | 25 | TTCTGACCACTGACGTGGACAGCTT |
| chr22 | 20053456 | 20053480 | + | 25 | TCTGACCACTGACGTGGACAGCTTG |
| chr22 | 20053457 | 20053481 | + | 25 | CTGACCACTGACGTGGACAGCTTGT |
| chr22 | 20053458 | 20053482 | + | 25 | TGACCACTGACGTGGACAGCTTGTC |
| chr22 | 20053459 | 20053483 | + | 25 | GACCACTGACGTGGACAGCTTGTCC |
| chr22 | 20053460 | 20053484 | + | 25 | ACCACTGACGTGGACAGCTTGTCCT |
| chr22 | 20053461 | 20053485 | + | 25 | CCACTGACGTGGACAGCTTGTCCTA |
| chr22 | 20053462 | 20053486 | + | 25 | CACTGACGTGGACAGCTTGTCCTAC |
| chr22 | 20053463 | 20053487 | + | 25 | ACTGACGTGGACAGCTTGTCCTACC |
| chr22 | 20053464 | 20053488 | + | 25 | CTGACGTGGACAGCTTGTCCTACCT |
| chr22 | 20053465 | 20053489 | + | 25 | TGACGTGGACAGCTTGTCCTACCTG |
| chr22 | 20053466 | 20053490 | + | 25 | GACGTGGACAGCTTGTCCTACCTGA |
| chr22 | 20053467 | 20053491 | + | 25 | ACGTGGACAGCTTGTCCTACCTGAA |
| chr22 | 20053468 | 20053492 | + | 25 | CGTGGACAGCTTGTCCTACCTGAAG |
| chr22 | 20053469 | 20053493 | + | 25 | GTGGACAGCTTGTCCTACCTGAAGA |
| chr22 | 20053470 | 20053494 | + | 25 | TGGACAGCTTGTCCTACCTGAAGAA |
| chr22 | 20053471 | 20053495 | + | 25 | GGACAGCTTGTCCTACCTGAAGAAG |
| chr22 | 20053472 | 20053496 | + | 25 | GACAGCTTGTCCTACCTGAAGAAGG |
| chr22 | 20053473 | 20053497 | + | 25 | ACAGCTTGTCCTACCTGAAGAAGGT |
| chr22 | 20053474 | 20053498 | + | 25 | CAGCTTGTCCTACCTGAAGAAGGTC |
| chr22 | 20053475 | 20053499 | + | 25 | AGCTTGTCCTACCTGAAGAAGGTCT |
| chr22 | 20053476 | 20053500 | + | 25 | GCTTGTCCTACCTGAAGAAGGTCTC |
| chr22 | 20053477 | 20053501 | + | 25 | CTTGTCCTACCTGAAGAAGGTCTCT |
| chr22 | 20053478 | 20053502 | + | 25 | TTGTCCTACCTGAAGAAGGTCTCTA |
| chr22 | 20053479 | 20053503 | + | 25 | TGTCCTACCTGAAGAAGGTCTCTAT |
| chr22 | 20053480 | 20053504 | + | 25 | GTCCTACCTGAAGAAGGTCTCTATG |
| chr22 | 20053481 | 20053505 | + | 25 | TCCTACCTGAAGAAGGTCTCTATGG |
| chr22 | 20053482 | 20053506 | + | 25 | CCTACCTGAAGAAGGTCTCTATGGA |
| chr22 | 20053483 | 20053507 | + | 25 | CTACCTGAAGAAGGTCTCTATGGAG |
| chr22 | 20053484 | 20053508 | + | 25 | TACCTGAAGAAGGTCTCTATGGAGG |
| chr22 | 20053485 | 20053509 | + | 25 | ACCTGAAGAAGGTCTCTATGGAGGG |
| chr22 | 20053486 | 20053510 | + | 25 | CCTGAAGAAGGTCTCTATGGAGGGC |
| chr22 | 20053487 | 20053511 | + | 25 | CTGAAGAAGGTCTCTATGGAGGGCC |
| chr22 | 20053488 | 20053512 | + | 25 | TGAAGAAGGTCTCTATGGAGGGCCA |
| chr22 | 20053489 | 20053513 | + | 25 | GAAGAAGGTCTCTATGGAGGGCCAT |
| chr22 | 20053490 | 20053514 | + | 25 | AAGAAGGTCTCTATGGAGGGCCATC |
| chr22 | 20053491 | 20053515 | + | 25 | AGAAGGTCTCTATGGAGGGCCATCT |
| chr22 | 20053492 | 20053516 | + | 25 | GAAGGTCTCTATGGAGGGCCATCTG |
| chr22 | 20053493 | 20053517 | + | 25 | AAGGTCTCTATGGAGGGCCATCTGT |
| chr22 | 20053494 | 20053518 | + | 25 | AGGTCTCTATGGAGGGCCATCTGTA |
| chr22 | 20053495 | 20053519 | + | 25 | GGTCTCTATGGAGGGCCATCTGTAC |
| chr22 | 20053496 | 20053520 | + | 25 | GTCTCTATGGAGGGCCATCTGTACA |
| chr22 | 20053497 | 20053521 | + | 25 | TCTCTATGGAGGGCCATCTGTACAA |
| chr22 | 20053498 | 20053522 | + | 25 | CTCTATGGAGGGCCATCTGTACAAT |
| chr22 | 20053499 | 20053523 | + | 25 | TCTATGGAGGGCCATCTGTACAATG |
| chr22 | 20053500 | 20053524 | + | 25 | CTATGGAGGGCCATCTGTACAATGG |
| chr22 | 20053501 | 20053525 | + | 25 | TATGGAGGGCCATCTGTACAATGGC |
| chr22 | 20053502 | 20053526 | + | 25 | ATGGAGGGCCATCTGTACAATGGCT |
| chr22 | 20053503 | 20053527 | + | 25 | TGGAGGGCCATCTGTACAATGGCTT |
| chr22 | 20053504 | 20053528 | + | 25 | GGAGGGCCATCTGTACAATGGCTTC |
| chr22 | 20053505 | 20053529 | + | 25 | GAGGGCCATCTGTACAATGGCTTCA |
| chr22 | 20053506 | 20053530 | + | 25 | AGGGCCATCTGTACAATGGCTTCAA |
| chr22 | 20053507 | 20053531 | + | 25 | GGGCCATCTGTACAATGGCTTCAAC |
| chr22 | 20053508 | 20053532 | + | 25 | GGCCATCTGTACAATGGCTTCAACC |
| chr22 | 20053509 | 20053533 | + | 25 | GCCATCTGTACAATGGCTTCAACCT |
| chr22 | 20053510 | 20053534 | + | 25 | CCATCTGTACAATGGCTTCAACCTC |
| chr22 | 20053511 | 20053535 | + | 25 | CATCTGTACAATGGCTTCAACCTCA |
| chr22 | 20053512 | 20053536 | + | 25 | ATCTGTACAATGGCTTCAACCTCAT |
| chr22 | 20053513 | 20053537 | + | 25 | TCTGTACAATGGCTTCAACCTCATA |
| chr22 | 20053514 | 20053538 | + | 25 | CTGTACAATGGCTTCAACCTCATAG |
| chr22 | 20053515 | 20053539 | + | 25 | TGTACAATGGCTTCAACCTCATAGC |
| chr22 | 20053516 | 20053540 | + | 25 | GTACAATGGCTTCAACCTCATAGCA |
| chr22 | 20053517 | 20053541 | + | 25 | TACAATGGCTTCAACCTCATAGCAG |
| chr22 | 20053518 | 20053542 | + | 25 | ACAATGGCTTCAACCTCATAGCAGC |
| chr22 | 20053519 | 20053543 | + | 25 | CAATGGCTTCAACCTCATAGCAGCC |
| chr22 | 20053520 | 20053544 | + | 25 | AATGGCTTCAACCTCATAGCAGCCG |
| chr22 | 20053521 | 20053545 | + | 25 | ATGGCTTCAACCTCATAGCAGCCGA |
| chr22 | 20053522 | 20053546 | + | 25 | TGGCTTCAACCTCATAGCAGCCGAC |
| chr22 | 20053523 | 20053547 | + | 25 | GGCTTCAACCTCATAGCAGCCGACC |
| chr22 | 20053524 | 20053548 | + | 25 | GCTTCAACCTCATAGCAGCCGACCT |
| chr22 | 20053525 | 20053549 | + | 25 | CTTCAACCTCATAGCAGCCGACCTG |
| chr22 | 20053526 | 20053550 | + | 25 | TTCAACCTCATAGCAGCCGACCTGA |
| chr22 | 20053527 | 20053551 | + | 25 | TCAACCTCATAGCAGCCGACCTGAG |
| chr22 | 20053528 | 20053552 | + | 25 | CAACCTCATAGCAGCCGACCTGAGG |
| chr22 | 20053529 | 20053553 | + | 25 | AACCTCATAGCAGCCGACCTGAGGT |
| chr22 | 20053530 | 20053554 | + | 25 | ACCTCATAGCAGCCGACCTGAGGTG |
| chr22 | 20053531 | 20053555 | + | 25 | CCTCATAGCAGCCGACCTGAGGTGG |
| chr22 | 20053532 | 20053556 | + | 25 | CTCATAGCAGCCGACCTGAGGTGGG |
| chr22 | 20053533 | 20053557 | + | 25 | TCATAGCAGCCGACCTGAGGTGGGT |
| chr22 | 20053534 | 20053558 | + | 25 | CATAGCAGCCGACCTGAGGTGGGTC |
| chr22 | 20053535 | 20053559 | + | 25 | ATAGCAGCCGACCTGAGGTGGGTCT |
| chr22 | 20053536 | 20053560 | + | 25 | TAGCAGCCGACCTGAGGTGGGTCTG |
| chr22 | 20053537 | 20053561 | + | 25 | AGCAGCCGACCTGAGGTGGGTCTGC |
| chr22 | 20053538 | 20053562 | + | 25 | GCAGCCGACCTGAGGTGGGTCTGCC |
| chr22 | 20053539 | 20053563 | + | 25 | CAGCCGACCTGAGGTGGGTCTGCCA |
| chr22 | 20053540 | 20053564 | + | 25 | AGCCGACCTGAGGTGGGTCTGCCAG |
| chr22 | 20053541 | 20053565 | + | 25 | GCCGACCTGAGGTGGGTCTGCCAGA |
| chr22 | 20053542 | 20053566 | + | 25 | CCGACCTGAGGTGGGTCTGCCAGAG |
| chr22 | 20053543 | 20053567 | + | 25 | CGACCTGAGGTGGGTCTGCCAGAGG |
| chr22 | 20053544 | 20053568 | + | 25 | GACCTGAGGTGGGTCTGCCAGAGGG |
| chr22 | 20053545 | 20053569 | + | 25 | ACCTGAGGTGGGTCTGCCAGAGGGG |
| chr22 | 20053546 | 20053570 | + | 25 | CCTGAGGTGGGTCTGCCAGAGGGGG |
| chr22 | 20053547 | 20053571 | + | 25 | CTGAGGTGGGTCTGCCAGAGGGGGA |
| chr22 | 20053548 | 20053572 | + | 25 | TGAGGTGGGTCTGCCAGAGGGGGAT |
| chr22 | 20053549 | 20053573 | + | 25 | GAGGTGGGTCTGCCAGAGGGGGATG |
| chr22 | 20053550 | 20053574 | + | 25 | AGGTGGGTCTGCCAGAGGGGGATGG |
| chr22 | 20053551 | 20053575 | + | 25 | GGTGGGTCTGCCAGAGGGGGATGGC |
| chr22 | 20053552 | 20053576 | + | 25 | GTGGGTCTGCCAGAGGGGGATGGCG |
| chr22 | 20053553 | 20053577 | + | 25 | TGGGTCTGCCAGAGGGGGATGGCGG |
| chr22 | 20053554 | 20053578 | + | 25 | GGGTCTGCCAGAGGGGGATGGCGGC |
| chr22 | 20053555 | 20053579 | + | 25 | GGTCTGCCAGAGGGGGATGGCGGCT |
| chr22 | 20053556 | 20053580 | + | 25 | GTCTGCCAGAGGGGGATGGCGGCTC |
| chr22 | 20053557 | 20053581 | + | 25 | TCTGCCAGAGGGGGATGGCGGCTCT |
| chr22 | 20053558 | 20053582 | + | 25 | CTGCCAGAGGGGGATGGCGGCTCTG |
| chr22 | 20053559 | 20053583 | + | 25 | TGCCAGAGGGGGATGGCGGCTCTGC |
| chr22 | 20053560 | 20053584 | + | 25 | GCCAGAGGGGGATGGCGGCTCTGCC |
| chr22 | 20053561 | 20053585 | + | 25 | CCAGAGGGGGATGGCGGCTCTGCCC |
| chr22 | 20053562 | 20053586 | + | 25 | CAGAGGGGGATGGCGGCTCTGCCCA |
| chr22 | 20053563 | 20053587 | + | 25 | AGAGGGGGATGGCGGCTCTGCCCAG |
| chr22 | 20053564 | 20053588 | + | 25 | GAGGGGGATGGCGGCTCTGCCCAGC |
| chr22 | 20053565 | 20053589 | + | 25 | AGGGGGATGGCGGCTCTGCCCAGCA |
| chr22 | 20053566 | 20053590 | + | 25 | GGGGGATGGCGGCTCTGCCCAGCAC |
| chr22 | 20053567 | 20053591 | + | 25 | GGGGATGGCGGCTCTGCCCAGCACT |
| chr22 | 20053568 | 20053592 | + | 25 | GGGATGGCGGCTCTGCCCAGCACTG |
| chr22 | 20053569 | 20053593 | + | 25 | GGATGGCGGCTCTGCCCAGCACTGC |
| chr22 | 20053570 | 20053594 | + | 25 | GATGGCGGCTCTGCCCAGCACTGCC |
| chr22 | 20053571 | 20053595 | + | 25 | ATGGCGGCTCTGCCCAGCACTGCCT |
| chr22 | 20053572 | 20053596 | + | 25 | TGGCGGCTCTGCCCAGCACTGCCTC |
| chr22 | 20053573 | 20053597 | + | 25 | GGCGGCTCTGCCCAGCACTGCCTCG |
| chr22 | 20053574 | 20053598 | + | 25 | GCGGCTCTGCCCAGCACTGCCTCGG |
| chr22 | 20053575 | 20053599 | + | 25 | CGGCTCTGCCCAGCACTGCCTCGGG |
| chr22 | 20053576 | 20053600 | + | 25 | GGCTCTGCCCAGCACTGCCTCGGGG |
| chr22 | 20053577 | 20053601 | + | 25 | GCTCTGCCCAGCACTGCCTCGGGGG |

**Table 21: 25-mer target-specific ASOs**

| **CHR** | **START** | **END** | **STRAND** | **kmer** | **SEQUENCE** |
|---|---|---|---|---|---|
| chr1 | 155061367 | 155061391 | + | 25 | TGCTTCCTCTTCCCCCTCTGTGCCT |
| chr1 | 155061368 | 155061392 | + | 25 | GCTTCCTCTTCCCCCTCTGTGCCTA |
| chr1 | 155061369 | 155061393 | + | 25 | CTTCCTCTTCCCCCTCTGTGCCTAT |
| chr1 | 155061370 | 155061394 | + | 25 | TTCCTCTTCCCCCTCTGTGCCTATC |
| chr1 | 155061371 | 155061395 | + | 25 | TCCTCTTCCCCCTCTGTGCCTATCT |
| chr1 | 155061372 | 155061396 | + | 25 | CCTCTTCCCCCTCTGTGCCTATCTG |
| chr1 | 155061373 | 155061397 | + | 25 | CTCTTCCCCCTCTGTGCCTATCTGC |
| chr1 | 155061374 | 155061398 | + | 25 | TCTTCCCCCTCTGTGCCTATCTGCC |
| chr1 | 155061375 | 155061399 | + | 25 | CTTCCCCCTCTGTGCCTATCTGCCC |
| chr1 | 155061376 | 155061400 | + | 25 | TTCCCCCTCTGTGCCTATCTGCCCC |
| chr1 | 155061377 | 155061401 | + | 25 | TCCCCCTCTGTGCCTATCTGCCCCT |
| chr1 | 155061378 | 155061402 | + | 25 | CCCCCTCTGTGCCTATCTGCCCCTC |
| chr1 | 155061379 | 155061403 | + | 25 | CCCCTCTGTGCCTATCTGCCCCTCC |
| chr1 | 155061380 | 155061404 | + | 25 | CCCTCTGTGCCTATCTGCCCCTCCT |
| chr1 | 155061381 | 155061405 | + | 25 | CCTCTGTGCCTATCTGCCCCTCCTG |
| chr1 | 155061382 | 155061406 | + | 25 | CTCTGTGCCTATCTGCCCCTCCTGC |
| chr1 | 155061383 | 155061407 | + | 25 | TCTGTGCCTATCTGCCCCTCCTGCC |
| chr1 | 155061384 | 155061408 | + | 25 | CTGTGCCTATCTGCCCCTCCTGCCC |
| chr1 | 155061385 | 155061409 | + | 25 | TGTGCCTATCTGCCCCTCCTGCCCT |
| chr1 | 155061386 | 155061410 | + | 25 | GTGCCTATCTGCCCCTCCTGCCCTC |
| chr1 | 155061387 | 155061411 | + | 25 | TGCCTATCTGCCCCTCCTGCCCTCT |
| chr1 | 155061388 | 155061412 | + | 25 | GCCTATCTGCCCCTCCTGCCCTCTC |
| chr1 | 155061389 | 155061413 | + | 25 | CCTATCTGCCCCTCCTGCCCTCTCA |
| chr1 | 155061390 | 155061414 | + | 25 | CTATCTGCCCCTCCTGCCCTCTCAG |
| chr1 | 155061391 | 155061415 | + | 25 | TATCTGCCCCTCCTGCCCTCTCAGC |
| chr1 | 155061392 | 155061416 | + | 25 | ATCTGCCCCTCCTGCCCTCTCAGCA |
| chr1 | 155061393 | 155061417 | + | 25 | TCTGCCCCTCCTGCCCTCTCAGCAG |
| chr1 | 155061394 | 155061418 | + | 25 | CTGCCCCTCCTGCCCTCTCAGCAGG |
| chr1 | 155061395 | 155061419 | + | 25 | TGCCCCTCCTGCCCTCTCAGCAGGC |
| chr1 | 155061396 | 155061420 | + | 25 | GCCCCTCCTGCCCTCTCAGCAGGCT |
| chr1 | 155061397 | 155061421 | + | 25 | CCCCTCCTGCCCTCTCAGCAGGCTA |
| chr1 | 155061398 | 155061422 | + | 25 | CCCTCCTGCCCTCTCAGCAGGCTAG |
| chr1 | 155061399 | 155061423 | + | 25 | CCTCCTGCCCTCTCAGCAGGCTAGT |
| chr1 | 155061400 | 155061424 | + | 25 | CTCCTGCCCTCTCAGCAGGCTAGTG |
| chr1 | 155061401 | 155061425 | + | 25 | TCCTGCCCTCTCAGCAGGCTAGTGC |
| chr1 | 155061402 | 155061426 | + | 25 | CCTGCCCTCTCAGCAGGCTAGTGCT |
| chr1 | 155061403 | 155061427 | + | 25 | CTGCCCTCTCAGCAGGCTAGTGCTC |
| chr1 | 155061404 | 155061428 | + | 25 | TGCCCTCTCAGCAGGCTAGTGCTCT |
| chr1 | 155061405 | 155061429 | + | 25 | GCCCTCTCAGCAGGCTAGTGCTCTC |
| chr1 | 155061406 | 155061430 | + | 25 | CCCTCTCAGCAGGCTAGTGCTCTCA |
| chr1 | 155061407 | 155061431 | + | 25 | CCTCTCAGCAGGCTAGTGCTCTCAG |
| chr1 | 155061408 | 155061432 | + | 25 | CTCTCAGCAGGCTAGTGCTCTCAGC |
| chr1 | 155061409 | 155061433 | + | 25 | TCTCAGCAGGCTAGTGCTCTCAGCT |
| chr1 | 155061410 | 155061434 | + | 25 | CTCAGCAGGCTAGTGCTCTCAGCTT |
| chr1 | 155061411 | 155061435 | + | 25 | TCAGCAGGCTAGTGCTCTCAGCTTC |
| chr1 | 155061412 | 155061436 | + | 25 | CAGCAGGCTAGTGCTCTCAGCTTCC |
| chr1 | 155061413 | 155061437 | + | 25 | AGCAGGCTAGTGCTCTCAGCTTCCC |
| chr1 | 155061414 | 155061438 | + | 25 | GCAGGCTAGTGCTCTCAGCTTCCCG |
| chr1 | 155061415 | 155061439 | + | 25 | CAGGCTAGTGCTCTCAGCTTCCCGG |
| chr1 | 155061416 | 155061440 | + | 25 | AGGCTAGTGCTCTCAGCTTCCCGGC |
| chr1 | 155061417 | 155061441 | + | 25 | GGCTAGTGCTCTCAGCTTCCCGGCC |
| chr1 | 155061418 | 155061442 | + | 25 | GCTAGTGCTCTCAGCTTCCCGGCCC |
| chr1 | 155061419 | 155061443 | + | 25 | CTAGTGCTCTCAGCTTCCCGGCCCC |
| chr1 | 155061420 | 155061444 | + | 25 | TAGTGCTCTCAGCTTCCCGGCCCCC |
| chr1 | 155061421 | 155061445 | + | 25 | AGTGCTCTCAGCTTCCCGGCCCCCC |
| chr1 | 155061422 | 155061446 | + | 25 | GTGCTCTCAGCTTCCCGGCCCCCCC |
| chr1 | 155061423 | 155061447 | + | 25 | TGCTCTCAGCTTCCCGGCCCCCCCT |
| chr1 | 155061424 | 155061448 | + | 25 | GCTCTCAGCTTCCCGGCCCCCCCTT |
| chr1 | 155061425 | 155061449 | + | 25 | CTCTCAGCTTCCCGGCCCCCCCTTC |
| chr1 | 155061426 | 155061450 | + | 25 | TCTCAGCTTCCCGGCCCCCCCTTCC |
| chr1 | 155061427 | 155061451 | + | 25 | CTCAGCTTCCCGGCCCCCCCTTCCA |
| chr1 | 155061428 | 155061452 | + | 25 | TCAGCTTCCCGGCCCCCCCTTCCAG |
| chr1 | 155061429 | 155061453 | + | 25 | CAGCTTCCCGGCCCCCCCTTCCAGG |
| chr1 | 155061430 | 155061454 | + | 25 | AGCTTCCCGGCCCCCCCTTCCAGGC |
| chr1 | 155061431 | 155061455 | + | 25 | GCTTCCCGGCCCCCCCTTCCAGGCC |
| chr1 | 155061432 | 155061456 | + | 25 | CTTCCCGGCCCCCCCTTCCAGGCCG |
| chr1 | 155061433 | 155061457 | + | 25 | TTCCCGGCCCCCCCTTCCAGGCCGC |
| chr1 | 155061434 | 155061458 | + | 25 | TCCCGGCCCCCCCTTCCAGGCCGCT |
| chr1 | 155061435 | 155061459 | + | 25 | CCCGGCCCCCCCTTCCAGGCCGCTG |
| chr1 | 155061436 | 155061460 | + | 25 | CCGGCCCCCCCTTCCAGGCCGCTGC |
| chr1 | 155061437 | 155061461 | + | 25 | CGGCCCCCCCTTCCAGGCCGCTGCC |
| chr1 | 155061438 | 155061462 | + | 25 | GGCCCCCCCTTCCAGGCCGCTGCCG |
| chr1 | 155061439 | 155061463 | + | 25 | GCCCCCCCTTCCAGGCCGCTGCCGC |
| chr1 | 155061440 | 155061464 | + | 25 | CCCCCCCTTCCAGGCCGCTGCCGCC |
| chr1 | 155061441 | 155061465 | + | 25 | CCCCCCTTCCAGGCCGCTGCCGCCT |
| chr1 | 155061442 | 155061466 | + | 25 | CCCCCTTCCAGGCCGCTGCCGCCTG |
| chr1 | 155061443 | 155061467 | + | 25 | CCCCTTCCAGGCCGCTGCCGCCTGA |
| chr1 | 155061444 | 155061468 | + | 25 | CCCTTCCAGGCCGCTGCCGCCTGAC |
| chr1 | 155061445 | 155061469 | + | 25 | CCTTCCAGGCCGCTGCCGCCTGACC |
| chr1 | 155061446 | 155061470 | + | 25 | CTTCCAGGCCGCTGCCGCCTGACCC |
| chr1 | 155061447 | 155061471 | + | 25 | TTCCAGGCCGCTGCCGCCTGACCCT |
| chr1 | 155061448 | 155061472 | + | 25 | TCCAGGCCGCTGCCGCCTGACCCTG |
| chr1 | 155061449 | 155061473 | + | 25 | CCAGGCCGCTGCCGCCTGACCCTGT |
| chr1 | 155061450 | 155061474 | + | 25 | CAGGCCGCTGCCGCCTGACCCTGTG |
| chr1 | 155061451 | 155061475 | + | 25 | AGGCCGCTGCCGCCTGACCCTGTGT |
| chr1 | 155061452 | 155061476 | + | 25 | GGCCGCTGCCGCCTGACCCTGTGTC |
| chr1 | 155061453 | 155061477 | + | 25 | GCCGCTGCCGCCTGACCCTGTGTCC |
| chr1 | 155061454 | 155061478 | + | 25 | CCGCTGCCGCCTGACCCTGTGTCCA |
| chr1 | 155061455 | 155061479 | + | 25 | CGCTGCCGCCTGACCCTGTGTCCAA |
| chr1 | 155061456 | 155061480 | + | 25 | GCTGCCGCCTGACCCTGTGTCCAAG |
| chr1 | 155061457 | 155061481 | + | 25 | CTGCCGCCTGACCCTGTGTCCAAGA |
| chr1 | 155061458 | 155061482 | + | 25 | TGCCGCCTGACCCTGTGTCCAAGAG |
| chr1 | 155061459 | 155061483 | + | 25 | GCCGCCTGACCCTGTGTCCAAGAGA |
| chr1 | 155061460 | 155061484 | + | 25 | CCGCCTGACCCTGTGTCCAAGAGAC |
| chr1 | 155061461 | 155061485 | + | 25 | CGCCTGACCCTGTGTCCAAGAGACT |
| chr1 | 155061462 | 155061486 | + | 25 | GCCTGACCCTGTGTCCAAGAGACTC |
| chr1 | 155061463 | 155061487 | + | 25 | CCTGACCCTGTGTCCAAGAGACTCC |
| chr1 | 155061464 | 155061488 | + | 25 | CTGACCCTGTGTCCAAGAGACTCCA |
| chr1 | 155061465 | 155061489 | + | 25 | TGACCCTGTGTCCAAGAGACTCCAG |
| chr1 | 155061466 | 155061490 | + | 25 | GACCCTGTGTCCAAGAGACTCCAGG |
| chr1 | 155061467 | 155061491 | + | 25 | ACCCTGTGTCCAAGAGACTCCAGGT |
| chr1 | 155061468 | 155061492 | + | 25 | CCCTGTGTCCAAGAGACTCCAGGTA |
| chr1 | 155061469 | 155061493 | + | 25 | CCTGTGTCCAAGAGACTCCAGGTAA |
| chr1 | 155061470 | 155061494 | + | 25 | CTGTGTCCAAGAGACTCCAGGTAAA |
| chr1 | 155061471 | 155061495 | + | 25 | TGTGTCCAAGAGACTCCAGGTAAAT |
| chr1 | 155061472 | 155061496 | + | 25 | GTGTCCAAGAGACTCCAGGTAAATC |
| chr1 | 155061473 | 155061497 | + | 25 | TGTCCAAGAGACTCCAGGTAAATCT |
| chr1 | 155061474 | 155061498 | + | 25 | GTCCAAGAGACTCCAGGTAAATCTG |
| chr1 | 155061475 | 155061499 | + | 25 | TCCAAGAGACTCCAGGTAAATCTGG |
| chr1 | 155061476 | 155061500 | + | 25 | CCAAGAGACTCCAGGTAAATCTGGG |
| chr1 | 155061477 | 155061501 | + | 25 | CAAGAGACTCCAGGTAAATCTGGGC |
| chr1 | 155061478 | 155061502 | + | 25 | AAGAGACTCCAGGTAAATCTGGGCC |
| chr1 | 155061479 | 155061503 | + | 25 | AGAGACTCCAGGTAAATCTGGGCCA |
| chr1 | 155061480 | 155061504 | + | 25 | GAGACTCCAGGTAAATCTGGGCCAG |
| chr1 | 155061481 | 155061505 | + | 25 | AGACTCCAGGTAAATCTGGGCCAGG |
| chr1 | 155061482 | 155061506 | + | 25 | GACTCCAGGTAAATCTGGGCCAGGG |
| chr1 | 155061483 | 155061507 | + | 25 | ACTCCAGGTAAATCTGGGCCAGGGC |
| chr1 | 155061484 | 155061508 | + | 25 | CTCCAGGTAAATCTGGGCCAGGGCC |
| chr1 | 155061485 | 155061509 | + | 25 | TCCAGGTAAATCTGGGCCAGGGCCC |
| chr1 | 155061486 | 155061510 | + | 25 | CCAGGTAAATCTGGGCCAGGGCCCG |
| chr1 | 155061487 | 155061511 | + | 25 | CAGGTAAATCTGGGCCAGGGCCCGC |
| chr1 | 155061488 | 155061512 | + | 25 | AGGTAAATCTGGGCCAGGGCCCGCC |
| chr1 | 155061489 | 155061513 | + | 25 | GGTAAATCTGGGCCAGGGCCCGCCC |
| chr1 | 155061490 | 155061514 | + | 25 | GTAAATCTGGGCCAGGGCCCGCCCT |
| chr1 | 155061491 | 155061515 | + | 25 | TAAATCTGGGCCAGGGCCCGCCCTG |
| chr1 | 155061492 | 155061516 | + | 25 | AAATCTGGGCCAGGGCCCGCCCTGA |
| chr1 | 155061493 | 155061517 | + | 25 | AATCTGGGCCAGGGCCCGCCCTGAG |
| chr1 | 155061494 | 155061518 | + | 25 | ATCTGGGCCAGGGCCCGCCCTGAGC |
| chr1 | 155061495 | 155061519 | + | 25 | TCTGGGCCAGGGCCCGCCCTGAGCC |
| chr1 | 155061496 | 155061520 | + | 25 | CTGGGCCAGGGCCCGCCCTGAGCCA |
| chr1 | 155061497 | 155061521 | + | 25 | TGGGCCAGGGCCCGCCCTGAGCCAA |
| chr1 | 155061498 | 155061522 | + | 25 | GGGCCAGGGCCCGCCCTGAGCCAAG |
| chr1 | 155061499 | 155061523 | + | 25 | GGCCAGGGCCCGCCCTGAGCCAAGG |
| chr1 | 155061500 | 155061524 | + | 25 | GCCAGGGCCCGCCCTGAGCCAAGGC |
| chr1 | 155061501 | 155061525 | + | 25 | CCAGGGCCCGCCCTGAGCCAAGGCA |
| chr1 | 155061502 | 155061526 | + | 25 | CAGGGCCCGCCCTGAGCCAAGGCAG |
| chr1 | 155061503 | 155061527 | + | 25 | AGGGCCCGCCCTGAGCCAAGGCAGG |
| chr1 | 155061504 | 155061528 | + | 25 | GGGCCCGCCCTGAGCCAAGGCAGGT |
| chr1 | 155061505 | 155061529 | + | 25 | GGCCCGCCCTGAGCCAAGGCAGGTG |
| chr1 | 155061506 | 155061530 | + | 25 | GCCCGCCCTGAGCCAAGGCAGGTGG |
| chr1 | 155061507 | 155061531 | + | 25 | CCCGCCCTGAGCCAAGGCAGGTGGG |
| chr1 | 155061508 | 155061532 | + | 25 | CCGCCCTGAGCCAAGGCAGGTGGGA |
| chr1 | 155061509 | 155061533 | + | 25 | CGCCCTGAGCCAAGGCAGGTGGGAG |
| chr1 | 155061510 | 155061534 | + | 25 | GCCCTGAGCCAAGGCAGGTGGGAGG |
| chr1 | 155061511 | 155061535 | + | 25 | CCCTGAGCCAAGGCAGGTGGGAGGC |
| chr1 | 155061512 | 155061536 | + | 25 | CCTGAGCCAAGGCAGGTGGGAGGCT |
| chr1 | 155061513 | 155061537 | + | 25 | CTGAGCCAAGGCAGGTGGGAGGCTT |
| chr1 | 155061514 | 155061538 | + | 25 | TGAGCCAAGGCAGGTGGGAGGCTTG |
| chr1 | 155061515 | 155061539 | + | 25 | GAGCCAAGGCAGGTGGGAGGCTTGG |

**Table 22: 25-mer target-specific ASOs**

| **CHR** | **START** | **END** | **STRAND** | **kmer** | **SEQUENCE** |
|---|---|---|---|---|---|
| chr20 | 36209437 | 36209461 | + | 25 | CTCTCTCTCCCCACCCCACATCCCC |
| chr20 | 36209438 | 36209462 | + | 25 | TCTCTCTCCCCACCCCACATCCCCA |
| chr20 | 36209439 | 36209463 | + | 25 | CTCTCTCCCCACCCCACATCCCCAT |
| chr20 | 36209440 | 36209464 | + | 25 | TCTCTCCCCACCCCACATCCCCATT |
| chr20 | 36209441 | 36209465 | + | 25 | CTCTCCCCACCCCACATCCCCATTC |
| chr20 | 36209442 | 36209466 | + | 25 | TCTCCCCACCCCACATCCCCATTCT |
| chr20 | 36209443 | 36209467 | + | 25 | CTCCCCACCCCACATCCCCATTCTT |
| chr20 | 36209444 | 36209468 | + | 25 | TCCCCACCCCACATCCCCATTCTTT |
| chr20 | 36209445 | 36209469 | + | 25 | CCCCACCCCACATCCCCATTCTTTT |
| chr20 | 36209446 | 36209470 | + | 25 | CCCACCCCACATCCCCATTCTTTTG |
| chr20 | 36209447 | 36209471 | + | 25 | CCACCCCACATCCCCATTCTTTTGA |
| chr20 | 36209448 | 36209472 | + | 25 | CACCCCACATCCCCATTCTTTTGAT |
| chr20 | 36209449 | 36209473 | + | 25 | ACCCCACATCCCCATTCTTTTGATT |
| chr20 | 36209450 | 36209474 | + | 25 | CCCCACATCCCCATTCTTTTGATTT |
| chr20 | 36209451 | 36209475 | + | 25 | CCCACATCCCCATTCTTTTGATTTT |
| chr20 | 36209452 | 36209476 | + | 25 | CCACATCCCCATTCTTTTGATTTTA |
| chr20 | 36209453 | 36209477 | + | 25 | CACATCCCCATTCTTTTGATTTTAT |
| chr20 | 36209454 | 36209478 | + | 25 | ACATCCCCATTCTTTTGATTTTATC |
| chr20 | 36209455 | 36209479 | + | 25 | CATCCCCATTCTTTTGATTTTATCT |
| chr20 | 36209456 | 36209480 | + | 25 | ATCCCCATTCTTTTGATTTTATCTG |
| chr20 | 36209457 | 36209481 | + | 25 | TCCCCATTCTTTTGATTTTATCTGG |
| chr20 | 36209458 | 36209482 | + | 25 | CCCCATTCTTTTGATTTTATCTGGC |
| chr20 | 36209459 | 36209483 | + | 25 | CCCATTCTTTTGATTTTATCTGGCC |
| chr20 | 36209460 | 36209484 | + | 25 | CCATTCTTTTGATTTTATCTGGCCA |
| chr20 | 36209461 | 36209485 | + | 25 | CATTCTTTTGATTTTATCTGGCCAT |
| chr20 | 36209462 | 36209486 | + | 25 | ATTCTTTTGATTTTATCTGGCCATA |
| chr20 | 36209463 | 36209487 | + | 25 | TTCTTTTGATTTTATCTGGCCATAG |
| chr20 | 36209464 | 36209488 | + | 25 | TCTTTTGATTTTATCTGGCCATAGA |
| chr20 | 36209465 | 36209489 | + | 25 | CTTTTGATTTTATCTGGCCATAGAG |
| chr20 | 36209466 | 36209490 | + | 25 | TTTTGATTTTATCTGGCCATAGAGA |
| chr20 | 36209467 | 36209491 | + | 25 | TTTGATTTTATCTGGCCATAGAGAG |
| chr20 | 36209468 | 36209492 | + | 25 | TTGATTTTATCTGGCCATAGAGAGC |
| chr20 | 36209469 | 36209493 | + | 25 | TGATTTTATCTGGCCATAGAGAGCA |
| chr20 | 36209470 | 36209494 | + | 25 | GATTTTATCTGGCCATAGAGAGCAG |
| chr20 | 36209471 | 36209495 | + | 25 | ATTTTATCTGGCCATAGAGAGCAGG |
| chr20 | 36209472 | 36209496 | + | 25 | TTTTATCTGGCCATAGAGAGCAGGG |
| chr20 | 36209473 | 36209497 | + | 25 | TTTATCTGGCCATAGAGAGCAGGGC |
| chr20 | 36209474 | 36209498 | + | 25 | TTATCTGGCCATAGAGAGCAGGGCT |
| chr20 | 36209475 | 36209499 | + | 25 | TATCTGGCCATAGAGAGCAGGGCTG |
| chr20 | 36209476 | 36209500 | + | 25 | ATCTGGCCATAGAGAGCAGGGCTGA |
| chr20 | 36209477 | 36209501 | + | 25 | TCTGGCCATAGAGAGCAGGGCTGAG |
| chr20 | 36209478 | 36209502 | + | 25 | CTGGCCATAGAGAGCAGGGCTGAGG |
| chr20 | 36209479 | 36209503 | + | 25 | TGGCCATAGAGAGCAGGGCTGAGGG |
| chr20 | 36209480 | 36209504 | + | 25 | GGCCATAGAGAGCAGGGCTGAGGGA |
| chr20 | 36209481 | 36209505 | + | 25 | GCCATAGAGAGCAGGGCTGAGGGAG |
| chr20 | 36209482 | 36209506 | + | 25 | CCATAGAGAGCAGGGCTGAGGGAGG |
| chr20 | 36209483 | 36209507 | + | 25 | CATAGAGAGCAGGGCTGAGGGAGGG |
| chr20 | 36209484 | 36209508 | + | 25 | ATAGAGAGCAGGGCTGAGGGAGGGC |
| chr20 | 36209485 | 36209509 | + | 25 | TAGAGAGCAGGGCTGAGGGAGGGCT |
| chr20 | 36209486 | 36209510 | + | 25 | AGAGAGCAGGGCTGAGGGAGGGCTC |
| chr20 | 36209487 | 36209511 | + | 25 | GAGAGCAGGGCTGAGGGAGGGCTCC |
| chr20 | 36209488 | 36209512 | + | 25 | AGAGCAGGGCTGAGGGAGGGCTCCG |
| chr20 | 36209489 | 36209513 | + | 25 | GAGCAGGGCTGAGGGAGGGCTCCGA |
| chr20 | 36209490 | 36209514 | + | 25 | AGCAGGGCTGAGGGAGGGCTCCGAG |
| chr20 | 36209491 | 36209515 | + | 25 | GCAGGGCTGAGGGAGGGCTCCGAGG |
| chr20 | 36209492 | 36209516 | + | 25 | CAGGGCTGAGGGAGGGCTCCGAGGA |
| chr20 | 36209493 | 36209517 | + | 25 | AGGGCTGAGGGAGGGCTCCGAGGAG |
| chr20 | 36209494 | 36209518 | + | 25 | GGGCTGAGGGAGGGCTCCGAGGAGA |
| chr20 | 36209495 | 36209519 | + | 25 | GGCTGAGGGAGGGCTCCGAGGAGAA |
| chr20 | 36209496 | 36209520 | + | 25 | GCTGAGGGAGGGCTCCGAGGAGAAA |
| chr20 | 36209497 | 36209521 | + | 25 | CTGAGGGAGGGCTCCGAGGAGAAAG |
| chr20 | 36209498 | 36209522 | + | 25 | TGAGGGAGGGCTCCGAGGAGAAAGT |
| chr20 | 36209499 | 36209523 | + | 25 | GAGGGAGGGCTCCGAGGAGAAAGTC |
| chr20 | 36209500 | 36209524 | + | 25 | AGGGAGGGCTCCGAGGAGAAAGTCA |
| chr20 | 36209501 | 36209525 | + | 25 | GGGAGGGCTCCGAGGAGAAAGTCAA |
| chr20 | 36209502 | 36209526 | + | 25 | GGAGGGCTCCGAGGAGAAAGTCAAA |
| chr20 | 36209503 | 36209527 | + | 25 | GAGGGCTCCGAGGAGAAAGTCAAAC |
| chr20 | 36209504 | 36209528 | + | 25 | AGGGCTCCGAGGAGAAAGTCAAACC |
| chr20 | 36209505 | 36209529 | + | 25 | GGGCTCCGAGGAGAAAGTCAAACCA |
| chr20 | 36209506 | 36209530 | + | 25 | GGCTCCGAGGAGAAAGTCAAACCAC |
| chr20 | 36209507 | 36209531 | + | 25 | GCTCCGAGGAGAAAGTCAAACCACC |
| chr20 | 36209508 | 36209532 | + | 25 | CTCCGAGGAGAAAGTCAAACCACCA |
| chr20 | 36209509 | 36209533 | + | 25 | TCCGAGGAGAAAGTCAAACCACCAC |
| chr20 | 36209510 | 36209534 | + | 25 | CCGAGGAGAAAGTCAAACCACCACG |
| chr20 | 36209511 | 36209535 | + | 25 | CGAGGAGAAAGTCAAACCACCACGT |
| chr20 | 36209512 | 36209536 | + | 25 | GAGGAGAAAGTCAAACCACCACGTC |
| chr20 | 36209513 | 36209537 | + | 25 | AGGAGAAAGTCAAACCACCACGTCC |
| chr20 | 36209514 | 36209538 | + | 25 | GGAGAAAGTCAAACCACCACGTCCC |
| chr20 | 36209515 | 36209539 | + | 25 | GAGAAAGTCAAACCACCACGTCCCC |
| chr20 | 36209516 | 36209540 | + | 25 | AGAAAGTCAAACCACCACGTCCCCG |
| chr20 | 36209517 | 36209541 | + | 25 | GAAAGTCAAACCACCACGTCCCCGG |
| chr20 | 36209518 | 36209542 | + | 25 | AAAGTCAAACCACCACGTCCCCGGG |
| chr20 | 36209519 | 36209543 | + | 25 | AAGTCAAACCACCACGTCCCCGGGC |
| chr20 | 36209520 | 36209544 | + | 25 | AGTCAAACCACCACGTCCCCGGGCC |
| chr20 | 36209521 | 36209545 | + | 25 | GTCAAACCACCACGTCCCCGGGCCC |
| chr20 | 36209522 | 36209546 | + | 25 | TCAAACCACCACGTCCCCGGGCCCC |
| chr20 | 36209523 | 36209547 | + | 25 | CAAACCACCACGTCCCCGGGCCCCA |
| chr20 | 36209524 | 36209548 | + | 25 | AAACCACCACGTCCCCGGGCCCCAG |
| chr20 | 36209525 | 36209549 | + | 25 | AACCACCACGTCCCCGGGCCCCAGA |
| chr20 | 36209526 | 36209550 | + | 25 | ACCACCACGTCCCCGGGCCCCAGAG |
| chr20 | 36209527 | 36209551 | + | 25 | CCACCACGTCCCCGGGCCCCAGAGA |
| chr20 | 36209528 | 36209552 | + | 25 | CACCACGTCCCCGGGCCCCAGAGAG |
| chr20 | 36209529 | 36209553 | + | 25 | ACCACGTCCCCGGGCCCCAGAGAGT |
| chr20 | 36209530 | 36209554 | + | 25 | CCACGTCCCCGGGCCCCAGAGAGTG |
| chr20 | 36209531 | 36209555 | + | 25 | CACGTCCCCGGGCCCCAGAGAGTGA |
| chr20 | 36209532 | 36209556 | + | 25 | ACGTCCCCGGGCCCCAGAGAGTGAC |
| chr20 | 36209533 | 36209557 | + | 25 | CGTCCCCGGGCCCCAGAGAGTGACA |
| chr20 | 36209534 | 36209558 | + | 25 | GTCCCCGGGCCCCAGAGAGTGACAC |
| chr20 | 36209535 | 36209559 | + | 25 | TCCCCGGGCCCCAGAGAGTGACACA |
| chr20 | 36209536 | 36209560 | + | 25 | CCCCGGGCCCCAGAGAGTGACACAG |
| chr20 | 36209537 | 36209561 | + | 25 | CCCGGGCCCCAGAGAGTGACACAGG |
| chr20 | 36209538 | 36209562 | + | 25 | CCGGGCCCCAGAGAGTGACACAGGC |
| chr20 | 36209539 | 36209563 | + | 25 | CGGGCCCCAGAGAGTGACACAGGCG |
| chr20 | 36209540 | 36209564 | + | 25 | GGGCCCCAGAGAGTGACACAGGCGA |
| chr20 | 36209541 | 36209565 | + | 25 | GGCCCCAGAGAGTGACACAGGCGAT |
| chr20 | 36209542 | 36209566 | + | 25 | GCCCCAGAGAGTGACACAGGCGATG |
| chr20 | 36209543 | 36209567 | + | 25 | CCCCAGAGAGTGACACAGGCGATGA |
| chr20 | 36209544 | 36209568 | + | 25 | CCCAGAGAGTGACACAGGCGATGAG |
| chr20 | 36209545 | 36209569 | + | 25 | CCAGAGAGTGACACAGGCGATGAGG |
| chr20 | 36209546 | 36209570 | + | 25 | CAGAGAGTGACACAGGCGATGAGGA |
| chr20 | 36209547 | 36209571 | + | 25 | AGAGAGTGACACAGGCGATGAGGAC |
| chr20 | 36209548 | 36209572 | + | 25 | GAGAGTGACACAGGCGATGAGGACC |
| chr20 | 36209549 | 36209573 | + | 25 | AGAGTGACACAGGCGATGAGGACCA |
| chr20 | 36209550 | 36209574 | + | 25 | GAGTGACACAGGCGATGAGGACCAG |
| chr20 | 36209551 | 36209575 | + | 25 | AGTGACACAGGCGATGAGGACCAGG |
| chr20 | 36209552 | 36209576 | + | 25 | GTGACACAGGCGATGAGGACCAGGA |
| chr20 | 36209553 | 36209577 | + | 25 | TGACACAGGCGATGAGGACCAGGAC |
| chr20 | 36209554 | 36209578 | + | 25 | GACACAGGCGATGAGGACCAGGACC |
| chr20 | 36209555 | 36209579 | + | 25 | ACACAGGCGATGAGGACCAGGACCA |
| chr20 | 36209556 | 36209580 | + | 25 | CACAGGCGATGAGGACCAGGACCAG |
| chr20 | 36209557 | 36209581 | + | 25 | ACAGGCGATGAGGACCAGGACCAGG |
| chr20 | 36209558 | 36209582 | + | 25 | CAGGCGATGAGGACCAGGACCAGGA |
| chr20 | 36209559 | 36209583 | + | 25 | AGGCGATGAGGACCAGGACCAGGAG |
| chr20 | 36209560 | 36209584 | + | 25 | GGCGATGAGGACCAGGACCAGGAGA |
| chr20 | 36209561 | 36209585 | + | 25 | GCGATGAGGACCAGGACCAGGAGAG |
| chr20 | 36209562 | 36209586 | + | 25 | CGATGAGGACCAGGACCAGGAGAGG |
| chr20 | 36209563 | 36209587 | + | 25 | GATGAGGACCAGGACCAGGAGAGGG |
| chr20 | 36209564 | 36209588 | + | 25 | ATGAGGACCAGGACCAGGAGAGGGA |
| chr20 | 36209565 | 36209589 | + | 25 | TGAGGACCAGGACCAGGAGAGGGAC |
| chr20 | 36209566 | 36209590 | + | 25 | GAGGACCAGGACCAGGAGAGGGACA |
| chr20 | 36209567 | 36209591 | + | 25 | AGGACCAGGACCAGGAGAGGGACAC |
| chr20 | 36209568 | 36209592 | + | 25 | GGACCAGGACCAGGAGAGGGACACG |
| chr20 | 36209569 | 36209593 | + | 25 | GACCAGGACCAGGAGAGGGACACGG |
| chr20 | 36209570 | 36209594 | + | 25 | ACCAGGACCAGGAGAGGGACACGGT |
| chr20 | 36209571 | 36209595 | + | 25 | CCAGGACCAGGAGAGGGACACGGTG |
| chr20 | 36209572 | 36209596 | + | 25 | CAGGACCAGGAGAGGGACACGGTGT |
| chr20 | 36209573 | 36209597 | + | 25 | AGGACCAGGAGAGGGACACGGTGTT |
| chr20 | 36209574 | 36209598 | + | 25 | GGACCAGGAGAGGGACACGGTGTTC |
| chr20 | 36209575 | 36209599 | + | 25 | GACCAGGAGAGGGACACGGTGTTCC |
| chr20 | 36209576 | 36209600 | + | 25 | ACCAGGAGAGGGACACGGTGTTCCT |
| chr20 | 36209577 | 36209601 | + | 25 | CCAGGAGAGGGACACGGTGTTCCTG |
| chr20 | 36209578 | 36209602 | + | 25 | CAGGAGAGGGACACGGTGTTCCTGA |
| chr20 | 36209579 | 36209603 | + | 25 | AGGAGAGGGACACGGTGTTCCTGAA |
| chr20 | 36209580 | 36209604 | + | 25 | GGAGAGGGACACGGTGTTCCTGAAG |
| chr20 | 36209581 | 36209605 | + | 25 | GAGAGGGACACGGTGTTCCTGAAGG |
| chr20 | 36209582 | 36209606 | + | 25 | AGAGGGACACGGTGTTCCTGAAGGA |
| chr20 | 36209583 | 36209607 | + | 25 | GAGGGACACGGTGTTCCTGAAGGAC |
| chr20 | 36209584 | 36209608 | + | 25 | AGGGACACGGTGTTCCTGAAGGACA |
| chr20 | 36209585 | 36209609 | + | 25 | GGGACACGGTGTTCCTGAAGGACAA |
| chr20 | 36209586 | 36209610 | + | 25 | GGACACGGTGTTCCTGAAGGACAAC |
| chr20 | 36209587 | 36209611 | + | 25 | GACACGGTGTTCCTGAAGGACAACC |
| chr20 | 36209588 | 36209612 | + | 25 | ACACGGTGTTCCTGAAGGACAACCA |
| chr20 | 36209589 | 36209613 | + | 25 | CACGGTGTTCCTGAAGGACAACCAC |
| chr20 | 36209590 | 36209614 | + | 25 | ACGGTGTTCCTGAAGGACAACCACC |
| chr20 | 36209591 | 36209615 | + | 25 | CGGTGTTCCTGAAGGACAACCACCT |
| chr20 | 36209592 | 36209616 | + | 25 | GGTGTTCCTGAAGGACAACCACCTG |
| chr20 | 36209593 | 36209617 | + | 25 | GTGTTCCTGAAGGACAACCACCTGG |
| chr20 | 36209594 | 36209618 | + | 25 | TGTTCCTGAAGGACAACCACCTGGC |
| chr20 | 36209595 | 36209619 | + | 25 | GTTCCTGAAGGACAACCACCTGGCC |
| chr20 | 36209596 | 36209620 | + | 25 | TTCCTGAAGGACAACCACCTGGCCA |
| chr20 | 36209597 | 36209621 | + | 25 | TCCTGAAGGACAACCACCTGGCCAT |
| chr20 | 36209598 | 36209622 | + | 25 | CCTGAAGGACAACCACCTGGCCATT |
| chr20 | 36209599 | 36209623 | + | 25 | CTGAAGGACAACCACCTGGCCATTG |
| chr20 | 36209600 | 36209624 | + | 25 | TGAAGGACAACCACCTGGCCATTGA |
| chr20 | 36209601 | 36209625 | + | 25 | GAAGGACAACCACCTGGCCATTGAG |
| chr20 | 36209602 | 36209626 | + | 25 | AAGGACAACCACCTGGCCATTGAGC |
| chr20 | 36209603 | 36209627 | + | 25 | AGGACAACCACCTGGCCATTGAGCG |
| chr20 | 36209604 | 36209628 | + | 25 | GGACAACCACCTGGCCATTGAGCGC |
| chr20 | 36209605 | 36209629 | + | 25 | GACAACCACCTGGCCATTGAGCGCA |
| chr20 | 36209606 | 36209630 | + | 25 | ACAACCACCTGGCCATTGAGCGCAA |
| chr20 | 36209607 | 36209631 | + | 25 | CAACCACCTGGCCATTGAGCGCAAG |
| chr20 | 36209608 | 36209632 | + | 25 | AACCACCTGGCCATTGAGCGCAAGT |
| chr20 | 36209609 | 36209633 | + | 25 | ACCACCTGGCCATTGAGCGCAAGTG |
| chr20 | 36209610 | 36209634 | + | 25 | CCACCTGGCCATTGAGCGCAAGTGC |
| chr20 | 36209611 | 36209635 | + | 25 | CACCTGGCCATTGAGCGCAAGTGCT |
| chr20 | 36209612 | 36209636 | + | 25 | ACCTGGCCATTGAGCGCAAGTGCTC |
| chr20 | 36209613 | 36209637 | + | 25 | CCTGGCCATTGAGCGCAAGTGCTCC |
| chr20 | 36209614 | 36209638 | + | 25 | CTGGCCATTGAGCGCAAGTGCTCCA |
| chr20 | 36209615 | 36209639 | + | 25 | TGGCCATTGAGCGCAAGTGCTCCAG |
| chr20 | 36209616 | 36209640 | + | 25 | GGCCATTGAGCGCAAGTGCTCCAGC |
| chr20 | 36209617 | 36209641 | + | 25 | GCCATTGAGCGCAAGTGCTCCAGCA |
| chr20 | 36209618 | 36209642 | + | 25 | CCATTGAGCGCAAGTGCTCCAGCAT |
| chr20 | 36209619 | 36209643 | + | 25 | CATTGAGCGCAAGTGCTCCAGCATC |
| chr20 | 36209620 | 36209644 | + | 25 | ATTGAGCGCAAGTGCTCCAGCATCA |
| chr20 | 36209621 | 36209645 | + | 25 | TTGAGCGCAAGTGCTCCAGCATCAC |
| chr20 | 36209622 | 36209646 | + | 25 | TGAGCGCAAGTGCTCCAGCATCACG |
| chr20 | 36209623 | 36209647 | + | 25 | GAGCGCAAGTGCTCCAGCATCACGG |
| chr20 | 36209624 | 36209648 | + | 25 | AGCGCAAGTGCTCCAGCATCACGGT |
| chr20 | 36209625 | 36209649 | + | 25 | GCGCAAGTGCTCCAGCATCACGGTC |
| chr20 | 36209626 | 36209650 | + | 25 | CGCAAGTGCTCCAGCATCACGGTCA |
| chr20 | 36209627 | 36209651 | + | 25 | GCAAGTGCTCCAGCATCACGGTCAG |
| chr20 | 36209628 | 36209652 | + | 25 | CAAGTGCTCCAGCATCACGGTCAGC |
| chr20 | 36209629 | 36209653 | + | 25 | AAGTGCTCCAGCATCACGGTCAGCT |
| chr20 | 36209630 | 36209654 | + | 25 | AGTGCTCCAGCATCACGGTCAGCTC |
| chr20 | 36209631 | 36209655 | + | 25 | GTGCTCCAGCATCACGGTCAGCTCT |
| chr20 | 36209632 | 36209656 | + | 25 | TGCTCCAGCATCACGGTCAGCTCTA |
| chr20 | 36209633 | 36209657 | + | 25 | GCTCCAGCATCACGGTCAGCTCTAC |
| chr20 | 36209634 | 36209658 | + | 25 | CTCCAGCATCACGGTCAGCTCTACG |
| chr20 | 36209635 | 36209659 | + | 25 | TCCAGCATCACGGTCAGCTCTACGT |
| chr20 | 36209636 | 36209660 | + | 25 | CCAGCATCACGGTCAGCTCTACGTC |
| chr20 | 36209637 | 36209661 | + | 25 | CAGCATCACGGTCAGCTCTACGTCT |
| chr20 | 36209638 | 36209662 | + | 25 | AGCATCACGGTCAGCTCTACGTCTA |
| chr20 | 36209639 | 36209663 | + | 25 | GCATCACGGTCAGCTCTACGTCTAG |
| chr20 | 36209640 | 36209664 | + | 25 | CATCACGGTCAGCTCTACGTCTAGC |
| chr20 | 36209641 | 36209665 | + | 25 | ATCACGGTCAGCTCTACGTCTAGCC |
| chr20 | 36209642 | 36209666 | + | 25 | TCACGGTCAGCTCTACGTCTAGCCT |
| chr20 | 36209643 | 36209667 | + | 25 | CACGGTCAGCTCTACGTCTAGCCTG |
| chr20 | 36209644 | 36209668 | + | 25 | ACGGTCAGCTCTACGTCTAGCCTGG |
| chr20 | 36209645 | 36209669 | + | 25 | CGGTCAGCTCTACGTCTAGCCTGGA |
| chr20 | 36209646 | 36209670 | + | 25 | GGTCAGCTCTACGTCTAGCCTGGAG |
| chr20 | 36209647 | 36209671 | + | 25 | GTCAGCTCTACGTCTAGCCTGGAGG |
| chr20 | 36209648 | 36209672 | + | 25 | TCAGCTCTACGTCTAGCCTGGAGGC |
| chr20 | 36209649 | 36209673 | + | 25 | CAGCTCTACGTCTAGCCTGGAGGCT |
| chr20 | 36209650 | 36209674 | + | 25 | AGCTCTACGTCTAGCCTGGAGGCTG |
| chr20 | 36209651 | 36209675 | + | 25 | GCTCTACGTCTAGCCTGGAGGCTGA |
| chr20 | 36209652 | 36209676 | + | 25 | CTCTACGTCTAGCCTGGAGGCTGAG |
| chr20 | 36209653 | 36209677 | + | 25 | TCTACGTCTAGCCTGGAGGCTGAGG |
| chr20 | 36209654 | 36209678 | + | 25 | CTACGTCTAGCCTGGAGGCTGAGGT |
| chr20 | 36209655 | 36209679 | + | 25 | TACGTCTAGCCTGGAGGCTGAGGTG |
| chr20 | 36209656 | 36209680 | + | 25 | ACGTCTAGCCTGGAGGCTGAGGTGG |
| chr20 | 36209657 | 36209681 | + | 25 | CGTCTAGCCTGGAGGCTGAGGTGGA |
| chr20 | 36209658 | 36209682 | + | 25 | GTCTAGCCTGGAGGCTGAGGTGGAC |
| chr20 | 36209659 | 36209683 | + | 25 | TCTAGCCTGGAGGCTGAGGTGGACT |
| chr20 | 36209660 | 36209684 | + | 25 | CTAGCCTGGAGGCTGAGGTGGACTT |
| chr20 | 36209661 | 36209685 | + | 25 | TAGCCTGGAGGCTGAGGTGGACTTC |
| chr20 | 36209662 | 36209686 | + | 25 | AGCCTGGAGGCTGAGGTGGACTTCA |
| chr20 | 36209663 | 36209687 | + | 25 | GCCTGGAGGCTGAGGTGGACTTCAC |
| chr20 | 36209664 | 36209688 | + | 25 | CCTGGAGGCTGAGGTGGACTTCACG |
| chr20 | 36209665 | 36209689 | + | 25 | CTGGAGGCTGAGGTGGACTTCACGG |
| chr20 | 36209666 | 36209690 | + | 25 | TGGAGGCTGAGGTGGACTTCACGGT |
| chr20 | 36209667 | 36209691 | + | 25 | GGAGGCTGAGGTGGACTTCACGGTC |
| chr20 | 36209668 | 36209692 | + | 25 | GAGGCTGAGGTGGACTTCACGGTCA |
| chr20 | 36209669 | 36209693 | + | 25 | AGGCTGAGGTGGACTTCACGGTCAT |
| chr20 | 36209670 | 36209694 | + | 25 | GGCTGAGGTGGACTTCACGGTCATT |
| chr20 | 36209671 | 36209695 | + | 25 | GCTGAGGTGGACTTCACGGTCATTG |
| chr20 | 36209672 | 36209696 | + | 25 | CTGAGGTGGACTTCACGGTCATTGG |
| chr20 | 36209673 | 36209697 | + | 25 | TGAGGTGGACTTCACGGTCATTGGT |
| chr20 | 36209674 | 36209698 | + | 25 | GAGGTGGACTTCACGGTCATTGGTG |
| chr20 | 36209675 | 36209699 | + | 25 | AGGTGGACTTCACGGTCATTGGTGA |
| chr20 | 36209676 | 36209700 | + | 25 | GGTGGACTTCACGGTCATTGGTGAC |
| chr20 | 36209677 | 36209701 | + | 25 | GTGGACTTCACGGTCATTGGTGACT |
| chr20 | 36209678 | 36209702 | + | 25 | TGGACTTCACGGTCATTGGTGACTA |
| chr20 | 36209679 | 36209703 | + | 25 | GGACTTCACGGTCATTGGTGACTAC |
| chr20 | 36209680 | 36209704 | + | 25 | GACTTCACGGTCATTGGTGACTACC |
| chr20 | 36209681 | 36209705 | + | 25 | ACTTCACGGTCATTGGTGACTACCA |
| chr20 | 36209682 | 36209706 | + | 25 | CTTCACGGTCATTGGTGACTACCAT |
| chr20 | 36209683 | 36209707 | + | 25 | TTCACGGTCATTGGTGACTACCATG |
| chr20 | 36209684 | 36209708 | + | 25 | TCACGGTCATTGGTGACTACCATGG |
| chr20 | 36209685 | 36209709 | + | 25 | CACGGTCATTGGTGACTACCATGGC |
| chr20 | 36209686 | 36209710 | + | 25 | ACGGTCATTGGTGACTACCATGGCA |
| chr20 | 36209687 | 36209711 | + | 25 | CGGTCATTGGTGACTACCATGGCAG |
| chr20 | 36209688 | 36209712 | + | 25 | GGTCATTGGTGACTACCATGGCAGC |
| chr20 | 36209689 | 36209713 | + | 25 | GTCATTGGTGACTACCATGGCAGCG |
| chr20 | 36209690 | 36209714 | + | 25 | TCATTGGTGACTACCATGGCAGCGC |
| chr20 | 36209691 | 36209715 | + | 25 | CATTGGTGACTACCATGGCAGCGCC |
| chr20 | 36209692 | 36209716 | + | 25 | ATTGGTGACTACCATGGCAGCGCCT |
| chr20 | 36209693 | 36209717 | + | 25 | TTGGTGACTACCATGGCAGCGCCTT |
| chr20 | 36209694 | 36209718 | + | 25 | TGGTGACTACCATGGCAGCGCCTTC |
| chr20 | 36209695 | 36209719 | + | 25 | GGTGACTACCATGGCAGCGCCTTCG |
| chr20 | 36209696 | 36209720 | + | 25 | GTGACTACCATGGCAGCGCCTTCGA |
| chr20 | 36209697 | 36209721 | + | 25 | TGACTACCATGGCAGCGCCTTCGAA |
| chr20 | 36209698 | 36209722 | + | 25 | GACTACCATGGCAGCGCCTTCGAAG |
| chr20 | 36209699 | 36209723 | + | 25 | ACTACCATGGCAGCGCCTTCGAAGA |
| chr20 | 36209700 | 36209724 | + | 25 | CTACCATGGCAGCGCCTTCGAAGAC |
| chr20 | 36209701 | 36209725 | + | 25 | TACCATGGCAGCGCCTTCGAAGACT |
| chr20 | 36209702 | 36209726 | + | 25 | ACCATGGCAGCGCCTTCGAAGACTT |
| chr20 | 36209703 | 36209727 | + | 25 | CCATGGCAGCGCCTTCGAAGACTTC |
| chr20 | 36209704 | 36209728 | + | 25 | CATGGCAGCGCCTTCGAAGACTTCT |
| chr20 | 36209705 | 36209729 | + | 25 | ATGGCAGCGCCTTCGAAGACTTCTC |
| chr20 | 36209706 | 36209730 | + | 25 | TGGCAGCGCCTTCGAAGACTTCTCC |
| chr20 | 36209707 | 36209731 | + | 25 | GGCAGCGCCTTCGAAGACTTCTCCC |
| chr20 | 36209708 | 36209732 | + | 25 | GCAGCGCCTTCGAAGACTTCTCCCG |
| chr20 | 36209709 | 36209733 | + | 25 | CAGCGCCTTCGAAGACTTCTCCCGC |
| chr20 | 36209710 | 36209734 | + | 25 | AGCGCCTTCGAAGACTTCTCCCGCA |
| chr20 | 36209711 | 36209735 | + | 25 | GCGCCTTCGAAGACTTCTCCCGCAG |
| chr20 | 36209712 | 36209736 | + | 25 | CGCCTTCGAAGACTTCTCCCGCAGC |
| chr20 | 36209713 | 36209737 | + | 25 | GCCTTCGAAGACTTCTCCCGCAGCC |
| chr20 | 36209714 | 36209738 | + | 25 | CCTTCGAAGACTTCTCCCGCAGCCT |
| chr20 | 36209715 | 36209739 | + | 25 | CTTCGAAGACTTCTCCCGCAGCCTG |
| chr20 | 36209716 | 36209740 | + | 25 | TTCGAAGACTTCTCCCGCAGCCTGC |
| chr20 | 36209717 | 36209741 | + | 25 | TCGAAGACTTCTCCCGCAGCCTGCC |
| chr20 | 36209718 | 36209742 | + | 25 | CGAAGACTTCTCCCGCAGCCTGCCT |
| chr20 | 36209719 | 36209743 | + | 25 | GAAGACTTCTCCCGCAGCCTGCCTG |
| chr20 | 36209720 | 36209744 | + | 25 | AAGACTTCTCCCGCAGCCTGCCTGA |
| chr20 | 36209721 | 36209745 | + | 25 | AGACTTCTCCCGCAGCCTGCCTGAG |
| chr20 | 36209722 | 36209746 | + | 25 | GACTTCTCCCGCAGCCTGCCTGAGC |
| chr20 | 36209723 | 36209747 | + | 25 | ACTTCTCCCGCAGCCTGCCTGAGCT |
| chr20 | 36209724 | 36209748 | + | 25 | CTTCTCCCGCAGCCTGCCTGAGCTC |
| chr20 | 36209725 | 36209749 | + | 25 | TTCTCCCGCAGCCTGCCTGAGCTCG |
| chr20 | 36209726 | 36209750 | + | 25 | TCTCCCGCAGCCTGCCTGAGCTCGA |
| chr20 | 36209727 | 36209751 | + | 25 | CTCCCGCAGCCTGCCTGAGCTCGAC |
| chr20 | 36209728 | 36209752 | + | 25 | TCCCGCAGCCTGCCTGAGCTCGACC |
| chr20 | 36209729 | 36209753 | + | 25 | CCCGCAGCCTGCCTGAGCTCGACCG |
| chr20 | 36209730 | 36209754 | + | 25 | CCGCAGCCTGCCTGAGCTCGACCGG |
| chr20 | 36209731 | 36209755 | + | 25 | CGCAGCCTGCCTGAGCTCGACCGGG |
| chr20 | 36209732 | 36209756 | + | 25 | GCAGCCTGCCTGAGCTCGACCGGGA |
| chr20 | 36209733 | 36209757 | + | 25 | CAGCCTGCCTGAGCTCGACCGGGAC |
| chr20 | 36209734 | 36209758 | + | 25 | AGCCTGCCTGAGCTCGACCGGGACA |
| chr20 | 36209735 | 36209759 | + | 25 | GCCTGCCTGAGCTCGACCGGGACAA |
| chr20 | 36209736 | 36209760 | + | 25 | CCTGCCTGAGCTCGACCGGGACAAA |
| chr20 | 36209737 | 36209761 | + | 25 | CTGCCTGAGCTCGACCGGGACAAAA |
| chr20 | 36209738 | 36209762 | + | 25 | TGCCTGAGCTCGACCGGGACAAAAG |
| chr20 | 36209739 | 36209763 | + | 25 | GCCTGAGCTCGACCGGGACAAAAGC |
| chr20 | 36209740 | 36209764 | + | 25 | CCTGAGCTCGACCGGGACAAAAGCG |
| chr20 | 36209741 | 36209765 | + | 25 | CTGAGCTCGACCGGGACAAAAGCGA |
| chr20 | 36209742 | 36209766 | + | 25 | TGAGCTCGACCGGGACAAAAGCGAC |
| chr20 | 36209743 | 36209767 | + | 25 | GAGCTCGACCGGGACAAAAGCGACT |
| chr20 | 36209744 | 36209768 | + | 25 | AGCTCGACCGGGACAAAAGCGACTC |
| chr20 | 36209745 | 36209769 | + | 25 | GCTCGACCGGGACAAAAGCGACTCG |
| chr20 | 36209746 | 36209770 | + | 25 | CTCGACCGGGACAAAAGCGACTCGG |
| chr20 | 36209747 | 36209771 | + | 25 | TCGACCGGGACAAAAGCGACTCGGA |
| chr20 | 36209748 | 36209772 | + | 25 | CGACCGGGACAAAAGCGACTCGGAC |
| chr20 | 36209749 | 36209773 | + | 25 | GACCGGGACAAAAGCGACTCGGACA |
| chr20 | 36209750 | 36209774 | + | 25 | ACCGGGACAAAAGCGACTCGGACAC |
| chr20 | 36209751 | 36209775 | + | 25 | CCGGGACAAAAGCGACTCGGACACT |
| chr20 | 36209752 | 36209776 | + | 25 | CGGGACAAAAGCGACTCGGACACTG |
| chr20 | 36209753 | 36209777 | + | 25 | GGGACAAAAGCGACTCGGACACTGA |
| chr20 | 36209754 | 36209778 | + | 25 | GGACAAAAGCGACTCGGACACTGAG |
| chr20 | 36209755 | 36209779 | + | 25 | GACAAAAGCGACTCGGACACTGAGG |
| chr20 | 36209756 | 36209780 | + | 25 | ACAAAAGCGACTCGGACACTGAGGG |
| chr20 | 36209757 | 36209781 | + | 25 | CAAAAGCGACTCGGACACTGAGGGC |
| chr20 | 36209758 | 36209782 | + | 25 | AAAAGCGACTCGGACACTGAGGGCC |
| chr20 | 36209759 | 36209783 | + | 25 | AAAGCGACTCGGACACTGAGGGCCT |
| chr20 | 36209760 | 36209784 | + | 25 | AAGCGACTCGGACACTGAGGGCCTG |
| chr20 | 36209761 | 36209785 | + | 25 | AGCGACTCGGACACTGAGGGCCTGC |
| chr20 | 36209762 | 36209786 | + | 25 | GCGACTCGGACACTGAGGGCCTGCT |
| chr20 | 36209763 | 36209787 | + | 25 | CGACTCGGACACTGAGGGCCTGCTG |
| chr20 | 36209764 | 36209788 | + | 25 | GACTCGGACACTGAGGGCCTGCTGT |
| chr20 | 36209765 | 36209789 | + | 25 | ACTCGGACACTGAGGGCCTGCTGTT |
| chr20 | 36209766 | 36209790 | + | 25 | CTCGGACACTGAGGGCCTGCTGTTC |
| chr20 | 36209767 | 36209791 | + | 25 | TCGGACACTGAGGGCCTGCTGTTCT |
| chr20 | 36209768 | 36209792 | + | 25 | CGGACACTGAGGGCCTGCTGTTCTC |
| chr20 | 36209769 | 36209793 | + | 25 | GGACACTGAGGGCCTGCTGTTCTCC |
| chr20 | 36209770 | 36209794 | + | 25 | GACACTGAGGGCCTGCTGTTCTCCC |
| chr20 | 36209771 | 36209795 | + | 25 | ACACTGAGGGCCTGCTGTTCTCCCG |
| chr20 | 36209772 | 36209796 | + | 25 | CACTGAGGGCCTGCTGTTCTCCCGG |
| chr20 | 36209773 | 36209797 | + | 25 | ACTGAGGGCCTGCTGTTCTCCCGGG |
| chr20 | 36209774 | 36209798 | + | 25 | CTGAGGGCCTGCTGTTCTCCCGGGA |
| chr20 | 36209775 | 36209799 | + | 25 | TGAGGGCCTGCTGTTCTCCCGGGAT |
| chr20 | 36209776 | 36209800 | + | 25 | GAGGGCCTGCTGTTCTCCCGGGATC |
| chr20 | 36209777 | 36209801 | + | 25 | AGGGCCTGCTGTTCTCCCGGGATCT |
| chr20 | 36209778 | 36209802 | + | 25 | GGGCCTGCTGTTCTCCCGGGATCTC |
| chr20 | 36209779 | 36209803 | + | 25 | GGCCTGCTGTTCTCCCGGGATCTCA |
| chr20 | 36209780 | 36209804 | + | 25 | GCCTGCTGTTCTCCCGGGATCTCAA |
| chr20 | 36209781 | 36209805 | + | 25 | CCTGCTGTTCTCCCGGGATCTCAAC |
| chr20 | 36209782 | 36209806 | + | 25 | CTGCTGTTCTCCCGGGATCTCAACA |
| chr20 | 36209783 | 36209807 | + | 25 | TGCTGTTCTCCCGGGATCTCAACAA |
| chr20 | 36209784 | 36209808 | + | 25 | GCTGTTCTCCCGGGATCTCAACAAG |
| chr20 | 36209785 | 36209809 | + | 25 | CTGTTCTCCCGGGATCTCAACAAGG |
| chr20 | 36209786 | 36209810 | + | 25 | TGTTCTCCCGGGATCTCAACAAGGG |
| chr20 | 36209787 | 36209811 | + | 25 | GTTCTCCCGGGATCTCAACAAGGGG |
| chr20 | 36209788 | 36209812 | + | 25 | TTCTCCCGGGATCTCAACAAGGGGG |
| chr20 | 36209789 | 36209813 | + | 25 | TCTCCCGGGATCTCAACAAGGGGGC |
| chr20 | 36209790 | 36209814 | + | 25 | CTCCCGGGATCTCAACAAGGGGGCC |
| chr20 | 36209791 | 36209815 | + | 25 | TCCCGGGATCTCAACAAGGGGGCCC |
| chr20 | 36209792 | 36209816 | + | 25 | CCCGGGATCTCAACAAGGGGGCCCC |
| chr20 | 36209793 | 36209817 | + | 25 | CCGGGATCTCAACAAGGGGGCCCCC |
| chr20 | 36209794 | 36209818 | + | 25 | CGGGATCTCAACAAGGGGGCCCCCA |
| chr20 | 36209795 | 36209819 | + | 25 | GGGATCTCAACAAGGGGGCCCCCAG |
| chr20 | 36209796 | 36209820 | + | 25 | GGATCTCAACAAGGGGGCCCCCAGC |
| chr20 | 36209797 | 36209821 | + | 25 | GATCTCAACAAGGGGGCCCCCAGCC |
| chr20 | 36209798 | 36209822 | + | 25 | ATCTCAACAAGGGGGCCCCCAGCCA |
| chr20 | 36209799 | 36209823 | + | 25 | TCTCAACAAGGGGGCCCCCAGCCAG |
| chr20 | 36209800 | 36209824 | + | 25 | CTCAACAAGGGGGCCCCCAGCCAGG |
| chr20 | 36209801 | 36209825 | + | 25 | TCAACAAGGGGGCCCCCAGCCAGGA |
| chr20 | 36209802 | 36209826 | + | 25 | CAACAAGGGGGCCCCCAGCCAGGAT |
| chr20 | 36209803 | 36209827 | + | 25 | AACAAGGGGGCCCCCAGCCAGGATG |
| chr20 | 36209804 | 36209828 | + | 25 | ACAAGGGGGCCCCCAGCCAGGATGA |
| chr20 | 36209805 | 36209829 | + | 25 | CAAGGGGGCCCCCAGCCAGGATGAT |
| chr20 | 36209806 | 36209830 | + | 25 | AAGGGGGCCCCCAGCCAGGATGATG |
| chr20 | 36209807 | 36209831 | + | 25 | AGGGGGCCCCCAGCCAGGATGATGA |
| chr20 | 36209808 | 36209832 | + | 25 | GGGGGCCCCCAGCCAGGATGATGAG |
| chr20 | 36209809 | 36209833 | + | 25 | GGGGCCCCCAGCCAGGATGATGAGT |
| chr20 | 36209810 | 36209834 | + | 25 | GGGCCCCCAGCCAGGATGATGAGTC |
| chr20 | 36209811 | 36209835 | + | 25 | GGCCCCCAGCCAGGATGATGAGTCT |
| chr20 | 36209812 | 36209836 | + | 25 | GCCCCCAGCCAGGATGATGAGTCTG |
| chr20 | 36209813 | 36209837 | + | 25 | CCCCCAGCCAGGATGATGAGTCTGG |
| chr20 | 36209814 | 36209838 | + | 25 | CCCCAGCCAGGATGATGAGTCTGGG |
| chr20 | 36209815 | 36209839 | + | 25 | CCCAGCCAGGATGATGAGTCTGGGG |
| chr20 | 36209816 | 36209840 | + | 25 | CCAGCCAGGATGATGAGTCTGGGGG |
| chr20 | 36209817 | 36209841 | + | 25 | CAGCCAGGATGATGAGTCTGGGGGC |
| chr20 | 36209818 | 36209842 | + | 25 | AGCCAGGATGATGAGTCTGGGGGCA |
| chr20 | 36209819 | 36209843 | + | 25 | GCCAGGATGATGAGTCTGGGGGCAT |
| chr20 | 36209820 | 36209844 | + | 25 | CCAGGATGATGAGTCTGGGGGCATT |
| chr20 | 36209821 | 36209845 | + | 25 | CAGGATGATGAGTCTGGGGGCATTG |
| chr20 | 36209822 | 36209846 | + | 25 | AGGATGATGAGTCTGGGGGCATTGA |
| chr20 | 36209823 | 36209847 | + | 25 | GGATGATGAGTCTGGGGGCATTGAG |
| chr20 | 36209824 | 36209848 | + | 25 | GATGATGAGTCTGGGGGCATTGAGG |
| chr20 | 36209825 | 36209849 | + | 25 | ATGATGAGTCTGGGGGCATTGAGGA |
| chr20 | 36209826 | 36209850 | + | 25 | TGATGAGTCTGGGGGCATTGAGGAC |
| chr20 | 36209827 | 36209851 | + | 25 | GATGAGTCTGGGGGCATTGAGGACA |
| chr20 | 36209828 | 36209852 | + | 25 | ATGAGTCTGGGGGCATTGAGGACAG |
| chr20 | 36209829 | 36209853 | + | 25 | TGAGTCTGGGGGCATTGAGGACAGC |
| chr20 | 36209830 | 36209854 | + | 25 | GAGTCTGGGGGCATTGAGGACAGCC |
| chr20 | 36209831 | 36209855 | + | 25 | AGTCTGGGGGCATTGAGGACAGCCC |
| chr20 | 36209832 | 36209856 | + | 25 | GTCTGGGGGCATTGAGGACAGCCCG |
| chr20 | 36209833 | 36209857 | + | 25 | TCTGGGGGCATTGAGGACAGCCCGG |
| chr20 | 36209834 | 36209858 | + | 25 | CTGGGGGCATTGAGGACAGCCCGGA |
| chr20 | 36209835 | 36209859 | + | 25 | TGGGGGCATTGAGGACAGCCCGGAT |
| chr20 | 36209836 | 36209860 | + | 25 | GGGGGCATTGAGGACAGCCCGGATC |
| chr20 | 36209837 | 36209861 | + | 25 | GGGGCATTGAGGACAGCCCGGATCG |
| chr20 | 36209838 | 36209862 | + | 25 | GGGCATTGAGGACAGCCCGGATCGA |
| chr20 | 36209839 | 36209863 | + | 25 | GGCATTGAGGACAGCCCGGATCGAG |
| chr20 | 36209840 | 36209864 | + | 25 | GCATTGAGGACAGCCCGGATCGAGG |
| chr20 | 36209841 | 36209865 | + | 25 | CATTGAGGACAGCCCGGATCGAGGG |
| chr20 | 36209842 | 36209866 | + | 25 | ATTGAGGACAGCCCGGATCGAGGGG |
| chr20 | 36209843 | 36209867 | + | 25 | TTGAGGACAGCCCGGATCGAGGGGC |
| chr20 | 36209844 | 36209868 | + | 25 | TGAGGACAGCCCGGATCGAGGGGCC |
| chr20 | 36209845 | 36209869 | + | 25 | GAGGACAGCCCGGATCGAGGGGCCT |
| chr20 | 36209846 | 36209870 | + | 25 | AGGACAGCCCGGATCGAGGGGCCTG |
| chr20 | 36209847 | 36209871 | + | 25 | GGACAGCCCGGATCGAGGGGCCTGC |
| chr20 | 36209848 | 36209872 | + | 25 | GACAGCCCGGATCGAGGGGCCTGCT |
| chr20 | 36209849 | 36209873 | + | 25 | ACAGCCCGGATCGAGGGGCCTGCTC |
| chr20 | 36209850 | 36209874 | + | 25 | CAGCCCGGATCGAGGGGCCTGCTCC |
| chr20 | 36209851 | 36209875 | + | 25 | AGCCCGGATCGAGGGGCCTGCTCCA |
| chr20 | 36209852 | 36209876 | + | 25 | GCCCGGATCGAGGGGCCTGCTCCAC |
| chr20 | 36209853 | 36209877 | + | 25 | CCCGGATCGAGGGGCCTGCTCCACC |
| chr20 | 36209854 | 36209878 | + | 25 | CCGGATCGAGGGGCCTGCTCCACCC |
| chr20 | 36209855 | 36209879 | + | 25 | CGGATCGAGGGGCCTGCTCCACCCC |
| chr20 | 36209856 | 36209880 | + | 25 | GGATCGAGGGGCCTGCTCCACCCCG |
| chr20 | 36209857 | 36209881 | + | 25 | GATCGAGGGGCCTGCTCCACCCCGG |
| chr20 | 36209858 | 36209882 | + | 25 | ATCGAGGGGCCTGCTCCACCCCGGA |
| chr20 | 36209859 | 36209883 | + | 25 | TCGAGGGGCCTGCTCCACCCCGGAT |
| chr20 | 36209860 | 36209884 | + | 25 | CGAGGGGCCTGCTCCACCCCGGATA |
| chr20 | 36209861 | 36209885 | + | 25 | GAGGGGCCTGCTCCACCCCGGATAT |
| chr20 | 36209862 | 36209886 | + | 25 | AGGGGCCTGCTCCACCCCGGATATG |
| chr20 | 36209863 | 36209887 | + | 25 | GGGGCCTGCTCCACCCCGGATATGC |
| chr20 | 36209864 | 36209888 | + | 25 | GGGCCTGCTCCACCCCGGATATGCC |
| chr20 | 36209865 | 36209889 | + | 25 | GGCCTGCTCCACCCCGGATATGCCC |
| chr20 | 36209866 | 36209890 | + | 25 | GCCTGCTCCACCCCGGATATGCCCC |
| chr20 | 36209867 | 36209891 | + | 25 | CCTGCTCCACCCCGGATATGCCCCA |
| chr20 | 36209868 | 36209892 | + | 25 | CTGCTCCACCCCGGATATGCCCCAG |
| chr20 | 36209869 | 36209893 | + | 25 | TGCTCCACCCCGGATATGCCCCAGT |
| chr20 | 36209870 | 36209894 | + | 25 | GCTCCACCCCGGATATGCCCCAGTT |
| chr20 | 36209871 | 36209895 | + | 25 | CTCCACCCCGGATATGCCCCAGTTT |
| chr20 | 36209872 | 36209896 | + | 25 | TCCACCCCGGATATGCCCCAGTTTG |
| chr20 | 36209873 | 36209897 | + | 25 | CCACCCCGGATATGCCCCAGTTTGA |
| chr20 | 36209874 | 36209898 | + | 25 | CACCCCGGATATGCCCCAGTTTGAG |
| chr20 | 36209875 | 36209899 | + | 25 | ACCCCGGATATGCCCCAGTTTGAGG |
| chr20 | 36209876 | 36209900 | + | 25 | CCCCGGATATGCCCCAGTTTGAGGT |
| chr20 | 36209877 | 36209901 | + | 25 | CCCGGATATGCCCCAGTTTGAGGTA |
| chr20 | 36209878 | 36209902 | + | 25 | CCGGATATGCCCCAGTTTGAGGTAC |
| chr20 | 36209879 | 36209903 | + | 25 | CGGATATGCCCCAGTTTGAGGTACA |
| chr20 | 36209880 | 36209904 | + | 25 | GGATATGCCCCAGTTTGAGGTACAG |
| chr20 | 36209881 | 36209905 | + | 25 | GATATGCCCCAGTTTGAGGTACAGT |
| chr20 | 36209882 | 36209906 | + | 25 | ATATGCCCCAGTTTGAGGTACAGTG |
| chr20 | 36209883 | 36209907 | + | 25 | TATGCCCCAGTTTGAGGTACAGTGG |
| chr20 | 36209884 | 36209908 | + | 25 | ATGCCCCAGTTTGAGGTACAGTGGA |
| chr20 | 36209885 | 36209909 | + | 25 | TGCCCCAGTTTGAGGTACAGTGGAG |
| chr20 | 36209886 | 36209910 | + | 25 | GCCCCAGTTTGAGGTACAGTGGAGC |
| chr20 | 36209887 | 36209911 | + | 25 | CCCCAGTTTGAGGTACAGTGGAGCT |
| chr20 | 36209888 | 36209912 | + | 25 | CCCAGTTTGAGGTACAGTGGAGCTT |
| chr20 | 36209889 | 36209913 | + | 25 | CCAGTTTGAGGTACAGTGGAGCTTC |
| chr20 | 36209890 | 36209914 | + | 25 | CAGTTTGAGGTACAGTGGAGCTTCC |
| chr20 | 36209891 | 36209915 | + | 25 | AGTTTGAGGTACAGTGGAGCTTCCT |
| chr20 | 36209892 | 36209916 | + | 25 | GTTTGAGGTACAGTGGAGCTTCCTC |
| chr20 | 36209893 | 36209917 | + | 25 | TTTGAGGTACAGTGGAGCTTCCTCA |
| chr20 | 36209894 | 36209918 | + | 25 | TTGAGGTACAGTGGAGCTTCCTCAA |
| chr20 | 36209895 | 36209919 | + | 25 | TGAGGTACAGTGGAGCTTCCTCAAG |
| chr20 | 36209896 | 36209920 | + | 25 | GAGGTACAGTGGAGCTTCCTCAAGA |
| chr20 | 36209897 | 36209921 | + | 25 | AGGTACAGTGGAGCTTCCTCAAGAG |
| chr20 | 36209898 | 36209922 | + | 25 | GGTACAGTGGAGCTTCCTCAAGAGC |
| chr20 | 36209899 | 36209923 | + | 25 | GTACAGTGGAGCTTCCTCAAGAGCC |
| chr20 | 36209900 | 36209924 | + | 25 | TACAGTGGAGCTTCCTCAAGAGCCA |
| chr20 | 36209901 | 36209925 | + | 25 | ACAGTGGAGCTTCCTCAAGAGCCAG |
| chr20 | 36209902 | 36209926 | + | 25 | CAGTGGAGCTTCCTCAAGAGCCAGG |
| chr20 | 36209903 | 36209927 | + | 25 | AGTGGAGCTTCCTCAAGAGCCAGGC |
| chr20 | 36209904 | 36209928 | + | 25 | GTGGAGCTTCCTCAAGAGCCAGGCC |
| chr20 | 36209905 | 36209929 | + | 25 | TGGAGCTTCCTCAAGAGCCAGGCCC |
| chr20 | 36209906 | 36209930 | + | 25 | GGAGCTTCCTCAAGAGCCAGGCCCG |
| chr20 | 36209907 | 36209931 | + | 25 | GAGCTTCCTCAAGAGCCAGGCCCGC |
| chr20 | 36209908 | 36209932 | + | 25 | AGCTTCCTCAAGAGCCAGGCCCGCT |
| chr20 | 36209909 | 36209933 | + | 25 | GCTTCCTCAAGAGCCAGGCCCGCTG |
| chr20 | 36209910 | 36209934 | + | 25 | CTTCCTCAAGAGCCAGGCCCGCTGG |
| chr20 | 36209911 | 36209935 | + | 25 | TTCCTCAAGAGCCAGGCCCGCTGGG |
| chr20 | 36209912 | 36209936 | + | 25 | TCCTCAAGAGCCAGGCCCGCTGGGC |
| chr20 | 36209913 | 36209937 | + | 25 | CCTCAAGAGCCAGGCCCGCTGGGCA |
| chr20 | 36209914 | 36209938 | + | 25 | CTCAAGAGCCAGGCCCGCTGGGCAC |
| chr20 | 36209915 | 36209939 | + | 25 | TCAAGAGCCAGGCCCGCTGGGCACC |
| chr20 | 36209916 | 36209940 | + | 25 | CAAGAGCCAGGCCCGCTGGGCACCG |
| chr20 | 36209917 | 36209941 | + | 25 | AAGAGCCAGGCCCGCTGGGCACCGC |
| chr20 | 36209918 | 36209942 | + | 25 | AGAGCCAGGCCCGCTGGGCACCGCA |
| chr20 | 36209919 | 36209943 | + | 25 | GAGCCAGGCCCGCTGGGCACCGCAT |
| chr20 | 36209920 | 36209944 | + | 25 | AGCCAGGCCCGCTGGGCACCGCATG |
| chr20 | 36209921 | 36209945 | + | 25 | GCCAGGCCCGCTGGGCACCGCATGC |
| chr20 | 36209922 | 36209946 | + | 25 | CCAGGCCCGCTGGGCACCGCATGCT |
| chr20 | 36209923 | 36209947 | + | 25 | CAGGCCCGCTGGGCACCGCATGCTC |
| chr20 | 36209924 | 36209948 | + | 25 | AGGCCCGCTGGGCACCGCATGCTCA |

**Table 23: 25-mer target-specific ASOs**

| **CHR** | **START** | **END** | **STRAND** | **kmer** | **SEQUENCE** |
|---|---|---|---|---|---|
| chr5 | 75399259 | 75399283 | + | 25 | TGAAATATAGCACAGAAAGACTCAA |
| chr5 | 75399260 | 75399284 | + | 25 | GAAATATAGCACAGAAAGACTCAAG |
| chr5 | 75399261 | 75399285 | + | 25 | AAATATAGCACAGAAAGACTCAAGT |
| chr5 | 75399262 | 75399286 | + | 25 | AATATAGCACAGAAAGACTCAAGTC |
| chr5 | 75399263 | 75399287 | + | 25 | ATATAGCACAGAAAGACTCAAGTCC |
| chr5 | 75399264 | 75399288 | + | 25 | TATAGCACAGAAAGACTCAAGTCCA |
| chr5 | 75399265 | 75399289 | + | 25 | ATAGCACAGAAAGACTCAAGTCCAC |
| chr5 | 75399266 | 75399290 | + | 25 | TAGCACAGAAAGACTCAAGTCCACT |
| chr5 | 75399267 | 75399291 | + | 25 | AGCACAGAAAGACTCAAGTCCACTC |
| chr5 | 75399268 | 75399292 | + | 25 | GCACAGAAAGACTCAAGTCCACTCT |
| chr5 | 75399269 | 75399293 | + | 25 | CACAGAAAGACTCAAGTCCACTCTA |
| chr5 | 75399270 | 75399294 | + | 25 | ACAGAAAGACTCAAGTCCACTCTAT |
| chr5 | 75399271 | 75399295 | + | 25 | CAGAAAGACTCAAGTCCACTCTATA |
| chr5 | 75399272 | 75399296 | + | 25 | AGAAAGACTCAAGTCCACTCTATAC |
| chr5 | 75399273 | 75399297 | + | 25 | GAAAGACTCAAGTCCACTCTATACA |
| chr5 | 75399274 | 75399298 | + | 25 | AAAGACTCAAGTCCACTCTATACAT |
| chr5 | 75399275 | 75399299 | + | 25 | AAGACTCAAGTCCACTCTATACATT |
| chr5 | 75399276 | 75399300 | + | 25 | AGACTCAAGTCCACTCTATACATTC |
| chr5 | 75399277 | 75399301 | + | 25 | GACTCAAGTCCACTCTATACATTCA |
| chr5 | 75399278 | 75399302 | + | 25 | ACTCAAGTCCACTCTATACATTCAT |
| chr5 | 75399279 | 75399303 | + | 25 | CTCAAGTCCACTCTATACATTCATA |
| chr5 | 75399280 | 75399304 | + | 25 | TCAAGTCCACTCTATACATTCATAC |
| chr5 | 75399281 | 75399305 | + | 25 | CAAGTCCACTCTATACATTCATACA |
| chr5 | 75399282 | 75399306 | + | 25 | AAGTCCACTCTATACATTCATACAG |
| chr5 | 75399283 | 75399307 | + | 25 | AGTCCACTCTATACATTCATACAGT |
| chr5 | 75399284 | 75399308 | + | 25 | GTCCACTCTATACATTCATACAGTA |
| chr5 | 75399285 | 75399309 | + | 25 | TCCACTCTATACATTCATACAGTAC |
| chr5 | 75399286 | 75399310 | + | 25 | CCACTCTATACATTCATACAGTACC |
| chr5 | 75399287 | 75399311 | + | 25 | CACTCTATACATTCATACAGTACCT |
| chr5 | 75399288 | 75399312 | + | 25 | ACTCTATACATTCATACAGTACCTG |
| chr5 | 75399289 | 75399313 | + | 25 | CTCTATACATTCATACAGTACCTGG |
| chr5 | 75399290 | 75399314 | + | 25 | TCTATACATTCATACAGTACCTGGG |
| chr5 | 75399291 | 75399315 | + | 25 | CTATACATTCATACAGTACCTGGGA |
| chr5 | 75399292 | 75399316 | + | 25 | TATACATTCATACAGTACCTGGGAG |
| chr5 | 75399293 | 75399317 | + | 25 | ATACATTCATACAGTACCTGGGAGC |
| chr5 | 75399294 | 75399318 | + | 25 | TACATTCATACAGTACCTGGGAGCT |
| chr5 | 75399295 | 75399319 | + | 25 | ACATTCATACAGTACCTGGGAGCTG |
| chr5 | 75399296 | 75399320 | + | 25 | CATTCATACAGTACCTGGGAGCTGA |
| chr5 | 75399297 | 75399321 | + | 25 | ATTCATACAGTACCTGGGAGCTGAA |
| chr5 | 75399298 | 75399322 | + | 25 | TTCATACAGTACCTGGGAGCTGAAT |
| chr5 | 75399299 | 75399323 | + | 25 | TCATACAGTACCTGGGAGCTGAATC |
| chr5 | 75399300 | 75399324 | + | 25 | CATACAGTACCTGGGAGCTGAATCT |
| chr5 | 75399301 | 75399325 | + | 25 | ATACAGTACCTGGGAGCTGAATCTG |
| chr5 | 75399302 | 75399326 | + | 25 | TACAGTACCTGGGAGCTGAATCTGT |
| chr5 | 75399303 | 75399327 | + | 25 | ACAGTACCTGGGAGCTGAATCTGTG |
| chr5 | 75399304 | 75399328 | + | 25 | CAGTACCTGGGAGCTGAATCTGTGA |
| chr5 | 75399305 | 75399329 | + | 25 | AGTACCTGGGAGCTGAATCTGTGAA |
| chr5 | 75399306 | 75399330 | + | 25 | GTACCTGGGAGCTGAATCTGTGAAC |
| chr5 | 75399307 | 75399331 | + | 25 | TACCTGGGAGCTGAATCTGTGAACA |
| chr5 | 75399308 | 75399332 | + | 25 | ACCTGGGAGCTGAATCTGTGAACAT |
| chr5 | 75399309 | 75399333 | + | 25 | CCTGGGAGCTGAATCTGTGAACATC |
| chr5 | 75399310 | 75399334 | + | 25 | CTGGGAGCTGAATCTGTGAACATCA |
| chr5 | 75399311 | 75399335 | + | 25 | TGGGAGCTGAATCTGTGAACATCAT |
| chr5 | 75399312 | 75399336 | + | 25 | GGGAGCTGAATCTGTGAACATCATC |
| chr5 | 75399313 | 75399337 | + | 25 | GGAGCTGAATCTGTGAACATCATCA |
| chr5 | 75399314 | 75399338 | + | 25 | GAGCTGAATCTGTGAACATCATCAG |
| chr5 | 75399315 | 75399339 | + | 25 | AGCTGAATCTGTGAACATCATCAGA |
| chr5 | 75399316 | 75399340 | + | 25 | GCTGAATCTGTGAACATCATCAGAG |
| chr5 | 75399317 | 75399341 | + | 25 | CTGAATCTGTGAACATCATCAGAGG |
| chr5 | 75399318 | 75399342 | + | 25 | TGAATCTGTGAACATCATCAGAGGC |
| chr5 | 75399319 | 75399343 | + | 25 | GAATCTGTGAACATCATCAGAGGCA |
| chr5 | 75399320 | 75399344 | + | 25 | AATCTGTGAACATCATCAGAGGCAC |
| chr5 | 75399321 | 75399345 | + | 25 | ATCTGTGAACATCATCAGAGGCACT |
| chr5 | 75399322 | 75399346 | + | 25 | TCTGTGAACATCATCAGAGGCACTG |
| chr5 | 75399323 | 75399347 | + | 25 | CTGTGAACATCATCAGAGGCACTGA |
| chr5 | 75399324 | 75399348 | + | 25 | TGTGAACATCATCAGAGGCACTGAC |
| chr5 | 75399325 | 75399349 | + | 25 | GTGAACATCATCAGAGGCACTGACT |
| chr5 | 75399326 | 75399350 | + | 25 | TGAACATCATCAGAGGCACTGACTA |
| chr5 | 75399327 | 75399351 | + | 25 | GAACATCATCAGAGGCACTGACTAG |
| chr5 | 75399328 | 75399352 | + | 25 | AACATCATCAGAGGCACTGACTAGA |
| chr5 | 75399329 | 75399353 | + | 25 | ACATCATCAGAGGCACTGACTAGAT |
| chr5 | 75399330 | 75399354 | + | 25 | CATCATCAGAGGCACTGACTAGATC |
| chr5 | 75399331 | 75399355 | + | 25 | ATCATCAGAGGCACTGACTAGATCA |
| chr5 | 75399332 | 75399356 | + | 25 | TCATCAGAGGCACTGACTAGATCAA |
| chr5 | 75399333 | 75399357 | + | 25 | CATCAGAGGCACTGACTAGATCAAT |
| chr5 | 75399334 | 75399358 | + | 25 | ATCAGAGGCACTGACTAGATCAATG |
| chr5 | 75399335 | 75399359 | + | 25 | TCAGAGGCACTGACTAGATCAATGG |
| chr5 | 75399336 | 75399360 | + | 25 | CAGAGGCACTGACTAGATCAATGGA |
| chr5 | 75399337 | 75399361 | + | 25 | AGAGGCACTGACTAGATCAATGGAA |
| chr5 | 75399338 | 75399362 | + | 25 | GAGGCACTGACTAGATCAATGGAAG |
| chr5 | 75399339 | 75399363 | + | 25 | AGGCACTGACTAGATCAATGGAAGA |
| chr5 | 75399340 | 75399364 | + | 25 | GGCACTGACTAGATCAATGGAAGAC |
| chr5 | 75399341 | 75399365 | + | 25 | GCACTGACTAGATCAATGGAAGACA |
| chr5 | 75399342 | 75399366 | + | 25 | CACTGACTAGATCAATGGAAGACAT |
| chr5 | 75399343 | 75399367 | + | 25 | ACTGACTAGATCAATGGAAGACATG |
| chr5 | 75399344 | 75399368 | + | 25 | CTGACTAGATCAATGGAAGACATGG |
| chr5 | 75399345 | 75399369 | + | 25 | TGACTAGATCAATGGAAGACATGGA |
| chr5 | 75399346 | 75399370 | + | 25 | GACTAGATCAATGGAAGACATGGAG |
| chr5 | 75399347 | 75399371 | + | 25 | ACTAGATCAATGGAAGACATGGAGG |
| chr5 | 75399348 | 75399372 | + | 25 | CTAGATCAATGGAAGACATGGAGGA |
| chr5 | 75399349 | 75399373 | + | 25 | TAGATCAATGGAAGACATGGAGGAA |
| chr5 | 75399350 | 75399374 | + | 25 | AGATCAATGGAAGACATGGAGGAAG |
| chr5 | 75399351 | 75399375 | + | 25 | GATCAATGGAAGACATGGAGGAAGA |
| chr5 | 75399352 | 75399376 | + | 25 | ATCAATGGAAGACATGGAGGAAGAG |
| chr5 | 75399353 | 75399377 | + | 25 | TCAATGGAAGACATGGAGGAAGAGC |
| chr5 | 75399354 | 75399378 | + | 25 | CAATGGAAGACATGGAGGAAGAGCG |
| chr5 | 75399355 | 75399379 | + | 25 | AATGGAAGACATGGAGGAAGAGCGA |
| chr5 | 75399356 | 75399380 | + | 25 | ATGGAAGACATGGAGGAAGAGCGAC |
| chr5 | 75399357 | 75399381 | + | 25 | TGGAAGACATGGAGGAAGAGCGACT |
| chr5 | 75399358 | 75399382 | + | 25 | GGAAGACATGGAGGAAGAGCGACTA |
| chr5 | 75399359 | 75399383 | + | 25 | GAAGACATGGAGGAAGAGCGACTAT |
| chr5 | 75399360 | 75399384 | + | 25 | AAGACATGGAGGAAGAGCGACTATA |
| chr5 | 75399361 | 75399385 | + | 25 | AGACATGGAGGAAGAGCGACTATAG |
| chr5 | 75399362 | 75399386 | + | 25 | GACATGGAGGAAGAGCGACTATAGG |
| chr5 | 75399363 | 75399387 | + | 25 | ACATGGAGGAAGAGCGACTATAGGG |
| chr5 | 75399364 | 75399388 | + | 25 | CATGGAGGAAGAGCGACTATAGGGC |
| chr5 | 75399365 | 75399389 | + | 25 | ATGGAGGAAGAGCGACTATAGGGCT |
| chr5 | 75399366 | 75399390 | + | 25 | TGGAGGAAGAGCGACTATAGGGCTA |
| chr5 | 75399367 | 75399391 | + | 25 | GGAGGAAGAGCGACTATAGGGCTAC |
| chr5 | 75399368 | 75399392 | + | 25 | GAGGAAGAGCGACTATAGGGCTACC |
| chr5 | 75399369 | 75399393 | + | 25 | AGGAAGAGCGACTATAGGGCTACCA |
| chr5 | 75399370 | 75399394 | + | 25 | GGAAGAGCGACTATAGGGCTACCAG |
| chr5 | 75399371 | 75399395 | + | 25 | GAAGAGCGACTATAGGGCTACCAGA |
| chr5 | 75399372 | 75399396 | + | 25 | AAGAGCGACTATAGGGCTACCAGAG |
| chr5 | 75399373 | 75399397 | + | 25 | AGAGCGACTATAGGGCTACCAGAGA |
| chr5 | 75399374 | 75399398 | + | 25 | GAGCGACTATAGGGCTACCAGAGAC |
| chr5 | 75399375 | 75399399 | + | 25 | AGCGACTATAGGGCTACCAGAGACA |
| chr5 | 75399376 | 75399400 | + | 25 | GCGACTATAGGGCTACCAGAGACAG |
| chr5 | 75399377 | 75399401 | + | 25 | CGACTATAGGGCTACCAGAGACAGA |
| chr5 | 75399378 | 75399402 | + | 25 | GACTATAGGGCTACCAGAGACAGAC |
| chr5 | 75399379 | 75399403 | + | 25 | ACTATAGGGCTACCAGAGACAGACA |
| chr5 | 75399380 | 75399404 | + | 25 | CTATAGGGCTACCAGAGACAGACAA |
| chr5 | 75399381 | 75399405 | + | 25 | TATAGGGCTACCAGAGACAGACAAA |
| chr5 | 75399382 | 75399406 | + | 25 | ATAGGGCTACCAGAGACAGACAAAG |
| chr5 | 75399383 | 75399407 | + | 25 | TAGGGCTACCAGAGACAGACAAAGA |
| chr5 | 75399384 | 75399408 | + | 25 | AGGGCTACCAGAGACAGACAAAGAA |
| chr5 | 75399385 | 75399409 | + | 25 | GGGCTACCAGAGACAGACAAAGAAA |
| chr5 | 75399386 | 75399410 | + | 25 | GGCTACCAGAGACAGACAAAGAAAA |
| chr5 | 75399387 | 75399411 | + | 25 | GCTACCAGAGACAGACAAAGAAAAA |
| chr5 | 75399388 | 75399412 | + | 25 | CTACCAGAGACAGACAAAGAAAAAA |
| chr5 | 75399389 | 75399413 | + | 25 | TACCAGAGACAGACAAAGAAAAAAC |
| chr5 | 75399390 | 75399414 | + | 25 | ACCAGAGACAGACAAAGAAAAAACC |
| chr5 | 75399391 | 75399415 | + | 25 | CCAGAGACAGACAAAGAAAAAACCA |
| chr5 | 75399392 | 75399416 | + | 25 | CAGAGACAGACAAAGAAAAAACCAA |
| chr5 | 75399393 | 75399417 | + | 25 | AGAGACAGACAAAGAAAAAACCAAG |
| chr5 | 75399394 | 75399418 | + | 25 | GAGACAGACAAAGAAAAAACCAAGT |
| chr5 | 75399395 | 75399419 | + | 25 | AGACAGACAAAGAAAAAACCAAGTA |
| chr5 | 75399396 | 75399420 | + | 25 | GACAGACAAAGAAAAAACCAAGTAA |
| chr5 | 75399397 | 75399421 | + | 25 | ACAGACAAAGAAAAAACCAAGTAAT |
| chr5 | 75399398 | 75399422 | + | 25 | CAGACAAAGAAAAAACCAAGTAATC |
| chr5 | 75399399 | 75399423 | + | 25 | AGACAAAGAAAAAACCAAGTAATCA |
| chr5 | 75399400 | 75399424 | + | 25 | GACAAAGAAAAAACCAAGTAATCAG |
| chr5 | 75399401 | 75399425 | + | 25 | ACAAAGAAAAAACCAAGTAATCAGA |
| chr5 | 75399402 | 75399426 | + | 25 | CAAAGAAAAAACCAAGTAATCAGAA |
| chr5 | 75399403 | 75399427 | + | 25 | AAAGAAAAAACCAAGTAATCAGAAC |
| chr5 | 75399404 | 75399428 | + | 25 | AAGAAAAAACCAAGTAATCAGAACA |
| chr5 | 75399405 | 75399429 | + | 25 | AGAAAAAACCAAGTAATCAGAACAA |
| chr5 | 75399406 | 75399430 | + | 25 | GAAAAAACCAAGTAATCAGAACAAA |
| chr5 | 75399407 | 75399431 | + | 25 | AAAAAACCAAGTAATCAGAACAAAG |
| chr5 | 75399408 | 75399432 | + | 25 | AAAAACCAAGTAATCAGAACAAAGG |
| chr5 | 75399409 | 75399433 | + | 25 | AAAACCAAGTAATCAGAACAAAGGC |
| chr5 | 75399410 | 75399434 | + | 25 | AAACCAAGTAATCAGAACAAAGGCA |
| chr5 | 75399411 | 75399435 | + | 25 | AACCAAGTAATCAGAACAAAGGCAC |
| chr5 | 75399412 | 75399436 | + | 25 | ACCAAGTAATCAGAACAAAGGCACA |
| chr5 | 75399413 | 75399437 | + | 25 | CCAAGTAATCAGAACAAAGGCACAA |

**Table 24: 25-mer target-specific ASOs**

| **CHR** | **START** | **END** | **STRAND** | **kmer** | **SEQUENCE** |
|---|---|---|---|---|---|
| chr10 | 26755604 | 26755628 | + | 25 | CTATAAGGAGACAGCCTCTACTCTT |
| chr10 | 26755605 | 26755629 | + | 25 | TATAAGGAGACAGCCTCTACTCTTC |
| chr10 | 26755606 | 26755630 | + | 25 | ATAAGGAGACAGCCTCTACTCTTCC |
| chr10 | 26755607 | 26755631 | + | 25 | TAAGGAGACAGCCTCTACTCTTCCA |
| chr10 | 26755608 | 26755632 | + | 25 | AAGGAGACAGCCTCTACTCTTCCAT |
| chr10 | 26755609 | 26755633 | + | 25 | AGGAGACAGCCTCTACTCTTCCATA |
| chr10 | 26755610 | 26755634 | + | 25 | GGAGACAGCCTCTACTCTTCCATAG |
| chr10 | 26755611 | 26755635 | + | 25 | GAGACAGCCTCTACTCTTCCATAGC |
| chr10 | 26755612 | 26755636 | + | 25 | AGACAGCCTCTACTCTTCCATAGCT |
| chr10 | 26755613 | 26755637 | + | 25 | GACAGCCTCTACTCTTCCATAGCTC |
| chr10 | 26755614 | 26755638 | + | 25 | ACAGCCTCTACTCTTCCATAGCTCA |
| chr10 | 26755615 | 26755639 | + | 25 | CAGCCTCTACTCTTCCATAGCTCAG |
| chr10 | 26755616 | 26755640 | + | 25 | AGCCTCTACTCTTCCATAGCTCAGT |
| chr10 | 26755617 | 26755641 | + | 25 | GCCTCTACTCTTCCATAGCTCAGTT |
| chr10 | 26755618 | 26755642 | + | 25 | CCTCTACTCTTCCATAGCTCAGTTT |
| chr10 | 26755619 | 26755643 | + | 25 | CTCTACTCTTCCATAGCTCAGTTTT |
| chr10 | 26755620 | 26755644 | + | 25 | TCTACTCTTCCATAGCTCAGTTTTT |
| chr10 | 26755621 | 26755645 | + | 25 | CTACTCTTCCATAGCTCAGTTTTTC |
| chr10 | 26755622 | 26755646 | + | 25 | TACTCTTCCATAGCTCAGTTTTTCC |
| chr10 | 26755623 | 26755647 | + | 25 | ACTCTTCCATAGCTCAGTTTTTCCA |
| chr10 | 26755624 | 26755648 | + | 25 | CTCTTCCATAGCTCAGTTTTTCCAA |
| chr10 | 26755625 | 26755649 | + | 25 | TCTTCCATAGCTCAGTTTTTCCAAA |
| chr10 | 26755626 | 26755650 | + | 25 | CTTCCATAGCTCAGTTTTTCCAAAC |
| chr10 | 26755627 | 26755651 | + | 25 | TTCCATAGCTCAGTTTTTCCAAACT |
| chr10 | 26755628 | 26755652 | + | 25 | TCCATAGCTCAGTTTTTCCAAACTT |
| chr10 | 26755629 | 26755653 | + | 25 | CCATAGCTCAGTTTTTCCAAACTTA |
| chr10 | 26755630 | 26755654 | + | 25 | CATAGCTCAGTTTTTCCAAACTTAC |
| chr10 | 26755631 | 26755655 | + | 25 | ATAGCTCAGTTTTTCCAAACTTACT |
| chr10 | 26755632 | 26755656 | + | 25 | TAGCTCAGTTTTTCCAAACTTACTG |
| chr10 | 26755633 | 26755657 | + | 25 | AGCTCAGTTTTTCCAAACTTACTGT |
| chr10 | 26755634 | 26755658 | + | 25 | GCTCAGTTTTTCCAAACTTACTGTT |
| chr10 | 26755635 | 26755659 | + | 25 | CTCAGTTTTTCCAAACTTACTGTTT |
| chr10 | 26755636 | 26755660 | + | 25 | TCAGTTTTTCCAAACTTACTGTTTT |
| chr10 | 26755637 | 26755661 | + | 25 | CAGTTTTTCCAAACTTACTGTTTTC |
| chr10 | 26755638 | 26755662 | + | 25 | AGTTTTTCCAAACTTACTGTTTTCC |
| chr10 | 26755639 | 26755663 | + | 25 | GTTTTTCCAAACTTACTGTTTTCCT |
| chr10 | 26755640 | 26755664 | + | 25 | TTTTTCCAAACTTACTGTTTTCCTG |
| chr10 | 26755641 | 26755665 | + | 25 | TTTTCCAAACTTACTGTTTTCCTGC |
| chr10 | 26755642 | 26755666 | + | 25 | TTTCCAAACTTACTGTTTTCCTGCA |
| chr10 | 26755643 | 26755667 | + | 25 | TTCCAAACTTACTGTTTTCCTGCAC |
| chr10 | 26755644 | 26755668 | + | 25 | TCCAAACTTACTGTTTTCCTGCACC |
| chr10 | 26755645 | 26755669 | + | 25 | CCAAACTTACTGTTTTCCTGCACCC |
| chr10 | 26755646 | 26755670 | + | 25 | CAAACTTACTGTTTTCCTGCACCCT |
| chr10 | 26755647 | 26755671 | + | 25 | AAACTTACTGTTTTCCTGCACCCTG |
| chr10 | 26755648 | 26755672 | + | 25 | AACTTACTGTTTTCCTGCACCCTGG |
| chr10 | 26755649 | 26755673 | + | 25 | ACTTACTGTTTTCCTGCACCCTGGC |
| chr10 | 26755650 | 26755674 | + | 25 | CTTACTGTTTTCCTGCACCCTGGCC |
| chr10 | 26755651 | 26755675 | + | 25 | TTACTGTTTTCCTGCACCCTGGCCA |
| chr10 | 26755652 | 26755676 | + | 25 | TACTGTTTTCCTGCACCCTGGCCAC |
| chr10 | 26755653 | 26755677 | + | 25 | ACTGTTTTCCTGCACCCTGGCCACG |
| chr10 | 26755654 | 26755678 | + | 25 | CTGTTTTCCTGCACCCTGGCCACGA |
| chr10 | 26755655 | 26755679 | + | 25 | TGTTTTCCTGCACCCTGGCCACGAA |
| chr10 | 26755656 | 26755680 | + | 25 | GTTTTCCTGCACCCTGGCCACGAAG |
| chr10 | 26755657 | 26755681 | + | 25 | TTTTCCTGCACCCTGGCCACGAAGC |
| chr10 | 26755658 | 26755682 | + | 25 | TTTCCTGCACCCTGGCCACGAAGCC |
| chr10 | 26755659 | 26755683 | + | 25 | TTCCTGCACCCTGGCCACGAAGCCT |
| chr10 | 26755660 | 26755684 | + | 25 | TCCTGCACCCTGGCCACGAAGCCTG |
| chr10 | 26755661 | 26755685 | + | 25 | CCTGCACCCTGGCCACGAAGCCTGT |
| chr10 | 26755662 | 26755686 | + | 25 | CTGCACCCTGGCCACGAAGCCTGTG |
| chr10 | 26755663 | 26755687 | + | 25 | TGCACCCTGGCCACGAAGCCTGTGA |
| chr10 | 26755664 | 26755688 | + | 25 | GCACCCTGGCCACGAAGCCTGTGAG |
| chr10 | 26755665 | 26755689 | + | 25 | CACCCTGGCCACGAAGCCTGTGAGA |
| chr10 | 26755666 | 26755690 | + | 25 | ACCCTGGCCACGAAGCCTGTGAGAG |
| chr10 | 26755667 | 26755691 | + | 25 | CCCTGGCCACGAAGCCTGTGAGAGG |
| chr10 | 26755668 | 26755692 | + | 25 | CCTGGCCACGAAGCCTGTGAGAGGT |
| chr10 | 26755669 | 26755693 | + | 25 | CTGGCCACGAAGCCTGTGAGAGGTA |
| chr10 | 26755670 | 26755694 | + | 25 | TGGCCACGAAGCCTGTGAGAGGTAT |
| chr10 | 26755671 | 26755695 | + | 25 | GGCCACGAAGCCTGTGAGAGGTATC |
| chr10 | 26755672 | 26755696 | + | 25 | GCCACGAAGCCTGTGAGAGGTATCT |
| chr10 | 26755673 | 26755697 | + | 25 | CCACGAAGCCTGTGAGAGGTATCTG |
| chr10 | 26755674 | 26755698 | + | 25 | CACGAAGCCTGTGAGAGGTATCTGT |
| chr10 | 26755675 | 26755699 | + | 25 | ACGAAGCCTGTGAGAGGTATCTGTG |
| chr10 | 26755676 | 26755700 | + | 25 | CGAAGCCTGTGAGAGGTATCTGTGG |
| chr10 | 26755677 | 26755701 | + | 25 | GAAGCCTGTGAGAGGTATCTGTGGA |
| chr10 | 26755678 | 26755702 | + | 25 | AAGCCTGTGAGAGGTATCTGTGGAG |
| chr10 | 26755679 | 26755703 | + | 25 | AGCCTGTGAGAGGTATCTGTGGAGT |
| chr10 | 26755680 | 26755704 | + | 25 | GCCTGTGAGAGGTATCTGTGGAGTC |
| chr10 | 26755681 | 26755705 | + | 25 | CCTGTGAGAGGTATCTGTGGAGTCA |
| chr10 | 26755682 | 26755706 | + | 25 | CTGTGAGAGGTATCTGTGGAGTCAA |
| chr10 | 26755683 | 26755707 | + | 25 | TGTGAGAGGTATCTGTGGAGTCAAC |
| chr10 | 26755684 | 26755708 | + | 25 | GTGAGAGGTATCTGTGGAGTCAACT |
| chr10 | 26755685 | 26755709 | + | 25 | TGAGAGGTATCTGTGGAGTCAACTG |
| chr10 | 26755686 | 26755710 | + | 25 | GAGAGGTATCTGTGGAGTCAACTGA |
| chr10 | 26755687 | 26755711 | + | 25 | AGAGGTATCTGTGGAGTCAACTGAG |
| chr10 | 26755688 | 26755712 | + | 25 | GAGGTATCTGTGGAGTCAACTGAGG |
| chr10 | 26755689 | 26755713 | + | 25 | AGGTATCTGTGGAGTCAACTGAGGC |
| chr10 | 26755690 | 26755714 | + | 25 | GGTATCTGTGGAGTCAACTGAGGCA |
| chr10 | 26755691 | 26755715 | + | 25 | GTATCTGTGGAGTCAACTGAGGCAT |
| chr10 | 26755692 | 26755716 | + | 25 | TATCTGTGGAGTCAACTGAGGCATA |
| chr10 | 26755693 | 26755717 | + | 25 | ATCTGTGGAGTCAACTGAGGCATAG |
| chr10 | 26755694 | 26755718 | + | 25 | TCTGTGGAGTCAACTGAGGCATAGG |
| chr10 | 26755695 | 26755719 | + | 25 | CTGTGGAGTCAACTGAGGCATAGGG |
| chr10 | 26755696 | 26755720 | + | 25 | TGTGGAGTCAACTGAGGCATAGGGG |
| chr10 | 26755697 | 26755721 | + | 25 | GTGGAGTCAACTGAGGCATAGGGGG |
| chr10 | 26755698 | 26755722 | + | 25 | TGGAGTCAACTGAGGCATAGGGGGA |
| chr10 | 26755699 | 26755723 | + | 25 | GGAGTCAACTGAGGCATAGGGGGAG |
| chr10 | 26755700 | 26755724 | + | 25 | GAGTCAACTGAGGCATAGGGGGAGG |
| chr10 | 26755701 | 26755725 | + | 25 | AGTCAACTGAGGCATAGGGGGAGGG |
| chr10 | 26755702 | 26755726 | + | 25 | GTCAACTGAGGCATAGGGGGAGGGG |
| chr10 | 26755703 | 26755727 | + | 25 | TCAACTGAGGCATAGGGGGAGGGGG |
| chr10 | 26755704 | 26755728 | + | 25 | CAACTGAGGCATAGGGGGAGGGGGT |
| chr10 | 26755705 | 26755729 | + | 25 | AACTGAGGCATAGGGGGAGGGGGTG |
| chr10 | 26755706 | 26755730 | + | 25 | ACTGAGGCATAGGGGGAGGGGGTGG |
| chr10 | 26755707 | 26755731 | + | 25 | CTGAGGCATAGGGGGAGGGGGTGGA |
| chr10 | 26755708 | 26755732 | + | 25 | TGAGGCATAGGGGGAGGGGGTGGAG |
| chr10 | 26755709 | 26755733 | + | 25 | GAGGCATAGGGGGAGGGGGTGGAGC |
| chr10 | 26755710 | 26755734 | + | 25 | AGGCATAGGGGGAGGGGGTGGAGCA |
| chr10 | 26755711 | 26755735 | + | 25 | GGCATAGGGGGAGGGGGTGGAGCAA |
| chr10 | 26755712 | 26755736 | + | 25 | GCATAGGGGGAGGGGGTGGAGCAAT |
| chr10 | 26755713 | 26755737 | + | 25 | CATAGGGGGAGGGGGTGGAGCAATA |
| chr10 | 26755714 | 26755738 | + | 25 | ATAGGGGGAGGGGGTGGAGCAATAG |
| chr10 | 26755715 | 26755739 | + | 25 | TAGGGGGAGGGGGTGGAGCAATAGA |
| chr10 | 26755716 | 26755740 | + | 25 | AGGGGGAGGGGGTGGAGCAATAGAA |
| chr10 | 26755717 | 26755741 | + | 25 | GGGGGAGGGGGTGGAGCAATAGAAA |
| chr10 | 26755718 | 26755742 | + | 25 | GGGGAGGGGGTGGAGCAATAGAAAC |
| chr10 | 26755719 | 26755743 | + | 25 | GGGAGGGGGTGGAGCAATAGAAACT |
| chr10 | 26755720 | 26755744 | + | 25 | GGAGGGGGTGGAGCAATAGAAACTG |
| chr10 | 26755721 | 26755745 | + | 25 | GAGGGGGTGGAGCAATAGAAACTGG |
| chr10 | 26755722 | 26755746 | + | 25 | AGGGGGTGGAGCAATAGAAACTGGT |
| chr10 | 26755723 | 26755747 | + | 25 | GGGGGTGGAGCAATAGAAACTGGTA |
| chr10 | 26755724 | 26755748 | + | 25 | GGGGTGGAGCAATAGAAACTGGTAG |
| chr10 | 26755725 | 26755749 | + | 25 | GGGTGGAGCAATAGAAACTGGTAGC |
| chr10 | 26755726 | 26755750 | + | 25 | GGTGGAGCAATAGAAACTGGTAGCA |
| chr10 | 26755727 | 26755751 | + | 25 | GTGGAGCAATAGAAACTGGTAGCAA |
| chr10 | 26755728 | 26755752 | + | 25 | TGGAGCAATAGAAACTGGTAGCAAC |
| chr10 | 26755729 | 26755753 | + | 25 | GGAGCAATAGAAACTGGTAGCAACA |
| chr10 | 26755730 | 26755754 | + | 25 | GAGCAATAGAAACTGGTAGCAACAA |
| chr10 | 26755731 | 26755755 | + | 25 | AGCAATAGAAACTGGTAGCAACAAC |
| chr10 | 26755732 | 26755756 | + | 25 | GCAATAGAAACTGGTAGCAACAACA |
| chr10 | 26755733 | 26755757 | + | 25 | CAATAGAAACTGGTAGCAACAACAC |
| chr10 | 26755734 | 26755758 | + | 25 | AATAGAAACTGGTAGCAACAACACA |
| chr10 | 26755735 | 26755759 | + | 25 | ATAGAAACTGGTAGCAACAACACAG |
| chr10 | 26755736 | 26755760 | + | 25 | TAGAAACTGGTAGCAACAACACAGT |
| chr10 | 26755737 | 26755761 | + | 25 | AGAAACTGGTAGCAACAACACAGTA |
| chr10 | 26755738 | 26755762 | + | 25 | GAAACTGGTAGCAACAACACAGTAT |
| chr10 | 26755739 | 26755763 | + | 25 | AAACTGGTAGCAACAACACAGTATG |
| chr10 | 26755740 | 26755764 | + | 25 | AACTGGTAGCAACAACACAGTATGG |
| chr10 | 26755741 | 26755765 | + | 25 | ACTGGTAGCAACAACACAGTATGGG |
| chr10 | 26755742 | 26755766 | + | 25 | CTGGTAGCAACAACACAGTATGGGG |
| chr10 | 26755743 | 26755767 | + | 25 | TGGTAGCAACAACACAGTATGGGGG |
| chr10 | 26755744 | 26755768 | + | 25 | GGTAGCAACAACACAGTATGGGGGA |
| chr10 | 26755745 | 26755769 | + | 25 | GTAGCAACAACACAGTATGGGGGAA |
| chr10 | 26755746 | 26755770 | + | 25 | TAGCAACAACACAGTATGGGGGAAG |
| chr10 | 26755747 | 26755771 | + | 25 | AGCAACAACACAGTATGGGGGAAGT |
| chr10 | 26755748 | 26755772 | + | 25 | GCAACAACACAGTATGGGGGAAGTA |
| chr10 | 26755749 | 26755773 | + | 25 | CAACAACACAGTATGGGGGAAGTAA |
| chr10 | 26755750 | 26755774 | + | 25 | AACAACACAGTATGGGGGAAGTAAA |
| chr10 | 26755751 | 26755775 | + | 25 | ACAACACAGTATGGGGGAAGTAAAA |
| chr10 | 26755752 | 26755776 | + | 25 | CAACACAGTATGGGGGAAGTAAAAC |
| chr10 | 26755753 | 26755777 | + | 25 | AACACAGTATGGGGGAAGTAAAACA |
| chr10 | 26755754 | 26755778 | + | 25 | ACACAGTATGGGGGAAGTAAAACAG |
| chr10 | 26755755 | 26755779 | + | 25 | CACAGTATGGGGGAAGTAAAACAGA |
| chr10 | 26755756 | 26755780 | + | 25 | ACAGTATGGGGGAAGTAAAACAGAT |
| chr10 | 26755757 | 26755781 | + | 25 | CAGTATGGGGGAAGTAAAACAGATA |
| chr10 | 26755758 | 26755782 | + | 25 | AGTATGGGGGAAGTAAAACAGATAA |
| chr10 | 26755759 | 26755783 | + | 25 | GTATGGGGGAAGTAAAACAGATAAA |
| chr10 | 26755760 | 26755784 | + | 25 | TATGGGGGAAGTAAAACAGATAAAG |
| chr10 | 26755761 | 26755785 | + | 25 | ATGGGGGAAGTAAAACAGATAAAGG |
| chr10 | 26755762 | 26755786 | + | 25 | TGGGGGAAGTAAAACAGATAAAGGA |
| chr10 | 26755763 | 26755787 | + | 25 | GGGGGAAGTAAAACAGATAAAGGAA |
| chr10 | 26755764 | 26755788 | + | 25 | GGGGAAGTAAAACAGATAAAGGAAA |
| chr10 | 26755765 | 26755789 | + | 25 | GGGAAGTAAAACAGATAAAGGAAAG |
| chr10 | 26755766 | 26755790 | + | 25 | GGAAGTAAAACAGATAAAGGAAAGA |
| chr10 | 26755767 | 26755791 | + | 25 | GAAGTAAAACAGATAAAGGAAAGAA |

**Table 25: 25-mer target-specific ASOs**

| **CHR** | **START** | **END** | **STRAND** | **kmer** | **SEQUENCE** |
|---|---|---|---|---|---|
| chr6 | 41080760 | 41080784 | + | 25 | TACACATTTCCTTCCTGACATATTT |
| chr6 | 41080761 | 41080785 | + | 25 | ACACATTTCCTTCCTGACATATTTC |
| chr6 | 41080762 | 41080786 | + | 25 | CACATTTCCTTCCTGACATATTTCA |
| chr6 | 41080763 | 41080787 | + | 25 | ACATTTCCTTCCTGACATATTTCAA |
| chr6 | 41080764 | 41080788 | + | 25 | CATTTCCTTCCTGACATATTTCAAG |
| chr6 | 41080765 | 41080789 | + | 25 | ATTTCCTTCCTGACATATTTCAAGC |
| chr6 | 41080766 | 41080790 | + | 25 | TTTCCTTCCTGACATATTTCAAGCT |
| chr6 | 41080767 | 41080791 | + | 25 | TTCCTTCCTGACATATTTCAAGCTC |
| chr6 | 41080768 | 41080792 | + | 25 | TCCTTCCTGACATATTTCAAGCTCT |
| chr6 | 41080769 | 41080793 | + | 25 | CCTTCCTGACATATTTCAAGCTCTT |
| chr6 | 41080770 | 41080794 | + | 25 | CTTCCTGACATATTTCAAGCTCTTC |
| chr6 | 41080771 | 41080795 | + | 25 | TTCCTGACATATTTCAAGCTCTTCC |
| chr6 | 41080772 | 41080796 | + | 25 | TCCTGACATATTTCAAGCTCTTCCT |
| chr6 | 41080773 | 41080797 | + | 25 | CCTGACATATTTCAAGCTCTTCCTG |
| chr6 | 41080774 | 41080798 | + | 25 | CTGACATATTTCAAGCTCTTCCTGT |
| chr6 | 41080775 | 41080799 | + | 25 | TGACATATTTCAAGCTCTTCCTGTT |
| chr6 | 41080776 | 41080800 | + | 25 | GACATATTTCAAGCTCTTCCTGTTC |
| chr6 | 41080777 | 41080801 | + | 25 | ACATATTTCAAGCTCTTCCTGTTCC |
| chr6 | 41080778 | 41080802 | + | 25 | CATATTTCAAGCTCTTCCTGTTCCT |
| chr6 | 41080779 | 41080803 | + | 25 | ATATTTCAAGCTCTTCCTGTTCCTG |
| chr6 | 41080780 | 41080804 | + | 25 | TATTTCAAGCTCTTCCTGTTCCTGT |
| chr6 | 41080781 | 41080805 | + | 25 | ATTTCAAGCTCTTCCTGTTCCTGTT |
| chr6 | 41080782 | 41080806 | + | 25 | TTTCAAGCTCTTCCTGTTCCTGTTC |
| chr6 | 41080783 | 41080807 | + | 25 | TTCAAGCTCTTCCTGTTCCTGTTCT |
| chr6 | 41080784 | 41080808 | + | 25 | TCAAGCTCTTCCTGTTCCTGTTCTC |
| chr6 | 41080785 | 41080809 | + | 25 | CAAGCTCTTCCTGTTCCTGTTCTCA |
| chr6 | 41080786 | 41080810 | + | 25 | AAGCTCTTCCTGTTCCTGTTCTCAG |
| chr6 | 41080787 | 41080811 | + | 25 | AGCTCTTCCTGTTCCTGTTCTCAGC |
| chr6 | 41080788 | 41080812 | + | 25 | GCTCTTCCTGTTCCTGTTCTCAGCA |
| chr6 | 41080789 | 41080813 | + | 25 | CTCTTCCTGTTCCTGTTCTCAGCAG |
| chr6 | 41080790 | 41080814 | + | 25 | TCTTCCTGTTCCTGTTCTCAGCAGC |
| chr6 | 41080791 | 41080815 | + | 25 | CTTCCTGTTCCTGTTCTCAGCAGCA |
| chr6 | 41080792 | 41080816 | + | 25 | TTCCTGTTCCTGTTCTCAGCAGCAG |
| chr6 | 41080793 | 41080817 | + | 25 | TCCTGTTCCTGTTCTCAGCAGCAGG |
| chr6 | 41080794 | 41080818 | + | 25 | CCTGTTCCTGTTCTCAGCAGCAGGG |
| chr6 | 41080795 | 41080819 | + | 25 | CTGTTCCTGTTCTCAGCAGCAGGGT |
| chr6 | 41080796 | 41080820 | + | 25 | TGTTCCTGTTCTCAGCAGCAGGGTG |
| chr6 | 41080797 | 41080821 | + | 25 | GTTCCTGTTCTCAGCAGCAGGGTGG |
| chr6 | 41080798 | 41080822 | + | 25 | TTCCTGTTCTCAGCAGCAGGGTGGT |
| chr6 | 41080799 | 41080823 | + | 25 | TCCTGTTCTCAGCAGCAGGGTGGTG |
| chr6 | 41080800 | 41080824 | + | 25 | CCTGTTCTCAGCAGCAGGGTGGTGT |
| chr6 | 41080801 | 41080825 | + | 25 | CTGTTCTCAGCAGCAGGGTGGTGTC |
| chr6 | 41080802 | 41080826 | + | 25 | TGTTCTCAGCAGCAGGGTGGTGTCA |
| chr6 | 41080803 | 41080827 | + | 25 | GTTCTCAGCAGCAGGGTGGTGTCAC |
| chr6 | 41080804 | 41080828 | + | 25 | TTCTCAGCAGCAGGGTGGTGTCACT |
| chr6 | 41080805 | 41080829 | + | 25 | TCTCAGCAGCAGGGTGGTGTCACTG |
| chr6 | 41080806 | 41080830 | + | 25 | CTCAGCAGCAGGGTGGTGTCACTGC |
| chr6 | 41080807 | 41080831 | + | 25 | TCAGCAGCAGGGTGGTGTCACTGCT |
| chr6 | 41080808 | 41080832 | + | 25 | CAGCAGCAGGGTGGTGTCACTGCTG |
| chr6 | 41080809 | 41080833 | + | 25 | AGCAGCAGGGTGGTGTCACTGCTGT |
| chr6 | 41080810 | 41080834 | + | 25 | GCAGCAGGGTGGTGTCACTGCTGTG |
| chr6 | 41080811 | 41080835 | + | 25 | CAGCAGGGTGGTGTCACTGCTGTGC |
| chr6 | 41080812 | 41080836 | + | 25 | AGCAGGGTGGTGTCACTGCTGTGCA |
| chr6 | 41080813 | 41080837 | + | 25 | GCAGGGTGGTGTCACTGCTGTGCAG |
| chr6 | 41080814 | 41080838 | + | 25 | CAGGGTGGTGTCACTGCTGTGCAGT |
| chr6 | 41080815 | 41080839 | + | 25 | AGGGTGGTGTCACTGCTGTGCAGTT |
| chr6 | 41080816 | 41080840 | + | 25 | GGGTGGTGTCACTGCTGTGCAGTTG |
| chr6 | 41080817 | 41080841 | + | 25 | GGTGGTGTCACTGCTGTGCAGTTGC |
| chr6 | 41080818 | 41080842 | + | 25 | GTGGTGTCACTGCTGTGCAGTTGCA |
| chr6 | 41080819 | 41080843 | + | 25 | TGGTGTCACTGCTGTGCAGTTGCAG |
| chr6 | 41080820 | 41080844 | + | 25 | GGTGTCACTGCTGTGCAGTTGCAGA |
| chr6 | 41080821 | 41080845 | + | 25 | GTGTCACTGCTGTGCAGTTGCAGAC |
| chr6 | 41080822 | 41080846 | + | 25 | TGTCACTGCTGTGCAGTTGCAGACT |
| chr6 | 41080823 | 41080847 | + | 25 | GTCACTGCTGTGCAGTTGCAGACTG |
| chr6 | 41080824 | 41080848 | + | 25 | TCACTGCTGTGCAGTTGCAGACTGA |
| chr6 | 41080825 | 41080849 | + | 25 | CACTGCTGTGCAGTTGCAGACTGAG |
| chr6 | 41080826 | 41080850 | + | 25 | ACTGCTGTGCAGTTGCAGACTGAGG |
| chr6 | 41080827 | 41080851 | + | 25 | CTGCTGTGCAGTTGCAGACTGAGGC |
| chr6 | 41080828 | 41080852 | + | 25 | TGCTGTGCAGTTGCAGACTGAGGCC |
| chr6 | 41080829 | 41080853 | + | 25 | GCTGTGCAGTTGCAGACTGAGGCCC |
| chr6 | 41080830 | 41080854 | + | 25 | CTGTGCAGTTGCAGACTGAGGCCCA |
| chr6 | 41080831 | 41080855 | + | 25 | TGTGCAGTTGCAGACTGAGGCCCAG |
| chr6 | 41080832 | 41080856 | + | 25 | GTGCAGTTGCAGACTGAGGCCCAGG |
| chr6 | 41080833 | 41080857 | + | 25 | TGCAGTTGCAGACTGAGGCCCAGGT |
| chr6 | 41080834 | 41080858 | + | 25 | GCAGTTGCAGACTGAGGCCCAGGTG |
| chr6 | 41080835 | 41080859 | + | 25 | CAGTTGCAGACTGAGGCCCAGGTGG |
| chr6 | 41080836 | 41080860 | + | 25 | AGTTGCAGACTGAGGCCCAGGTGGC |
| chr6 | 41080837 | 41080861 | + | 25 | GTTGCAGACTGAGGCCCAGGTGGCA |
| chr6 | 41080838 | 41080862 | + | 25 | TTGCAGACTGAGGCCCAGGTGGCAT |
| chr6 | 41080839 | 41080863 | + | 25 | TGCAGACTGAGGCCCAGGTGGCATC |
| chr6 | 41080840 | 41080864 | + | 25 | GCAGACTGAGGCCCAGGTGGCATCC |
| chr6 | 41080841 | 41080865 | + | 25 | CAGACTGAGGCCCAGGTGGCATCCG |
| chr6 | 41080842 | 41080866 | + | 25 | AGACTGAGGCCCAGGTGGCATCCGC |
| chr6 | 41080843 | 41080867 | + | 25 | GACTGAGGCCCAGGTGGCATCCGCC |
| chr6 | 41080844 | 41080868 | + | 25 | ACTGAGGCCCAGGTGGCATCCGCCT |
| chr6 | 41080845 | 41080869 | + | 25 | CTGAGGCCCAGGTGGCATCCGCCTC |
| chr6 | 41080846 | 41080870 | + | 25 | TGAGGCCCAGGTGGCATCCGCCTCA |
| chr6 | 41080847 | 41080871 | + | 25 | GAGGCCCAGGTGGCATCCGCCTCAG |
| chr6 | 41080848 | 41080872 | + | 25 | AGGCCCAGGTGGCATCCGCCTCAGG |
| chr6 | 41080849 | 41080873 | + | 25 | GGCCCAGGTGGCATCCGCCTCAGGC |
| chr6 | 41080850 | 41080874 | + | 25 | GCCCAGGTGGCATCCGCCTCAGGCC |
| chr6 | 41080851 | 41080875 | + | 25 | CCCAGGTGGCATCCGCCTCAGGCCA |
| chr6 | 41080852 | 41080876 | + | 25 | CCAGGTGGCATCCGCCTCAGGCCAG |
| chr6 | 41080853 | 41080877 | + | 25 | CAGGTGGCATCCGCCTCAGGCCAGC |
| chr6 | 41080854 | 41080878 | + | 25 | AGGTGGCATCCGCCTCAGGCCAGCA |
| chr6 | 41080855 | 41080879 | + | 25 | GGTGGCATCCGCCTCAGGCCAGCAA |
| chr6 | 41080856 | 41080880 | + | 25 | GTGGCATCCGCCTCAGGCCAGCAAG |
| chr6 | 41080857 | 41080881 | + | 25 | TGGCATCCGCCTCAGGCCAGCAAGT |
| chr6 | 41080858 | 41080882 | + | 25 | GGCATCCGCCTCAGGCCAGCAAGTC |
| chr6 | 41080859 | 41080883 | + | 25 | GCATCCGCCTCAGGCCAGCAAGTCC |
| chr6 | 41080860 | 41080884 | + | 25 | CATCCGCCTCAGGCCAGCAAGTCCA |
| chr6 | 41080861 | 41080885 | + | 25 | ATCCGCCTCAGGCCAGCAAGTCCAG |
| chr6 | 41080862 | 41080886 | + | 25 | TCCGCCTCAGGCCAGCAAGTCCAGA |
| chr6 | 41080863 | 41080887 | + | 25 | CCGCCTCAGGCCAGCAAGTCCAGAC |
| chr6 | 41080864 | 41080888 | + | 25 | CGCCTCAGGCCAGCAAGTCCAGACC |
| chr6 | 41080865 | 41080889 | + | 25 | GCCTCAGGCCAGCAAGTCCAGACCC |
| chr6 | 41080866 | 41080890 | + | 25 | CCTCAGGCCAGCAAGTCCAGACCCT |
| chr6 | 41080867 | 41080891 | + | 25 | CTCAGGCCAGCAAGTCCAGACCCTC |
| chr6 | 41080868 | 41080892 | + | 25 | TCAGGCCAGCAAGTCCAGACCCTCC |
| chr6 | 41080869 | 41080893 | + | 25 | CAGGCCAGCAAGTCCAGACCCTCCA |
| chr6 | 41080870 | 41080894 | + | 25 | AGGCCAGCAAGTCCAGACCCTCCAG |
| chr6 | 41080871 | 41080895 | + | 25 | GGCCAGCAAGTCCAGACCCTCCAGG |
| chr6 | 41080872 | 41080896 | + | 25 | GCCAGCAAGTCCAGACCCTCCAGGT |
| chr6 | 41080873 | 41080897 | + | 25 | CCAGCAAGTCCAGACCCTCCAGGTA |
| chr6 | 41080874 | 41080898 | + | 25 | CAGCAAGTCCAGACCCTCCAGGTAG |
| chr6 | 41080875 | 41080899 | + | 25 | AGCAAGTCCAGACCCTCCAGGTAGT |
| chr6 | 41080876 | 41080900 | + | 25 | GCAAGTCCAGACCCTCCAGGTAGTG |
| chr6 | 41080877 | 41080901 | + | 25 | CAAGTCCAGACCCTCCAGGTAGTGG |
| chr6 | 41080878 | 41080902 | + | 25 | AAGTCCAGACCCTCCAGGTAGTGGT |
| chr6 | 41080879 | 41080903 | + | 25 | AGTCCAGACCCTCCAGGTAGTGGTA |
| chr6 | 41080880 | 41080904 | + | 25 | GTCCAGACCCTCCAGGTAGTGGTAC |
| chr6 | 41080881 | 41080905 | + | 25 | TCCAGACCCTCCAGGTAGTGGTACC |
| chr6 | 41080882 | 41080906 | + | 25 | CCAGACCCTCCAGGTAGTGGTACCC |
| chr6 | 41080883 | 41080907 | + | 25 | CAGACCCTCCAGGTAGTGGTACCCT |
| chr6 | 41080884 | 41080908 | + | 25 | AGACCCTCCAGGTAGTGGTACCCTC |
| chr6 | 41080885 | 41080909 | + | 25 | GACCCTCCAGGTAGTGGTACCCTCT |
| chr6 | 41080886 | 41080910 | + | 25 | ACCCTCCAGGTAGTGGTACCCTCTC |
| chr6 | 41080887 | 41080911 | + | 25 | CCCTCCAGGTAGTGGTACCCTCTCT |
| chr6 | 41080888 | 41080912 | + | 25 | CCTCCAGGTAGTGGTACCCTCTCTG |
| chr6 | 41080889 | 41080913 | + | 25 | CTCCAGGTAGTGGTACCCTCTCTGA |
| chr6 | 41080890 | 41080914 | + | 25 | TCCAGGTAGTGGTACCCTCTCTGAT |
| chr6 | 41080891 | 41080915 | + | 25 | CCAGGTAGTGGTACCCTCTCTGATT |
| chr6 | 41080892 | 41080916 | + | 25 | CAGGTAGTGGTACCCTCTCTGATTC |
| chr6 | 41080893 | 41080917 | + | 25 | AGGTAGTGGTACCCTCTCTGATTCT |
| chr6 | 41080894 | 41080918 | + | 25 | GGTAGTGGTACCCTCTCTGATTCTC |
| chr6 | 41080895 | 41080919 | + | 25 | GTAGTGGTACCCTCTCTGATTCTCT |
| chr6 | 41080896 | 41080920 | + | 25 | TAGTGGTACCCTCTCTGATTCTCTG |
| chr6 | 41080897 | 41080921 | + | 25 | AGTGGTACCCTCTCTGATTCTCTGT |
| chr6 | 41080898 | 41080922 | + | 25 | GTGGTACCCTCTCTGATTCTCTGTG |
| chr6 | 41080899 | 41080923 | + | 25 | TGGTACCCTCTCTGATTCTCTGTGA |
| chr6 | 41080900 | 41080924 | + | 25 | GGTACCCTCTCTGATTCTCTGTGAG |
| chr6 | 41080901 | 41080925 | + | 25 | GTACCCTCTCTGATTCTCTGTGAGC |
| chr6 | 41080902 | 41080926 | + | 25 | TACCCTCTCTGATTCTCTGTGAGCA |
| chr6 | 41080903 | 41080927 | + | 25 | ACCCTCTCTGATTCTCTGTGAGCAC |
| chr6 | 41080904 | 41080928 | + | 25 | CCCTCTCTGATTCTCTGTGAGCACT |
| chr6 | 41080905 | 41080929 | + | 25 | CCTCTCTGATTCTCTGTGAGCACTG |
| chr6 | 41080906 | 41080930 | + | 25 | CTCTCTGATTCTCTGTGAGCACTGC |
| chr6 | 41080907 | 41080931 | + | 25 | TCTCTGATTCTCTGTGAGCACTGCA |
| chr6 | 41080908 | 41080932 | + | 25 | CTCTGATTCTCTGTGAGCACTGCAT |
| chr6 | 41080909 | 41080933 | + | 25 | TCTGATTCTCTGTGAGCACTGCATG |
| chr6 | 41080910 | 41080934 | + | 25 | CTGATTCTCTGTGAGCACTGCATGA |
| chr6 | 41080911 | 41080935 | + | 25 | TGATTCTCTGTGAGCACTGCATGAA |
| chr6 | 41080912 | 41080936 | + | 25 | GATTCTCTGTGAGCACTGCATGAAC |
| chr6 | 41080913 | 41080937 | + | 25 | ATTCTCTGTGAGCACTGCATGAACT |
| chr6 | 41080914 | 41080938 | + | 25 | TTCTCTGTGAGCACTGCATGAACTT |
| chr6 | 41080915 | 41080939 | + | 25 | TCTCTGTGAGCACTGCATGAACTTC |
| chr6 | 41080916 | 41080940 | + | 25 | CTCTGTGAGCACTGCATGAACTTCT |
| chr6 | 41080917 | 41080941 | + | 25 | TCTGTGAGCACTGCATGAACTTCTC |
| chr6 | 41080918 | 41080942 | + | 25 | CTGTGAGCACTGCATGAACTTCTCC |
| chr6 | 41080919 | 41080943 | + | 25 | TGTGAGCACTGCATGAACTTCTCCT |
| chr6 | 41080920 | 41080944 | + | 25 | GTGAGCACTGCATGAACTTCTCCTC |
| chr6 | 41080921 | 41080945 | + | 25 | TGAGCACTGCATGAACTTCTCCTCT |
| chr6 | 41080922 | 41080946 | + | 25 | GAGCACTGCATGAACTTCTCCTCTC |
| chr6 | 41080923 | 41080947 | + | 25 | AGCACTGCATGAACTTCTCCTCTCC |

**Table 26: 25-mer target-specific ASOs**

| **CHR** | **START** | **END** | **STRAND** | **kmer** | **SEQUENCE** |
|---|---|---|---|---|---|
| chr11 | 57791443 | 57791467 | + | 25 | TTACCCTGCCCTGCGGCGGCTCCGC |
| chr11 | 57791444 | 57791468 | + | 25 | TACCCTGCCCTGCGGCGGCTCCGCC |
| chr11 | 57791445 | 57791469 | + | 25 | ACCCTGCCCTGCGGCGGCTCCGCCC |
| chr11 | 57791446 | 57791470 | + | 25 | CCCTGCCCTGCGGCGGCTCCGCCCC |
| chr11 | 57791447 | 57791471 | + | 25 | CCTGCCCTGCGGCGGCTCCGCCCCT |
| chr11 | 57791448 | 57791472 | + | 25 | CTGCCCTGCGGCGGCTCCGCCCCTT |
| chr11 | 57791449 | 57791473 | + | 25 | TGCCCTGCGGCGGCTCCGCCCCTTA |
| chr11 | 57791450 | 57791474 | + | 25 | GCCCTGCGGCGGCTCCGCCCCTTAC |
| chr11 | 57791451 | 57791475 | + | 25 | CCCTGCGGCGGCTCCGCCCCTTACC |
| chr11 | 57791452 | 57791476 | + | 25 | CCTGCGGCGGCTCCGCCCCTTACCT |
| chr11 | 57791453 | 57791477 | + | 25 | CTGCGGCGGCTCCGCCCCTTACCTT |
| chr11 | 57791454 | 57791478 | + | 25 | TGCGGCGGCTCCGCCCCTTACCTTC |
| chr11 | 57791455 | 57791479 | + | 25 | GCGGCGGCTCCGCCCCTTACCTTCA |
| chr11 | 57791456 | 57791480 | + | 25 | CGGCGGCTCCGCCCCTTACCTTCAT |
| chr11 | 57791457 | 57791481 | + | 25 | GGCGGCTCCGCCCCTTACCTTCATG |
| chr11 | 57791458 | 57791482 | + | 25 | GCGGCTCCGCCCCTTACCTTCATGG |
| chr11 | 57791459 | 57791483 | + | 25 | CGGCTCCGCCCCTTACCTTCATGGA |
| chr11 | 57791460 | 57791484 | + | 25 | GGCTCCGCCCCTTACCTTCATGGAC |
| chr11 | 57791461 | 57791485 | + | 25 | GCTCCGCCCCTTACCTTCATGGACG |
| chr11 | 57791462 | 57791486 | + | 25 | CTCCGCCCCTTACCTTCATGGACGA |
| chr11 | 57791463 | 57791487 | + | 25 | TCCGCCCCTTACCTTCATGGACGAC |
| chr11 | 57791464 | 57791488 | + | 25 | CCGCCCCTTACCTTCATGGACGACT |
| chr11 | 57791465 | 57791489 | + | 25 | CGCCCCTTACCTTCATGGACGACTC |
| chr11 | 57791466 | 57791490 | + | 25 | GCCCCTTACCTTCATGGACGACTCA |
| chr11 | 57791467 | 57791491 | + | 25 | CCCCTTACCTTCATGGACGACTCAG |
| chr11 | 57791468 | 57791492 | + | 25 | CCCTTACCTTCATGGACGACTCAGA |
| chr11 | 57791469 | 57791493 | + | 25 | CCTTACCTTCATGGACGACTCAGAG |
| chr11 | 57791470 | 57791494 | + | 25 | CTTACCTTCATGGACGACTCAGAGG |
| chr11 | 57791471 | 57791495 | + | 25 | TTACCTTCATGGACGACTCAGAGGT |
| chr11 | 57791472 | 57791496 | + | 25 | TACCTTCATGGACGACTCAGAGGTG |
| chr11 | 57791473 | 57791497 | + | 25 | ACCTTCATGGACGACTCAGAGGTGG |
| chr11 | 57791474 | 57791498 | + | 25 | CCTTCATGGACGACTCAGAGGTGGA |
| chr11 | 57791475 | 57791499 | + | 25 | CTTCATGGACGACTCAGAGGTGGAG |
| chr11 | 57791476 | 57791500 | + | 25 | TTCATGGACGACTCAGAGGTGGAGT |
| chr11 | 57791477 | 57791501 | + | 25 | TCATGGACGACTCAGAGGTGGAGTC |
| chr11 | 57791478 | 57791502 | + | 25 | CATGGACGACTCAGAGGTGGAGTCG |
| chr11 | 57791479 | 57791503 | + | 25 | ATGGACGACTCAGAGGTGGAGTCGA |
| chr11 | 57791480 | 57791504 | + | 25 | TGGACGACTCAGAGGTGGAGTCGAC |
| chr11 | 57791481 | 57791505 | + | 25 | GGACGACTCAGAGGTGGAGTCGACC |
| chr11 | 57791482 | 57791506 | + | 25 | GACGACTCAGAGGTGGAGTCGACCG |
| chr11 | 57791483 | 57791507 | + | 25 | ACGACTCAGAGGTGGAGTCGACCGC |
| chr11 | 57791484 | 57791508 | + | 25 | CGACTCAGAGGTGGAGTCGACCGCC |
| chr11 | 57791485 | 57791509 | + | 25 | GACTCAGAGGTGGAGTCGACCGCCA |
| chr11 | 57791486 | 57791510 | + | 25 | ACTCAGAGGTGGAGTCGACCGCCAG |
| chr11 | 57791487 | 57791511 | + | 25 | CTCAGAGGTGGAGTCGACCGCCAGC |
| chr11 | 57791488 | 57791512 | + | 25 | TCAGAGGTGGAGTCGACCGCCAGCA |
| chr11 | 57791489 | 57791513 | + | 25 | CAGAGGTGGAGTCGACCGCCAGCAT |
| chr11 | 57791490 | 57791514 | + | 25 | AGAGGTGGAGTCGACCGCCAGCATC |
| chr11 | 57791491 | 57791515 | + | 25 | GAGGTGGAGTCGACCGCCAGCATCT |
| chr11 | 57791492 | 57791516 | + | 25 | AGGTGGAGTCGACCGCCAGCATCTT |
| chr11 | 57791493 | 57791517 | + | 25 | GGTGGAGTCGACCGCCAGCATCTTG |
| chr11 | 57791494 | 57791518 | + | 25 | GTGGAGTCGACCGCCAGCATCTTGG |
| chr11 | 57791495 | 57791519 | + | 25 | TGGAGTCGACCGCCAGCATCTTGGC |
| chr11 | 57791496 | 57791520 | + | 25 | GGAGTCGACCGCCAGCATCTTGGCC |
| chr11 | 57791497 | 57791521 | + | 25 | GAGTCGACCGCCAGCATCTTGGCCT |
| chr11 | 57791498 | 57791522 | + | 25 | AGTCGACCGCCAGCATCTTGGCCTC |
| chr11 | 57791499 | 57791523 | + | 25 | GTCGACCGCCAGCATCTTGGCCTCT |
| chr11 | 57791500 | 57791524 | + | 25 | TCGACCGCCAGCATCTTGGCCTCTG |
| chr11 | 57791501 | 57791525 | + | 25 | CGACCGCCAGCATCTTGGCCTCTGT |
| chr11 | 57791502 | 57791526 | + | 25 | GACCGCCAGCATCTTGGCCTCTGTG |
| chr11 | 57791503 | 57791527 | + | 25 | ACCGCCAGCATCTTGGCCTCTGTGA |
| chr11 | 57791504 | 57791528 | + | 25 | CCGCCAGCATCTTGGCCTCTGTGAA |
| chr11 | 57791505 | 57791529 | + | 25 | CGCCAGCATCTTGGCCTCTGTGAAG |
| chr11 | 57791506 | 57791530 | + | 25 | GCCAGCATCTTGGCCTCTGTGAAGG |
| chr11 | 57791507 | 57791531 | + | 25 | CCAGCATCTTGGCCTCTGTGAAGGA |
| chr11 | 57791508 | 57791532 | + | 25 | CAGCATCTTGGCCTCTGTGAAGGAA |
| chr11 | 57791509 | 57791533 | + | 25 | AGCATCTTGGCCTCTGTGAAGGAAC |
| chr11 | 57791510 | 57791534 | + | 25 | GCATCTTGGCCTCTGTGAAGGAACA |
| chr11 | 57791511 | 57791535 | + | 25 | CATCTTGGCCTCTGTGAAGGAACAA |
| chr11 | 57791512 | 57791536 | + | 25 | ATCTTGGCCTCTGTGAAGGAACAAG |
| chr11 | 57791513 | 57791537 | + | 25 | TCTTGGCCTCTGTGAAGGAACAAGA |
| chr11 | 57791514 | 57791538 | + | 25 | CTTGGCCTCTGTGAAGGAACAAGAG |
| chr11 | 57791515 | 57791539 | + | 25 | TTGGCCTCTGTGAAGGAACAAGAGG |
| chr11 | 57791516 | 57791540 | + | 25 | TGGCCTCTGTGAAGGAACAAGAGGC |
| chr11 | 57791517 | 57791541 | + | 25 | GGCCTCTGTGAAGGAACAAGAGGCC |
| chr11 | 57791518 | 57791542 | + | 25 | GCCTCTGTGAAGGAACAAGAGGCCC |
| chr11 | 57791519 | 57791543 | + | 25 | CCTCTGTGAAGGAACAAGAGGCCCA |
| chr11 | 57791520 | 57791544 | + | 25 | CTCTGTGAAGGAACAAGAGGCCCAG |
| chr11 | 57791521 | 57791545 | + | 25 | TCTGTGAAGGAACAAGAGGCCCAGT |
| chr11 | 57791522 | 57791546 | + | 25 | CTGTGAAGGAACAAGAGGCCCAGTT |
| chr11 | 57791523 | 57791547 | + | 25 | TGTGAAGGAACAAGAGGCCCAGTTT |
| chr11 | 57791524 | 57791548 | + | 25 | GTGAAGGAACAAGAGGCCCAGTTTG |
| chr11 | 57791525 | 57791549 | + | 25 | TGAAGGAACAAGAGGCCCAGTTTGA |
| chr11 | 57791526 | 57791550 | + | 25 | GAAGGAACAAGAGGCCCAGTTTGAG |
| chr11 | 57791527 | 57791551 | + | 25 | AAGGAACAAGAGGCCCAGTTTGAGA |
| chr11 | 57791528 | 57791552 | + | 25 | AGGAACAAGAGGCCCAGTTTGAGAA |
| chr11 | 57791529 | 57791553 | + | 25 | GGAACAAGAGGCCCAGTTTGAGAAG |
| chr11 | 57791530 | 57791554 | + | 25 | GAACAAGAGGCCCAGTTTGAGAAGC |
| chr11 | 57791531 | 57791555 | + | 25 | AACAAGAGGCCCAGTTTGAGAAGCT |
| chr11 | 57791532 | 57791556 | + | 25 | ACAAGAGGCCCAGTTTGAGAAGCTG |
| chr11 | 57791533 | 57791557 | + | 25 | CAAGAGGCCCAGTTTGAGAAGCTGA |
| chr11 | 57791534 | 57791558 | + | 25 | AAGAGGCCCAGTTTGAGAAGCTGAC |
| chr11 | 57791535 | 57791559 | + | 25 | AGAGGCCCAGTTTGAGAAGCTGACC |
| chr11 | 57791536 | 57791560 | + | 25 | GAGGCCCAGTTTGAGAAGCTGACCC |
| chr11 | 57791537 | 57791561 | + | 25 | AGGCCCAGTTTGAGAAGCTGACCCG |
| chr11 | 57791538 | 57791562 | + | 25 | GGCCCAGTTTGAGAAGCTGACCCGG |
| chr11 | 57791539 | 57791563 | + | 25 | GCCCAGTTTGAGAAGCTGACCCGGG |
| chr11 | 57791540 | 57791564 | + | 25 | CCCAGTTTGAGAAGCTGACCCGGGC |
| chr11 | 57791541 | 57791565 | + | 25 | CCAGTTTGAGAAGCTGACCCGGGCG |
| chr11 | 57791542 | 57791566 | + | 25 | CAGTTTGAGAAGCTGACCCGGGCGC |
| chr11 | 57791543 | 57791567 | + | 25 | AGTTTGAGAAGCTGACCCGGGCGCT |
| chr11 | 57791544 | 57791568 | + | 25 | GTTTGAGAAGCTGACCCGGGCGCTG |
| chr11 | 57791545 | 57791569 | + | 25 | TTTGAGAAGCTGACCCGGGCGCTGG |
| chr11 | 57791546 | 57791570 | + | 25 | TTGAGAAGCTGACCCGGGCGCTGGA |
| chr11 | 57791547 | 57791571 | + | 25 | TGAGAAGCTGACCCGGGCGCTGGAG |
| chr11 | 57791548 | 57791572 | + | 25 | GAGAAGCTGACCCGGGCGCTGGAGG |
| chr11 | 57791549 | 57791573 | + | 25 | AGAAGCTGACCCGGGCGCTGGAGGA |
| chr11 | 57791550 | 57791574 | + | 25 | GAAGCTGACCCGGGCGCTGGAGGAG |
| chr11 | 57791551 | 57791575 | + | 25 | AAGCTGACCCGGGCGCTGGAGGAGG |
| chr11 | 57791552 | 57791576 | + | 25 | AGCTGACCCGGGCGCTGGAGGAGGA |
| chr11 | 57791553 | 57791577 | + | 25 | GCTGACCCGGGCGCTGGAGGAGGAA |
| chr11 | 57791554 | 57791578 | + | 25 | CTGACCCGGGCGCTGGAGGAGGAAC |
| chr11 | 57791555 | 57791579 | + | 25 | TGACCCGGGCGCTGGAGGAGGAACG |
| chr11 | 57791556 | 57791580 | + | 25 | GACCCGGGCGCTGGAGGAGGAACGG |
| chr11 | 57791557 | 57791581 | + | 25 | ACCCGGGCGCTGGAGGAGGAACGGC |
| chr11 | 57791558 | 57791582 | + | 25 | CCCGGGCGCTGGAGGAGGAACGGCG |
| chr11 | 57791559 | 57791583 | + | 25 | CCGGGCGCTGGAGGAGGAACGGCGC |
| chr11 | 57791560 | 57791584 | + | 25 | CGGGCGCTGGAGGAGGAACGGCGCC |
| chr11 | 57791561 | 57791585 | + | 25 | GGGCGCTGGAGGAGGAACGGCGCCA |
| chr11 | 57791562 | 57791586 | + | 25 | GGCGCTGGAGGAGGAACGGCGCCAC |
| chr11 | 57791563 | 57791587 | + | 25 | GCGCTGGAGGAGGAACGGCGCCACG |
| chr11 | 57791564 | 57791588 | + | 25 | CGCTGGAGGAGGAACGGCGCCACGT |
| chr11 | 57791565 | 57791589 | + | 25 | GCTGGAGGAGGAACGGCGCCACGTC |
| chr11 | 57791566 | 57791590 | + | 25 | CTGGAGGAGGAACGGCGCCACGTCT |
| chr11 | 57791567 | 57791591 | + | 25 | TGGAGGAGGAACGGCGCCACGTCTC |
| chr11 | 57791568 | 57791592 | + | 25 | GGAGGAGGAACGGCGCCACGTCTCG |
| chr11 | 57791569 | 57791593 | + | 25 | GAGGAGGAACGGCGCCACGTCTCGG |
| chr11 | 57791570 | 57791594 | + | 25 | AGGAGGAACGGCGCCACGTCTCGGC |
| chr11 | 57791571 | 57791595 | + | 25 | GGAGGAACGGCGCCACGTCTCGGCG |
| chr11 | 57791572 | 57791596 | + | 25 | GAGGAACGGCGCCACGTCTCGGCGC |
| chr11 | 57791573 | 57791597 | + | 25 | AGGAACGGCGCCACGTCTCGGCGCA |
| chr11 | 57791574 | 57791598 | + | 25 | GGAACGGCGCCACGTCTCGGCGCAG |
| chr11 | 57791575 | 57791599 | + | 25 | GAACGGCGCCACGTCTCGGCGCAGC |
| chr11 | 57791576 | 57791600 | + | 25 | AACGGCGCCACGTCTCGGCGCAGCT |
| chr11 | 57791577 | 57791601 | + | 25 | ACGGCGCCACGTCTCGGCGCAGCTG |
| chr11 | 57791578 | 57791602 | + | 25 | CGGCGCCACGTCTCGGCGCAGCTGG |
| chr11 | 57791579 | 57791603 | + | 25 | GGCGCCACGTCTCGGCGCAGCTGGA |
| chr11 | 57791580 | 57791604 | + | 25 | GCGCCACGTCTCGGCGCAGCTGGAA |
| chr11 | 57791581 | 57791605 | + | 25 | CGCCACGTCTCGGCGCAGCTGGAAC |
| chr11 | 57791582 | 57791606 | + | 25 | GCCACGTCTCGGCGCAGCTGGAACG |
| chr11 | 57791583 | 57791607 | + | 25 | CCACGTCTCGGCGCAGCTGGAACGC |
| chr11 | 57791584 | 57791608 | + | 25 | CACGTCTCGGCGCAGCTGGAACGCG |
| chr11 | 57791585 | 57791609 | + | 25 | ACGTCTCGGCGCAGCTGGAACGCGT |
| chr11 | 57791586 | 57791610 | + | 25 | CGTCTCGGCGCAGCTGGAACGCGTC |
| chr11 | 57791587 | 57791611 | + | 25 | GTCTCGGCGCAGCTGGAACGCGTCC |
| chr11 | 57791588 | 57791612 | + | 25 | TCTCGGCGCAGCTGGAACGCGTCCG |
| chr11 | 57791589 | 57791613 | + | 25 | CTCGGCGCAGCTGGAACGCGTCCGG |
| chr11 | 57791590 | 57791614 | + | 25 | TCGGCGCAGCTGGAACGCGTCCGGG |
| chr11 | 57791591 | 57791615 | + | 25 | CGGCGCAGCTGGAACGCGTCCGGGT |
| chr11 | 57791592 | 57791616 | + | 25 | GGCGCAGCTGGAACGCGTCCGGGTC |
| chr11 | 57791593 | 57791617 | + | 25 | GCGCAGCTGGAACGCGTCCGGGTCT |
| chr11 | 57791594 | 57791618 | + | 25 | CGCAGCTGGAACGCGTCCGGGTCTC |
| chr11 | 57791595 | 57791619 | + | 25 | GCAGCTGGAACGCGTCCGGGTCTCA |
| chr11 | 57791596 | 57791620 | + | 25 | CAGCTGGAACGCGTCCGGGTCTCAC |
| chr11 | 57791597 | 57791621 | + | 25 | AGCTGGAACGCGTCCGGGTCTCACC |
| chr11 | 57791598 | 57791622 | + | 25 | GCTGGAACGCGTCCGGGTCTCACCA |
| chr11 | 57791599 | 57791623 | + | 25 | CTGGAACGCGTCCGGGTCTCACCAC |
| chr11 | 57791600 | 57791624 | + | 25 | TGGAACGCGTCCGGGTCTCACCACA |
| chr11 | 57791601 | 57791625 | + | 25 | GGAACGCGTCCGGGTCTCACCACAA |
| chr11 | 57791602 | 57791626 | + | 25 | GAACGCGTCCGGGTCTCACCACAAG |
| chr11 | 57791603 | 57791627 | + | 25 | AACGCGTCCGGGTCTCACCACAAGA |
| chr11 | 57791604 | 57791628 | + | 25 | ACGCGTCCGGGTCTCACCACAAGAT |
| chr11 | 57791605 | 57791629 | + | 25 | CGCGTCCGGGTCTCACCACAAGATG |
| chr11 | 57791606 | 57791630 | + | 25 | GCGTCCGGGTCTCACCACAAGATGC |
| chr11 | 57791607 | 57791631 | + | 25 | CGTCCGGGTCTCACCACAAGATGCC |
| chr11 | 57791608 | 57791632 | + | 25 | GTCCGGGTCTCACCACAAGATGCCA |
| chr11 | 57791609 | 57791633 | + | 25 | TCCGGGTCTCACCACAAGATGCCAA |
| chr11 | 57791610 | 57791634 | + | 25 | CCGGGTCTCACCACAAGATGCCAAC |
| chr11 | 57791611 | 57791635 | + | 25 | CGGGTCTCACCACAAGATGCCAACC |
| chr11 | 57791612 | 57791636 | + | 25 | GGGTCTCACCACAAGATGCCAACCC |
| chr11 | 57791613 | 57791637 | + | 25 | GGTCTCACCACAAGATGCCAACCCA |
| chr11 | 57791614 | 57791638 | + | 25 | GTCTCACCACAAGATGCCAACCCAC |
| chr11 | 57791615 | 57791639 | + | 25 | TCTCACCACAAGATGCCAACCCACT |
| chr11 | 57791616 | 57791640 | + | 25 | CTCACCACAAGATGCCAACCCACTC |
| chr11 | 57791617 | 57791641 | + | 25 | TCACCACAAGATGCCAACCCACTCA |
| chr11 | 57791618 | 57791642 | + | 25 | CACCACAAGATGCCAACCCACTCAT |
| chr11 | 57791619 | 57791643 | + | 25 | ACCACAAGATGCCAACCCACTCATG |
| chr11 | 57791620 | 57791644 | + | 25 | CCACAAGATGCCAACCCACTCATGG |
| chr11 | 57791621 | 57791645 | + | 25 | CACAAGATGCCAACCCACTCATGGC |
| chr11 | 57791622 | 57791646 | + | 25 | ACAAGATGCCAACCCACTCATGGCC |
| chr11 | 57791623 | 57791647 | + | 25 | CAAGATGCCAACCCACTCATGGCCA |
| chr11 | 57791624 | 57791648 | + | 25 | AAGATGCCAACCCACTCATGGCCAA |
| chr11 | 57791625 | 57791649 | + | 25 | AGATGCCAACCCACTCATGGCCAAC |
| chr11 | 57791626 | 57791650 | + | 25 | GATGCCAACCCACTCATGGCCAACG |
| chr11 | 57791627 | 57791651 | + | 25 | ATGCCAACCCACTCATGGCCAACGG |
| chr11 | 57791628 | 57791652 | + | 25 | TGCCAACCCACTCATGGCCAACGGC |
| chr11 | 57791629 | 57791653 | + | 25 | GCCAACCCACTCATGGCCAACGGCA |
| chr11 | 57791630 | 57791654 | + | 25 | CCAACCCACTCATGGCCAACGGCAC |
| chr11 | 57791631 | 57791655 | + | 25 | CAACCCACTCATGGCCAACGGCACA |
| chr11 | 57791632 | 57791656 | + | 25 | AACCCACTCATGGCCAACGGCACAC |
| chr11 | 57791633 | 57791657 | + | 25 | ACCCACTCATGGCCAACGGCACACT |
| chr11 | 57791634 | 57791658 | + | 25 | CCCACTCATGGCCAACGGCACACTC |
| chr11 | 57791635 | 57791659 | + | 25 | CCACTCATGGCCAACGGCACACTCA |
| chr11 | 57791636 | 57791660 | + | 25 | CACTCATGGCCAACGGCACACTCAC |
| chr11 | 57791637 | 57791661 | + | 25 | ACTCATGGCCAACGGCACACTCACC |
| chr11 | 57791638 | 57791662 | + | 25 | CTCATGGCCAACGGCACACTCACCC |
| chr11 | 57791639 | 57791663 | + | 25 | TCATGGCCAACGGCACACTCACCCG |
| chr11 | 57791640 | 57791664 | + | 25 | CATGGCCAACGGCACACTCACCCGC |
| chr11 | 57791641 | 57791665 | + | 25 | ATGGCCAACGGCACACTCACCCGCC |
| chr11 | 57791642 | 57791666 | + | 25 | TGGCCAACGGCACACTCACCCGCCG |
| chr11 | 57791643 | 57791667 | + | 25 | GGCCAACGGCACACTCACCCGCCGG |
| chr11 | 57791644 | 57791668 | + | 25 | GCCAACGGCACACTCACCCGCCGGC |
| chr11 | 57791645 | 57791669 | + | 25 | CCAACGGCACACTCACCCGCCGGCA |
| chr11 | 57791646 | 57791670 | + | 25 | CAACGGCACACTCACCCGCCGGCAT |
| chr11 | 57791647 | 57791671 | + | 25 | AACGGCACACTCACCCGCCGGCATC |
| chr11 | 57791648 | 57791672 | + | 25 | ACGGCACACTCACCCGCCGGCATCA |
| chr11 | 57791649 | 57791673 | + | 25 | CGGCACACTCACCCGCCGGCATCAG |
| chr11 | 57791650 | 57791674 | + | 25 | GGCACACTCACCCGCCGGCATCAGG |
| chr11 | 57791651 | 57791675 | + | 25 | GCACACTCACCCGCCGGCATCAGGT |
| chr11 | 57791652 | 57791676 | + | 25 | CACACTCACCCGCCGGCATCAGGTA |
| chr11 | 57791653 | 57791677 | + | 25 | ACACTCACCCGCCGGCATCAGGTAA |
| chr11 | 57791654 | 57791678 | + | 25 | CACTCACCCGCCGGCATCAGGTAAC |
| chr11 | 57791655 | 57791679 | + | 25 | ACTCACCCGCCGGCATCAGGTAACC |
| chr11 | 57791656 | 57791680 | + | 25 | CTCACCCGCCGGCATCAGGTAACCC |
| chr11 | 57791657 | 57791681 | + | 25 | TCACCCGCCGGCATCAGGTAACCCC |
| chr11 | 57791658 | 57791682 | + | 25 | CACCCGCCGGCATCAGGTAACCCCT |
| chr11 | 57791659 | 57791683 | + | 25 | ACCCGCCGGCATCAGGTAACCCCTC |
| chr11 | 57791660 | 57791684 | + | 25 | CCCGCCGGCATCAGGTAACCCCTCT |
| chr11 | 57791661 | 57791685 | + | 25 | CCGCCGGCATCAGGTAACCCCTCTC |
| chr11 | 57791662 | 57791686 | + | 25 | CGCCGGCATCAGGTAACCCCTCTCT |
| chr11 | 57791663 | 57791687 | + | 25 | GCCGGCATCAGGTAACCCCTCTCTC |
| chr11 | 57791664 | 57791688 | + | 25 | CCGGCATCAGGTAACCCCTCTCTCC |
| chr11 | 57791665 | 57791689 | + | 25 | CGGCATCAGGTAACCCCTCTCTCCA |
| chr11 | 57791666 | 57791690 | + | 25 | GGCATCAGGTAACCCCTCTCTCCAT |
| chr11 | 57791667 | 57791691 | + | 25 | GCATCAGGTAACCCCTCTCTCCATC |
| chr11 | 57791668 | 57791692 | + | 25 | CATCAGGTAACCCCTCTCTCCATCA |
| chr11 | 57791669 | 57791693 | + | 25 | ATCAGGTAACCCCTCTCTCCATCAG |
| chr11 | 57791670 | 57791694 | + | 25 | TCAGGTAACCCCTCTCTCCATCAGA |
| chr11 | 57791671 | 57791695 | + | 25 | CAGGTAACCCCTCTCTCCATCAGAC |
| chr11 | 57791672 | 57791696 | + | 25 | AGGTAACCCCTCTCTCCATCAGACG |
| chr11 | 57791673 | 57791697 | + | 25 | GGTAACCCCTCTCTCCATCAGACGT |
| chr11 | 57791674 | 57791698 | + | 25 | GTAACCCCTCTCTCCATCAGACGTG |
| chr11 | 57791675 | 57791699 | + | 25 | TAACCCCTCTCTCCATCAGACGTGC |
| chr11 | 57791676 | 57791700 | + | 25 | AACCCCTCTCTCCATCAGACGTGCA |
| chr11 | 57791677 | 57791701 | + | 25 | ACCCCTCTCTCCATCAGACGTGCAG |
| chr11 | 57791678 | 57791702 | + | 25 | CCCCTCTCTCCATCAGACGTGCAGG |
| chr11 | 57791679 | 57791703 | + | 25 | CCCTCTCTCCATCAGACGTGCAGGT |
| chr11 | 57791680 | 57791704 | + | 25 | CCTCTCTCCATCAGACGTGCAGGTA |
| chr11 | 57791681 | 57791705 | + | 25 | CTCTCTCCATCAGACGTGCAGGTAG |
| chr11 | 57791682 | 57791706 | + | 25 | TCTCTCCATCAGACGTGCAGGTAGG |
| chr11 | 57791683 | 57791707 | + | 25 | CTCTCCATCAGACGTGCAGGTAGGA |
| chr11 | 57791684 | 57791708 | + | 25 | TCTCCATCAGACGTGCAGGTAGGAC |
| chr11 | 57791685 | 57791709 | + | 25 | CTCCATCAGACGTGCAGGTAGGACT |
| chr11 | 57791686 | 57791710 | + | 25 | TCCATCAGACGTGCAGGTAGGACTT |
| chr11 | 57791687 | 57791711 | + | 25 | CCATCAGACGTGCAGGTAGGACTTT |
| chr11 | 57791688 | 57791712 | + | 25 | CATCAGACGTGCAGGTAGGACTTTG |
| chr11 | 57791689 | 57791713 | + | 25 | ATCAGACGTGCAGGTAGGACTTTGG |
| chr11 | 57791690 | 57791714 | + | 25 | TCAGACGTGCAGGTAGGACTTTGGC |
| chr11 | 57791691 | 57791715 | + | 25 | CAGACGTGCAGGTAGGACTTTGGCA |
| chr11 | 57791692 | 57791716 | + | 25 | AGACGTGCAGGTAGGACTTTGGCAT |
| chr11 | 57791693 | 57791717 | + | 25 | GACGTGCAGGTAGGACTTTGGCATG |
| chr11 | 57791694 | 57791718 | + | 25 | ACGTGCAGGTAGGACTTTGGCATGG |
| chr11 | 57791695 | 57791719 | + | 25 | CGTGCAGGTAGGACTTTGGCATGGT |
| chr11 | 57791696 | 57791720 | + | 25 | GTGCAGGTAGGACTTTGGCATGGTC |
| chr11 | 57791697 | 57791721 | + | 25 | TGCAGGTAGGACTTTGGCATGGTCA |
| chr11 | 57791698 | 57791722 | + | 25 | GCAGGTAGGACTTTGGCATGGTCAC |
| chr11 | 57791699 | 57791723 | + | 25 | CAGGTAGGACTTTGGCATGGTCACA |

**Table 27: 25-mer target-specific ASOs**

| **CHR** | **START** | **END** | **STRAND** | **kmer** | **SEQUENCE** |
|---|---|---|---|---|---|
| chr17 | 77307090 | 77307114 | + | 25 | GGAGAGCGCCAGTGGCCTTGTGTGA |
| chr17 | 77307091 | 77307115 | + | 25 | GAGAGCGCCAGTGGCCTTGTGTGAC |
| chr17 | 77307092 | 77307116 | + | 25 | AGAGCGCCAGTGGCCTTGTGTGACC |
| chr17 | 77307093 | 77307117 | + | 25 | GAGCGCCAGTGGCCTTGTGTGACCT |
| chr17 | 77307094 | 77307118 | + | 25 | AGCGCCAGTGGCCTTGTGTGACCTT |
| chr17 | 77307095 | 77307119 | + | 25 | GCGCCAGTGGCCTTGTGTGACCTTT |
| chr17 | 77307096 | 77307120 | + | 25 | CGCCAGTGGCCTTGTGTGACCTTTG |
| chr17 | 77307097 | 77307121 | + | 25 | GCCAGTGGCCTTGTGTGACCTTTGC |
| chr17 | 77307098 | 77307122 | + | 25 | CCAGTGGCCTTGTGTGACCTTTGCC |
| chr17 | 77307099 | 77307123 | + | 25 | CAGTGGCCTTGTGTGACCTTTGCCC |
| chr17 | 77307100 | 77307124 | + | 25 | AGTGGCCTTGTGTGACCTTTGCCCT |
| chr17 | 77307101 | 77307125 | + | 25 | GTGGCCTTGTGTGACCTTTGCCCTT |
| chr17 | 77307102 | 77307126 | + | 25 | TGGCCTTGTGTGACCTTTGCCCTTT |
| chr17 | 77307103 | 77307127 | + | 25 | GGCCTTGTGTGACCTTTGCCCTTTG |
| chr17 | 77307104 | 77307128 | + | 25 | GCCTTGTGTGACCTTTGCCCTTTGT |
| chr17 | 77307105 | 77307129 | + | 25 | CCTTGTGTGACCTTTGCCCTTTGTC |
| chr17 | 77307106 | 77307130 | + | 25 | CTTGTGTGACCTTTGCCCTTTGTCT |
| chr17 | 77307107 | 77307131 | + | 25 | TTGTGTGACCTTTGCCCTTTGTCTC |
| chr17 | 77307108 | 77307132 | + | 25 | TGTGTGACCTTTGCCCTTTGTCTCT |
| chr17 | 77307109 | 77307133 | + | 25 | GTGTGACCTTTGCCCTTTGTCTCTG |
| chr17 | 77307110 | 77307134 | + | 25 | TGTGACCTTTGCCCTTTGTCTCTGT |
| chr17 | 77307111 | 77307135 | + | 25 | GTGACCTTTGCCCTTTGTCTCTGTC |
| chr17 | 77307112 | 77307136 | + | 25 | TGACCTTTGCCCTTTGTCTCTGTCT |
| chr17 | 77307113 | 77307137 | + | 25 | GACCTTTGCCCTTTGTCTCTGTCTT |
| chr17 | 77307114 | 77307138 | + | 25 | ACCTTTGCCCTTTGTCTCTGTCTTT |
| chr17 | 77307115 | 77307139 | + | 25 | CCTTTGCCCTTTGTCTCTGTCTTTA |
| chr17 | 77307116 | 77307140 | + | 25 | CTTTGCCCTTTGTCTCTGTCTTTAG |
| chr17 | 77307117 | 77307141 | + | 25 | TTTGCCCTTTGTCTCTGTCTTTAGG |
| chr17 | 77307118 | 77307142 | + | 25 | TTGCCCTTTGTCTCTGTCTTTAGGA |
| chr17 | 77307119 | 77307143 | + | 25 | TGCCCTTTGTCTCTGTCTTTAGGAG |
| chr17 | 77307120 | 77307144 | + | 25 | GCCCTTTGTCTCTGTCTTTAGGAGG |
| chr17 | 77307121 | 77307145 | + | 25 | CCCTTTGTCTCTGTCTTTAGGAGGC |
| chr17 | 77307122 | 77307146 | + | 25 | CCTTTGTCTCTGTCTTTAGGAGGCA |
| chr17 | 77307123 | 77307147 | + | 25 | CTTTGTCTCTGTCTTTAGGAGGCAC |
| chr17 | 77307124 | 77307148 | + | 25 | TTTGTCTCTGTCTTTAGGAGGCACG |
| chr17 | 77307125 | 77307149 | + | 25 | TTGTCTCTGTCTTTAGGAGGCACGC |
| chr17 | 77307126 | 77307150 | + | 25 | TGTCTCTGTCTTTAGGAGGCACGCG |
| chr17 | 77307127 | 77307151 | + | 25 | GTCTCTGTCTTTAGGAGGCACGCGG |
| chr17 | 77307128 | 77307152 | + | 25 | TCTCTGTCTTTAGGAGGCACGCGGA |
| chr17 | 77307129 | 77307153 | + | 25 | CTCTGTCTTTAGGAGGCACGCGGAC |
| chr17 | 77307130 | 77307154 | + | 25 | TCTGTCTTTAGGAGGCACGCGGACC |
| chr17 | 77307131 | 77307155 | + | 25 | CTGTCTTTAGGAGGCACGCGGACCT |
| chr17 | 77307132 | 77307156 | + | 25 | TGTCTTTAGGAGGCACGCGGACCTC |
| chr17 | 77307133 | 77307157 | + | 25 | GTCTTTAGGAGGCACGCGGACCTCC |
| chr17 | 77307134 | 77307158 | + | 25 | TCTTTAGGAGGCACGCGGACCTCCA |
| chr17 | 77307135 | 77307159 | + | 25 | CTTTAGGAGGCACGCGGACCTCCAG |
| chr17 | 77307136 | 77307160 | + | 25 | TTTAGGAGGCACGCGGACCTCCAGT |
| chr17 | 77307137 | 77307161 | + | 25 | TTAGGAGGCACGCGGACCTCCAGTG |
| chr17 | 77307138 | 77307162 | + | 25 | TAGGAGGCACGCGGACCTCCAGTGG |
| chr17 | 77307139 | 77307163 | + | 25 | AGGAGGCACGCGGACCTCCAGTGGC |
| chr17 | 77307140 | 77307164 | + | 25 | GGAGGCACGCGGACCTCCAGTGGCC |
| chr17 | 77307141 | 77307165 | + | 25 | GAGGCACGCGGACCTCCAGTGGCCG |
| chr17 | 77307142 | 77307166 | + | 25 | AGGCACGCGGACCTCCAGTGGCCGG |
| chr17 | 77307143 | 77307167 | + | 25 | GGCACGCGGACCTCCAGTGGCCGGC |
| chr17 | 77307144 | 77307168 | + | 25 | GCACGCGGACCTCCAGTGGCCGGCT |
| chr17 | 77307145 | 77307169 | + | 25 | CACGCGGACCTCCAGTGGCCGGCTC |
| chr17 | 77307146 | 77307170 | + | 25 | ACGCGGACCTCCAGTGGCCGGCTCC |
| chr17 | 77307147 | 77307171 | + | 25 | CGCGGACCTCCAGTGGCCGGCTCCG |
| chr17 | 77307148 | 77307172 | + | 25 | GCGGACCTCCAGTGGCCGGCTCCGG |
| chr17 | 77307149 | 77307173 | + | 25 | CGGACCTCCAGTGGCCGGCTCCGGA |
| chr17 | 77307150 | 77307174 | + | 25 | GGACCTCCAGTGGCCGGCTCCGGAG |
| chr17 | 77307151 | 77307175 | + | 25 | GACCTCCAGTGGCCGGCTCCGGAGG |
| chr17 | 77307152 | 77307176 | + | 25 | ACCTCCAGTGGCCGGCTCCGGAGGC |
| chr17 | 77307153 | 77307177 | + | 25 | CCTCCAGTGGCCGGCTCCGGAGGCT |
| chr17 | 77307154 | 77307178 | + | 25 | CTCCAGTGGCCGGCTCCGGAGGCTT |
| chr17 | 77307155 | 77307179 | + | 25 | TCCAGTGGCCGGCTCCGGAGGCTTG |
| chr17 | 77307156 | 77307180 | + | 25 | CCAGTGGCCGGCTCCGGAGGCTTGG |
| chr17 | 77307157 | 77307181 | + | 25 | CAGTGGCCGGCTCCGGAGGCTTGGT |
| chr17 | 77307158 | 77307182 | + | 25 | AGTGGCCGGCTCCGGAGGCTTGGTG |
| chr17 | 77307159 | 77307183 | + | 25 | GTGGCCGGCTCCGGAGGCTTGGTGA |
| chr17 | 77307160 | 77307184 | + | 25 | TGGCCGGCTCCGGAGGCTTGGTGAC |
| chr17 | 77307161 | 77307185 | + | 25 | GGCCGGCTCCGGAGGCTTGGTGACT |
| chr17 | 77307162 | 77307186 | + | 25 | GCCGGCTCCGGAGGCTTGGTGACTC |
| chr17 | 77307163 | 77307187 | + | 25 | CCGGCTCCGGAGGCTTGGTGACTCC |
| chr17 | 77307164 | 77307188 | + | 25 | CGGCTCCGGAGGCTTGGTGACTCCA |
| chr17 | 77307165 | 77307189 | + | 25 | GGCTCCGGAGGCTTGGTGACTCCAG |
| chr17 | 77307166 | 77307190 | + | 25 | GCTCCGGAGGCTTGGTGACTCCAGT |
| chr17 | 77307167 | 77307191 | + | 25 | CTCCGGAGGCTTGGTGACTCCAGTG |
| chr17 | 77307168 | 77307192 | + | 25 | TCCGGAGGCTTGGTGACTCCAGTGG |
| chr17 | 77307169 | 77307193 | + | 25 | CCGGAGGCTTGGTGACTCCAGTGGC |
| chr17 | 77307170 | 77307194 | + | 25 | CGGAGGCTTGGTGACTCCAGTGGCC |
| chr17 | 77307171 | 77307195 | + | 25 | GGAGGCTTGGTGACTCCAGTGGCCC |
| chr17 | 77307172 | 77307196 | + | 25 | GAGGCTTGGTGACTCCAGTGGCCCA |
| chr17 | 77307173 | 77307197 | + | 25 | AGGCTTGGTGACTCCAGTGGCCCAG |
| chr17 | 77307174 | 77307198 | + | 25 | GGCTTGGTGACTCCAGTGGCCCAGG |
| chr17 | 77307175 | 77307199 | + | 25 | GCTTGGTGACTCCAGTGGCCCAGGT |
| chr17 | 77307176 | 77307200 | + | 25 | CTTGGTGACTCCAGTGGCCCAGGTA |
| chr17 | 77307177 | 77307201 | + | 25 | TTGGTGACTCCAGTGGCCCAGGTAG |
| chr17 | 77307178 | 77307202 | + | 25 | TGGTGACTCCAGTGGCCCAGGTAGG |
| chr17 | 77307179 | 77307203 | + | 25 | GGTGACTCCAGTGGCCCAGGTAGGT |
| chr17 | 77307180 | 77307204 | + | 25 | GTGACTCCAGTGGCCCAGGTAGGTG |
| chr17 | 77307181 | 77307205 | + | 25 | TGACTCCAGTGGCCCAGGTAGGTGG |
| chr17 | 77307182 | 77307206 | + | 25 | GACTCCAGTGGCCCAGGTAGGTGGC |
| chr17 | 77307183 | 77307207 | + | 25 | ACTCCAGTGGCCCAGGTAGGTGGCT |
| chr17 | 77307184 | 77307208 | + | 25 | CTCCAGTGGCCCAGGTAGGTGGCTC |
| chr17 | 77307185 | 77307209 | + | 25 | TCCAGTGGCCCAGGTAGGTGGCTCG |
| chr17 | 77307186 | 77307210 | + | 25 | CCAGTGGCCCAGGTAGGTGGCTCGC |
| chr17 | 77307187 | 77307211 | + | 25 | CAGTGGCCCAGGTAGGTGGCTCGCT |
| chr17 | 77307188 | 77307212 | + | 25 | AGTGGCCCAGGTAGGTGGCTCGCTC |
| chr17 | 77307189 | 77307213 | + | 25 | GTGGCCCAGGTAGGTGGCTCGCTCC |
| chr17 | 77307190 | 77307214 | + | 25 | TGGCCCAGGTAGGTGGCTCGCTCCG |
| chr17 | 77307191 | 77307215 | + | 25 | GGCCCAGGTAGGTGGCTCGCTCCGC |
| chr17 | 77307192 | 77307216 | + | 25 | GCCCAGGTAGGTGGCTCGCTCCGCT |
| chr17 | 77307193 | 77307217 | + | 25 | CCCAGGTAGGTGGCTCGCTCCGCTC |
| chr17 | 77307194 | 77307218 | + | 25 | CCAGGTAGGTGGCTCGCTCCGCTCT |
| chr17 | 77307195 | 77307219 | + | 25 | CAGGTAGGTGGCTCGCTCCGCTCTG |
| chr17 | 77307196 | 77307220 | + | 25 | AGGTAGGTGGCTCGCTCCGCTCTGG |
| chr17 | 77307197 | 77307221 | + | 25 | GGTAGGTGGCTCGCTCCGCTCTGGC |
| chr17 | 77307198 | 77307222 | + | 25 | GTAGGTGGCTCGCTCCGCTCTGGCC |
| chr17 | 77307199 | 77307223 | + | 25 | TAGGTGGCTCGCTCCGCTCTGGCCC |
| chr17 | 77307200 | 77307224 | + | 25 | AGGTGGCTCGCTCCGCTCTGGCCCC |
| chr17 | 77307201 | 77307225 | + | 25 | GGTGGCTCGCTCCGCTCTGGCCCCA |
| chr17 | 77307202 | 77307226 | + | 25 | GTGGCTCGCTCCGCTCTGGCCCCAC |
| chr17 | 77307203 | 77307227 | + | 25 | TGGCTCGCTCCGCTCTGGCCCCACC |
| chr17 | 77307204 | 77307228 | + | 25 | GGCTCGCTCCGCTCTGGCCCCACCC |
| chr17 | 77307205 | 77307229 | + | 25 | GCTCGCTCCGCTCTGGCCCCACCCA |
| chr17 | 77307206 | 77307230 | + | 25 | CTCGCTCCGCTCTGGCCCCACCCAG |
| chr17 | 77307207 | 77307231 | + | 25 | TCGCTCCGCTCTGGCCCCACCCAGC |
| chr17 | 77307208 | 77307232 | + | 25 | CGCTCCGCTCTGGCCCCACCCAGCT |
| chr17 | 77307209 | 77307233 | + | 25 | GCTCCGCTCTGGCCCCACCCAGCTC |
| chr17 | 77307210 | 77307234 | + | 25 | CTCCGCTCTGGCCCCACCCAGCTCA |
| chr17 | 77307211 | 77307235 | + | 25 | TCCGCTCTGGCCCCACCCAGCTCAT |
| chr17 | 77307212 | 77307236 | + | 25 | CCGCTCTGGCCCCACCCAGCTCATG |
| chr17 | 77307213 | 77307237 | + | 25 | CGCTCTGGCCCCACCCAGCTCATGG |
| chr17 | 77307214 | 77307238 | + | 25 | GCTCTGGCCCCACCCAGCTCATGGG |
| chr17 | 77307215 | 77307239 | + | 25 | CTCTGGCCCCACCCAGCTCATGGGT |
| chr17 | 77307216 | 77307240 | + | 25 | TCTGGCCCCACCCAGCTCATGGGTG |
| chr17 | 77307217 | 77307241 | + | 25 | CTGGCCCCACCCAGCTCATGGGTGT |
| chr17 | 77307218 | 77307242 | + | 25 | TGGCCCCACCCAGCTCATGGGTGTG |
| chr17 | 77307219 | 77307243 | + | 25 | GGCCCCACCCAGCTCATGGGTGTGT |
| chr17 | 77307220 | 77307244 | + | 25 | GCCCCACCCAGCTCATGGGTGTGTT |
| chr17 | 77307221 | 77307245 | + | 25 | CCCCACCCAGCTCATGGGTGTGTTT |
| chr17 | 77307222 | 77307246 | + | 25 | CCCACCCAGCTCATGGGTGTGTTTG |
| chr17 | 77307223 | 77307247 | + | 25 | CCACCCAGCTCATGGGTGTGTTTGT |

**Table 28: 25-mer target-specific ASOs**

| **CHR** | **START** | **END** | **STRAND** | **kmer** | **SEQUENCE** |
|---|---|---|---|---|---|
| chr11 | 85717432 | 85717456 | + | 25 | ATATGTAAAGTGGAATGTTTAGTCA |
| chr11 | 85717433 | 85717457 | + | 25 | TATGTAAAGTGGAATGTTTAGTCAT |
| chr11 | 85717434 | 85717458 | + | 25 | ATGTAAAGTGGAATGTTTAGTCATT |
| chr11 | 85717435 | 85717459 | + | 25 | TGTAAAGTGGAATGTTTAGTCATTT |
| chr11 | 85717436 | 85717460 | + | 25 | GTAAAGTGGAATGTTTAGTCATTTG |
| chr11 | 85717437 | 85717461 | + | 25 | TAAAGTGGAATGTTTAGTCATTTGT |
| chr11 | 85717438 | 85717462 | + | 25 | AAAGTGGAATGTTTAGTCATTTGTG |
| chr11 | 85717439 | 85717463 | + | 25 | AAGTGGAATGTTTAGTCATTTGTGA |
| chr11 | 85717440 | 85717464 | + | 25 | AGTGGAATGTTTAGTCATTTGTGAG |
| chr11 | 85717441 | 85717465 | + | 25 | GTGGAATGTTTAGTCATTTGTGAGA |
| chr11 | 85717442 | 85717466 | + | 25 | TGGAATGTTTAGTCATTTGTGAGAA |
| chr11 | 85717443 | 85717467 | + | 25 | GGAATGTTTAGTCATTTGTGAGAAA |
| chr11 | 85717444 | 85717468 | + | 25 | GAATGTTTAGTCATTTGTGAGAAAG |
| chr11 | 85717445 | 85717469 | + | 25 | AATGTTTAGTCATTTGTGAGAAAGA |
| chr11 | 85717446 | 85717470 | + | 25 | ATGTTTAGTCATTTGTGAGAAAGAT |
| chr11 | 85717447 | 85717471 | + | 25 | TGTTTAGTCATTTGTGAGAAAGATC |
| chr11 | 85717448 | 85717472 | + | 25 | GTTTAGTCATTTGTGAGAAAGATCC |
| chr11 | 85717449 | 85717473 | + | 25 | TTTAGTCATTTGTGAGAAAGATCCT |
| chr11 | 85717450 | 85717474 | + | 25 | TTAGTCATTTGTGAGAAAGATCCTG |
| chr11 | 85717451 | 85717475 | + | 25 | TAGTCATTTGTGAGAAAGATCCTGC |
| chr11 | 85717452 | 85717476 | + | 25 | AGTCATTTGTGAGAAAGATCCTGCT |
| chr11 | 85717453 | 85717477 | + | 25 | GTCATTTGTGAGAAAGATCCTGCTA |
| chr11 | 85717454 | 85717478 | + | 25 | TCATTTGTGAGAAAGATCCTGCTAC |
| chr11 | 85717455 | 85717479 | + | 25 | CATTTGTGAGAAAGATCCTGCTACT |
| chr11 | 85717456 | 85717480 | + | 25 | ATTTGTGAGAAAGATCCTGCTACTC |
| chr11 | 85717457 | 85717481 | + | 25 | TTTGTGAGAAAGATCCTGCTACTCA |
| chr11 | 85717458 | 85717482 | + | 25 | TTGTGAGAAAGATCCTGCTACTCAC |
| chr11 | 85717459 | 85717483 | + | 25 | TGTGAGAAAGATCCTGCTACTCACT |
| chr11 | 85717460 | 85717484 | + | 25 | GTGAGAAAGATCCTGCTACTCACTT |
| chr11 | 85717461 | 85717485 | + | 25 | TGAGAAAGATCCTGCTACTCACTTC |
| chr11 | 85717462 | 85717486 | + | 25 | GAGAAAGATCCTGCTACTCACTTCT |
| chr11 | 85717463 | 85717487 | + | 25 | AGAAAGATCCTGCTACTCACTTCTT |
| chr11 | 85717464 | 85717488 | + | 25 | GAAAGATCCTGCTACTCACTTCTTG |
| chr11 | 85717465 | 85717489 | + | 25 | AAAGATCCTGCTACTCACTTCTTGG |
| chr11 | 85717466 | 85717490 | + | 25 | AAGATCCTGCTACTCACTTCTTGGT |
| chr11 | 85717467 | 85717491 | + | 25 | AGATCCTGCTACTCACTTCTTGGTA |
| chr11 | 85717468 | 85717492 | + | 25 | GATCCTGCTACTCACTTCTTGGTAC |
| chr11 | 85717469 | 85717493 | + | 25 | ATCCTGCTACTCACTTCTTGGTACG |
| chr11 | 85717470 | 85717494 | + | 25 | TCCTGCTACTCACTTCTTGGTACGC |
| chr11 | 85717471 | 85717495 | + | 25 | CCTGCTACTCACTTCTTGGTACGCA |
| chr11 | 85717472 | 85717496 | + | 25 | CTGCTACTCACTTCTTGGTACGCAT |
| chr11 | 85717473 | 85717497 | + | 25 | TGCTACTCACTTCTTGGTACGCATT |
| chr11 | 85717474 | 85717498 | + | 25 | GCTACTCACTTCTTGGTACGCATTC |
| chr11 | 85717475 | 85717499 | + | 25 | CTACTCACTTCTTGGTACGCATTCA |
| chr11 | 85717476 | 85717500 | + | 25 | TACTCACTTCTTGGTACGCATTCAT |
| chr11 | 85717477 | 85717501 | + | 25 | ACTCACTTCTTGGTACGCATTCATT |
| chr11 | 85717478 | 85717502 | + | 25 | CTCACTTCTTGGTACGCATTCATTT |
| chr11 | 85717479 | 85717503 | + | 25 | TCACTTCTTGGTACGCATTCATTTG |
| chr11 | 85717480 | 85717504 | + | 25 | CACTTCTTGGTACGCATTCATTTGT |
| chr11 | 85717481 | 85717505 | + | 25 | ACTTCTTGGTACGCATTCATTTGTA |
| chr11 | 85717482 | 85717506 | + | 25 | CTTCTTGGTACGCATTCATTTGTAA |
| chr11 | 85717483 | 85717507 | + | 25 | TTCTTGGTACGCATTCATTTGTAAC |
| chr11 | 85717484 | 85717508 | + | 25 | TCTTGGTACGCATTCATTTGTAACT |
| chr11 | 85717485 | 85717509 | + | 25 | CTTGGTACGCATTCATTTGTAACTG |
| chr11 | 85717486 | 85717510 | + | 25 | TTGGTACGCATTCATTTGTAACTGG |
| chr11 | 85717487 | 85717511 | + | 25 | TGGTACGCATTCATTTGTAACTGGC |
| chr11 | 85717488 | 85717512 | + | 25 | GGTACGCATTCATTTGTAACTGGCT |
| chr11 | 85717489 | 85717513 | + | 25 | GTACGCATTCATTTGTAACTGGCTT |
| chr11 | 85717490 | 85717514 | + | 25 | TACGCATTCATTTGTAACTGGCTTC |
| chr11 | 85717491 | 85717515 | + | 25 | ACGCATTCATTTGTAACTGGCTTCT |
| chr11 | 85717492 | 85717516 | + | 25 | CGCATTCATTTGTAACTGGCTTCTG |
| chr11 | 85717493 | 85717517 | + | 25 | GCATTCATTTGTAACTGGCTTCTGA |
| chr11 | 85717494 | 85717518 | + | 25 | CATTCATTTGTAACTGGCTTCTGAT |
| chr11 | 85717495 | 85717519 | + | 25 | ATTCATTTGTAACTGGCTTCTGATC |
| chr11 | 85717496 | 85717520 | + | 25 | TTCATTTGTAACTGGCTTCTGATCT |
| chr11 | 85717497 | 85717521 | + | 25 | TCATTTGTAACTGGCTTCTGATCTG |
| chr11 | 85717498 | 85717522 | + | 25 | CATTTGTAACTGGCTTCTGATCTGG |
| chr11 | 85717499 | 85717523 | + | 25 | ATTTGTAACTGGCTTCTGATCTGGT |
| chr11 | 85717500 | 85717524 | + | 25 | TTTGTAACTGGCTTCTGATCTGGTT |
| chr11 | 85717501 | 85717525 | + | 25 | TTGTAACTGGCTTCTGATCTGGTTT |
| chr11 | 85717502 | 85717526 | + | 25 | TGTAACTGGCTTCTGATCTGGTTTC |
| chr11 | 85717503 | 85717527 | + | 25 | GTAACTGGCTTCTGATCTGGTTTCT |
| chr11 | 85717504 | 85717528 | + | 25 | TAACTGGCTTCTGATCTGGTTTCTC |
| chr11 | 85717505 | 85717529 | + | 25 | AACTGGCTTCTGATCTGGTTTCTCA |
| chr11 | 85717506 | 85717530 | + | 25 | ACTGGCTTCTGATCTGGTTTCTCAT |
| chr11 | 85717507 | 85717531 | + | 25 | CTGGCTTCTGATCTGGTTTCTCATC |
| chr11 | 85717508 | 85717532 | + | 25 | TGGCTTCTGATCTGGTTTCTCATCT |
| chr11 | 85717509 | 85717533 | + | 25 | GGCTTCTGATCTGGTTTCTCATCTA |
| chr11 | 85717510 | 85717534 | + | 25 | GCTTCTGATCTGGTTTCTCATCTAC |
| chr11 | 85717511 | 85717535 | + | 25 | CTTCTGATCTGGTTTCTCATCTACT |
| chr11 | 85717512 | 85717536 | + | 25 | TTCTGATCTGGTTTCTCATCTACTC |
| chr11 | 85717513 | 85717537 | + | 25 | TCTGATCTGGTTTCTCATCTACTCA |
| chr11 | 85717514 | 85717538 | + | 25 | CTGATCTGGTTTCTCATCTACTCAG |
| chr11 | 85717515 | 85717539 | + | 25 | TGATCTGGTTTCTCATCTACTCAGG |
| chr11 | 85717516 | 85717540 | + | 25 | GATCTGGTTTCTCATCTACTCAGGA |
| chr11 | 85717517 | 85717541 | + | 25 | ATCTGGTTTCTCATCTACTCAGGAG |
| chr11 | 85717518 | 85717542 | + | 25 | TCTGGTTTCTCATCTACTCAGGAGG |
| chr11 | 85717519 | 85717543 | + | 25 | CTGGTTTCTCATCTACTCAGGAGGG |
| chr11 | 85717520 | 85717544 | + | 25 | TGGTTTCTCATCTACTCAGGAGGGC |
| chr11 | 85717521 | 85717545 | + | 25 | GGTTTCTCATCTACTCAGGAGGGCA |
| chr11 | 85717522 | 85717546 | + | 25 | GTTTCTCATCTACTCAGGAGGGCAA |
| chr11 | 85717523 | 85717547 | + | 25 | TTTCTCATCTACTCAGGAGGGCAAC |
| chr11 | 85717524 | 85717548 | + | 25 | TTCTCATCTACTCAGGAGGGCAACA |
| chr11 | 85717525 | 85717549 | + | 25 | TCTCATCTACTCAGGAGGGCAACAT |
| chr11 | 85717526 | 85717550 | + | 25 | CTCATCTACTCAGGAGGGCAACATT |
| chr11 | 85717527 | 85717551 | + | 25 | TCATCTACTCAGGAGGGCAACATTG |
| chr11 | 85717528 | 85717552 | + | 25 | CATCTACTCAGGAGGGCAACATTGA |
| chr11 | 85717529 | 85717553 | + | 25 | ATCTACTCAGGAGGGCAACATTGAG |
| chr11 | 85717530 | 85717554 | + | 25 | TCTACTCAGGAGGGCAACATTGAGA |
| chr11 | 85717531 | 85717555 | + | 25 | CTACTCAGGAGGGCAACATTGAGAA |
| chr11 | 85717532 | 85717556 | + | 25 | TACTCAGGAGGGCAACATTGAGAAT |
| chr11 | 85717533 | 85717557 | + | 25 | ACTCAGGAGGGCAACATTGAGAATA |
| chr11 | 85717534 | 85717558 | + | 25 | CTCAGGAGGGCAACATTGAGAATAA |
| chr11 | 85717535 | 85717559 | + | 25 | TCAGGAGGGCAACATTGAGAATAAA |
| chr11 | 85717536 | 85717560 | + | 25 | CAGGAGGGCAACATTGAGAATAAAT |
| chr11 | 85717537 | 85717561 | + | 25 | AGGAGGGCAACATTGAGAATAAATA |
| chr11 | 85717538 | 85717562 | + | 25 | GGAGGGCAACATTGAGAATAAATAC |
| chr11 | 85717539 | 85717563 | + | 25 | GAGGGCAACATTGAGAATAAATACC |
| chr11 | 85717540 | 85717564 | + | 25 | AGGGCAACATTGAGAATAAATACCA |
| chr11 | 85717541 | 85717565 | + | 25 | GGGCAACATTGAGAATAAATACCAT |
| chr11 | 85717542 | 85717566 | + | 25 | GGCAACATTGAGAATAAATACCATT |
| chr11 | 85717543 | 85717567 | + | 25 | GCAACATTGAGAATAAATACCATTT |
| chr11 | 85717544 | 85717568 | + | 25 | CAACATTGAGAATAAATACCATTTT |
| chr11 | 85717545 | 85717569 | + | 25 | AACATTGAGAATAAATACCATTTTA |
| chr11 | 85717546 | 85717570 | + | 25 | ACATTGAGAATAAATACCATTTTAA |
| chr11 | 85717547 | 85717571 | + | 25 | CATTGAGAATAAATACCATTTTAAC |
| chr11 | 85717548 | 85717572 | + | 25 | ATTGAGAATAAATACCATTTTAACA |
| chr11 | 85717549 | 85717573 | + | 25 | TTGAGAATAAATACCATTTTAACAG |
| chr11 | 85717550 | 85717574 | + | 25 | TGAGAATAAATACCATTTTAACAGA |
| chr11 | 85717551 | 85717575 | + | 25 | GAGAATAAATACCATTTTAACAGAA |
| chr11 | 85717552 | 85717576 | + | 25 | AGAATAAATACCATTTTAACAGAAG |
| chr11 | 85717553 | 85717577 | + | 25 | GAATAAATACCATTTTAACAGAAGG |
| chr11 | 85717554 | 85717578 | + | 25 | AATAAATACCATTTTAACAGAAGGA |
| chr11 | 85717555 | 85717579 | + | 25 | ATAAATACCATTTTAACAGAAGGAT |
| chr11 | 85717556 | 85717580 | + | 25 | TAAATACCATTTTAACAGAAGGATT |

**Table 29: 25-mer target-specific ASOs**

| **CHR** | **START** | **END** | **STRAND** | **kmer** | **SEQUENCE** |
|---|---|---|---|---|---|
| chr11 | 63903935 | 63903959 | + | 25 | CCGCCCTCACTCACCCCTAACACGG |
| chr11 | 63903936 | 63903960 | + | 25 | CGCCCTCACTCACCCCTAACACGGG |
| chr11 | 63903937 | 63903961 | + | 25 | GCCCTCACTCACCCCTAACACGGGC |
| chr11 | 63903938 | 63903962 | + | 25 | CCCTCACTCACCCCTAACACGGGCC |
| chr11 | 63903939 | 63903963 | + | 25 | CCTCACTCACCCCTAACACGGGCCT |
| chr11 | 63903940 | 63903964 | + | 25 | CTCACTCACCCCTAACACGGGCCTC |
| chr11 | 63903941 | 63903965 | + | 25 | TCACTCACCCCTAACACGGGCCTCT |
| chr11 | 63903942 | 63903966 | + | 25 | CACTCACCCCTAACACGGGCCTCTC |
| chr11 | 63903943 | 63903967 | + | 25 | ACTCACCCCTAACACGGGCCTCTCC |
| chr11 | 63903944 | 63903968 | + | 25 | CTCACCCCTAACACGGGCCTCTCCG |
| chr11 | 63903945 | 63903969 | + | 25 | TCACCCCTAACACGGGCCTCTCCGC |
| chr11 | 63903946 | 63903970 | + | 25 | CACCCCTAACACGGGCCTCTCCGCT |
| chr11 | 63903947 | 63903971 | + | 25 | ACCCCTAACACGGGCCTCTCCGCTG |
| chr11 | 63903948 | 63903972 | + | 25 | CCCCTAACACGGGCCTCTCCGCTGC |
| chr11 | 63903949 | 63903973 | + | 25 | CCCTAACACGGGCCTCTCCGCTGCT |
| chr11 | 63903950 | 63903974 | + | 25 | CCTAACACGGGCCTCTCCGCTGCTT |
| chr11 | 63903951 | 63903975 | + | 25 | CTAACACGGGCCTCTCCGCTGCTTT |
| chr11 | 63903952 | 63903976 | + | 25 | TAACACGGGCCTCTCCGCTGCTTTT |
| chr11 | 63903953 | 63903977 | + | 25 | AACACGGGCCTCTCCGCTGCTTTTG |
| chr11 | 63903954 | 63903978 | + | 25 | ACACGGGCCTCTCCGCTGCTTTTGT |
| chr11 | 63903955 | 63903979 | + | 25 | CACGGGCCTCTCCGCTGCTTTTGTT |
| chr11 | 63903956 | 63903980 | + | 25 | ACGGGCCTCTCCGCTGCTTTTGTTT |
| chr11 | 63903957 | 63903981 | + | 25 | CGGGCCTCTCCGCTGCTTTTGTTTC |
| chr11 | 63903958 | 63903982 | + | 25 | GGGCCTCTCCGCTGCTTTTGTTTCC |
| chr11 | 63903959 | 63903983 | + | 25 | GGCCTCTCCGCTGCTTTTGTTTCCT |
| chr11 | 63903960 | 63903984 | + | 25 | GCCTCTCCGCTGCTTTTGTTTCCTA |
| chr11 | 63903961 | 63903985 | + | 25 | CCTCTCCGCTGCTTTTGTTTCCTAG |
| chr11 | 63903962 | 63903986 | + | 25 | CTCTCCGCTGCTTTTGTTTCCTAGC |
| chr11 | 63903963 | 63903987 | + | 25 | TCTCCGCTGCTTTTGTTTCCTAGCC |
| chr11 | 63903964 | 63903988 | + | 25 | CTCCGCTGCTTTTGTTTCCTAGCCT |
| chr11 | 63903965 | 63903989 | + | 25 | TCCGCTGCTTTTGTTTCCTAGCCTA |
| chr11 | 63903966 | 63903990 | + | 25 | CCGCTGCTTTTGTTTCCTAGCCTAA |
| chr11 | 63903967 | 63903991 | + | 25 | CGCTGCTTTTGTTTCCTAGCCTAAC |
| chr11 | 63903968 | 63903992 | + | 25 | GCTGCTTTTGTTTCCTAGCCTAACC |
| chr11 | 63903969 | 63903993 | + | 25 | CTGCTTTTGTTTCCTAGCCTAACCA |
| chr11 | 63903970 | 63903994 | + | 25 | TGCTTTTGTTTCCTAGCCTAACCAT |
| chr11 | 63903971 | 63903995 | + | 25 | GCTTTTGTTTCCTAGCCTAACCATG |
| chr11 | 63903972 | 63903996 | + | 25 | CTTTTGTTTCCTAGCCTAACCATGC |
| chr11 | 63903973 | 63903997 | + | 25 | TTTTGTTTCCTAGCCTAACCATGCC |
| chr11 | 63903974 | 63903998 | + | 25 | TTTGTTTCCTAGCCTAACCATGCCA |
| chr11 | 63903975 | 63903999 | + | 25 | TTGTTTCCTAGCCTAACCATGCCAG |
| chr11 | 63903976 | 63904000 | + | 25 | TGTTTCCTAGCCTAACCATGCCAGG |
| chr11 | 63903977 | 63904001 | + | 25 | GTTTCCTAGCCTAACCATGCCAGGG |
| chr11 | 63903978 | 63904002 | + | 25 | TTTCCTAGCCTAACCATGCCAGGGT |
| chr11 | 63903979 | 63904003 | + | 25 | TTCCTAGCCTAACCATGCCAGGGTC |
| chr11 | 63903980 | 63904004 | + | 25 | TCCTAGCCTAACCATGCCAGGGTCC |
| chr11 | 63903981 | 63904005 | + | 25 | CCTAGCCTAACCATGCCAGGGTCCC |
| chr11 | 63903982 | 63904006 | + | 25 | CTAGCCTAACCATGCCAGGGTCCCG |
| chr11 | 63903983 | 63904007 | + | 25 | TAGCCTAACCATGCCAGGGTCCCGG |
| chr11 | 63903984 | 63904008 | + | 25 | AGCCTAACCATGCCAGGGTCCCGGG |
| chr11 | 63903985 | 63904009 | + | 25 | GCCTAACCATGCCAGGGTCCCGGGC |
| chr11 | 63903986 | 63904010 | + | 25 | CCTAACCATGCCAGGGTCCCGGGCC |
| chr11 | 63903987 | 63904011 | + | 25 | CTAACCATGCCAGGGTCCCGGGCCT |
| chr11 | 63903988 | 63904012 | + | 25 | TAACCATGCCAGGGTCCCGGGCCTC |
| chr11 | 63903989 | 63904013 | + | 25 | AACCATGCCAGGGTCCCGGGCCTCC |
| chr11 | 63903990 | 63904014 | + | 25 | ACCATGCCAGGGTCCCGGGCCTCCA |
| chr11 | 63903991 | 63904015 | + | 25 | CCATGCCAGGGTCCCGGGCCTCCAC |
| chr11 | 63903992 | 63904016 | + | 25 | CATGCCAGGGTCCCGGGCCTCCACG |
| chr11 | 63903993 | 63904017 | + | 25 | ATGCCAGGGTCCCGGGCCTCCACGG |
| chr11 | 63903994 | 63904018 | + | 25 | TGCCAGGGTCCCGGGCCTCCACGGC |
| chr11 | 63903995 | 63904019 | + | 25 | GCCAGGGTCCCGGGCCTCCACGGCT |
| chr11 | 63903996 | 63904020 | + | 25 | CCAGGGTCCCGGGCCTCCACGGCTT |
| chr11 | 63903997 | 63904021 | + | 25 | CAGGGTCCCGGGCCTCCACGGCTTC |
| chr11 | 63903998 | 63904022 | + | 25 | AGGGTCCCGGGCCTCCACGGCTTCT |
| chr11 | 63903999 | 63904023 | + | 25 | GGGTCCCGGGCCTCCACGGCTTCTG |
| chr11 | 63904000 | 63904024 | + | 25 | GGTCCCGGGCCTCCACGGCTTCTGC |
| chr11 | 63904001 | 63904025 | + | 25 | GTCCCGGGCCTCCACGGCTTCTGCT |
| chr11 | 63904002 | 63904026 | + | 25 | TCCCGGGCCTCCACGGCTTCTGCTT |
| chr11 | 63904003 | 63904027 | + | 25 | CCCGGGCCTCCACGGCTTCTGCTTC |
| chr11 | 63904004 | 63904028 | + | 25 | CCGGGCCTCCACGGCTTCTGCTTCT |
| chr11 | 63904005 | 63904029 | + | 25 | CGGGCCTCCACGGCTTCTGCTTCTG |
| chr11 | 63904006 | 63904030 | + | 25 | GGGCCTCCACGGCTTCTGCTTCTGC |
| chr11 | 63904007 | 63904031 | + | 25 | GGCCTCCACGGCTTCTGCTTCTGCC |
| chr11 | 63904008 | 63904032 | + | 25 | GCCTCCACGGCTTCTGCTTCTGCCG |
| chr11 | 63904009 | 63904033 | + | 25 | CCTCCACGGCTTCTGCTTCTGCCGC |
| chr11 | 63904010 | 63904034 | + | 25 | CTCCACGGCTTCTGCTTCTGCCGCA |
| chr11 | 63904011 | 63904035 | + | 25 | TCCACGGCTTCTGCTTCTGCCGCAG |
| chr11 | 63904012 | 63904036 | + | 25 | CCACGGCTTCTGCTTCTGCCGCAGT |
| chr11 | 63904013 | 63904037 | + | 25 | CACGGCTTCTGCTTCTGCCGCAGTC |
| chr11 | 63904014 | 63904038 | + | 25 | ACGGCTTCTGCTTCTGCCGCAGTCT |
| chr11 | 63904015 | 63904039 | + | 25 | CGGCTTCTGCTTCTGCCGCAGTCTC |
| chr11 | 63904016 | 63904040 | + | 25 | GGCTTCTGCTTCTGCCGCAGTCTCT |
| chr11 | 63904017 | 63904041 | + | 25 | GCTTCTGCTTCTGCCGCAGTCTCTG |
| chr11 | 63904018 | 63904042 | + | 25 | CTTCTGCTTCTGCCGCAGTCTCTGC |
| chr11 | 63904019 | 63904043 | + | 25 | TTCTGCTTCTGCCGCAGTCTCTGCG |
| chr11 | 63904020 | 63904044 | + | 25 | TCTGCTTCTGCCGCAGTCTCTGCGG |
| chr11 | 63904021 | 63904045 | + | 25 | CTGCTTCTGCCGCAGTCTCTGCGGC |
| chr11 | 63904022 | 63904046 | + | 25 | TGCTTCTGCCGCAGTCTCTGCGGCC |
| chr11 | 63904023 | 63904047 | + | 25 | GCTTCTGCCGCAGTCTCTGCGGCCC |
| chr11 | 63904024 | 63904048 | + | 25 | CTTCTGCCGCAGTCTCTGCGGCCCG |
| chr11 | 63904025 | 63904049 | + | 25 | TTCTGCCGCAGTCTCTGCGGCCCGG |
| chr11 | 63904026 | 63904050 | + | 25 | TCTGCCGCAGTCTCTGCGGCCCGGC |
| chr11 | 63904027 | 63904051 | + | 25 | CTGCCGCAGTCTCTGCGGCCCGGCC |
| chr11 | 63904028 | 63904052 | + | 25 | TGCCGCAGTCTCTGCGGCCCGGCCC |
| chr11 | 63904029 | 63904053 | + | 25 | GCCGCAGTCTCTGCGGCCCGGCCCC |
| chr11 | 63904030 | 63904054 | + | 25 | CCGCAGTCTCTGCGGCCCGGCCCCG |
| chr11 | 63904031 | 63904055 | + | 25 | CGCAGTCTCTGCGGCCCGGCCCCGC |
| chr11 | 63904032 | 63904056 | + | 25 | GCAGTCTCTGCGGCCCGGCCCCGCC |
| chr11 | 63904033 | 63904057 | + | 25 | CAGTCTCTGCGGCCCGGCCCCGCCA |
| chr11 | 63904034 | 63904058 | + | 25 | AGTCTCTGCGGCCCGGCCCCGCCAG |
| chr11 | 63904035 | 63904059 | + | 25 | GTCTCTGCGGCCCGGCCCCGCCAGC |
| chr11 | 63904036 | 63904060 | + | 25 | TCTCTGCGGCCCGGCCCCGCCAGCA |
| chr11 | 63904037 | 63904061 | + | 25 | CTCTGCGGCCCGGCCCCGCCAGCAC |
| chr11 | 63904038 | 63904062 | + | 25 | TCTGCGGCCCGGCCCCGCCAGCACC |
| chr11 | 63904039 | 63904063 | + | 25 | CTGCGGCCCGGCCCCGCCAGCACCA |
| chr11 | 63904040 | 63904064 | + | 25 | TGCGGCCCGGCCCCGCCAGCACCAG |
| chr11 | 63904041 | 63904065 | + | 25 | GCGGCCCGGCCCCGCCAGCACCAGA |
| chr11 | 63904042 | 63904066 | + | 25 | CGGCCCGGCCCCGCCAGCACCAGAA |
| chr11 | 63904043 | 63904067 | + | 25 | GGCCCGGCCCCGCCAGCACCAGAAA |
| chr11 | 63904044 | 63904068 | + | 25 | GCCCGGCCCCGCCAGCACCAGAAAT |
| chr11 | 63904045 | 63904069 | + | 25 | CCCGGCCCCGCCAGCACCAGAAATC |
| chr11 | 63904046 | 63904070 | + | 25 | CCGGCCCCGCCAGCACCAGAAATCC |
| chr11 | 63904047 | 63904071 | + | 25 | CGGCCCCGCCAGCACCAGAAATCCA |
| chr11 | 63904048 | 63904072 | + | 25 | GGCCCCGCCAGCACCAGAAATCCAT |
| chr11 | 63904049 | 63904073 | + | 25 | GCCCCGCCAGCACCAGAAATCCATG |
| chr11 | 63904050 | 63904074 | + | 25 | CCCCGCCAGCACCAGAAATCCATGT |
| chr11 | 63904051 | 63904075 | + | 25 | CCCGCCAGCACCAGAAATCCATGTC |
| chr11 | 63904052 | 63904076 | + | 25 | CCGCCAGCACCAGAAATCCATGTCG |
| chr11 | 63904053 | 63904077 | + | 25 | CGCCAGCACCAGAAATCCATGTCGG |
| chr11 | 63904054 | 63904078 | + | 25 | GCCAGCACCAGAAATCCATGTCGGC |
| chr11 | 63904055 | 63904079 | + | 25 | CCAGCACCAGAAATCCATGTCGGCC |
| chr11 | 63904056 | 63904080 | + | 25 | CAGCACCAGAAATCCATGTCGGCCT |
| chr11 | 63904057 | 63904081 | + | 25 | AGCACCAGAAATCCATGTCGGCCTC |
| chr11 | 63904058 | 63904082 | + | 25 | GCACCAGAAATCCATGTCGGCCTCC |
| chr11 | 63904059 | 63904083 | + | 25 | CACCAGAAATCCATGTCGGCCTCCG |
| chr11 | 63904060 | 63904084 | + | 25 | ACCAGAAATCCATGTCGGCCTCCGT |
| chr11 | 63904061 | 63904085 | + | 25 | CCAGAAATCCATGTCGGCCTCCGTG |
| chr11 | 63904062 | 63904086 | + | 25 | CAGAAATCCATGTCGGCCTCCGTGC |
| chr11 | 63904063 | 63904087 | + | 25 | AGAAATCCATGTCGGCCTCCGTGCA |
| chr11 | 63904064 | 63904088 | + | 25 | GAAATCCATGTCGGCCTCCGTGCAC |
| chr11 | 63904065 | 63904089 | + | 25 | AAATCCATGTCGGCCTCCGTGCACC |
| chr11 | 63904066 | 63904090 | + | 25 | AATCCATGTCGGCCTCCGTGCACCC |
| chr11 | 63904067 | 63904091 | + | 25 | ATCCATGTCGGCCTCCGTGCACCCC |
| chr11 | 63904068 | 63904092 | + | 25 | TCCATGTCGGCCTCCGTGCACCCCA |
| chr11 | 63904069 | 63904093 | + | 25 | CCATGTCGGCCTCCGTGCACCCCAA |
| chr11 | 63904070 | 63904094 | + | 25 | CATGTCGGCCTCCGTGCACCCCAAC |
| chr11 | 63904071 | 63904095 | + | 25 | ATGTCGGCCTCCGTGCACCCCAACA |
| chr11 | 63904072 | 63904096 | + | 25 | TGTCGGCCTCCGTGCACCCCAACAA |
| chr11 | 63904073 | 63904097 | + | 25 | GTCGGCCTCCGTGCACCCCAACAAG |
| chr11 | 63904074 | 63904098 | + | 25 | TCGGCCTCCGTGCACCCCAACAAGG |
| chr11 | 63904075 | 63904099 | + | 25 | CGGCCTCCGTGCACCCCAACAAGGC |
| chr11 | 63904076 | 63904100 | + | 25 | GGCCTCCGTGCACCCCAACAAGGCC |
| chr11 | 63904077 | 63904101 | + | 25 | GCCTCCGTGCACCCCAACAAGGCCT |
| chr11 | 63904078 | 63904102 | + | 25 | CCTCCGTGCACCCCAACAAGGCCTC |
| chr11 | 63904079 | 63904103 | + | 25 | CTCCGTGCACCCCAACAAGGCCTCT |
| chr11 | 63904080 | 63904104 | + | 25 | TCCGTGCACCCCAACAAGGCCTCTG |
| chr11 | 63904081 | 63904105 | + | 25 | CCGTGCACCCCAACAAGGCCTCTGG |
| chr11 | 63904082 | 63904106 | + | 25 | CGTGCACCCCAACAAGGCCTCTGGG |
| chr11 | 63904083 | 63904107 | + | 25 | GTGCACCCCAACAAGGCCTCTGGGC |
| chr11 | 63904084 | 63904108 | + | 25 | TGCACCCCAACAAGGCCTCTGGGCT |
| chr11 | 63904085 | 63904109 | + | 25 | GCACCCCAACAAGGCCTCTGGGCTG |
| chr11 | 63904086 | 63904110 | + | 25 | CACCCCAACAAGGCCTCTGGGCTGC |
| chr11 | 63904087 | 63904111 | + | 25 | ACCCCAACAAGGCCTCTGGGCTGCC |
| chr11 | 63904088 | 63904112 | + | 25 | CCCCAACAAGGCCTCTGGGCTGCCC |
| chr11 | 63904089 | 63904113 | + | 25 | CCCAACAAGGCCTCTGGGCTGCCCC |
| chr11 | 63904090 | 63904114 | + | 25 | CCAACAAGGCCTCTGGGCTGCCCCC |
| chr11 | 63904091 | 63904115 | + | 25 | CAACAAGGCCTCTGGGCTGCCCCCC |
| chr11 | 63904092 | 63904116 | + | 25 | AACAAGGCCTCTGGGCTGCCCCCCA |
| chr11 | 63904093 | 63904117 | + | 25 | ACAAGGCCTCTGGGCTGCCCCCCAC |
| chr11 | 63904094 | 63904118 | + | 25 | CAAGGCCTCTGGGCTGCCCCCCACG |
| chr11 | 63904095 | 63904119 | + | 25 | AAGGCCTCTGGGCTGCCCCCCACGG |
| chr11 | 63904096 | 63904120 | + | 25 | AGGCCTCTGGGCTGCCCCCCACGGA |
| chr11 | 63904097 | 63904121 | + | 25 | GGCCTCTGGGCTGCCCCCCACGGAG |
| chr11 | 63904098 | 63904122 | + | 25 | GCCTCTGGGCTGCCCCCCACGGAGA |
| chr11 | 63904099 | 63904123 | + | 25 | CCTCTGGGCTGCCCCCCACGGAGAG |
| chr11 | 63904100 | 63904124 | + | 25 | CTCTGGGCTGCCCCCCACGGAGAGT |
| chr11 | 63904101 | 63904125 | + | 25 | TCTGGGCTGCCCCCCACGGAGAGTA |
| chr11 | 63904102 | 63904126 | + | 25 | CTGGGCTGCCCCCCACGGAGAGTAA |
| chr11 | 63904103 | 63904127 | + | 25 | TGGGCTGCCCCCCACGGAGAGTAAC |
| chr11 | 63904104 | 63904128 | + | 25 | GGGCTGCCCCCCACGGAGAGTAACT |
| chr11 | 63904105 | 63904129 | + | 25 | GGCTGCCCCCCACGGAGAGTAACTG |
| chr11 | 63904106 | 63904130 | + | 25 | GCTGCCCCCCACGGAGAGTAACTGT |
| chr11 | 63904107 | 63904131 | + | 25 | CTGCCCCCCACGGAGAGTAACTGTG |
| chr11 | 63904108 | 63904132 | + | 25 | TGCCCCCCACGGAGAGTAACTGTGA |
| chr11 | 63904109 | 63904133 | + | 25 | GCCCCCCACGGAGAGTAACTGTGAG |
| chr11 | 63904110 | 63904134 | + | 25 | CCCCCCACGGAGAGTAACTGTGAGG |
| chr11 | 63904111 | 63904135 | + | 25 | CCCCCACGGAGAGTAACTGTGAGGT |
| chr11 | 63904112 | 63904136 | + | 25 | CCCCACGGAGAGTAACTGTGAGGTG |
| chr11 | 63904113 | 63904137 | + | 25 | CCCACGGAGAGTAACTGTGAGGTGC |
| chr11 | 63904114 | 63904138 | + | 25 | CCACGGAGAGTAACTGTGAGGTGCC |
| chr11 | 63904115 | 63904139 | + | 25 | CACGGAGAGTAACTGTGAGGTGCCG |
| chr11 | 63904116 | 63904140 | + | 25 | ACGGAGAGTAACTGTGAGGTGCCGC |
| chr11 | 63904117 | 63904141 | + | 25 | CGGAGAGTAACTGTGAGGTGCCGCG |
| chr11 | 63904118 | 63904142 | + | 25 | GGAGAGTAACTGTGAGGTGCCGCGG |
| chr11 | 63904119 | 63904143 | + | 25 | GAGAGTAACTGTGAGGTGCCGCGGC |
| chr11 | 63904120 | 63904144 | + | 25 | AGAGTAACTGTGAGGTGCCGCGGCC |
| chr11 | 63904121 | 63904145 | + | 25 | GAGTAACTGTGAGGTGCCGCGGCCC |
| chr11 | 63904122 | 63904146 | + | 25 | AGTAACTGTGAGGTGCCGCGGCCCA |
| chr11 | 63904123 | 63904147 | + | 25 | GTAACTGTGAGGTGCCGCGGCCCAG |
| chr11 | 63904124 | 63904148 | + | 25 | TAACTGTGAGGTGCCGCGGCCCAGG |
| chr11 | 63904125 | 63904149 | + | 25 | AACTGTGAGGTGCCGCGGCCCAGGC |
| chr11 | 63904126 | 63904150 | + | 25 | ACTGTGAGGTGCCGCGGCCCAGGCA |
| chr11 | 63904127 | 63904151 | + | 25 | CTGTGAGGTGCCGCGGCCCAGGCAA |
| chr11 | 63904128 | 63904152 | + | 25 | TGTGAGGTGCCGCGGCCCAGGCAAG |
| chr11 | 63904129 | 63904153 | + | 25 | GTGAGGTGCCGCGGCCCAGGCAAGT |
| chr11 | 63904130 | 63904154 | + | 25 | TGAGGTGCCGCGGCCCAGGCAAGTG |
| chr11 | 63904131 | 63904155 | + | 25 | GAGGTGCCGCGGCCCAGGCAAGTGT |
| chr11 | 63904132 | 63904156 | + | 25 | AGGTGCCGCGGCCCAGGCAAGTGTG |
| chr11 | 63904133 | 63904157 | + | 25 | GGTGCCGCGGCCCAGGCAAGTGTGC |
| chr11 | 63904134 | 63904158 | + | 25 | GTGCCGCGGCCCAGGCAAGTGTGCT |
| chr11 | 63904135 | 63904159 | + | 25 | TGCCGCGGCCCAGGCAAGTGTGCTG |
| chr11 | 63904136 | 63904160 | + | 25 | GCCGCGGCCCAGGCAAGTGTGCTGG |
| chr11 | 63904137 | 63904161 | + | 25 | CCGCGGCCCAGGCAAGTGTGCTGGG |
| chr11 | 63904138 | 63904162 | + | 25 | CGCGGCCCAGGCAAGTGTGCTGGGG |
| chr11 | 63904139 | 63904163 | + | 25 | GCGGCCCAGGCAAGTGTGCTGGGGC |
| chr11 | 63904140 | 63904164 | + | 25 | CGGCCCAGGCAAGTGTGCTGGGGCA |
| chr11 | 63904141 | 63904165 | + | 25 | GGCCCAGGCAAGTGTGCTGGGGCAG |
| chr11 | 63904142 | 63904166 | + | 25 | GCCCAGGCAAGTGTGCTGGGGCAGC |
| chr11 | 63904143 | 63904167 | + | 25 | CCCAGGCAAGTGTGCTGGGGCAGCT |
| chr11 | 63904144 | 63904168 | + | 25 | CCAGGCAAGTGTGCTGGGGCAGCTG |
| chr11 | 63904145 | 63904169 | + | 25 | CAGGCAAGTGTGCTGGGGCAGCTGG |
| chr11 | 63904146 | 63904170 | + | 25 | AGGCAAGTGTGCTGGGGCAGCTGGT |
| chr11 | 63904147 | 63904171 | + | 25 | GGCAAGTGTGCTGGGGCAGCTGGTG |
| chr11 | 63904148 | 63904172 | + | 25 | GCAAGTGTGCTGGGGCAGCTGGTGC |
| chr11 | 63904149 | 63904173 | + | 25 | CAAGTGTGCTGGGGCAGCTGGTGCA |
| chr11 | 63904150 | 63904174 | + | 25 | AAGTGTGCTGGGGCAGCTGGTGCAC |
| chr11 | 63904151 | 63904175 | + | 25 | AGTGTGCTGGGGCAGCTGGTGCACC |
| chr11 | 63904152 | 63904176 | + | 25 | GTGTGCTGGGGCAGCTGGTGCACCT |
| chr11 | 63904153 | 63904177 | + | 25 | TGTGCTGGGGCAGCTGGTGCACCTG |
| chr11 | 63904154 | 63904178 | + | 25 | GTGCTGGGGCAGCTGGTGCACCTGC |
| chr11 | 63904155 | 63904179 | + | 25 | TGCTGGGGCAGCTGGTGCACCTGCT |
| chr11 | 63904156 | 63904180 | + | 25 | GCTGGGGCAGCTGGTGCACCTGCTG |
| chr11 | 63904157 | 63904181 | + | 25 | CTGGGGCAGCTGGTGCACCTGCTGC |
| chr11 | 63904158 | 63904182 | + | 25 | TGGGGCAGCTGGTGCACCTGCTGCC |
| chr11 | 63904159 | 63904183 | + | 25 | GGGGCAGCTGGTGCACCTGCTGCCC |
| chr11 | 63904160 | 63904184 | + | 25 | GGGCAGCTGGTGCACCTGCTGCCCT |
| chr11 | 63904161 | 63904185 | + | 25 | GGCAGCTGGTGCACCTGCTGCCCTC |
| chr11 | 63904162 | 63904186 | + | 25 | GCAGCTGGTGCACCTGCTGCCCTCA |
| chr11 | 63904163 | 63904187 | + | 25 | CAGCTGGTGCACCTGCTGCCCTCAG |
| chr11 | 63904164 | 63904188 | + | 25 | AGCTGGTGCACCTGCTGCCCTCAGC |
| chr11 | 63904165 | 63904189 | + | 25 | GCTGGTGCACCTGCTGCCCTCAGCC |
| chr11 | 63904166 | 63904190 | + | 25 | CTGGTGCACCTGCTGCCCTCAGCCC |
| chr11 | 63904167 | 63904191 | + | 25 | TGGTGCACCTGCTGCCCTCAGCCCA |
| chr11 | 63904168 | 63904192 | + | 25 | GGTGCACCTGCTGCCCTCAGCCCAC |
| chr11 | 63904169 | 63904193 | + | 25 | GTGCACCTGCTGCCCTCAGCCCACC |
| chr11 | 63904170 | 63904194 | + | 25 | TGCACCTGCTGCCCTCAGCCCACCC |
| chr11 | 63904171 | 63904195 | + | 25 | GCACCTGCTGCCCTCAGCCCACCCT |
| chr11 | 63904172 | 63904196 | + | 25 | CACCTGCTGCCCTCAGCCCACCCTA |
| chr11 | 63904173 | 63904197 | + | 25 | ACCTGCTGCCCTCAGCCCACCCTAC |

**Table 30: 25-mer target-specific ASOs**

| **CHR** | **START** | **END** | **STRAND** | **kmer** | SEQUENCE |
|---|---|---|---|---|---|
| chr7 | 117134073 | 117134097 | + | 25 | AATGTTCCTATCAATTTTCATTTTA |
| chr7 | 117134074 | 117134098 | + | 25 | ATGTTCCTATCAATTTTCATTTTAC |
| chr7 | 117134075 | 117134099 | + | 25 | TGTTCCTATCAATTTTCATTTTACC |
| chr7 | 117134076 | 117134100 | + | 25 | GTTCCTATCAATTTTCATTTTACCA |
| chr7 | 117134077 | 117134101 | + | 25 | TTCCTATCAATTTTCATTTTACCAA |
| chr7 | 117134078 | 117134102 | + | 25 | TCCTATCAATTTTCATTTTACCAAC |
| chr7 | 117134079 | 117134103 | + | 25 | CCTATCAATTTTCATTTTACCAACT |
| chr7 | 117134080 | 117134104 | + | 25 | CTATCAATTTTCATTTTACCAACTA |
| chr7 | 117134081 | 117134105 | + | 25 | TATCAATTTTCATTTTACCAACTAT |
| chr7 | 117134082 | 117134106 | + | 25 | ATCAATTTTCATTTTACCAACTATT |
| chr7 | 117134083 | 117134107 | + | 25 | TCAATTTTCATTTTACCAACTATTT |
| chr7 | 117134084 | 117134108 | + | 25 | CAATTTTCATTTTACCAACTATTTT |
| chr7 | 117134085 | 117134109 | + | 25 | AATTTTCATTTTACCAACTATTTTT |
| chr7 | 117134086 | 117134110 | + | 25 | ATTTTCATTTTACCAACTATTTTTT |
| chr7 | 117134087 | 117134111 | + | 25 | TTTTCATTTTACCAACTATTTTTTT |
| chr7 | 117134088 | 117134112 | + | 25 | TTTCATTTTACCAACTATTTTTTTC |
| chr7 | 117134089 | 117134113 | + | 25 | TTCATTTTACCAACTATTTTTTTCT |
| chr7 | 117134090 | 117134114 | + | 25 | TCATTTTACCAACTATTTTTTTCTT |
| chr7 | 117134091 | 117134115 | + | 25 | CATTTTACCAACTATTTTTTTCTTC |
| chr7 | 117134092 | 117134116 | + | 25 | ATTTTACCAACTATTTTTTTCTTCT |
| chr7 | 117134093 | 117134117 | + | 25 | TTTTACCAACTATTTTTTTCTTCTT |
| chr7 | 117134094 | 117134118 | + | 25 | TTTACCAACTATTTTTTTCTTCTTG |
| chr7 | 117134095 | 117134119 | + | 25 | TTACCAACTATTTTTTTCTTCTTGG |
| chr7 | 117134096 | 117134120 | + | 25 | TACCAACTATTTTTTTCTTCTTGGT |
| chr7 | 117134097 | 117134121 | + | 25 | ACCAACTATTTTTTTCTTCTTGGTC |
| chr7 | 117134098 | 117134122 | + | 25 | CCAACTATTTTTTTCTTCTTGGTCA |
| chr7 | 117134099 | 117134123 | + | 25 | CAACTATTTTTTTCTTCTTGGTCAG |
| chr7 | 117134100 | 117134124 | + | 25 | AACTATTTTTTTCTTCTTGGTCAGA |
| chr7 | 117134101 | 117134125 | + | 25 | ACTATTTTTTTCTTCTTGGTCAGAA |
| chr7 | 117134102 | 117134126 | + | 25 | CTATTTTTTTCTTCTTGGTCAGAAT |
| chr7 | 117134103 | 117134127 | + | 25 | TATTTTTTTCTTCTTGGTCAGAATT |
| chr7 | 117134104 | 117134128 | + | 25 | ATTTTTTTCTTCTTGGTCAGAATTA |
| chr7 | 117134105 | 117134129 | + | 25 | TTTTTTTCTTCTTGGTCAGAATTAG |
| chr7 | 117134106 | 117134130 | + | 25 | TTTTTTCTTCTTGGTCAGAATTAGG |
| chr7 | 117134107 | 117134131 | + | 25 | TTTTTCTTCTTGGTCAGAATTAGGT |
| chr7 | 117134108 | 117134132 | + | 25 | TTTTCTTCTTGGTCAGAATTAGGTC |
| chr7 | 117134109 | 117134133 | + | 25 | TTTCTTCTTGGTCAGAATTAGGTCC |
| chr7 | 117134110 | 117134134 | + | 25 | TTCTTCTTGGTCAGAATTAGGTCCA |
| chr7 | 117134111 | 117134135 | + | 25 | TCTTCTTGGTCAGAATTAGGTCCAG |
| chr7 | 117134112 | 117134136 | + | 25 | CTTCTTGGTCAGAATTAGGTCCAGA |
| chr7 | 117134113 | 117134137 | + | 25 | TTCTTGGTCAGAATTAGGTCCAGAG |
| chr7 | 117134114 | 117134138 | + | 25 | TCTTGGTCAGAATTAGGTCCAGAGT |
| chr7 | 117134115 | 117134139 | + | 25 | CTTGGTCAGAATTAGGTCCAGAGTT |
| chr7 | 117134116 | 117134140 | + | 25 | TTGGTCAGAATTAGGTCCAGAGTTG |
| chr7 | 117134117 | 117134141 | + | 25 | TGGTCAGAATTAGGTCCAGAGTTGA |
| chr7 | 117134118 | 117134142 | + | 25 | GGTCAGAATTAGGTCCAGAGTTGAA |
| chr7 | 117134119 | 117134143 | + | 25 | GTCAGAATTAGGTCCAGAGTTGAAG |
| chr7 | 117134120 | 117134144 | + | 25 | TCAGAATTAGGTCCAGAGTTGAAGT |
| chr7 | 117134121 | 117134145 | + | 25 | CAGAATTAGGTCCAGAGTTGAAGTT |
| chr7 | 117134122 | 117134146 | + | 25 | AGAATTAGGTCCAGAGTTGAAGTTC |
| chr7 | 117134123 | 117134147 | + | 25 | GAATTAGGTCCAGAGTTGAAGTTCC |
| chr7 | 117134124 | 117134148 | + | 25 | AATTAGGTCCAGAGTTGAAGTTCCC |
| chr7 | 117134125 | 117134149 | + | 25 | ATTAGGTCCAGAGTTGAAGTTCCCC |
| chr7 | 117134126 | 117134150 | + | 25 | TTAGGTCCAGAGTTGAAGTTCCCCT |
| chr7 | 117134127 | 117134151 | + | 25 | TAGGTCCAGAGTTGAAGTTCCCCTA |
| chr7 | 117134128 | 117134152 | + | 25 | AGGTCCAGAGTTGAAGTTCCCCTAA |
| chr7 | 117134129 | 117134153 | + | 25 | GGTCCAGAGTTGAAGTTCCCCTAAT |
| chr7 | 117134130 | 117134154 | + | 25 | GTCCAGAGTTGAAGTTCCCCTAATT |
| chr7 | 117134131 | 117134155 | + | 25 | TCCAGAGTTGAAGTTCCCCTAATTG |
| chr7 | 117134132 | 117134156 | + | 25 | CCAGAGTTGAAGTTCCCCTAATTGC |
| chr7 | 117134133 | 117134157 | + | 25 | CAGAGTTGAAGTTCCCCTAATTGCT |
| chr7 | 117134134 | 117134158 | + | 25 | AGAGTTGAAGTTCCCCTAATTGCTT |
| chr7 | 117134135 | 117134159 | + | 25 | GAGTTGAAGTTCCCCTAATTGCTTC |
| chr7 | 117134136 | 117134160 | + | 25 | AGTTGAAGTTCCCCTAATTGCTTCC |
| chr7 | 117134137 | 117134161 | + | 25 | GTTGAAGTTCCCCTAATTGCTTCCT |
| chr7 | 117134138 | 117134162 | + | 25 | TTGAAGTTCCCCTAATTGCTTCCTC |
| chr7 | 117134139 | 117134163 | + | 25 | TGAAGTTCCCCTAATTGCTTCCTCT |
| chr7 | 117134140 | 117134164 | + | 25 | GAAGTTCCCCTAATTGCTTCCTCTA |
| chr7 | 117134141 | 117134165 | + | 25 | AAGTTCCCCTAATTGCTTCCTCTAC |
| chr7 | 117134142 | 117134166 | + | 25 | AGTTCCCCTAATTGCTTCCTCTACC |
| chr7 | 117134143 | 117134167 | + | 25 | GTTCCCCTAATTGCTTCCTCTACCA |
| chr7 | 117134144 | 117134168 | + | 25 | TTCCCCTAATTGCTTCCTCTACCAT |
| chr7 | 117134145 | 117134169 | + | 25 | TCCCCTAATTGCTTCCTCTACCATC |
| chr7 | 117134146 | 117134170 | + | 25 | CCCCTAATTGCTTCCTCTACCATCT |
| chr7 | 117134147 | 117134171 | + | 25 | CCCTAATTGCTTCCTCTACCATCTG |
| chr7 | 117134148 | 117134172 | + | 25 | CCTAATTGCTTCCTCTACCATCTGG |
| chr7 | 117134149 | 117134173 | + | 25 | CTAATTGCTTCCTCTACCATCTGGG |
| chr7 | 117134150 | 117134174 | + | 25 | TAATTGCTTCCTCTACCATCTGGGA |
| chr7 | 117134151 | 117134175 | + | 25 | AATTGCTTCCTCTACCATCTGGGAG |
| chr7 | 117134152 | 117134176 | + | 25 | ATTGCTTCCTCTACCATCTGGGAGA |
| chr7 | 117134153 | 117134177 | + | 25 | TTGCTTCCTCTACCATCTGGGAGAT |
| chr7 | 117134154 | 117134178 | + | 25 | TGCTTCCTCTACCATCTGGGAGATA |
| chr7 | 117134155 | 117134179 | + | 25 | GCTTCCTCTACCATCTGGGAGATAA |
| chr7 | 117134156 | 117134180 | + | 25 | CTTCCTCTACCATCTGGGAGATAAA |
| chr7 | 117134157 | 117134181 | + | 25 | TTCCTCTACCATCTGGGAGATAAAA |
| chr7 | 117134158 | 117134182 | + | 25 | TCCTCTACCATCTGGGAGATAAAAT |
| chr7 | 117134159 | 117134183 | + | 25 | CCTCTACCATCTGGGAGATAAAATT |
| chr7 | 117134160 | 117134184 | + | 25 | CTCTACCATCTGGGAGATAAAATTG |
| chr7 | 117134161 | 117134185 | + | 25 | TCTACCATCTGGGAGATAAAATTGT |
| chr7 | 117134162 | 117134186 | + | 25 | CTACCATCTGGGAGATAAAATTGTT |
| chr7 | 117134163 | 117134187 | + | 25 | TACCATCTGGGAGATAAAATTGTTA |
| chr7 | 117134164 | 117134188 | + | 25 | ACCATCTGGGAGATAAAATTGTTAC |
| chr7 | 117134165 | 117134189 | + | 25 | CCATCTGGGAGATAAAATTGTTACC |
| chr7 | 117134166 | 117134190 | + | 25 | CATCTGGGAGATAAAATTGTTACCA |
| chr7 | 117134167 | 117134191 | + | 25 | ATCTGGGAGATAAAATTGTTACCAA |
| chr7 | 117134168 | 117134192 | + | 25 | TCTGGGAGATAAAATTGTTACCAAA |
| chr7 | 117134169 | 117134193 | + | 25 | CTGGGAGATAAAATTGTTACCAAAG |
| chr7 | 117134170 | 117134194 | + | 25 | TGGGAGATAAAATTGTTACCAAAGT |
| chr7 | 117134171 | 117134195 | + | 25 | GGGAGATAAAATTGTTACCAAAGTA |
| chr7 | 117134172 | 117134196 | + | 25 | GGAGATAAAATTGTTACCAAAGTAA |
| chr7 | 117134173 | 117134197 | + | 25 | GAGATAAAATTGTTACCAAAGTAAG |
| chr7 | 117134174 | 117134198 | + | 25 | AGATAAAATTGTTACCAAAGTAAGT |
| chr7 | 117134175 | 117134199 | + | 25 | GATAAAATTGTTACCAAAGTAAGTC |
| chr7 | 117134176 | 117134200 | + | 25 | ATAAAATTGTTACCAAAGTAAGTCA |
| chr7 | 117134177 | 117134201 | + | 25 | TAAAATTGTTACCAAAGTAAGTCAA |
| chr7 | 117134178 | 117134202 | + | 25 | AAAATTGTTACCAAAGTAAGTCAAG |
| chr7 | 117134179 | 117134203 | + | 25 | AAATTGTTACCAAAGTAAGTCAAGA |
| chr7 | 117134180 | 117134204 | + | 25 | AATTGTTACCAAAGTAAGTCAAGAA |
| chr7 | 117134181 | 117134205 | + | 25 | ATTGTTACCAAAGTAAGTCAAGAAC |
| chr7 | 117134182 | 117134206 | + | 25 | TTGTTACCAAAGTAAGTCAAGAACC |
| chr7 | 117134183 | 117134207 | + | 25 | TGTTACCAAAGTAAGTCAAGAACCT |
| chr7 | 117134184 | 117134208 | + | 25 | GTTACCAAAGTAAGTCAAGAACCTG |
| chr7 | 117134185 | 117134209 | + | 25 | TTACCAAAGTAAGTCAAGAACCTGT |
| chr7 | 117134186 | 117134210 | + | 25 | TACCAAAGTAAGTCAAGAACCTGTT |
| chr7 | 117134187 | 117134211 | + | 25 | ACCAAAGTAAGTCAAGAACCTGTTG |
| chr7 | 117134188 | 117134212 | + | 25 | CCAAAGTAAGTCAAGAACCTGTTGG |
| chr7 | 117134189 | 117134213 | + | 25 | CAAAGTAAGTCAAGAACCTGTTGGG |
| chr7 | 117134190 | 117134214 | + | 25 | AAAGTAAGTCAAGAACCTGTTGGGT |
| chr7 | 117134191 | 117134215 | + | 25 | AAGTAAGTCAAGAACCTGTTGGGTG |
| chr7 | 117134192 | 117134216 | + | 25 | AGTAAGTCAAGAACCTGTTGGGTGC |
| chr7 | 117134193 | 117134217 | + | 25 | GTAAGTCAAGAACCTGTTGGGTGCC |
| chr7 | 117134194 | 117134218 | + | 25 | TAAGTCAAGAACCTGTTGGGTGCCC |
| chr7 | 117134195 | 117134219 | + | 25 | AAGTCAAGAACCTGTTGGGTGCCCT |
| chr7 | 117134196 | 117134220 | + | 25 | AGTCAAGAACCTGTTGGGTGCCCTA |
| chr7 | 117134197 | 117134221 | + | 25 | GTCAAGAACCTGTTGGGTGCCCTAC |
| chr7 | 117134198 | 117134222 | + | 25 | TCAAGAACCTGTTGGGTGCCCTACT |
| chr7 | 117134199 | 117134223 | + | 25 | CAAGAACCTGTTGGGTGCCCTACTT |
| chr7 | 117134200 | 117134224 | + | 25 | AAGAACCTGTTGGGTGCCCTACTTC |
| chr7 | 117134201 | 117134225 | + | 25 | AGAACCTGTTGGGTGCCCTACTTCT |
| chr7 | 117134202 | 117134226 | + | 25 | GAACCTGTTGGGTGCCCTACTTCTA |
| chr7 | 117134203 | 117134227 | + | 25 | AACCTGTTGGGTGCCCTACTTCTAA |
| chr7 | 117134204 | 117134228 | + | 25 | ACCTGTTGGGTGCCCTACTTCTAAC |
| chr7 | 117134205 | 117134229 | + | 25 | CCTGTTGGGTGCCCTACTTCTAACC |
| chr7 | 117134206 | 117134230 | + | 25 | CTGTTGGGTGCCCTACTTCTAACCA |
| chr7 | 117134207 | 117134231 | + | 25 | TGTTGGGTGCCCTACTTCTAACCAA |
| chr7 | 117134208 | 117134232 | + | 25 | GTTGGGTGCCCTACTTCTAACCAAA |
| chr7 | 117134209 | 117134233 | + | 25 | TTGGGTGCCCTACTTCTAACCAAAT |
| chr7 | 117134210 | 117134234 | + | 25 | TGGGTGCCCTACTTCTAACCAAATG |
| chr7 | 117134211 | 117134235 | + | 25 | GGGTGCCCTACTTCTAACCAAATGA |
| chr7 | 117134212 | 117134236 | + | 25 | GGTGCCCTACTTCTAACCAAATGAG |
| chr7 | 117134213 | 117134237 | + | 25 | GTGCCCTACTTCTAACCAAATGAGA |
| chr7 | 117134214 | 117134238 | + | 25 | TGCCCTACTTCTAACCAAATGAGAC |
| chr7 | 117134215 | 117134239 | + | 25 | GCCCTACTTCTAACCAAATGAGACT |
| chr7 | 117134216 | 117134240 | + | 25 | CCCTACTTCTAACCAAATGAGACTT |
| chr7 | 117134217 | 117134241 | + | 25 | CCTACTTCTAACCAAATGAGACTTC |
| chr7 | 117134218 | 117134242 | + | 25 | CTACTTCTAACCAAATGAGACTTCT |

**Table 31: 25-mer target-specific ASOs**

| **CHR** | **START** | **END** | **STRAND** | **kmer** | **SEQUENCE** |
|---|---|---|---|---|---|
| chr7 | 158752730 | 158752754 | + | 25 | TCTTTCAATATGTTTTCATTAACTC |
| chr7 | 158752731 | 158752755 | + | 25 | CTTTCAATATGTTTTCATTAACTCT |
| chr7 | 158752732 | 158752756 | + | 25 | TTTCAATATGTTTTCATTAACTCTG |
| chr7 | 158752733 | 158752757 | + | 25 | TTCAATATGTTTTCATTAACTCTGT |
| chr7 | 158752734 | 158752758 | + | 25 | TCAATATGTTTTCATTAACTCTGTG |
| chr7 | 158752735 | 158752759 | + | 25 | CAATATGTTTTCATTAACTCTGTGA |
| chr7 | 158752736 | 158752760 | + | 25 | AATATGTTTTCATTAACTCTGTGAC |
| chr7 | 158752737 | 158752761 | + | 25 | ATATGTTTTCATTAACTCTGTGACC |
| chr7 | 158752738 | 158752762 | + | 25 | TATGTTTTCATTAACTCTGTGACCT |
| chr7 | 158752739 | 158752763 | + | 25 | ATGTTTTCATTAACTCTGTGACCTG |
| chr7 | 158752740 | 158752764 | + | 25 | TGTTTTCATTAACTCTGTGACCTGA |
| chr7 | 158752741 | 158752765 | + | 25 | GTTTTCATTAACTCTGTGACCTGAA |
| chr7 | 158752742 | 158752766 | + | 25 | TTTTCATTAACTCTGTGACCTGAAG |
| chr7 | 158752743 | 158752767 | + | 25 | TTTCATTAACTCTGTGACCTGAAGG |
| chr7 | 158752744 | 158752768 | + | 25 | TTCATTAACTCTGTGACCTGAAGGG |
| chr7 | 158752745 | 158752769 | + | 25 | TCATTAACTCTGTGACCTGAAGGGA |
| chr7 | 158752746 | 158752770 | + | 25 | CATTAACTCTGTGACCTGAAGGGAA |
| chr7 | 158752747 | 158752771 | + | 25 | ATTAACTCTGTGACCTGAAGGGAAA |
| chr7 | 158752748 | 158752772 | + | 25 | TTAACTCTGTGACCTGAAGGGAAAA |
| chr7 | 158752749 | 158752773 | + | 25 | TAACTCTGTGACCTGAAGGGAAAAC |
| chr7 | 158752750 | 158752774 | + | 25 | AACTCTGTGACCTGAAGGGAAAACA |
| chr7 | 158752751 | 158752775 | + | 25 | ACTCTGTGACCTGAAGGGAAAACAG |
| chr7 | 158752752 | 158752776 | + | 25 | CTCTGTGACCTGAAGGGAAAACAGT |
| chr7 | 158752753 | 158752777 | + | 25 | TCTGTGACCTGAAGGGAAAACAGTT |
| chr7 | 158752754 | 158752778 | + | 25 | CTGTGACCTGAAGGGAAAACAGTTT |
| chr7 | 158752755 | 158752779 | + | 25 | TGTGACCTGAAGGGAAAACAGTTTA |
| chr7 | 158752756 | 158752780 | + | 25 | GTGACCTGAAGGGAAAACAGTTTAC |
| chr7 | 158752757 | 158752781 | + | 25 | TGACCTGAAGGGAAAACAGTTTACC |
| chr7 | 158752758 | 158752782 | + | 25 | GACCTGAAGGGAAAACAGTTTACCT |
| chr7 | 158752759 | 158752783 | + | 25 | ACCTGAAGGGAAAACAGTTTACCTT |
| chr7 | 158752760 | 158752784 | + | 25 | CCTGAAGGGAAAACAGTTTACCTTT |
| chr7 | 158752761 | 158752785 | + | 25 | CTGAAGGGAAAACAGTTTACCTTTA |
| chr7 | 158752762 | 158752786 | + | 25 | TGAAGGGAAAACAGTTTACCTTTAA |
| chr7 | 158752763 | 158752787 | + | 25 | GAAGGGAAAACAGTTTACCTTTAAA |
| chr7 | 158752764 | 158752788 | + | 25 | AAGGGAAAACAGTTTACCTTTAAAG |
| chr7 | 158752765 | 158752789 | + | 25 | AGGGAAAACAGTTTACCTTTAAAGC |
| chr7 | 158752766 | 158752790 | + | 25 | GGGAAAACAGTTTACCTTTAAAGCT |
| chr7 | 158752767 | 158752791 | + | 25 | GGAAAACAGTTTACCTTTAAAGCTC |
| chr7 | 158752768 | 158752792 | + | 25 | GAAAACAGTTTACCTTTAAAGCTCT |
| chr7 | 158752769 | 158752793 | + | 25 | AAAACAGTTTACCTTTAAAGCTCTC |
| chr7 | 158752770 | 158752794 | + | 25 | AAACAGTTTACCTTTAAAGCTCTCT |
| chr7 | 158752771 | 158752795 | + | 25 | AACAGTTTACCTTTAAAGCTCTCTG |
| chr7 | 158752772 | 158752796 | + | 25 | ACAGTTTACCTTTAAAGCTCTCTGA |
| chr7 | 158752773 | 158752797 | + | 25 | CAGTTTACCTTTAAAGCTCTCTGAA |
| chr7 | 158752774 | 158752798 | + | 25 | AGTTTACCTTTAAAGCTCTCTGAAC |
| chr7 | 158752775 | 158752799 | + | 25 | GTTTACCTTTAAAGCTCTCTGAACT |
| chr7 | 158752776 | 158752800 | + | 25 | TTTACCTTTAAAGCTCTCTGAACTG |
| chr7 | 158752777 | 158752801 | + | 25 | TTACCTTTAAAGCTCTCTGAACTGC |
| chr7 | 158752778 | 158752802 | + | 25 | TACCTTTAAAGCTCTCTGAACTGCA |
| chr7 | 158752779 | 158752803 | + | 25 | ACCTTTAAAGCTCTCTGAACTGCAG |
| chr7 | 158752780 | 158752804 | + | 25 | CCTTTAAAGCTCTCTGAACTGCAGT |
| chr7 | 158752781 | 158752805 | + | 25 | CTTTAAAGCTCTCTGAACTGCAGTC |
| chr7 | 158752782 | 158752806 | + | 25 | TTTAAAGCTCTCTGAACTGCAGTCT |
| chr7 | 158752783 | 158752807 | + | 25 | TTAAAGCTCTCTGAACTGCAGTCTT |
| chr7 | 158752784 | 158752808 | + | 25 | TAAAGCTCTCTGAACTGCAGTCTTC |
| chr7 | 158752785 | 158752809 | + | 25 | AAAGCTCTCTGAACTGCAGTCTTCT |
| chr7 | 158752786 | 158752810 | + | 25 | AAGCTCTCTGAACTGCAGTCTTCTT |
| chr7 | 158752787 | 158752811 | + | 25 | AGCTCTCTGAACTGCAGTCTTCTTC |
| chr7 | 158752788 | 158752812 | + | 25 | GCTCTCTGAACTGCAGTCTTCTTCA |
| chr7 | 158752789 | 158752813 | + | 25 | CTCTCTGAACTGCAGTCTTCTTCAA |
| chr7 | 158752790 | 158752814 | + | 25 | TCTCTGAACTGCAGTCTTCTTCAAG |
| chr7 | 158752791 | 158752815 | + | 25 | CTCTGAACTGCAGTCTTCTTCAAGA |
| chr7 | 158752792 | 158752816 | + | 25 | TCTGAACTGCAGTCTTCTTCAAGAC |
| chr7 | 158752793 | 158752817 | + | 25 | CTGAACTGCAGTCTTCTTCAAGACA |
| chr7 | 158752794 | 158752818 | + | 25 | TGAACTGCAGTCTTCTTCAAGACAT |
| chr7 | 158752795 | 158752819 | + | 25 | GAACTGCAGTCTTCTTCAAGACATC |
| chr7 | 158752796 | 158752820 | + | 25 | AACTGCAGTCTTCTTCAAGACATCG |
| chr7 | 158752797 | 158752821 | + | 25 | ACTGCAGTCTTCTTCAAGACATCGG |
| chr7 | 158752798 | 158752822 | + | 25 | CTGCAGTCTTCTTCAAGACATCGGG |
| chr7 | 158752799 | 158752823 | + | 25 | TGCAGTCTTCTTCAAGACATCGGGG |
| chr7 | 158752800 | 158752824 | + | 25 | GCAGTCTTCTTCAAGACATCGGGGT |
| chr7 | 158752801 | 158752825 | + | 25 | CAGTCTTCTTCAAGACATCGGGGTT |
| chr7 | 158752802 | 158752826 | + | 25 | AGTCTTCTTCAAGACATCGGGGTTA |
| chr7 | 158752803 | 158752827 | + | 25 | GTCTTCTTCAAGACATCGGGGTTAA |
| chr7 | 158752804 | 158752828 | + | 25 | TCTTCTTCAAGACATCGGGGTTAAA |
| chr7 | 158752805 | 158752829 | + | 25 | CTTCTTCAAGACATCGGGGTTAAAT |
| chr7 | 158752806 | 158752830 | + | 25 | TTCTTCAAGACATCGGGGTTAAATT |
| chr7 | 158752807 | 158752831 | + | 25 | TCTTCAAGACATCGGGGTTAAATTC |
| chr7 | 158752808 | 158752832 | + | 25 | CTTCAAGACATCGGGGTTAAATTCT |
| chr7 | 158752809 | 158752833 | + | 25 | TTCAAGACATCGGGGTTAAATTCTA |
| chr7 | 158752810 | 158752834 | + | 25 | TCAAGACATCGGGGTTAAATTCTAA |
| chr7 | 158752811 | 158752835 | + | 25 | CAAGACATCGGGGTTAAATTCTAAT |
| chr7 | 158752812 | 158752836 | + | 25 | AAGACATCGGGGTTAAATTCTAATG |
| chr7 | 158752813 | 158752837 | + | 25 | AGACATCGGGGTTAAATTCTAATGG |
| chr7 | 158752814 | 158752838 | + | 25 | GACATCGGGGTTAAATTCTAATGGG |
| chr7 | 158752815 | 158752839 | + | 25 | ACATCGGGGTTAAATTCTAATGGGT |
| chr7 | 158752816 | 158752840 | + | 25 | CATCGGGGTTAAATTCTAATGGGTT |
| chr7 | 158752817 | 158752841 | + | 25 | ATCGGGGTTAAATTCTAATGGGTTA |
| chr7 | 158752818 | 158752842 | + | 25 | TCGGGGTTAAATTCTAATGGGTTAC |
| chr7 | 158752819 | 158752843 | + | 25 | CGGGGTTAAATTCTAATGGGTTACT |
| chr7 | 158752820 | 158752844 | + | 25 | GGGGTTAAATTCTAATGGGTTACTC |
| chr7 | 158752821 | 158752845 | + | 25 | GGGTTAAATTCTAATGGGTTACTCT |
| chr7 | 158752822 | 158752846 | + | 25 | GGTTAAATTCTAATGGGTTACTCTT |
| chr7 | 158752823 | 158752847 | + | 25 | GTTAAATTCTAATGGGTTACTCTTT |
| chr7 | 158752824 | 158752848 | + | 25 | TTAAATTCTAATGGGTTACTCTTTC |
| chr7 | 158752825 | 158752849 | + | 25 | TAAATTCTAATGGGTTACTCTTTCA |
| chr7 | 158752826 | 158752850 | + | 25 | AAATTCTAATGGGTTACTCTTTCAA |
| chr7 | 158752827 | 158752851 | + | 25 | AATTCTAATGGGTTACTCTTTCAAG |
| chr7 | 158752828 | 158752852 | + | 25 | ATTCTAATGGGTTACTCTTTCAAGT |
| chr7 | 158752829 | 158752853 | + | 25 | TTCTAATGGGTTACTCTTTCAAGTC |
| chr7 | 158752830 | 158752854 | + | 25 | TCTAATGGGTTACTCTTTCAAGTCA |
| chr7 | 158752831 | 158752855 | + | 25 | CTAATGGGTTACTCTTTCAAGTCAG |
| chr7 | 158752832 | 158752856 | + | 25 | TAATGGGTTACTCTTTCAAGTCAGA |
| chr7 | 158752833 | 158752857 | + | 25 | AATGGGTTACTCTTTCAAGTCAGAA |
| chr7 | 158752834 | 158752858 | + | 25 | ATGGGTTACTCTTTCAAGTCAGAAG |
| chr7 | 158752835 | 158752859 | + | 25 | TGGGTTACTCTTTCAAGTCAGAAGA |
| chr7 | 158752836 | 158752860 | + | 25 | GGGTTACTCTTTCAAGTCAGAAGAA |
| chr7 | 158752837 | 158752861 | + | 25 | GGTTACTCTTTCAAGTCAGAAGAAA |
| chr7 | 158752838 | 158752862 | + | 25 | GTTACTCTTTCAAGTCAGAAGAAAA |
| chr7 | 158752839 | 158752863 | + | 25 | TTACTCTTTCAAGTCAGAAGAAAAC |
| chr7 | 158752840 | 158752864 | + | 25 | TACTCTTTCAAGTCAGAAGAAAACG |
| chr7 | 158752841 | 158752865 | + | 25 | ACTCTTTCAAGTCAGAAGAAAACGA |
| chr7 | 158752842 | 158752866 | + | 25 | CTCTTTCAAGTCAGAAGAAAACGAA |
| chr7 | 158752843 | 158752867 | + | 25 | TCTTTCAAGTCAGAAGAAAACGAAT |
| chr7 | 158752844 | 158752868 | + | 25 | CTTTCAAGTCAGAAGAAAACGAATA |
| chr7 | 158752845 | 158752869 | + | 25 | TTTCAAGTCAGAAGAAAACGAATAT |
| chr7 | 158752846 | 158752870 | + | 25 | TTCAAGTCAGAAGAAAACGAATATG |
| chr7 | 158752847 | 158752871 | + | 25 | TCAAGTCAGAAGAAAACGAATATGC |
| chr7 | 158752848 | 158752872 | + | 25 | CAAGTCAGAAGAAAACGAATATGCC |
| chr7 | 158752849 | 158752873 | + | 25 | AAGTCAGAAGAAAACGAATATGCCA |
| chr7 | 158752850 | 158752874 | + | 25 | AGTCAGAAGAAAACGAATATGCCAA |
| chr7 | 158752851 | 158752875 | + | 25 | GTCAGAAGAAAACGAATATGCCAAG |
| chr7 | 158752852 | 158752876 | + | 25 | TCAGAAGAAAACGAATATGCCAAGG |
| chr7 | 158752853 | 158752877 | + | 25 | CAGAAGAAAACGAATATGCCAAGGG |
| chr7 | 158752854 | 158752878 | + | 25 | AGAAGAAAACGAATATGCCAAGGGT |
| chr7 | 158752855 | 158752879 | + | 25 | GAAGAAAACGAATATGCCAAGGGTT |
| chr7 | 158752856 | 158752880 | + | 25 | AAGAAAACGAATATGCCAAGGGTTA |
| chr7 | 158752857 | 158752881 | + | 25 | AGAAAACGAATATGCCAAGGGTTAA |
| chr7 | 158752858 | 158752882 | + | 25 | GAAAACGAATATGCCAAGGGTTAAT |
| chr7 | 158752859 | 158752883 | + | 25 | AAAACGAATATGCCAAGGGTTAATA |
| chr7 | 158752860 | 158752884 | + | 25 | AAACGAATATGCCAAGGGTTAATAA |
| chr7 | 158752861 | 158752885 | + | 25 | AACGAATATGCCAAGGGTTAATAAA |
| chr7 | 158752862 | 158752886 | + | 25 | ACGAATATGCCAAGGGTTAATAAAA |
| chr7 | 158752863 | 158752887 | + | 25 | CGAATATGCCAAGGGTTAATAAAAC |
| chr7 | 158752864 | 158752888 | + | 25 | GAATATGCCAAGGGTTAATAAAACA |
| chr7 | 158752865 | 158752889 | + | 25 | AATATGCCAAGGGTTAATAAAACAT |
| chr7 | 158752866 | 158752890 | + | 25 | ATATGCCAAGGGTTAATAAAACATG |
| chr7 | 158752867 | 158752891 | + | 25 | TATGCCAAGGGTTAATAAAACATGC |
| chr7 | 158752868 | 158752892 | + | 25 | ATGCCAAGGGTTAATAAAACATGCA |
| chr7 | 158752869 | 158752893 | + | 25 | TGCCAAGGGTTAATAAAACATGCAA |

**Table 32: 25-mer target-specific ASOs**

| **CHR** | **START** | **END** | **STRAND** | **kmer** | **SEQUENCE** |
|---|---|---|---|---|---|
| chr22 | 19971165 | 19971189 | + | 25 | TGGCCCAGAGGGCAGGAACAGGTGG |
| chr22 | 19971166 | 19971190 | + | 25 | GGCCCAGAGGGCAGGAACAGGTGGA |
| chr22 | 19971167 | 19971191 | + | 25 | GCCCAGAGGGCAGGAACAGGTGGAC |
| chr22 | 19971168 | 19971192 | + | 25 | CCCAGAGGGCAGGAACAGGTGGACG |
| chr22 | 19971169 | 19971193 | + | 25 | CCAGAGGGCAGGAACAGGTGGACGA |
| chr22 | 19971170 | 19971194 | + | 25 | CAGAGGGCAGGAACAGGTGGACGAA |
| chr22 | 19971171 | 19971195 | + | 25 | AGAGGGCAGGAACAGGTGGACGAAC |
| chr22 | 19971172 | 19971196 | + | 25 | GAGGGCAGGAACAGGTGGACGAACA |
| chr22 | 19971173 | 19971197 | + | 25 | AGGGCAGGAACAGGTGGACGAACAA |
| chr22 | 19971174 | 19971198 | + | 25 | GGGCAGGAACAGGTGGACGAACAAC |
| chr22 | 19971175 | 19971199 | + | 25 | GGCAGGAACAGGTGGACGAACAACC |
| chr22 | 19971176 | 19971200 | + | 25 | GCAGGAACAGGTGGACGAACAACCA |
| chr22 | 19971177 | 19971201 | + | 25 | CAGGAACAGGTGGACGAACAACCAG |
| chr22 | 19971178 | 19971202 | + | 25 | AGGAACAGGTGGACGAACAACCAGA |
| chr22 | 19971179 | 19971203 | + | 25 | GGAACAGGTGGACGAACAACCAGAT |
| chr22 | 19971180 | 19971204 | + | 25 | GAACAGGTGGACGAACAACCAGATG |
| chr22 | 19971181 | 19971205 | + | 25 | AACAGGTGGACGAACAACCAGATGA |
| chr22 | 19971182 | 19971206 | + | 25 | ACAGGTGGACGAACAACCAGATGAG |
| chr22 | 19971183 | 19971207 | + | 25 | CAGGTGGACGAACAACCAGATGAGA |
| chr22 | 19971184 | 19971208 | + | 25 | AGGTGGACGAACAACCAGATGAGAG |
| chr22 | 19971185 | 19971209 | + | 25 | GGTGGACGAACAACCAGATGAGAGA |
| chr22 | 19971186 | 19971210 | + | 25 | GTGGACGAACAACCAGATGAGAGAA |
| chr22 | 19971187 | 19971211 | + | 25 | TGGACGAACAACCAGATGAGAGAAC |
| chr22 | 19971188 | 19971212 | + | 25 | GGACGAACAACCAGATGAGAGAACG |
| chr22 | 19971189 | 19971213 | + | 25 | GACGAACAACCAGATGAGAGAACGT |
| chr22 | 19971190 | 19971214 | + | 25 | ACGAACAACCAGATGAGAGAACGTA |
| chr22 | 19971191 | 19971215 | + | 25 | CGAACAACCAGATGAGAGAACGTAC |
| chr22 | 19971192 | 19971216 | + | 25 | GAACAACCAGATGAGAGAACGTACC |
| chr22 | 19971193 | 19971217 | + | 25 | AACAACCAGATGAGAGAACGTACCA |
| chr22 | 19971194 | 19971218 | + | 25 | ACAACCAGATGAGAGAACGTACCAG |
| chr22 | 19971195 | 19971219 | + | 25 | CAACCAGATGAGAGAACGTACCAGG |
| chr22 | 19971196 | 19971220 | + | 25 | AACCAGATGAGAGAACGTACCAGGC |
| chr22 | 19971197 | 19971221 | + | 25 | ACCAGATGAGAGAACGTACCAGGCA |
| chr22 | 19971198 | 19971222 | + | 25 | CCAGATGAGAGAACGTACCAGGCAT |
| chr22 | 19971199 | 19971223 | + | 25 | CAGATGAGAGAACGTACCAGGCATG |
| chr22 | 19971200 | 19971224 | + | 25 | AGATGAGAGAACGTACCAGGCATGC |
| chr22 | 19971201 | 19971225 | + | 25 | GATGAGAGAACGTACCAGGCATGCA |
| chr22 | 19971202 | 19971226 | + | 25 | ATGAGAGAACGTACCAGGCATGCAA |
| chr22 | 19971203 | 19971227 | + | 25 | TGAGAGAACGTACCAGGCATGCAAG |
| chr22 | 19971204 | 19971228 | + | 25 | GAGAGAACGTACCAGGCATGCAAGC |
| chr22 | 19971205 | 19971229 | + | 25 | AGAGAACGTACCAGGCATGCAAGCT |
| chr22 | 19971206 | 19971230 | + | 25 | GAGAACGTACCAGGCATGCAAGCTA |
| chr22 | 19971207 | 19971231 | + | 25 | AGAACGTACCAGGCATGCAAGCTAG |
| chr22 | 19971208 | 19971232 | + | 25 | GAACGTACCAGGCATGCAAGCTAGA |
| chr22 | 19971209 | 19971233 | + | 25 | AACGTACCAGGCATGCAAGCTAGAC |
| chr22 | 19971210 | 19971234 | + | 25 | ACGTACCAGGCATGCAAGCTAGACC |
| chr22 | 19971211 | 19971235 | + | 25 | CGTACCAGGCATGCAAGCTAGACCC |
| chr22 | 19971212 | 19971236 | + | 25 | GTACCAGGCATGCAAGCTAGACCCA |
| chr22 | 19971213 | 19971237 | + | 25 | TACCAGGCATGCAAGCTAGACCCAG |
| chr22 | 19971214 | 19971238 | + | 25 | ACCAGGCATGCAAGCTAGACCCAGG |
| chr22 | 19971215 | 19971239 | + | 25 | CCAGGCATGCAAGCTAGACCCAGGA |
| chr22 | 19971216 | 19971240 | + | 25 | CAGGCATGCAAGCTAGACCCAGGAA |
| chr22 | 19971217 | 19971241 | + | 25 | AGGCATGCAAGCTAGACCCAGGAAT |
| chr22 | 19971218 | 19971242 | + | 25 | GGCATGCAAGCTAGACCCAGGAATC |
| chr22 | 19971219 | 19971243 | + | 25 | GCATGCAAGCTAGACCCAGGAATCA |
| chr22 | 19971220 | 19971244 | + | 25 | CATGCAAGCTAGACCCAGGAATCAA |
| chr22 | 19971221 | 19971245 | + | 25 | ATGCAAGCTAGACCCAGGAATCAAC |
| chr22 | 19971222 | 19971246 | + | 25 | TGCAAGCTAGACCCAGGAATCAACG |
| chr22 | 19971223 | 19971247 | + | 25 | GCAAGCTAGACCCAGGAATCAACGG |
| chr22 | 19971224 | 19971248 | + | 25 | CAAGCTAGACCCAGGAATCAACGGG |
| chr22 | 19971225 | 19971249 | + | 25 | AAGCTAGACCCAGGAATCAACGGGC |
| chr22 | 19971226 | 19971250 | + | 25 | AGCTAGACCCAGGAATCAACGGGCT |
| chr22 | 19971227 | 19971251 | + | 25 | GCTAGACCCAGGAATCAACGGGCTG |
| chr22 | 19971228 | 19971252 | + | 25 | CTAGACCCAGGAATCAACGGGCTGA |
| chr22 | 19971229 | 19971253 | + | 25 | TAGACCCAGGAATCAACGGGCTGAG |
| chr22 | 19971230 | 19971254 | + | 25 | AGACCCAGGAATCAACGGGCTGAGG |
| chr22 | 19971231 | 19971255 | + | 25 | GACCCAGGAATCAACGGGCTGAGGC |
| chr22 | 19971232 | 19971256 | + | 25 | ACCCAGGAATCAACGGGCTGAGGCT |
| chr22 | 19971233 | 19971257 | + | 25 | CCCAGGAATCAACGGGCTGAGGCTT |
| chr22 | 19971234 | 19971258 | + | 25 | CCAGGAATCAACGGGCTGAGGCTTA |
| chr22 | 19971235 | 19971259 | + | 25 | CAGGAATCAACGGGCTGAGGCTTAG |
| chr22 | 19971236 | 19971260 | + | 25 | AGGAATCAACGGGCTGAGGCTTAGC |
| chr22 | 19971237 | 19971261 | + | 25 | GGAATCAACGGGCTGAGGCTTAGCG |
| chr22 | 19971238 | 19971262 | + | 25 | GAATCAACGGGCTGAGGCTTAGCGT |
| chr22 | 19971239 | 19971263 | + | 25 | AATCAACGGGCTGAGGCTTAGCGTC |
| chr22 | 19971240 | 19971264 | + | 25 | ATCAACGGGCTGAGGCTTAGCGTCC |
| chr22 | 19971241 | 19971265 | + | 25 | TCAACGGGCTGAGGCTTAGCGTCCC |
| chr22 | 19971242 | 19971266 | + | 25 | CAACGGGCTGAGGCTTAGCGTCCCC |
| chr22 | 19971243 | 19971267 | + | 25 | AACGGGCTGAGGCTTAGCGTCCCCT |
| chr22 | 19971244 | 19971268 | + | 25 | ACGGGCTGAGGCTTAGCGTCCCCTA |
| chr22 | 19971245 | 19971269 | + | 25 | CGGGCTGAGGCTTAGCGTCCCCTAC |
| chr22 | 19971246 | 19971270 | + | 25 | GGGCTGAGGCTTAGCGTCCCCTACG |
| chr22 | 19971247 | 19971271 | + | 25 | GGCTGAGGCTTAGCGTCCCCTACGG |
| chr22 | 19971248 | 19971272 | + | 25 | GCTGAGGCTTAGCGTCCCCTACGGC |
| chr22 | 19971249 | 19971273 | + | 25 | CTGAGGCTTAGCGTCCCCTACGGCG |
| chr22 | 19971250 | 19971274 | + | 25 | TGAGGCTTAGCGTCCCCTACGGCGT |
| chr22 | 19971251 | 19971275 | + | 25 | GAGGCTTAGCGTCCCCTACGGCGTC |
| chr22 | 19971252 | 19971276 | + | 25 | AGGCTTAGCGTCCCCTACGGCGTCC |
| chr22 | 19971253 | 19971277 | + | 25 | GGCTTAGCGTCCCCTACGGCGTCCA |
| chr22 | 19971254 | 19971278 | + | 25 | GCTTAGCGTCCCCTACGGCGTCCAC |
| chr22 | 19971255 | 19971279 | + | 25 | CTTAGCGTCCCCTACGGCGTCCACC |
| chr22 | 19971256 | 19971280 | + | 25 | TTAGCGTCCCCTACGGCGTCCACCA |
| chr22 | 19971257 | 19971281 | + | 25 | TAGCGTCCCCTACGGCGTCCACCAG |
| chr22 | 19971258 | 19971282 | + | 25 | AGCGTCCCCTACGGCGTCCACCAGC |
| chr22 | 19971259 | 19971283 | + | 25 | GCGTCCCCTACGGCGTCCACCAGCC |
| chr22 | 19971260 | 19971284 | + | 25 | CGTCCCCTACGGCGTCCACCAGCCT |
| chr22 | 19971261 | 19971285 | + | 25 | GTCCCCTACGGCGTCCACCAGCCTG |
| chr22 | 19971262 | 19971286 | + | 25 | TCCCCTACGGCGTCCACCAGCCTGA |
| chr22 | 19971263 | 19971287 | + | 25 | CCCCTACGGCGTCCACCAGCCTGAC |
| chr22 | 19971264 | 19971288 | + | 25 | CCCTACGGCGTCCACCAGCCTGACC |
| chr22 | 19971265 | 19971289 | + | 25 | CCTACGGCGTCCACCAGCCTGACCG |
| chr22 | 19971266 | 19971290 | + | 25 | CTACGGCGTCCACCAGCCTGACCGC |
| chr22 | 19971267 | 19971291 | + | 25 | TACGGCGTCCACCAGCCTGACCGCG |
| chr22 | 19971268 | 19971292 | + | 25 | ACGGCGTCCACCAGCCTGACCGCGG |
| chr22 | 19971269 | 19971293 | + | 25 | CGGCGTCCACCAGCCTGACCGCGGG |
| chr22 | 19971270 | 19971294 | + | 25 | GGCGTCCACCAGCCTGACCGCGGGC |
| chr22 | 19971271 | 19971295 | + | 25 | GCGTCCACCAGCCTGACCGCGGGCC |
| chr22 | 19971272 | 19971296 | + | 25 | CGTCCACCAGCCTGACCGCGGGCCT |
| chr22 | 19971273 | 19971297 | + | 25 | GTCCACCAGCCTGACCGCGGGCCTG |
| chr22 | 19971274 | 19971298 | + | 25 | TCCACCAGCCTGACCGCGGGCCTGC |
| chr22 | 19971275 | 19971299 | + | 25 | CCACCAGCCTGACCGCGGGCCTGCT |
| chr22 | 19971276 | 19971300 | + | 25 | CACCAGCCTGACCGCGGGCCTGCTG |
| chr22 | 19971277 | 19971301 | + | 25 | ACCAGCCTGACCGCGGGCCTGCTGG |
| chr22 | 19971278 | 19971302 | + | 25 | CCAGCCTGACCGCGGGCCTGCTGGG |
| chr22 | 19971279 | 19971303 | + | 25 | CAGCCTGACCGCGGGCCTGCTGGGC |
| chr22 | 19971280 | 19971304 | + | 25 | AGCCTGACCGCGGGCCTGCTGGGCC |
| chr22 | 19971281 | 19971305 | + | 25 | GCCTGACCGCGGGCCTGCTGGGCCC |
| chr22 | 19971282 | 19971306 | + | 25 | CCTGACCGCGGGCCTGCTGGGCCCG |
| chr22 | 19971283 | 19971307 | + | 25 | CTGACCGCGGGCCTGCTGGGCCCGG |
| chr22 | 19971284 | 19971308 | + | 25 | TGACCGCGGGCCTGCTGGGCCCGGG |
| chr22 | 19971285 | 19971309 | + | 25 | GACCGCGGGCCTGCTGGGCCCGGGG |
| chr22 | 19971286 | 19971310 | + | 25 | ACCGCGGGCCTGCTGGGCCCGGGGG |
| chr22 | 19971287 | 19971311 | + | 25 | CCGCGGGCCTGCTGGGCCCGGGGGG |
| chr22 | 19971288 | 19971312 | + | 25 | CGCGGGCCTGCTGGGCCCGGGGGGA |
| chr22 | 19971289 | 19971313 | + | 25 | GCGGGCCTGCTGGGCCCGGGGGGAG |
| chr22 | 19971290 | 19971314 | + | 25 | CGGGCCTGCTGGGCCCGGGGGGAGG |
| chr22 | 19971291 | 19971315 | + | 25 | GGGCCTGCTGGGCCCGGGGGGAGGG |
| chr22 | 19971292 | 19971316 | + | 25 | GGCCTGCTGGGCCCGGGGGGAGGGG |
| chr22 | 19971293 | 19971317 | + | 25 | GCCTGCTGGGCCCGGGGGGAGGGGC |
| chr22 | 19971294 | 19971318 | + | 25 | CCTGCTGGGCCCGGGGGGAGGGGCC |
| chr22 | 19971295 | 19971319 | + | 25 | CTGCTGGGCCCGGGGGGAGGGGCCT |
| chr22 | 19971296 | 19971320 | + | 25 | TGCTGGGCCCGGGGGGAGGGGCCTT |
| chr22 | 19971297 | 19971321 | + | 25 | GCTGGGCCCGGGGGGAGGGGCCTTC |
| chr22 | 19971298 | 19971322 | + | 25 | CTGGGCCCGGGGGGAGGGGCCTTCC |
| chr22 | 19971299 | 19971323 | + | 25 | TGGGCCCGGGGGGAGGGGCCTTCCT |
| chr22 | 19971300 | 19971324 | + | 25 | GGGCCCGGGGGGAGGGGCCTTCCTG |
| chr22 | 19971301 | 19971325 | + | 25 | GGCCCGGGGGGAGGGGCCTTCCTGC |
| chr22 | 19971302 | 19971326 | + | 25 | GCCCGGGGGGAGGGGCCTTCCTGCT |
| chr22 | 19971303 | 19971327 | + | 25 | CCCGGGGGGAGGGGCCTTCCTGCTG |
| chr22 | 19971304 | 19971328 | + | 25 | CCGGGGGGAGGGGCCTTCCTGCTGG |
| chr22 | 19971305 | 19971329 | + | 25 | CGGGGGGAGGGGCCTTCCTGCTGGG |
| chr22 | 19971306 | 19971330 | + | 25 | GGGGGGAGGGGCCTTCCTGCTGGGG |
| chr22 | 19971307 | 19971331 | + | 25 | GGGGGAGGGGCCTTCCTGCTGGGGT |
| chr22 | 19971308 | 19971332 | + | 25 | GGGGAGGGGCCTTCCTGCTGGGGTC |
| chr22 | 19971309 | 19971333 | + | 25 | GGGAGGGGCCTTCCTGCTGGGGTCG |
| chr22 | 19971310 | 19971334 | + | 25 | GGAGGGGCCTTCCTGCTGGGGTCGA |
| chr22 | 19971311 | 19971335 | + | 25 | GAGGGGCCTTCCTGCTGGGGTCGAG |
| chr22 | 19971312 | 19971336 | + | 25 | AGGGGCCTTCCTGCTGGGGTCGAGC |
| chr22 | 19971313 | 19971337 | + | 25 | GGGGCCTTCCTGCTGGGGTCGAGCT |
| chr22 | 19971314 | 19971338 | + | 25 | GGGCCTTCCTGCTGGGGTCGAGCTG |
| chr22 | 19971315 | 19971339 | + | 25 | GGCCTTCCTGCTGGGGTCGAGCTGC |
| chr22 | 19971316 | 19971340 | + | 25 | GCCTTCCTGCTGGGGTCGAGCTGCA |
| chr22 | 19971317 | 19971341 | + | 25 | CCTTCCTGCTGGGGTCGAGCTGCAG |
| chr22 | 19971318 | 19971342 | + | 25 | CTTCCTGCTGGGGTCGAGCTGCAGC |
| chr22 | 19971319 | 19971343 | + | 25 | TTCCTGCTGGGGTCGAGCTGCAGCG |
| chr22 | 19971320 | 19971344 | + | 25 | TCCTGCTGGGGTCGAGCTGCAGCGC |
| chr22 | 19971321 | 19971345 | + | 25 | CCTGCTGGGGTCGAGCTGCAGCGCA |
| chr22 | 19971322 | 19971346 | + | 25 | CTGCTGGGGTCGAGCTGCAGCGCAC |
| chr22 | 19971323 | 19971347 | + | 25 | TGCTGGGGTCGAGCTGCAGCGCACG |
| chr22 | 19971324 | 19971348 | + | 25 | GCTGGGGTCGAGCTGCAGCGCACGG |
| chr22 | 19971325 | 19971349 | + | 25 | CTGGGGTCGAGCTGCAGCGCACGGG |
| chr22 | 19971326 | 19971350 | + | 25 | TGGGGTCGAGCTGCAGCGCACGGGT |
| chr22 | 19971327 | 19971351 | + | 25 | GGGGTCGAGCTGCAGCGCACGGGTG |
| chr22 | 19971328 | 19971352 | + | 25 | GGGTCGAGCTGCAGCGCACGGGTGG |
| chr22 | 19971329 | 19971353 | + | 25 | GGTCGAGCTGCAGCGCACGGGTGGG |
| chr22 | 19971330 | 19971354 | + | 25 | GTCGAGCTGCAGCGCACGGGTGGGC |
| chr22 | 19971331 | 19971355 | + | 25 | TCGAGCTGCAGCGCACGGGTGGGCA |
| chr22 | 19971332 | 19971356 | + | 25 | CGAGCTGCAGCGCACGGGTGGGCAT |
| chr22 | 19971333 | 19971357 | + | 25 | GAGCTGCAGCGCACGGGTGGGCATT |
| chr22 | 19971334 | 19971358 | + | 25 | AGCTGCAGCGCACGGGTGGGCATTA |
| chr22 | 19971335 | 19971359 | + | 25 | GCTGCAGCGCACGGGTGGGCATTAG |
| chr22 | 19971336 | 19971360 | + | 25 | CTGCAGCGCACGGGTGGGCATTAGA |
| chr22 | 19971337 | 19971361 | + | 25 | TGCAGCGCACGGGTGGGCATTAGAG |
| chr22 | 19971338 | 19971362 | + | 25 | GCAGCGCACGGGTGGGCATTAGAGG |
| chr22 | 19971339 | 19971363 | + | 25 | CAGCGCACGGGTGGGCATTAGAGGC |
| chr22 | 19971340 | 19971364 | + | 25 | AGCGCACGGGTGGGCATTAGAGGCA |
| chr22 | 19971341 | 19971365 | + | 25 | GCGCACGGGTGGGCATTAGAGGCAC |
| chr22 | 19971342 | 19971366 | + | 25 | CGCACGGGTGGGCATTAGAGGCACA |
| chr22 | 19971343 | 19971367 | + | 25 | GCACGGGTGGGCATTAGAGGCACAA |
| chr22 | 19971344 | 19971368 | + | 25 | CACGGGTGGGCATTAGAGGCACAAT |
| chr22 | 19971345 | 19971369 | + | 25 | ACGGGTGGGCATTAGAGGCACAATA |
| chr22 | 19971346 | 19971370 | + | 25 | CGGGTGGGCATTAGAGGCACAATAG |
| chr22 | 19971347 | 19971371 | + | 25 | GGGTGGGCATTAGAGGCACAATAGA |
| chr22 | 19971348 | 19971372 | + | 25 | GGTGGGCATTAGAGGCACAATAGAG |
| chr22 | 19971349 | 19971373 | + | 25 | GTGGGCATTAGAGGCACAATAGAGC |
| chr22 | 19971350 | 19971374 | + | 25 | TGGGCATTAGAGGCACAATAGAGCA |
| chr22 | 19971351 | 19971375 | + | 25 | GGGCATTAGAGGCACAATAGAGCAG |
| chr22 | 19971352 | 19971376 | + | 25 | GGCATTAGAGGCACAATAGAGCAGG |
| chr22 | 19971353 | 19971377 | + | 25 | GCATTAGAGGCACAATAGAGCAGGT |
| chr22 | 19971354 | 19971378 | + | 25 | CATTAGAGGCACAATAGAGCAGGTT |
| chr22 | 19971355 | 19971379 | + | 25 | ATTAGAGGCACAATAGAGCAGGTTA |
| chr22 | 19971356 | 19971380 | + | 25 | TTAGAGGCACAATAGAGCAGGTTAG |
| chr22 | 19971357 | 19971381 | + | 25 | TAGAGGCACAATAGAGCAGGTTAGT |
| chr22 | 19971358 | 19971382 | + | 25 | AGAGGCACAATAGAGCAGGTTAGTT |
| chr22 | 19971359 | 19971383 | + | 25 | GAGGCACAATAGAGCAGGTTAGTTA |
| chr22 | 19971360 | 19971384 | + | 25 | AGGCACAATAGAGCAGGTTAGTTAG |
| chr22 | 19971361 | 19971385 | + | 25 | GGCACAATAGAGCAGGTTAGTTAGA |

**Table 33: 25-mer target-specific ASOs**

| **CHR** | **START** | **END** | **STRAND** | **kmer** | **SEQUENCE** |
|---|---|---|---|---|---|
| chr2 | 135641485 | 135641509 | + | 25 | TTTTTTATATGAGGTTTAAAAAATC |
| chr2 | 135641486 | 135641510 | + | 25 | TTTTTATATGAGGTTTAAAAAATCC |
| chr2 | 135641487 | 135641511 | + | 25 | TTTTATATGAGGTTTAAAAAATCCA |
| chr2 | 135641488 | 135641512 | + | 25 | TTTATATGAGGTTTAAAAAATCCAT |
| chr2 | 135641489 | 135641513 | + | 25 | TTATATGAGGTTTAAAAAATCCATA |
| chr2 | 135641490 | 135641514 | + | 25 | TATATGAGGTTTAAAAAATCCATAT |
| chr2 | 135641491 | 135641515 | + | 25 | ATATGAGGTTTAAAAAATCCATATT |
| chr2 | 135641492 | 135641516 | + | 25 | TATGAGGTTTAAAAAATCCATATTT |
| chr2 | 135641493 | 135641517 | + | 25 | ATGAGGTTTAAAAAATCCATATTTT |
| chr2 | 135641494 | 135641518 | + | 25 | TGAGGTTTAAAAAATCCATATTTTT |
| chr2 | 135641495 | 135641519 | + | 25 | GAGGTTTAAAAAATCCATATTTTTC |
| chr2 | 135641496 | 135641520 | + | 25 | AGGTTTAAAAAATCCATATTTTTCA |
| chr2 | 135641497 | 135641521 | + | 25 | GGTTTAAAAAATCCATATTTTTCAT |
| chr2 | 135641498 | 135641522 | + | 25 | GTTTAAAAAATCCATATTTTTCATT |
| chr2 | 135641499 | 135641523 | + | 25 | TTTAAAAAATCCATATTTTTCATTA |
| chr2 | 135641500 | 135641524 | + | 25 | TTAAAAAATCCATATTTTTCATTAC |
| chr2 | 135641501 | 135641525 | + | 25 | TAAAAAATCCATATTTTTCATTACT |
| chr2 | 135641502 | 135641526 | + | 25 | AAAAAATCCATATTTTTCATTACTC |
| chr2 | 135641503 | 135641527 | + | 25 | AAAAATCCATATTTTTCATTACTCC |
| chr2 | 135641504 | 135641528 | + | 25 | AAAATCCATATTTTTCATTACTCCT |
| chr2 | 135641505 | 135641529 | + | 25 | AAATCCATATTTTTCATTACTCCTC |
| chr2 | 135641506 | 135641530 | + | 25 | AATCCATATTTTTCATTACTCCTCT |
| chr2 | 135641507 | 135641531 | + | 25 | ATCCATATTTTTCATTACTCCTCTT |
| chr2 | 135641508 | 135641532 | + | 25 | TCCATATTTTTCATTACTCCTCTTC |
| chr2 | 135641509 | 135641533 | + | 25 | CCATATTTTTCATTACTCCTCTTCT |
| chr2 | 135641510 | 135641534 | + | 25 | CATATTTTTCATTACTCCTCTTCTA |
| chr2 | 135641511 | 135641535 | + | 25 | ATATTTTTCATTACTCCTCTTCTAG |
| chr2 | 135641512 | 135641536 | + | 25 | TATTTTTCATTACTCCTCTTCTAGG |
| chr2 | 135641513 | 135641537 | + | 25 | ATTTTTCATTACTCCTCTTCTAGGT |
| chr2 | 135641514 | 135641538 | + | 25 | TTTTTCATTACTCCTCTTCTAGGTT |
| chr2 | 135641515 | 135641539 | + | 25 | TTTTCATTACTCCTCTTCTAGGTTC |
| chr2 | 135641516 | 135641540 | + | 25 | TTTCATTACTCCTCTTCTAGGTTCT |
| chr2 | 135641517 | 135641541 | + | 25 | TTCATTACTCCTCTTCTAGGTTCTG |
| chr2 | 135641518 | 135641542 | + | 25 | TCATTACTCCTCTTCTAGGTTCTGA |
| chr2 | 135641519 | 135641543 | + | 25 | CATTACTCCTCTTCTAGGTTCTGAG |
| chr2 | 135641520 | 135641544 | + | 25 | ATTACTCCTCTTCTAGGTTCTGAGT |
| chr2 | 135641521 | 135641545 | + | 25 | TTACTCCTCTTCTAGGTTCTGAGTC |
| chr2 | 135641522 | 135641546 | + | 25 | TACTCCTCTTCTAGGTTCTGAGTCT |
| chr2 | 135641523 | 135641547 | + | 25 | ACTCCTCTTCTAGGTTCTGAGTCTT |
| chr2 | 135641524 | 135641548 | + | 25 | CTCCTCTTCTAGGTTCTGAGTCTTC |
| chr2 | 135641525 | 135641549 | + | 25 | TCCTCTTCTAGGTTCTGAGTCTTCT |
| chr2 | 135641526 | 135641550 | + | 25 | CCTCTTCTAGGTTCTGAGTCTTCTG |
| chr2 | 135641527 | 135641551 | + | 25 | CTCTTCTAGGTTCTGAGTCTTCTGG |
| chr2 | 135641528 | 135641552 | + | 25 | TCTTCTAGGTTCTGAGTCTTCTGGT |
| chr2 | 135641529 | 135641553 | + | 25 | CTTCTAGGTTCTGAGTCTTCTGGTA |
| chr2 | 135641530 | 135641554 | + | 25 | TTCTAGGTTCTGAGTCTTCTGGTAG |
| chr2 | 135641531 | 135641555 | + | 25 | TCTAGGTTCTGAGTCTTCTGGTAGT |
| chr2 | 135641532 | 135641556 | + | 25 | CTAGGTTCTGAGTCTTCTGGTAGTG |
| chr2 | 135641533 | 135641557 | + | 25 | TAGGTTCTGAGTCTTCTGGTAGTGT |
| chr2 | 135641534 | 135641558 | + | 25 | AGGTTCTGAGTCTTCTGGTAGTGTA |
| chr2 | 135641535 | 135641559 | + | 25 | GGTTCTGAGTCTTCTGGTAGTGTAG |
| chr2 | 135641536 | 135641560 | + | 25 | GTTCTGAGTCTTCTGGTAGTGTAGG |
| chr2 | 135641537 | 135641561 | + | 25 | TTCTGAGTCTTCTGGTAGTGTAGGG |
| chr2 | 135641538 | 135641562 | + | 25 | TCTGAGTCTTCTGGTAGTGTAGGGT |
| chr2 | 135641539 | 135641563 | + | 25 | CTGAGTCTTCTGGTAGTGTAGGGTC |
| chr2 | 135641540 | 135641564 | + | 25 | TGAGTCTTCTGGTAGTGTAGGGTCA |
| chr2 | 135641541 | 135641565 | + | 25 | GAGTCTTCTGGTAGTGTAGGGTCAT |
| chr2 | 135641542 | 135641566 | + | 25 | AGTCTTCTGGTAGTGTAGGGTCATC |
| chr2 | 135641543 | 135641567 | + | 25 | GTCTTCTGGTAGTGTAGGGTCATCT |
| chr2 | 135641544 | 135641568 | + | 25 | TCTTCTGGTAGTGTAGGGTCATCTA |
| chr2 | 135641545 | 135641569 | + | 25 | CTTCTGGTAGTGTAGGGTCATCTAC |
| chr2 | 135641546 | 135641570 | + | 25 | TTCTGGTAGTGTAGGGTCATCTACA |
| chr2 | 135641547 | 135641571 | + | 25 | TCTGGTAGTGTAGGGTCATCTACAG |
| chr2 | 135641548 | 135641572 | + | 25 | CTGGTAGTGTAGGGTCATCTACAGG |
| chr2 | 135641549 | 135641573 | + | 25 | TGGTAGTGTAGGGTCATCTACAGGC |
| chr2 | 135641550 | 135641574 | + | 25 | GGTAGTGTAGGGTCATCTACAGGCT |
| chr2 | 135641551 | 135641575 | + | 25 | GTAGTGTAGGGTCATCTACAGGCTC |
| chr2 | 135641552 | 135641576 | + | 25 | TAGTGTAGGGTCATCTACAGGCTCT |
| chr2 | 135641553 | 135641577 | + | 25 | AGTGTAGGGTCATCTACAGGCTCTC |
| chr2 | 135641554 | 135641578 | + | 25 | GTGTAGGGTCATCTACAGGCTCTCT |
| chr2 | 135641555 | 135641579 | + | 25 | TGTAGGGTCATCTACAGGCTCTCTT |
| chr2 | 135641556 | 135641580 | + | 25 | GTAGGGTCATCTACAGGCTCTCTTT |
| chr2 | 135641557 | 135641581 | + | 25 | TAGGGTCATCTACAGGCTCTCTTTC |
| chr2 | 135641558 | 135641582 | + | 25 | AGGGTCATCTACAGGCTCTCTTTCT |
| chr2 | 135641559 | 135641583 | + | 25 | GGGTCATCTACAGGCTCTCTTTCTC |
| chr2 | 135641560 | 135641584 | + | 25 | GGTCATCTACAGGCTCTCTTTCTCA |
| chr2 | 135641561 | 135641585 | + | 25 | GTCATCTACAGGCTCTCTTTCTCAC |
| chr2 | 135641562 | 135641586 | + | 25 | TCATCTACAGGCTCTCTTTCTCACA |
| chr2 | 135641563 | 135641587 | + | 25 | CATCTACAGGCTCTCTTTCTCACAT |
| chr2 | 135641564 | 135641588 | + | 25 | ATCTACAGGCTCTCTTTCTCACATC |
| chr2 | 135641565 | 135641589 | + | 25 | TCTACAGGCTCTCTTTCTCACATCC |
| chr2 | 135641566 | 135641590 | + | 25 | CTACAGGCTCTCTTTCTCACATCCA |
| chr2 | 135641567 | 135641591 | + | 25 | TACAGGCTCTCTTTCTCACATCCAG |
| chr2 | 135641568 | 135641592 | + | 25 | ACAGGCTCTCTTTCTCACATCCAGC |
| chr2 | 135641569 | 135641593 | + | 25 | CAGGCTCTCTTTCTCACATCCAGCA |
| chr2 | 135641570 | 135641594 | + | 25 | AGGCTCTCTTTCTCACATCCAGCAG |
| chr2 | 135641571 | 135641595 | + | 25 | GGCTCTCTTTCTCACATCCAGCAGC |
| chr2 | 135641572 | 135641596 | + | 25 | GCTCTCTTTCTCACATCCAGCAGCC |
| chr2 | 135641573 | 135641597 | + | 25 | CTCTCTTTCTCACATCCAGCAGCCT |
| chr2 | 135641574 | 135641598 | + | 25 | TCTCTTTCTCACATCCAGCAGCCTC |
| chr2 | 135641575 | 135641599 | + | 25 | CTCTTTCTCACATCCAGCAGCCTCT |
| chr2 | 135641576 | 135641600 | + | 25 | TCTTTCTCACATCCAGCAGCCTCTT |
| chr2 | 135641577 | 135641601 | + | 25 | CTTTCTCACATCCAGCAGCCTCTTC |
| chr2 | 135641578 | 135641602 | + | 25 | TTTCTCACATCCAGCAGCCTCTTCC |
| chr2 | 135641579 | 135641603 | + | 25 | TTCTCACATCCAGCAGCCTCTTCCA |
| chr2 | 135641580 | 135641604 | + | 25 | TCTCACATCCAGCAGCCTCTTCCAG |
| chr2 | 135641581 | 135641605 | + | 25 | CTCACATCCAGCAGCCTCTTCCAGG |
| chr2 | 135641582 | 135641606 | + | 25 | TCACATCCAGCAGCCTCTTCCAGGT |
| chr2 | 135641583 | 135641607 | + | 25 | CACATCCAGCAGCCTCTTCCAGGTA |
| chr2 | 135641584 | 135641608 | + | 25 | ACATCCAGCAGCCTCTTCCAGGTAC |
| chr2 | 135641585 | 135641609 | + | 25 | CATCCAGCAGCCTCTTCCAGGTACA |
| chr2 | 135641586 | 135641610 | + | 25 | ATCCAGCAGCCTCTTCCAGGTACAG |
| chr2 | 135641587 | 135641611 | + | 25 | TCCAGCAGCCTCTTCCAGGTACAGC |
| chr2 | 135641588 | 135641612 | + | 25 | CCAGCAGCCTCTTCCAGGTACAGCT |
| chr2 | 135641589 | 135641613 | + | 25 | CAGCAGCCTCTTCCAGGTACAGCTC |
| chr2 | 135641590 | 135641614 | + | 25 | AGCAGCCTCTTCCAGGTACAGCTCT |
| chr2 | 135641591 | 135641615 | + | 25 | GCAGCCTCTTCCAGGTACAGCTCTC |
| chr2 | 135641592 | 135641616 | + | 25 | CAGCCTCTTCCAGGTACAGCTCTCA |
| chr2 | 135641593 | 135641617 | + | 25 | AGCCTCTTCCAGGTACAGCTCTCAG |
| chr2 | 135641594 | 135641618 | + | 25 | GCCTCTTCCAGGTACAGCTCTCAGC |
| chr2 | 135641595 | 135641619 | + | 25 | CCTCTTCCAGGTACAGCTCTCAGCC |
| chr2 | 135641596 | 135641620 | + | 25 | CTCTTCCAGGTACAGCTCTCAGCCA |
| chr2 | 135641597 | 135641621 | + | 25 | TCTTCCAGGTACAGCTCTCAGCCAG |
| chr2 | 135641598 | 135641622 | + | 25 | CTTCCAGGTACAGCTCTCAGCCAGT |
| chr2 | 135641599 | 135641623 | + | 25 | TTCCAGGTACAGCTCTCAGCCAGTC |
| chr2 | 135641600 | 135641624 | + | 25 | TCCAGGTACAGCTCTCAGCCAGTCT |
| chr2 | 135641601 | 135641625 | + | 25 | CCAGGTACAGCTCTCAGCCAGTCTT |
| chr2 | 135641602 | 135641626 | + | 25 | CAGGTACAGCTCTCAGCCAGTCTTC |
| chr2 | 135641603 | 135641627 | + | 25 | AGGTACAGCTCTCAGCCAGTCTTCT |
| chr2 | 135641604 | 135641628 | + | 25 | GGTACAGCTCTCAGCCAGTCTTCTC |
| chr2 | 135641605 | 135641629 | + | 25 | GTACAGCTCTCAGCCAGTCTTCTCA |
| chr2 | 135641606 | 135641630 | + | 25 | TACAGCTCTCAGCCAGTCTTCTCAT |
| chr2 | 135641607 | 135641631 | + | 25 | ACAGCTCTCAGCCAGTCTTCTCATG |
| chr2 | 135641608 | 135641632 | + | 25 | CAGCTCTCAGCCAGTCTTCTCATGG |
| chr2 | 135641609 | 135641633 | + | 25 | AGCTCTCAGCCAGTCTTCTCATGGC |
| chr2 | 135641610 | 135641634 | + | 25 | GCTCTCAGCCAGTCTTCTCATGGCG |
| chr2 | 135641611 | 135641635 | + | 25 | CTCTCAGCCAGTCTTCTCATGGCGC |
| chr2 | 135641612 | 135641636 | + | 25 | TCTCAGCCAGTCTTCTCATGGCGCA |
| chr2 | 135641613 | 135641637 | + | 25 | CTCAGCCAGTCTTCTCATGGCGCAC |
| chr2 | 135641614 | 135641638 | + | 25 | TCAGCCAGTCTTCTCATGGCGCACC |
| chr2 | 135641615 | 135641639 | + | 25 | CAGCCAGTCTTCTCATGGCGCACCT |
| chr2 | 135641616 | 135641640 | + | 25 | AGCCAGTCTTCTCATGGCGCACCTG |
| chr2 | 135641617 | 135641641 | + | 25 | GCCAGTCTTCTCATGGCGCACCTGT |
| chr2 | 135641618 | 135641642 | + | 25 | CCAGTCTTCTCATGGCGCACCTGTC |
| chr2 | 135641619 | 135641643 | + | 25 | CAGTCTTCTCATGGCGCACCTGTCG |
| chr2 | 135641620 | 135641644 | + | 25 | AGTCTTCTCATGGCGCACCTGTCGT |
| chr2 | 135641621 | 135641645 | + | 25 | GTCTTCTCATGGCGCACCTGTCGTC |
| chr2 | 135641622 | 135641646 | + | 25 | TCTTCTCATGGCGCACCTGTCGTCT |
| chr2 | 135641623 | 135641647 | + | 25 | CTTCTCATGGCGCACCTGTCGTCTA |
| chr2 | 135641624 | 135641648 | + | 25 | TTCTCATGGCGCACCTGTCGTCTAT |
| chr2 | 135641625 | 135641649 | + | 25 | TCTCATGGCGCACCTGTCGTCTATC |
| chr2 | 135641626 | 135641650 | + | 25 | CTCATGGCGCACCTGTCGTCTATCC |
| chr2 | 135641627 | 135641651 | + | 25 | TCATGGCGCACCTGTCGTCTATCCA |
| chr2 | 135641628 | 135641652 | + | 25 | CATGGCGCACCTGTCGTCTATCCAA |
| chr2 | 135641629 | 135641653 | + | 25 | ATGGCGCACCTGTCGTCTATCCAAC |
| chr2 | 135641630 | 135641654 | + | 25 | TGGCGCACCTGTCGTCTATCCAACT |

While preferred aspects of the present disclosure have been shown and described herein, it will be obvious to those skilled in the art that such aspects are provided by way of example only. It is not intended that the disclosure be limited by the specific examples provided within the specification. While the disclosure has been described with reference to the aforementioned specification, the descriptions and illustrations of the aspects herein are not meant to be construed in a limiting sense. Numerous variations, changes, and substitutions will now occur to those skilled in the art. Furthermore, it shall be understood that aspects of the disclosure are not limited to the specific depictions, configurations or relative proportions set forth herein which depend upon a variety of conditions and variables. It should be understood that various alternatives to the aspects of the disclosure described herein may be employed. The invention is defined by the appended claims.

## Claims

1. An antisense compound comprising an oligonucleotide having a sequence selected from the group consisting of:
5'-GTGGGTTTCAGGGATTCTGA-3' (SEQ ID NO: 1),
5'-CCCTGATTCAGACAGCAGGG-3' (SEQ ID NO: 2),
5'-GCTGGCTTTGTCTGGATAGG-3' (SEQ ID NO: 3),
5'-TCTCACGTCACCTGCCTTAC-3' (SEQ ID NO: 4), and
5'-AGCGCTGCCACAGCAGTGGG-3' (SEQ ID NO: 5);
or a complement thereof.

2. The antisense compound of claim 1, wherein the oligonucleotide comprises 20-50 nucleotides; optionally, wherein the oligonucleotide comprises 25-30 nucleotides.

3. The antisense compound of any one of claims 1 or 2, wherein the oligonucleotide comprises deoxyribonucleic acid (DNA), ribonucleic acid (RNA), PNA, or a combination or hybrid thereof.

4. The antisense compound of any one of claims 1-3, wherein the oligonucleotide comprises one or more modified oligonucleotides.

5. The antisense compound of any one of claims 1-4, wherein the oligonucleotide comprises at least one modified internucleotide linkage, optionally wherein all internucleotide linkages are modified.

6. The antisense compound of claim 5, wherein the modified internucleotide linkage comprises a phosphorothioate linkage.

7. The antisense compound of any one of claims 1-6, wherein the oligonucleotide comprises one or more modified nucleosides selected from the group consisting of 2'-O-methoxyethyl nucleoside, 2'-fluoro nucleoside, 2'-dimethylaminooxyethoxy nucleoside, 2'-dimethylaminoethoxyethoxy nucleoside, 2'-guanidinium nucleoside, 2'-O-guanidinium ethyl nucleoside, 2'-carbamate nucleoside, 2'aminooxy nucleoside, 2'-acetamido nucleoside, and locked nucleic acid.

8. The antisense compound of any one of claims 1-7, wherein the oligonucleotide is capable of modulating splicing of NEDD4L mRNA in a cell.

9. The antisense compound of claim 8, wherein the modulation of splicing comprises promoting a splicing switch.

10. A pharmaceutically acceptable composition comprising the antisense compound according to any one of claims 1-9, and a pharmaceutically acceptable diluent or carrier.

11. The antisense compound of any one of claims 1-9 or the pharmaceutical composition of claim 10 for use in the treatment of cancer.

12. The antisense compound or the pharmaceutical composition for use of claim 11, wherein the cancer comprises lung cancer, kidney cancer, or breast cancer.

13. The antisense compound or the pharmaceutical composition for use of claim 12, wherein the breast cancer is triple-negative breast cancer.

14. The antisense compound or the pharmaceutical composition for use of claim 11, wherein the cancer shows a disease-specific splicing event of the pre-mRNA encoded by the NEDD4L gene resulting in a short pre-mRNA isoform, and the antisense compound binds to a segment of the pre-mRNA and modulates splicing of the pre-mRNA from the skipping isoform to a inclusion isoform, thereby treating the cancer; optionally, wherein the antisense compound promotes inclusion of an excluded middle exon of an identified exon trio in the pre-mRNA.

15. An *ex vivo* or *in vitro* method of modulating splicing of a NEDD4L pre-mRNA in a cell, the method comprising: contacting the cell with an antisense compound having a sequence selected from the group consisting of:
5'-GTGGGTTTCAGGGATTCTGA-3' (SEQ ID NO: 1),
5'-CCCTGATTCAGACAGCAGGG-3' (SEQ ID NO: 2),
5'-GCTGGCTTTGTCTGGATAGG-3' (SEQ ID NO: 3),
5'-TCTCACGTCACCTGCCTTAC-3' (SEQ ID NO: 4), and
5'-AGCGCTGCCACAGCAGTGGG-3' (SEQ ID NO: 5)
or a complement thereof;
thereby modulating splicing in the NEDD4L pre-mRNA of the cell.

16. An antisense compound for use in a method of therapy, wherein the antisense compound comprises a nucleic acid sequence selected from the group consisting of:
5'-GTGGGTTTCAGGGATTCTGA-3' (SEQ ID NO: 1),
5'-CCCTGATTCAGACAGCAGGG-3' (SEQ ID NO: 2),
5'-GCTGGCTTTGTCTGGATAGG-3' (SEQ ID NO: 3),
5'-TCTCACGTCACCTGCCTTAC-3' (SEQ ID NO: 4), and
5'-AGCGCTGCCACAGCAGTGGG-3' (SEQ ID NO: 5);
or a complement thereof.

## Patentansprüche

1. Antisense-Verbindung, umfassend ein Oligonukleotid, das eine Sequenz aufweist, die aus der aus Folgenden bestehenden Gruppe ausgewählt ist:
5'-GTGGGTTTCAGGGATTCTGA-3' (SEQ ID NO: 1),
5'-CCCTGATTCAGACAGCAGGG-3' (SEQ ID NO: 2),
5'-GCTGGCTTTGTCTGGATAGG-3' (SEQ ID NO: 3),
5'-TCTCACGTCACCTGCCTTAC-3' (SEQ ID NO: 4) und
5'-AGCGCTGCCACAGCAGTGGG-3' (SEQ ID NO: 5);
oder einem Komplement davon.

2. Antisense-Verbindung nach Anspruch 1, wobei das Oligonukleotid 20 bis 50 Nukleotide umfasst; wobei das Oligonukleotid gegebenenfalls 25 bis 30 Nukleotide umfasst.

3. Antisense-Verbindung nach einem der Ansprüche 1 oder 2, wobei das Oligonukleotid Desoxyribonukleinsäure (DNA), Ribonukleinsäure (RNA), PNA oder eine Kombination oder ein Hybrid davon umfasst.

4. Antisense-Verbindung nach einem der Ansprüche 1 bis 3, wobei das Oligonukleotid ein oder mehrere modifizierte Oligonukleotide umfasst.

5. Antisense-Verbindung nach einem der Ansprüche 1 bis 4, wobei das Oligonukleotid zumindest eine modifizierte Internukleotidbindung umfasst, wobei alle Internukleotidbindungen gegebenenfalls modifiziert sind.

6. Antisense-Verbindung nach Anspruch 5, wobei die modifizierte Internukleotidbindung eine Thiophosphatbindung umfasst.

7. Antisense-Verbindung nach einem der Ansprüche 1 bis 6, wobei das Oligonukleotid ein oder mehrere modifizierte Nukleoside umfasst, die aus der aus 2'-O-Methoxyethylnukleosid, 2'-Fluornukleosid, 2'- Dimethylaminooxyethoxynukleosid, 2'-Dimethylaminoethoxyethoxynukleosid, 2'-Guanidiniumnukleosid, 2'-O-Guanidiniumethylnukleosid, 2'-Carbamatnukleosid, 2'-Aminooxynukleosid, 2'-Acetamidonukleosid und einer verbrückten Nukleinsäure bestehenden Gruppe ausgewählt sind.

8. Antisense-Verbindung nach einem der Ansprüche 1 bis 7, wobei das Oligonukleotid dazu in der Lage ist, das Splicing von NEDD4L-mRNA in einer Zelle zu modulieren.

9. Antisense-Verbindung nach Anspruch 8, wobei die Modulation des Splicings das Fördern eines Splicing-Schalters umfasst.

10. Pharmazeutisch annehmbare Zusammensetzung, umfassend eine Antisense-Verbindung nach einem der Ansprüche 1 bis 9 und einen pharmazeutisch annehmbaren Verdünner oder Träger.

11. Antisense-Verbindung nach einem der Ansprüche 1 bis 9 oder pharmazeutische Zusammensetzung nach Anspruch 10 zur Verwendung bei der Behandlung von Krebs.

12. Antisense-Verbindung oder pharmazeutische Zusammensetzung zur Verwendung nach Anspruch 11, wobei der Krebs Lungenkrebs, Nierenkrebs oder Brustkrebs umfasst.

13. Antisense-Verbindung oder pharmazeutische Zusammensetzung zur Verwendung nach Anspruch 12, wobei der Brustkrebs dreifach negativer Brustkrebs ist.

14. Antisense-Verbindung oder pharmazeutische Zusammensetzung zur Verwendung nach Anspruch 11, wobei der Krebs ein krankheitsspezifisches Splicingereignis der Prä-mRNA aufweist, die durch das NEDD4L-Gen kodiert wird, was zu einer kurzen Prä-mRNA-Isoform führt, und wobei die Antisense-Verbindung an ein Segment der Prä-mRNA bindet und das Splicing der Prä-mRNA von der überspringenden Isoform in eine einschließende Isoform moduliert, wodurch der Krebs behandelt wird, wobei die Antisense-Verbindung gegebenenfalls den Einschluss eines ausgeschlossenen mittleren Exons eines identifizierten Exon-Trios in die Prä-mRNA fördert.

15. Ex-vivo- oder In-vitro-Verfahren zum Modulieren des Splicings einer NEDD4L-Prä-mRNA in einer Zelle, wobei das Verfahren Folgendes umfasst: Kontaktieren der Zelle mit einer Antisense-Verbindung, die eine Sequenz aufweist, die aus der aus Folgenden bestehenden Gruppe ausgewählt ist:
5'-GTGGGTTTCAGGGATTCTGA-3' (SEQ ID NO: 1),
5'-CCCTGATTCAGACAGCAGGG-3' (SEQ ID NO: 2),
5'-GCTGGCTTTGTCTGGATAGG-3' (SEQ ID NO: 3),
5'-TCTCACGTCACCTGCCTTAC-3' (SEQ ID NO: 4) und
5'-AGCGCTGCCACAGCAGTGGG-3' (SEQ ID NO: 5);
oder einem Komplement davon;
wodurch das Splicing in der NEDD4L-Prä-mRNA der Zelle moduliert wird.

16. Antisense-Verbindung zur Verwendung in einem Verfahren der Therapie, wobei die Antisense-Verbindung eine Nukleinsäuresequenz umfasst, die aus der aus Folgenden bestehenden Gruppe ausgewählt ist:
5'-GTGGGTTTCAGGGATTCTGA-3' (SEQ ID NO: 1),
5'-CCCTGATTCAGACAGCAGGG-3' (SEQ ID NO: 2),
5'-GCTGGCTTTGTCTGGATAGG-3' (SEQ ID NO: 3),
5'-TCTCACGTCACCTGCCTTAC-3' (SEQ ID NO: 4) und
5'-AGCGCTGCCACAGCAGTGGG-3' (SEQ ID NO: 5);
oder einem Komplement davon.

## Revendications

1. Composé antisens comprenant un oligonucléotide présentant une séquence choisie dans le groupe constitué de :
5'-GTGGGTTTCAGGGATTCTGA-3' (SEQ ID NO: 1),
5'-CCCTGATTCAGACAGCAGGG-3' (SEQ ID NO: 2),
5'-GCTGGCTTTGTCTGGATAGG-3' (SEQ ID NO: 3),
5'-TCTCACGTCACCTGCCTTAC-3' (SEQ ID NO: 4), et
5'-AGCGCTGCCACAGCAGTGGG-3' (SEQ ID NO: 5) ;
ou un complément de celui-ci

2. Composé antisens selon la revendication 1, dans lequel l'oligonucléotide comprend 20 à 50 nucléotides ; facultativement dans lequel l'oligonucléotide comprend 25 à 30 nucléotides.

3. Composé antisens selon l'une quelconque des revendications 1 ou 2, dans lequel l'oligonucléotide comprend de l'acide désoxyribonucléique (ADN), de l'acide ribonucléique (ARN), du PNA, ou une combinaison ou un hybride de ceux-ci.

4. Composé antisens selon l'une quelconque des revendications 1 à 3, dans lequel l'oligonucléotide comprend un ou plusieurs oligonucléotides modifiés.

5. Composé antisens selon l'une quelconque des revendications 1 à 4, dans lequel l'oligonucléotide comprend au moins une liaison internucléotidique modifiée, facultativement dans lequel toutes les liaisons internucléotidiques sont modifiées.

6. Composé antisens selon la revendication 5, dans lequel la liaison internucléotidique modifiée comprend une liaison phosphorothioate.

7. Composé antisens selon l'une quelconque des revendications 1 à 6, dans lequel l'oligonucléotide comprend un ou plusieurs nucléosides modifiés choisis dans le groupe constitué de 2'-O-méthoxyéthyl nucléoside, 2'-fluoro nucléoside, 2'-diméthylaminooxyéthoxy nucléoside, 2'-diméthylaminoéthoxyéthoxy nucléoside, 2'-guanidinium nucléoside, 2'-O-guanidinium éthyl nucléoside, 2'-carbamate nucléoside, 2'aminooxy nucléoside, 2'-acétamido nucléoside et acide nucléique verrouillé.

8. Composé antisens selon l'une quelconque des revendications 1 à 7, dans lequel l'oligonucléotide est capable de moduler l'épissage de l'ARNm NEDD4L dans une cellule.

9. Composé antisens selon la revendication 8, dans lequel la modulation de l'épissage comprend la promotion d'un commutateur d'épissage.

10. Composition pharmaceutiquement acceptable comprenant le composé antisens selon l'une quelconque des revendications 1 à 9, et un diluant ou support pharmaceutiquement acceptable.

11. Composé antisens selon l'une quelconque des revendications 1 à 9 ou composition pharmaceutique selon la revendication 10 pour utilisation dans le traitement du cancer.

12. Composé antisens ou composition pharmaceutique pour utilisation selon la revendication 11, dans lequel le cancer comprend le cancer du poumon, le cancer du rein ou le cancer du sein.

13. Composé antisens ou composition pharmaceutique pour utilisation selon la revendication 12, dans lequel le cancer du sein est un cancer du sein triple négatif.

14. Composé antisens ou composition pharmaceutique pour utilisation selon la revendication 11, dans lequel le cancer présente un événement d'épissage spécifique à une maladie du pré-ARNm codé par le gène NEDD4L résultant en une isoforme de pré-ARNm courte, et le composé antisens se lie à un segment du pré-ARNm et module l'épissage du pré-ARNm de l'isoforme de saut à une isoforme d'inclusion, en traitant ainsi le cancer ; facultativement dans lequel le composé antisens favorise l'inclusion d'un exon moyen exclu d'un trio d'exons identifié dans le pré-ARNm.

15. Procédé *ex vivo* ou *in vitro* de modulation de l'épissage d'un pré-ARNm NEDD4L dans une cellule, le procédé comprenant : une mise en contact de la cellule avec un composé antisens présentant une séquence choisie dans le groupe constitué de :
5'-GTGGGTTTCAGGGATTCTGA-3' (SEQ ID NO: 1),
5'-CCCTGATTCAGACAGCAGGG-3' (SEQ ID NO: 2),
5'-GCTGGCTTTGTCTGGATAGG-3' (SEQ ID NO: 3),
5'-TCTCACGTCACCTGCCTTAC-3' (SEQ ID NO: 4), et
5'-AGCGCTGCCACAGCAGTGGG-3' (SEQ ID NO: 5)
ou un complément de celui-ci ;
en modulant ainsi l'épissage dans le pré-ARNm NEDD4L de la cellule.

16. Composé antisens pour utilisation dans un procédé de thérapie, dans lequel le composé antisens comprend une séquence d'acides nucléiques choisie dans le groupe comprenant :
5'-GTGGGTTTCAGGGATTCTGA-3' (SEQ ID NO: 1),
5'-CCCTGATTCAGACAGCAGGG-3' (SEQ ID NO: 2),
5'-GCTGGCTTTGTCTGGATAGG-3' (SEQ ID NO: 3),
5'-TCTCACGTCACCTGCCTTAC-3' (SEQ ID NO: 4), et
5'-AGCGCTGCCACAGCAGTGGG-3' (SEQ ID NO: 5) ;
ou un complément de celui-ci.
